# EUROPEAN PATENT SPECIFICATION

(11) **EP 0 723 545 B1**
(45) Date of publication and mention of the grant of the patent: **08.05.2002**
(21) Application number: 94929998.6
(22) Date of filing: 30.09.1994
(51) Int. Cl.: C07D 455/02, C07D 213/68, C07D 213/61, C07D 471/04, C07D 491/16, A61K 31/535, A61K 31/495, A61K 31/435

(54) **QUINOLIZINONE TYPE COMPOUNDS**
VERBINDUNGEN DES CHINOLIZINON-TYPS
COMPOSES DU TYPE DE LA QUINOLIZINONE

(30) Priority: 14.10.1993 US 137236
(43) Date of publication of application: 31.07.1996
(73) Proprietor: ABBOTT LABORATORIES, Abbott Park, Illinois 60064-3500 (US)
(72) Inventor: CHU, Daniel T., Santa Clara, CA 95051 (US); LI, Qun, Gurnee, IL 60031 (US); COOPER, Curt S., Gurnee, IL 60031 (US); FUNG, Anthony K. L., Gurnee, IL 60031 (US); LEE, Cheuk M., Libertyville, IL (US); PLATTNER, Jacob J., Libertyville, IL 60048 (US)
(74) Representative: Modiano, Guido, Dr.-Ing.
(86) International application number: US9411166
(87) International publication number: WO9510519

(56) References cited:
- EP-A- 0 187 376
- EP-A- 0 308 019
- WO-A-91/16894

## Description

### TECHNICAL FIELD

The present invention relates to compounds having antimicrobial activity, pharmaceutical compositions containing such compounds, and processes for their chemical synthesis. More particularly, this invention relates to novel 4-oxo-4H-quinolizine-3-carboxylic acid compounds which are highly effective in the treatment of microbial and especially bacterial infections, as well as compositions containing the same and the use of such compounds for manufacturing a medicament for treating such infections.

### BACKGROUND OF THE INVENTION

There is a continuing need for new antibacterial agents. Although many compounds are known which are useful in the treatment of Gram-positive and Gram-negative bacterial infections as well as other microbial infections, the widespread use of such compounds continues to give rise to resistant strains of microorganisms, *i.e.,* strains of microorganisms against which a particular antibiotic or group of antibiotics, which was previously effective, is no longer useful. Also, known antibiotics may be effective against only certain strains of microorganisms or have limited activity against either Gram-positive or Gram-negative, aerobic or anaerobic organisms.

The therapeutic use of certain quinolizinone derivatives has been described previously. For example, Y. Kitaura *et al*., in U.S. Patent No. 4,650,804, issued March 17, 1987, have disclosed quinolizinone compounds having a tetrazolylcarbamoyl substituent which are useful for the treatment of allergic and ulcer diseases. J.V. Heck and E.D. Thorsett, in European Patent Application No. 0308019, published March 22, 1989, have disclosed the use of certain 4-oxo-4H-quinolizine-3-carboxylic acids and derivatives thereof for treating bacterial infections. In the International Application No. PCT/US91/02998, published November 14, 1991, (Publication No. WO91/16894) a wide variety of 8-heterosubstituted 4-oxo-4H-quinolizine-3-carboxylic acid compounds are disclosed as antibacterial agents. However, there remains an ongoing need for novel compounds which have improved antimicrobial potency and/or different spectra of activity, and in particular antibacterial agents which are effective against the growing number of bacterial pathogens which are resistant to frequently-used therapeutic agents.

### SUMMARY OF THE INVENTION

In one aspect of the present invention are disclosed compounds represented by the following structural formula (I): 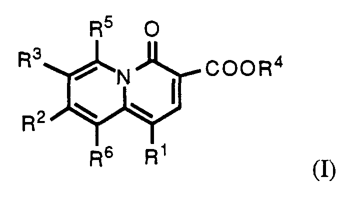 as well as the pharmaceutically acceptable salts, esters and amides thereof.
**R**^{**1**} in formula (I) is selected from (a) loweralkyl, (b) loweralkenyl, (c) halo(lower-alkyl), (d) loweralkoxy, (e) cycloalkyl of from three to eight carbon atoms, (f) phenyl, (g) substituted phenyl, (h) halo, (i) cyano, (j) nitro, (k) bicycloalkyl, (1) loweralkynyl, (m) loweralkoxycarbonyl, (n) nitrogen-containing aromatic heterocycle, (o) halo-substituted nitrogen-containing aromatic heterocycle, (p) a 4-, 5- or 6-membered cyclic ether, and (q) -NR⁷R⁸. The radicals **R**^{**7**} and **R**^{**8**} are independently selected from hydrogen, loweralkyl and alkanoyl of from one to eight carbon atoms or, taken together with the nitrogen atom to which they are attached, R⁷ and R⁸ may form a 5-, 6- or 7-membered heterocycle in which the remainder of the ring atoms carbon atoms.
**R**^{**2**} in formula (I) is selected from (a) halogen, (b) loweralkyl, (c) loweralkenyl, (d) cycloalkyl of from three to eight carbons, (e) cycloalkenyl of from four to eight carbons, (f) loweralkoxy, (g) aryloxy, (h) aryl(loweralkyl)oxy, (i) aryl(loweralkyl), (j) cycloalkyl(loweralkyl), (k) amino, (1) (loweralkyl)amino, (m) aryl(loweralkyl)-amino, (n) hydroxy-substituted (loweralkyl)amino, (o) phenyl, (p) substituted phenyl, (q) bicyclic nitrogen-containing heterocycle, (r) nitrogen-containing aromatic heterocycle, and (s) nitrogen-containing heterocycle having the formula 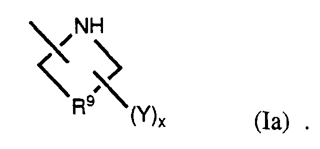 In subformula (Ia) above, **x** is between zero, one, two or three, and **R**^{**9**} is either (i) -(CH₂)ₘ-where **m** is between one and three, or (ii) -(CH₂)ₙR¹⁰(CH₂)ₚ- where **R**^{**10**} is selected from -S-, -O- and -NH-, **n** is one or two, and **p** is one or two. When present, the radical(s) **Y** is/are independently selected at each occurrence from among the following:
   (i) loweralkyl.
   (ii) hydroxy,
   (iii) halogen,
   (iv) halo(loweralkyl),
   (v) loweralkoxy,
   (vi) loweralkoxy(loweralkyl),
   (vii) loweralkoxy(loweralkoxy)(loweralkyl),
   (viii) hydroxy-substituted loweralkyl,
   (ix) imino,
   (x) amino(loweralkyl),
   (xi) halo(loweralkyl)amino(loweralkyl),
   (xii) thioloweralkoxy(loweralkyl),
   (xiii) thioloweralkoxy(loweralkoxy);
   (xiv) aminothioloweralkoxy,
   (xv) cycloalkyl of from three to six carbon atoms,
   (xvi)cycloalkyl(loweralkyl),
   (xvii) phenyl,
   (xviii) substituted phenyl,
   (xix) nitrogen-containing aromatic heterocycle,
   (xx) -NR¹¹R¹² where **R**^{**11**} and **R**^{**12**} are independently selected from hydrogen and loweralkyl or, when one of R¹¹ and R¹² is hydrogen, the other is alkanoyl of from one to eight carbon atoms, an alpha-amino acid, or a polypeptide residue of from two to five amino acids, and
   (xxi) -C(R²¹)(R²²)NH₂ where **R**^{**21**} and **R**^{**22**} are independently selected from among hydrogen, loweralkyl, hydroxy-substituted loweralkyl, amino(loweralkyl), loweralkoxy-(loweralkyl), thioloweralkoxy(loweralkyl), cycloalkyl of from three to six carbon atoms, and loweralkyl substituted with nitrogen-containing aromatic heterocycle (or, taken together with the carbon atom to which they are attached, R²¹ and R²² form a ring structure selected from cycloalkyl of from three to six carbon atoms and nitrogen-containing heterocycle).
**R**^{**3**} in formula (I) is selected from among hydrogen, halogen and loweralkoxy, while **R**^{**4**} is selected from hydrogen, loweralkyl, a pharmaceutically acceptable cation, and a prodrug ester group.
**R**^{**5**} in formula (I) is selected from (a) hydrogen, (b) halogen, (c) hydroxy, (d) loweralkyl. (e) halo(loweralkyl), (f) loweralkoxy, and (g) -NR¹³R¹⁴ where R¹³ and R¹⁴ are independently selected from among hydrogen, loweralkyl, hydroxy-substituted loweralkyl, loweralkoxy(loweralkyl), and alkanoyl of from one to eight carbon atoms.
**R**^{**6**} in formula (I) is loweralkyl.

The above 9-loweralkyl and, in particular, 9-methyl compounds of the invention are found to have a surprising degree of antimicrobial activity against a wide spectrum of Gram-positive and Gram-negative bacteria as well as enterobacteria, including certain bacterial pathogens with known resistance to quinolone-class antibacterial agents. Other susceptible organisms whose growth can be inhibited generally include both aerobic and anaerobic pathogens of the genera *Staphylococcus*, *Lactobacillus*, *Micrococcus, Enterococcus, Streptococcus, Sarcina, Escherichia, Enterobacter, Klebsiella, Pseudomonas, Acinobacter, Proteus, Providencia, Citrobacter, Nisseria, Baccillus, Bacteroides, Camphylobacter, Peptococcus, Clostridium, Salmonella, Shigella, Legionella, Serratia, Haemophilus, Brucella* and the like. It is therefore expected that the compounds of the present invention will be useful in the treatment and prevention of susceptible bacterial infections in both humans and lower animals. In addition, the compounds, by reason of their *in vitro* activity, may be used in scrub solutions for surface inhibition of bacterial growth.

Accordingly, in a further aspect of the present invention are disclosed pharmaceutical compositions which are useful in the treatment and prophylaxis of bacterial and/or fungal infection in humans and animals, comprising a compound of the invention in combination with a pharmaceutically acceptable carrier.

In yet another aspect of the present invention is disclosed the use of the compounds according to the invention for manufacturing a medicament for treating and/or preventing microbial infections in human or animal patients in need of such treatment, said treatment and prevention comprising the administration to such patients of a therapeutically effective amount of a compound of the invention in amounts and for such a period of time as are sufficient to produce the desired result.

In still another aspect of the present invention are disclosed synthetic schemes and processes which are useful in the preparation of the compounds of the invention, as well as synthetic (chemical) intermediates which can be utilized therein.

### DETAILED DESCRIPTION OF THE INVENTION

Among the compounds of the present invention, preferred sub-classes include those in which **R**^{**3**} is halogen (especially fluoro); **R**^{**5**} is hydrogen, loweralkyl, halo-(loweralkyl), or -NR¹³R¹⁴ (where R¹³ and R¹⁴ are as previously defined); and **R**^{**1**} is either cycloalkyl of from three to eight carbon atoms or substituted phenyl. The radical **R**^{**2**} in the above compounds is preferably bicyclic nitrogen-containing heterocycle or a nitrogen-containing heterocycle of the formula 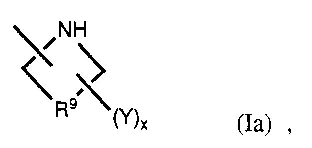 or, even more preferably, **R**^{**2**} is selected from among radicals of the formulae 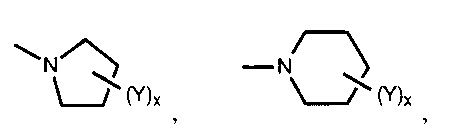 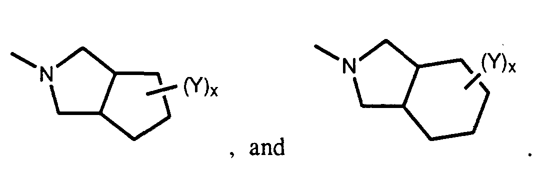 In these radicals **R**^{**2**}**, x** is preferably one or two, and **Y** is preferably either -NR¹¹R¹² or -C(R²¹)(R²²)NH₂, where R¹¹, R¹², R²¹ and R²² are as defined above. As to each of the above compounds, those in which **R**^{**6**} is methyl are especially preferred.

A particularly favored sub-class of the compounds of the present invention are those having the general formula 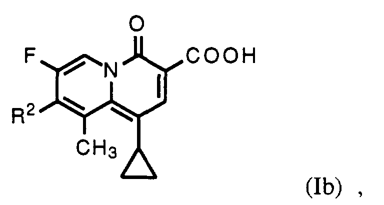 as well as the pharmaceutically acceptable salts, esters and amides thereof, in which **R**^{**2**} is either bicyclic nitrogen-containing heterocycle or a nitrogen-containing heterocycle having the formula 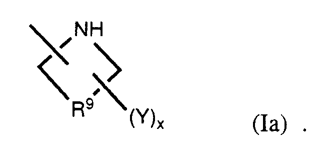 Of these, preferred compounds are those in which **R**^{**2**} is selected from among radicals having the formulae 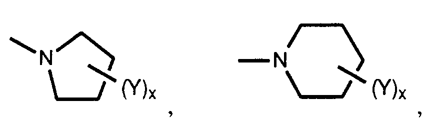 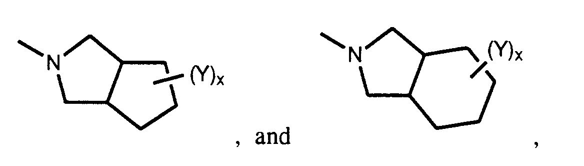 and especially those in which **x** is one or two and **Y** is -NR¹¹R¹² or -C(R²¹)(R²²)NH₂.

Particular compounds which are representative of the compounds of the present invention include the following:
8-(3-amino-1-pyrrolidinyl)-1-cyclopropyl-7-fluoro-9-methyl-4-oxo-4H-quinolizine-3-carboxylic acid hydrochloride;
8-(3-(aminomethyl)pyrrolidinyl)-1-cyclopropyl-7-fluoro-9-methyl-4-oxo-4H-quinolizine-3-carboxylic acid hydrochloride;
8-(2S,4S-4-amino-2-methylpyrrolidinyl)-1-cyclopropyl-7-fluoro-9-methyl-4-oxo-4H-quinolizine-3-carboxylic acid hydrochloride;
8-(3-aminoazetidinyl)-1-cyclopropyl-7-fluoro-9-methyl-4-oxo-4H-quinolizine-3-carboxylic acid hydrochloride;
8-(3(S)-aminopyrrolidinyl)-1-cyclopropyl-7-fluoro-9-methyl-4-oxo-4H-quinolizine-3-carboxylic acid hydrochloride;
1-cyclopropyl-7-fluoro-9-methyl-4-oxo-8-(3-methyl-1-piperazinyl)-4H-quinolizine-3-carboxylic acid hydrochloride;
1-cyclopropyl-7-fluoro-9-methyl-4-oxo-8-piperazinyl-4H-quinolizine-3-carboxylic acid hydrochloride;
1-cyclopropyl-7-fluoro-9-methyl-8-(2-((N-methyl)aminomethyl)-4-morpholinyl)-4-oxo-4H-quinolizine-3-carboxylic acid hydrochloride;
1-cyclopropyl-7-fluoro-9-methyl-4-oxo-8-(1,2,3,4-tetrahydro-2-isoquinolinyl)-4H-quinolizine-3-carboxylic acid ;
1-cyclopropyl-7-fluoro-9-methyl-4-oxo-8-(4-amino-1-piperdinyl)-4H-quinolizine-3-carboxylic acid hydrochloride;
1-cyclopropyl-7-fluoro-9-methyl-4-oxo-8-(3-amino-1-piperdinyl)-4H-quinolizine-3-carboxylic acid hydrochloride;
1-cyclopropyl-7-fluoro-9-methyl-4-oxo-8-(4-(aminomethyl)-1-piperdinyl)-4H-quinolizine-3-carboxylic acid hydrochloride;
1-cyclopropyl-7-fluoro-9-methyl-4-oxo-8-(5-amino-1,2,3,4-tetrahydro-2-isoquinolinyl)-4H-quinolizine-3-carboxylic acid hydrochloride;
1-cyclopropyl-7-fluoro-9-methyl-4-oxo-8-(4-(1-pyrrolyl)-1-piperidinyl)-4H-quinolizine-3-carboxylic acid;
1-cyclopropyl-8-(*cis*-3.5-dimethyl-1-piperazinyl)-7-fluoro-9-methyl-4-oxo-4H-quinolizine-3-carboxylic acid hydrochloride;
1-cyclopropyl-8-(2,7-diaza-7-bicyclo[3.3.0]octyl)-7-fluoro-9-methyl-4-oxo-4H-quinolizine-3-carboxylic acid hydrochloride;
1-cyclopropyl-8-(2,8-diaza-8-bicyclo[4.3.0]nonyl)-7-fluoro-9-methyl-4-oxo-4H-quinolizine-3-carboxylic acid hydrochloride;
1-cyclopropyl-7-fluoro-9-methyl-4-oxo-8-(3(S)-(1-pyrrolyl)-1-pyrrolidinyl)-4H-quinolizine-3-carboxylic acid;
1-cyclopropyl-7-fluoro-8-(3-hydroxy-1-pyrrolidinyl)-9-methyl-4-oxo-4H-quinolizine-3-carboxylic acid hydrochloride;
1-cyclopropyl-7-fluoro-8-(4-methyl-1-piperazinyl)-9-methyl-4-oxo-4H-quinolizine-3-carboxylic acid hydrochloride;
1-cyclopropyl-8-(2,7-diaza-7-bicyclo[3.3.0]octyl)-7-fluoro-9-methyl-4-oxo-4H-quinolizine-3-carboxylic acid hydrochloride;
1-cyclopropyl-8-(2,8-diaza-8-bicyclo[4.3.0]nonyl)-7-fluoro-9-methyl-4-oxo-4H-quinolizine-3-carboxylic acid hydrochloride;
1-cyclopropyl-7-fluoro-9-methyl-4-oxo-8-(3(S)-( 1-pyrrolyl)-1-pyrrolidinyl)-4H-quinolizine-3-carboxylic acid;
1-cyclopropyl-7-fluoro-8-(3-hydroxy-1-pyrrolidinyl)-9-methyl-4-oxo-4H-quinolizine-3-carboxylic acid hydrochloride;
1-cyclopropyl-7-fluoro-8-(4-methyl-1-piperazinyl)-9-methyl-4-oxo-4H-quinolizine-3-carboxylic acid hydrochloride;
1-cyclopropyl-7-fluoro-9-methyl-8-(3(S)-methylamino-1-pyrrolidinyl)-4-oxo-4H-quinolizine-3-carboxylic acid hydrochloride;
1-cyclopropyl-7-fluoro-9-methyl-8-(3(R)-amino-1-pyrrolidinyl)-4-oxo-4H-quinolizine-3-carboxylic acid hydrochloride;
8-(2,4-dimethyl-1-piperazinyl)-1-cyclopropyl-7-fluoro-9-methyl-4-oxo-4H-quinolizine-3-carboxylic acid hydrochloride;
8-(3-(methylamino)-1-piperazinyl)-1-cyclopropyl-7-fluoro-9-methyl-4-oxo-4H-quinolizine-3-carboxylic acid hydrochloride;
8-(3-(methylamino)-1-morpholinyl)-1-cyclopropyl-7-fluoro-9-methyl-4-oxo-4H-quinolizine-3-carboxylic acid hydrochloride;
8-(3-(S)-(methylamino)-1-pyrrolidinyl)-1-cyclopropyl-7-fluoro-9-methyl-4-oxo-4H-quinolizine-3-carboxylic acid hydrochloride;
8-(3-(S)-(1-(methylamino)methyl)-1-pyrrolidinyl)-1-cyclopropyl-7-fluoro-9-methyl-4-oxo-4H-quinolizine-3-carboxylic acid hydrochloride;
8-(3-(S)-(1-(ethylamino)methyl)-1-pyrrolidinyl)-1-cyclopropyl-7-fluoro-9-methyl-4-oxo-4H-quinolizine-3-carboxylic acid hydrochloride;
8-(octahydropyrrolo[3,4-c]pyrrol-1-yl)-1-cyclopropyl-7-fluoro-9-methyl-4-oxo-4H-quinolizine-3-carboxylic acid hydrochloride;
8-(octahydropyrrolo[3,4-c]pyridin-5-yl)-1-cyclopropyl-7-fluoro-9-methyl-4-oxo-4H-quinolizine-3-carboxylic acid hydrochloride;
8-(cis-4-amino-3-methylpyrrolidinyl)-1-cyclopropyl-7-fluoro-9-methyl-4-oxo-4H-quinolizine-3-carboxylic acid hydrochloride;
8-(trans-4-amino-3-methylpyrrolidinyl)-1-cyclopropyl-7-fluoro-9-methyl-4-oxo-4H-quinolizine-3-carboxylic acid hydrochloride;
8-(3-methyl-4-spirocyclopropylpyrrolidinyl)-1-cyclopropyl-7-fluoro-9-methyl-4-oxo-4H-quinolizine-3-carboxylic acid hydrochloride;
8-(3-dimethylaminopyrrolidinyl)-1-cyclopropyl-7-fluoro-9-methyl-4-oxo-4H-quinolizine-3-carboxylic acid, acetic acid salt;
(3R)-8-(3-dimethylaminopyrrolidinyl)-1-cyclopropyl-7-fluoro-9-methyl-4-oxo-4H-quinolizine-3-carboxylic acid hydrochloride;
(3R,1S)-8-(3-(1-aminoethyl)pyrrolidinyl)-1-cyclopropyl-7-fluoro-9-methyl-4-oxo-4H-quinolizine-3-carboxylic acid hydrochloride;
(3S,1R)-8-(3-(1-aminoethyl)pyrrolidinyl)-1-cyclopropyl-7-fluoro-9-methyl-4-oxo-4H-quinolizine-3-carboxylic acid hydrochloride;
(3R,1R)-8-(3-(1-aminoethyl)pyrrolidinyl)-1-cyclopropyl-7-fluoro-9-methyl-4-oxo-4H-quinolizine-3-carboxylic acid hydrochloride;
1-cyclopropyl-8-((R,S)-3-fluoropyrrolidine)-7-fluoro-9-methyl-4-oxo-4H-quinolizine-3-carboxylic acid;
8-(4-(1-piperidyl)-1-piperidyl)-1-cyclopropyl-7-fluoro-9-methyl-4-oxo-4H-quinolizine-3-carboxylic acid;
8-(4-(1-piperidyl)-1-piperidyl)-1-cyclopropyl-7-fluoro-9-methyl-4-oxo-4H-quinolizine-3-carboxylic acid trifluoroacetic acid salt;
8-(4-(2-pyridyl)-1-piperazinyl)-1-cyclopropyl-7-fluoro-9-methyl-4-oxo-4H-quinolizine-3-carboxylic acid;
8-((2-amino)thioethoxy)-1-cyclopropyl-7-fluoro-9-methyl-4-oxo-4H-quinolizine-3-carboxylic acid trifluoroacetic acid salt;
(3R,1S)-8-(3-(1-amino)propyl)pyrrolidinyl)-1-cyclopropyl-7-fluoro-9-methyl-4-oxo-4H-quinolizine-3-carboxylic acid hydrochloride;
(3R,1S)-8-(3-(1-(N-methyl)amino)propyl)pyrrolidinyl)-1-cyclopropyl-7-fluoro-9-methyl-4-oxo-4H-quinolizine-3-carboxylic acid hydrochloride;
(3R,1S)-8-(3-(1-amino-3-methylpropyl)pyrrolidinyl)-1-cyclopropyl-7-fluoro-9-methyl-4-oxo-4H-quinolizine-3-carboxylic acid hydrochloride;
8-(3-(1-aminocyclopropyl)pyrrolidinyl)-1-cyclopropyl-7-fluoro-9-methyl-4-oxo-4H-quinolizine-3-carboxylic acid hydrochloride;
(3R,1S)-8-(3-(1-amino-2-hydroxyethyl)pyrrolidinyl)-1-cyclopropyl-7-fluoro-9-methyl-4-oxo-4H-quinolizine-3-carboxylic acid hydrochloride;
(8-(3-(1-amino-1-methylethyl)pyrrolidinyl)-1-cyclopropyl-7-fluoro-9-methyl-4-oxo-4H-quinolizine-3-carboxylic acid hydrochloride;
8-(3-(1-aminobutyl)pyrrolidinyl)-1-cyclopropyl-7-fluoro-9-methyl-4-oxo-4H-quinolizine-3-carboxylic acid hydrochloride;
1-cyclopropyl-7-fluoro-9-methyl-4-oxo-8-(trans-4-trifluoromethyl-3-aminopyrrolidinyl)-4H-quinolizine-3-carboxylic acid hydrochloride;
1-cyclopropyl-7-fluoro-9-methyl-4-oxo-8-(trans-4-trifluoromethyl-3-aminomethylpyrrolidinyl)-4H-quinolizine-3-carboxylic acid hydrochloride;
3(S)-1-cyclopropyl-7-fluoro-9-methyl-4-oxo-8-(3-(N-(S)-norvalylamino)pyrrolidinyl)-4H-quinolizine-3-carboxylic acid hydrochloride;
3(S)-1-cyclopropyl-7-fluoro-9-methyl-4-oxo-8-(3-(N-(S)-alanylamino)pyrrolidinyl)-4H-quinolizine-3-carboxylic acid hydrochloride;
3(S)-1-cyclopropyl-7-fluoro-9-methyl-4-oxo-8-(3-(N-(S)-alanyl-(S)-alanylamino)pyrrolidinyl)-4H-quinolizine-3-carboxylic acid hydrochloride;
1-cyclopropyl-7-fluoro-4H-8-(1-imidazolyl)-9-methyl-4-oxo-quinolizine-3-carboxylic acid hydrochloride;
8-(3-amino-1-pyrrolidinyl)-1-ethyl-7-fluoro-4H-4-oxo-9-methyl-quinolizine-3-carboxylic acid hydrochloride;
8-(3-amino-1-pyrrolidinyl)-1-cyclopropyl-9-ethyl-7-fluoro-4H-4-oxo-quinolizine-3-carboxylic acid hydrochloride;
1-cyclopropyl-7-fluoro-4H-9-methyl-4-oxo-8-(3-(1,2,3-triazol-1-yl)-1-pyrrolidinyl)-quinolizine-3-carboxylic acid;
1-cyclopropyl-7-fluoro-4H-9-methyl-4-oxo-8-(cis-3-amino-4-methyl-1-pyrrolidinyl)-quinolizine-3-carboxylic acid hydrochloride;
8-(2-aminoethyl)-1-cyclopropyl-7-fluoro-4H-9-methyl-4-oxo-quinolizine-3-carboxylic acid hydrochloride;
8-(3-(ethylaminomethyl)pyrrolidinyl)-1-cyclopropyl-7-fluoro-4H-9-methyl-4-oxo-quinolizine-3-carboxylic acid hydrochloride;
8-(3-(1-aminoethyl)pyrrolidinyl)- 1-cyclopropyl-7-fluoro-4H-9-methyl-4-oxo-quinolizine-3-carboxylic acid hydrochloride;
1-cyclopropyl-7-fluoro-4H-9-methyl-8-(2-methyl-2,8-diaza-8-bicyclo[4.3.0]nonyl)-4-oxo-quinolizine-3-carboxylic acid hydrochloride;
1-cyclopropyl-7-fluoro-4H-8-((1S,4S)-2,5-diaza-bicyclo[2.2.1]heptan-2-yl)-9-methyl-4-oxo-quinolizine-3-carboxylic acid hydrochloride;
1-cyclopropyl-7-fluoro-4H-9-methyl-4-oxo-8-(3-(2-pyridinyl)-1-pyrrolidinyl)-quinolizine-3-carboxylic acid hydrochloride;
8-((1R*,2S*,6R*)-2-amino-8-azabicyclo[4.3.0]nonan-8-yl))-1-cyclopropyl-7-fluoro-4H-9-methyl-4-oxo-quinolizine-3-carboxylic acid hydrochloride;
8-((1R*,2R*,6R*)-2-amino-8-azabicyclo[4.3.0]nonan-8-yl))-1-cyclopropyl-7-fluoro-4H-9-methyl-4-oxo-quinolizine-3-carboxylic acid hydrochloride;
8-((1a,5a,6a)-6-amino-3-azabicyclo[3.1.0]hexan-3-yl))-1-cyclopropyl-9-methyl-7-fluoro-4H-4-oxo-quinolizine-3-carboxylic acid hydrochloride;
8-(*cis*-3-amino-4-fluoro-1-pyrrolidinyl))-1-cyclopropyl-9-methyl-7-fluoro-4H-4-oxo-quinolizine-3-carboxylic acid hydrochloride;
1-cyclopropyl-7-fluoro-4H-8-(1-homopiperazinyl))-9-methyl-4-oxo-quinolizine-3-carboxylic acid, acetic acid salt;
8-(spiro-1,3-dioxacyclopentane[2.3]-1-piperidinyl)-1-cyclopropyl-7-fluoro-4H-9-methyl-4-oxo-quinolizine-3-carboxylic acid;
8-(3-amino-4-methoxypyrrolidinyl)-1-cyclopropyl-7-fluoro-4H-9-methyl-4-oxo-quinolizine-3-carboxylic acid hydrochloride;
8-(4-amino-4-methylpyrrolidinyl)-1-cyclopropyl-7-fluoro-4H-9-methyl-4-oxo-quinolizine-3-carboxylic acid hydrochloride;
8-(4-(2-hydroxyethyl)piperidinyl)-1-cyclopropyl-7-fluoro-4H-9-methyl-4-oxo-quinolizine-3-carboxylic acid;
8-(4-(methoxymethyl)piperidinyl)-1-cyclopropyl-7-fluoro-4H-9-methyl-4-oxo-quinolizine-3-carboxylic acid;
8-(3-amino-3-methylpiperidinyl)-1-cyclopropyl-7-fluoro-4H-9-methyl-4-oxo-quinolizine-3-carboxylic acid hydrochloride;
8-(3-pyrrolylpiperidinyl)-1-cyclopropyl-7-fluoro-4H-9-methyl-4-oxo-quinolizine-3-carboxylic acid;
8-(3-aminopiperidinyl)-1-cyclopropyl-7-fluoro-4H-9-methyl-4-oxo-quinolizine-3-carboxylic acid hydrochloride;
8-(3-amino-3-methylpyrrolidinyl)-1-cyclopropyl-7-fluoro-4H-9-methyl-4-oxo-quinolizine-3-carboxylic acid hydrochloride;
8-(3-amino-4-(1',3'-dioxolanyl)pyrrolidinyl)-1-cyclopropyl-7-fluoro-4H-9-methyl-4-oxo-quinolizine-3-carboxylic acid hydrochloride;
8-(3-amino-4-hydroxy-pyrrolidinyl)-1-cyclopropyl-7-fluoro-4H-9-methyl-4-oxo-quinolizine-3-carboxylic acid hydrochloride;
8-(4-(1-(N-ethylamino)methyl)piperidinyl)-1-cyclopropyl-7-fluoro-4H-9-methyl-4-oxo-quinolizine-3-carboxylic acid hydrochloride;
1-cyclopropyl-7-fluoro-8-(3-hydroxy-4-methylaminopyrrolidinyl)-4H-9-methyl-4-oxo-quinolizine-3-carboxylic acid hydrochloride;
8-(3-aminomethylpiperidinyl)-1-cyclopropyl-7-fluoro-4H-9-methyl-4-oxo-quinolizine-3-carboxylic acid hydrochloride;
8-(2-aminomethyl-4-morpholinyl)-1-cyclopropyl-7-fluoro-4H-9-methyl-4-oxo-quinolizine-3-carboxylic acid hydrochloride;
8-(3-(1-(methylamino)methypiperidinyl)-1-cyclopropyl-7-fluoro-4H-9-methyl-4-oxo-quinolizine-3-carboxylic acid hydrochloride;
8-(3-(methyl(methylenedioxy)methyl)piperidinyl)-1-cyclopropyl-7-fluoro-4H-9-methyl-4-oxo-quinolizine-3-carboxylic acid hydrochloride;
8-(3-(S)-aminopiperidinyl)-1-cyclopropyl-7-fluoro-4H-9-methyl-4-oxo-quinolizine-3-carboxylic acid hydrochloride;
8-(3-(S)-(N-ethyl-N-methylamino)piperidinyl)-1-cyclopropyl-7-fluoro-4H-9-methyl-4-oxo-quinolizine-3-carboxylic acid;
1-cyclopropyl-8-(4-(2'-(N-methylamino)methyl-1',3'-dioxolanyl)piperidinyl)-7-fluoro-9-methyl-4-oxo-4H-quinolizine-3-carboxylic acid hydrochloride;
1-cyclopropyl-8-(3-aza-6-amino-6-methylbicyclo[3.3.0]octan-1-yl)-7-fluoro-9-methyl-4-oxo-4H-quinolizine-3-carboxylic acid hydrochloride;
1-cyclopropyl-8-(3-fluoromethylpiperidinyl)-7-fluoro-9-methyl-4-oxo-4H-quinolizine;
1-cyclopropyl-8-(4-(N,N-dimethyl)aminopiperidinyl)-7-fluoro-9-methyl-4-oxo-4H-quinolizine-3-carboxylic acid hydrochloride;
1-cyclopropyl-8-(6-amino-3-azabicyclo[3.3.0]octyl)-7-fluoro-9-methyl-4-oxo-4H-quinolizine-3-carboxylic acid hydrochloride;
1-cyclopropyl-8-((2-aza-4-(dimethylaminomethyl)bicyclo[4.3.0]non-2-yl)-7-fluoro-9-methyl-4-oxo-4H-quinolizine carboxylic acid hydrochloride;
1-cyclopropyl-8-(3-aza-6-(L-alanylamino)-6-methylbicyclo[3.3.0] octane)-7-fluoro-9-methyl-4-oxo-4H-quinolizine carboxylic acid hydrochloride;
(3R,1R)-8-(3-(1-(N-methyl)amino)propyl)pyrrolidinyl)-1-cyclopropyl-7-fluoro-9-methyl-4-oxo-4H-quinolizine-3-carboxylic acid hydrochloride; and
(3R,1S)-8-(3-(1-amino-2-methoxyethyl)pyrrolidinyl)-1-cyclopropyl-7-fluoro-9-methyl-4-oxo-4H-quinolizine-3-carboxylic acid hydrochloride;
as well as the pharmaceutically acceptable salts, esters and amides thereof.

Preferred among the above representative compounds of the invention are the following:
8-(3-(aminomethyl)pyrrolidinyl)-1-cyclopropyl-7-fluoro-9-methyl-4-oxo-4H-quinolizine-3-carboxylic acid hydrochloride;
8-(3(S)-amino-1-pyrrolidinyl)-1-cyclopropyl-7-fluoro-9-methyl-4-oxo-4H-quinolizine-3-carboxylic acid hydrochloride;
8-(3-amino-1-pyrrolidinyl)-1-cyclopropyl-7-fluoro-9-methyl-4-oxo-4H-quinolizine-3-carboxylic acid hydrochloride;
(3R,1S)-8-(3-(1-amino)propyl)pyrrolidinyl)-1-cyclopropyl-7-fluoro-9-methyl-4-oxo-4H-quinolizine-3-carboxylic acid hydrochloride;
8-(3-(1-aminobutyl)pyrrolidinyl)-1-cyclopropyl-7-fluoro-9-methyl-4-oxo-4H-quinolizine-3-carboxylic acid hydrochloride;
(3R, 1S)-8-(3-(1-amino-2-methoxyethyl)pyrrolidinyl)-1-cyclopropyl-7-fluoro-9-methyl-4-oxo-4H-quinolizine-3-carboxylic acid hydrochloride;
1-cyclopropyl-7-fluoro-9-methyl-4-oxo-8-(4-amino-1-piperdinyl)-4H-quinolizine-3-carboxylic acid hydrochloride;
1-cyclopropyl-7-fluoro-9-methyl-4-oxo-8-(4-(aminomethyl)-1-piperdinyl)-4H-quinolizine-3-carboxylic acid hydrochloride;
1-cyclopropyl-7-fluoro-9-methyl-4-oxo-8-(3-amino-1-piperdinyl)-4H-quinolizine-3-carboxylic acid hydrochloride;
8-(3-(S)-aminopiperidinyl)-1-cyclopropyl-7-fluoro-4H-9-methyl-4-oxo-quinolizine-3-carboxylic acid hydrochloride;
1-cyclopropyl-8-(3-aza-6-amino-6-methylbicyclo[3.3.0] octan-1-yl)-7-fluoro-9-methyl-4-oxo-4H-quinolizine-3-carboxylic acid hydrochloride;
1-cyclopropyl-8-(6-amino-3-azabicyclo[3.3.0]octyl)-7-fluoro-9-methyl-4-oxo-4H-quinolizine-3-carboxylic acid hydrochloride;
8-((1R*,2S*,6R*)-2-amino-8-azabicyclo[4.3.0]nonan-8-yl))-1-cyclopropyl-7-fluoro-4H-9-methyl-4-oxo-quinolizine-3-carboxylic acid hydrochloride;
8-((1R*,2R*,6R*)-2-amino-8-azabicyclo[4.3.0]nonan-8-yl))-1-cyclopropyl-7-fluoro-4H-9-methyl-4-oxo-quinolizine-3-carboxylic acid hydrochloride;
8-(3-(1-aminoethyl)pyrrolidinyl)-1-cyclopropyl-7-fluoro-4H-9-methyl-4-oxo-quinolizine-3-carboxylic acid hydrochloride;
(8-(3-(1-amino-1-methylethyl)pyrrolidinyl)-1-cyclopropyl-7-fluoro-9-methyl-4-oxo-4H-quinolizine-3-carboxylic acid hydrochloride;
(3R,1S)-8-(3-(1-(N-methyl)amino)propyl)pyrrolidinyl)-1-cyclopropyl-7-fluoro-9-methyl-4-oxo-4H-quinolizine-3-carboxylic acid hydrochloride;
8-(3-aminopiperidinyl)-1-cyclopropyl-7-fluoro-4H-9-methyl-4-oxo-quinolizine-3-carboxylic acid hydrochloride;
8-(3-(1-aminocyclopropyl)pyrrolidinyl)-1-cyclopropyl-7-fluoro-9-methyl-4-oxo-4H-quinolizine-3-carboxylic acid hydrochloride;
(3S,1R)-8-(3-(1-aminoethyl)pyrrolidinyl)-1-cyclopropyl-7-fluoro-9-methyl-4-oxo-4H-quinolizine-3-carboxylic acid hydrochloride;
(3R,1S)-8-(3-(1-aminoethyl)pyrrolidinyl)-1-cyclopropyl-7-fluoro-9-methyl-4-oxo-4H-quinolizine-3-carboxylic acid hydrochloride; and
(3R,1R)-8-(3-(1-aminoethyl)pyrrolidinyl)-1-cyclopropyl-7-fluoro-9-methyl-4-oxo-4H-quinolizine-3-carboxylic acid hydrochloride;
as well as the pharmaceutically acceptable salts, esters and amides thereof.

Especially preferred among the representative compounds of the present invention are the following:
8-(3(S)-amino-1-pyrrolidinyl)-1-cyclopropyl-7-fluoro-9-methyl-4-oxo-4H-quinolizine-3-carboxylic acid hydrochloride;
8-(3-amino-1-pyrrolidinyl)-1-cyclopropyl-7-fluoro-9-methyl-4-oxo-4H-quinolizine-3-carboxylic acid hydrochloride;
(3R,1S)-8-(3-(1-amino-2-methoxyethyl)pyrrolidinyl)-1-cyclopropyl-7-fluoro-9-methyl-4-oxo-4H-quinolizine-3-carboxylic acid hydrochloride;
(8-(3-(1-amino-1-methylethyl)pyrrolidinyl)-1-cyclopropyl-7-fluoro-9-methyl-4-oxo-4H-quinolizine-3-carboxylic acid hydrochloride;
8-(3-(1-aminocyclopropyl)pyrrolidinyl)-1-cyclopropyl-7-fluoro-9-methyl-4-oxo-4H-quinolizine-3-carboxylic acid hydrochloride; and
(3R,1S)-8-(3-(1-aminoethyl)pyrrolidinyl)-1-cyclopropyl-7-fluoro-9-methyl-4-oxo-4H-quinolizine-3-carboxylic acid hydrochloride;
as well as the pharmaceutically acceptable salts, esters and amides thereof.

Of the compounds of the present invention, the most preferred is 8-(3(S)-aminopyrrolidinyl)- 1-cyclopropyl-7-fluoro-9-methyl-4-oxo-4H-quinolizine-3-carboxylic acid hydrochloride and its pharmaceutically acceptable salts (hydrochloride and otherwise), esters and amides.

It will be observed above and elsewhere in the disclosure that numerous asymmetric centers may exist in the compounds of the present invention which will be found in the R or S configurations. Except where otherwise noted, the present invention contemplates the various stereoisomers and mixtures thereof.

A number of defined terms are used herein to designate particular elements of the present invention. When so used, the following meanings are intended:

The term "alkanoyl of from one to eight carbons" refers to a radical of the formula -C(O)R¹⁵ where R¹⁵ is hydrogen or an alkyl radical of from one to eight carbon atoms including, but not limited to, acetyl and pivaloyl.

The term "alkyl" refers to saturated, straight- or branched-chain hydrocarbon radicals containing between one and ten carbon atoms including, but not limited to, methyl, ethyl, propyl, isopropyl, *n*-butyl, *tert*-butyl and neopentyl.

The terms "alpha-amino acid" and "polypeptide residue" refer, respectively, to a single amino acid and two to five amino acids each joined by amide (peptide) bonds. The amino acids may be any of the naturally-occurring amino acids such as valine, phenylalanine and glycine or synthetic alpha-amino acids such as cyclohexylalanine, and further may be in either the L or D configuration or a mixture of the two isomers. Preferably, amino acid substituents are optically active and have the L configuration.

The term "amino(loweralkyl)" refers to a loweralkyl radical having appended thereto at least one amino substituent which in turn is optionally substituted with one or two loweralkyl radicals or an alpha-amino acid or polypeptide residue. Examples of amino(loweralkyl) groups include aminoethyl, aminomethyl and N,N-dimethylaminoethyl.

The term "aminothioloweralkoxy" refers to a thioloweralkoxy radical having appended thereto an amino group, as for example aminothiomethoxy and 2-aminothioethoxy.

The term "aromatic group" refers to a C6-to-C10 cyclic radical which is aromatic according to Huckel's rule. Examples of aromatic groups include carbocyclic aromatic radicals such as phenyl and naphthyl as well as nitrogen-containing aromatic heterocyclic radicals, defined below.

The term "aryl(loweralkyl)" refers to a loweralkyl radical having appended thereto an aromatic hydrocarbon group, as for example benzyl and phenylethyl.

The term "aryl(loweralkyl)amino" refers to an amino radical having appended thereto an aryl(loweralkyl) group. Examples of aryl(loweralkyl)amino groups include benzylamino and phenylethylamino.

The term "aryl(loweralkyl)oxy" refers to an aryl(loweralkyl) radical which is joined to the rest of the molecule via an ether linkage *(i.e.,* through an oxygen atom). Examples of aryl(loweralkyl)oxy radicals include benzyloxy and phenylethyloxy.

The term "aryloxy" refers to an aromatic hydrocarbon radical which is joined to the rest of the molecule via an ether linkage *(i.e.,* through an oxygen atom), as for example phenoxy.

The term "bicycloalkyl" refers to a radical comprising a bridged, saturated or unsaturated hydrocarbon ring system having between five and nine carbon atoms in which two non-adjacent carbon atoms of a first ring are linked by an alkylene bridge of between one and three additional carbon atoms, the bicycloalkyl radical being optionally substituted with between one and three additional radicals selected from among aryl(loweralkyl), alkoxycarbonyl, loweralkyl, halo(loweralkyl), amino(loweralkyl), hydroxy-substituted loweralkyl, hydroxy, loweralkoxy, halogen, and amino, (loweralkyl)amino or alkanoylamino of from one to eight carbon atoms in which the amino group may be further substituted with alkanoyl of from one to eight carbons, an alpha-amino acid or a polypeptide. Examples of bicycloalkyl radicals include, but are not limited to, norbornyl, bicylo[2.2.1]hept-2-enyl and bicyclo[1.1.1]pentanyl.

The term "bicyclic nitrogen-containing heterocyclic group" refers to a radical comprising a bicyclic ring system in which the the rings are of the (a) fused, (b) bridged or (c) spiro form. Fused-ring bicyclic nitrogen-containing heterocyclic groups are those in which a first nitrogen-containing heterocycle or aromatic heterocycle has fused to it a second saturated or unsaturated carbocyclic or heterocyclic ring of between three and six atoms of which zero, one or two are heteratoms selected from S, O, and N. Both the first and the second ring may be optionally substituted with between one and three additional radicals A² independently selected from among loweralkyl, halo(loweralkyl), hydroxy-substituted loweralkyl, hydroxy, halogen, amino(loweralkyl), alkanoylamino of from one to eight carbons, phenyl and -NR¹⁷R¹⁸ where R¹⁷ and R¹⁸ are independently hydrogen or loweralkyl or, when one is hydrogen, the other is an alpha-amino acid or a polypeptide residue. Examples of fused-ring bicyclic nitrogen-containing heterocyclic radicals are those having 5:3, 5:4, 5:5, 5:6 and 6:5 ring systems and include, but are not limited to, radicals of the formulae

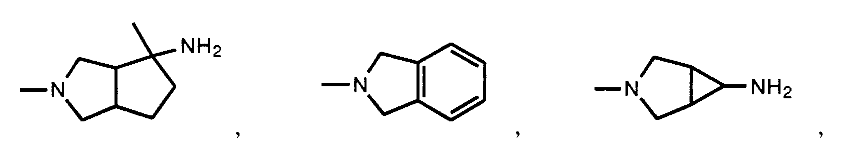

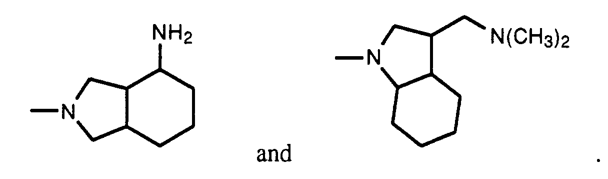

Bridged-ring bicyclic nitrogen-containing heterocyclic groups are those selected the formulae 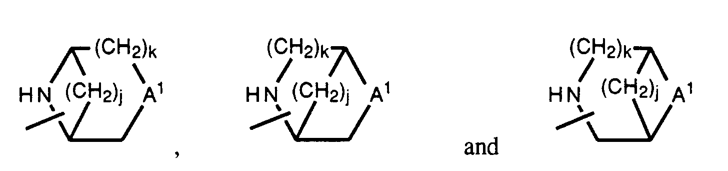 and unsaturated derivatives thereof, where j and k are independently one, two or three, and A¹ is a carbon atom or a heteroatom selected from S, O and N, optionally substituted at any position with between one and three additional radicals A² is as previously defined.

Spiro-ring bicyclic nitrogen-containing heterocyclic groups are those in which a first nitrogen-containing heterocycle or aromatic heterocycle to which is joined, by a single shared carbon atom, a second carbocyclic or heterocyclic ring of between three and six atoms of which zero, one or two are heteratoms selected from S, O, and N. Either the first or the second ring may be substituted with between one and three additional radicals A², where A² is as previously defined. Examples of spiro-ring bicyclic nitrogen-containing heterocyclic radicals include, but are not limited to, those having the formulae

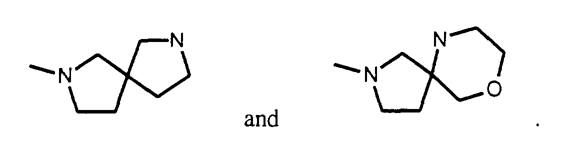

The term "cyclic ether" refers to a 4- to 6-membered monocyclic hydrocarbon radical containing an oxygen ring atom and joined to the rest of the molecule via any of the carbon atoms including, but not limited to, oxetane.

The term "cycloalkenyl of from four to eight carbons" refers to a mono-unsaturated monocyclic hydrocarbon radical having from four to eight carbon atoms in the ring, including, but not limited to, cyclobutenyl, cyclopentenyl, cyclohexenyl and cycloheptenyl, and optionally substituted with between one and three additionals radicals selected from among aryl(loweralkyl), alkoxycarbonyl, loweralkyl, halo(loweralkyl), amino(loweralkyl), hydroxy-substituted loweralkyl, hydroxy, loweralkoxy, halogen, amino, loweralkylamino, and amino, (loweralkyl)amino or alkanoylamino of from one to eight carbon atoms in which the amino group may be further substituted with alkanoyl of from one to eight carbons, an alpha-amino acid or a polypeptide.

The term "cycloalkyl of from three to eight carbons" refers to a saturated monocyclic hydrocarbon radical having from three to eight carbon atoms in the ring and optionally substituted with between one and three additional radicals selected from among aryl(loweralkyl), alkoxycarbonyl, loweralkyl, halo(loweralkyl), amino(loweralkyl), hydroxy-substituted loweralkyl, hydroxy, loweralkoxy, halogen, and amino, (loweralkyl)amino or alkanoylamino of from one to eight carbon atoms in which the amino group may be further substituted with alkanoyl of from one to eight carbons, an alpha-amino acid or a polypeptide. Examples of cycloalkyl radicals include, but are not limited to, cyclopropyl, cyclobutyl, cyclopentyl, cyclohexyl, cycloheptyl, 1-fluoro-cyclopropyl, 2-fluorocyclopropyl and 2-aminocyclopropyl.

The term "cycloalkyl(loweralkyl)" refers to a loweralkyl radical having appended thereto a cycloalkyl radical of from three to eight carbon atoms, which cycloalkyl radical may be optionally substituted as described above.

The term "fused" as used herein refers to two cyclic groups having two adjacent ring atoms in common.

The terms "halo" and "halogen" refer to a monovalent radical selected from among chloro (Cl), bromo (Br), fluoro (F) and iodo (I).

The term "halo(loweralkyl)" refers to a loweralkyl radical having appended thereto between one and three halogen atoms. Examples of halo(loweralkyl) radicals include fluoromethyl, trifluoromethyl, 1-fluoroethyl, 2-fluoroethyl and 1,2-difluoroethyl.

The term "halo(loweralkyl)amino(loweralkyl)" refers to an amino(loweralkyl) radical having appended thereto a halo(loweralkyl) group, as for example 2-fluoroethylaminomethyl.

The term "halo-substituted nitrogen-containing aromatic heterocycle" refers to a nitrogen-containing aromatic heterocycle radical having appended thereto between one and three halogen atoms including, but not limited to, 5-fluoro-2-pyrimidyl.

The term "hydroxy-substituted loweralkyl" refers to a loweralkyl radical having appended thereto between one and three hydroxyl groups, as for example hydroxymethyl and 2-hydroxyethyl.

The term "hydroxy-substituted (loweralkyl)amino" refers to a (loweralkyl)amino radical having appended thereto between one and three hydroxyl groups, as for example hydroxymethylamino and 2-hydroxyethylamino.

The term "imino" refers to a divalent radical of the formula =N-OH.

The term "loweralkenyl" refers to a straight- or branched-chain hydrocarbon radical containing between two and six carbon atoms and possessing at least one carbon-carbon double bond. Examples of loweralkenyl radicals include vinyl, allyl, 2- or 3-butenyl, 2-,3- or 4-pentenyl, 2-,3-,4- or 5-hexenyl and isomeric forms thereof.

The term "loweralkoxy" refers to a loweralkyl radical which is appended to the rest of the molecule via an ether linkage (*i.e.,* through an oxygen atom), as for example methoxy, ethoxy, propoxy, *tert*-butoxy, pentyloxy, hexyloxy, isomeric forms thereof and the like.

The term "loweralkoxycarbonyl" refers to a radical of the formula -C(O)R²⁵ wherein R²⁵ is a loweralkoxy group, as for example ethoxycarbonyl and methoxycarbonyl.

The term ""loweralkoxy(loweralkoxy)(loweralkyl)" refers to a loweralkoxy(loweralkyl) radical having appended thereto a loweralkoxy group, as for example methoxymethoxymethyl and ethoxymethoxymethyl.

The term "loweralkoxy(loweralkyl)" refers to a loweralkyl radical having appended thereto a loweralkoxy group and optionally substituted with an amino additional radical, as for example methoxyethyl, ethoxymethyl and 1-amino-2-methoxyethyl.

The term "loweralkyl" refers to an alkyl radical containing one to six carbon atoms including, but not limited to, methyl, ethyl, propyl, isopropyl, *n*-butyl, *tert*-butyl and neopentyl.

The term "(loweralkyl)amino" refers to an amino radical substituted with between one and two loweralkyl radicals including, but not limited to, methylamino, ethylamino, dimethylamino, propylamino and ethylmethylamino.

The tem "loweralkynyl" refers to a straight- or branched-chain hydrocarbon radical containing between two and six carbon atoms and possessing at least one carbon-carbon triple bond. Examples of loweralkynyl radicals include ethynyl, 2-hexyn-1-yl, 3,3-dimethyl-1-butyn-1-yl and 3-methylbutyn-3-yl.

The term "nitrogen-containing aromatic heterocycle" refers to a monocyclic aromatic radical having from five to seven ring atoms of which one ring atom is nitrogen; zero, one or two ring atoms are additional heteroatoms independently selected from S, O and N; and the remaining ring atoms are carbon, the radical being joined to the rest of the molecule via any of the ring atoms. Examples of nitrogen-containing aromatic heterocycles include pyridine, pyrazine, pyrimidine, pyrrole, pyrazole, imidazole, thiazole, oxazole, isooxazole, thiadiazole, oxadiazole and substituted derivatives thereof.

The term "nitrogen-containing heterocycle" refers to a monocyclic radical having from four to seven ring atoms of which one is nitrogen; zero, one or two are additional heteroatoms independently selected from S, O and N; and the remainder are carbon, the radical being joined to the rest of the molecule via any of the ring atoms and being optionally substituted, either on a nitrogen or a carbon atom, by an additional radical selected from among aryl(loweralkyl), alkoxycarbonyl, loweralkyl, halo(loweralkyl), amino(loweralkyl), hydroxy-substituted loweralkyl, hydroxy, loweralkoxy, halogen, amino, loweralkylamino, and amino, (loweralkyl)amino or alkanoylamino of from one to eight carbon atoms in which the amino group may be further substituted with alkanoyl of from one to eight carbons, an alpha-amino acid or a polypeptide. Examples of nitrogen-containing heterocycles include pyrrolidine, isooxazolidine, oxazolidine, piperidine, piperazine, morpholine, thiomorpholine, aziridine and azetidine.

The term "pharmaceutically acceptable cation" refers to a positively-charged inorganic or organic ion that is generally considered suitable for human consumption. Examples of pharmaceutically acceptable cations are hydrogen, alkali metal (lithium, sodium and potassium), magnesium, calcium, ferrous, ferric, ammonium, alkylammonium, dialkylammonium, trialkylammonium, tetraalkylammonium, diethanolammmonium, triethanolammonium, and guanidinium ions, and protonated forms of lysine, procaine and choline. Cations may be interchanged by methods known in the art, such as ion exchange. Where compounds of the present invention are prepared in the carboxylic acid form (that is, where R₄ is hydrogen) addition of a base form of the cation, (such as a hydroxide or a free amine) will yield the appropriate cationic form.

By "pharmaceutically acceptable salts, esters and amides", as of the compounds of formula I, is meant those carboxylate salts, amino acid addition salts, esters and amides which are, within the scope of sound medical judgement, suitable for use in contact with the tissues of humans and lower animals without undue toxicity, irritation, allergic response and the like, commensurate with a reasonable benefit/risk ratio, and effective for their intended use, as well as the zwitterionic forms thereof.

Pharmaceutically acceptable salts are well known in the art For example, S. M Berge *et al.* describe pharmaceutically acceptable salts in detail in J. Pharmaceutical Sciences, 66:1-19 (1977). Examples of pharmaceutically acceptable, nontoxic acid addition salts are salts of an amino group formed with inorganic acids such as hydrochloric acid, hydrobromic acid, phosphoric acid, sulfuric acid and perchloric acid or with organic acids such as acetic acid, oxalic acid, maleic acid, tartaric acid, citric acid, succinic acid or malonic acid or by using other methods used in the art such as ion exchange. Other pharmaceutically acceptable salts include nitrate, bisulfate, borate, formate, butyrate, valerate, 3-phenylpropionate, camphorate, adipate, benzoate, oleate, palmitate, stearate, laurate, lactate, fumarate, ascorbate, aspartate, nicotinate, p-toluenesulfonate, camphorsulfonate, methanesulfonate, 2-hydroxyethanesulfonate, gluconate, glucoheptonate, lactobionate, glycerophosphate, pectinate, lauryl sulfate and the like or metal salts such as sodium, potassium, magnesium or calcium salts or amino salts such as ammonium, triethylamine salts and the like, all of which may be prepared according to conventional methods.

Examples of pharmaceutically acceptable, non-toxic esters of the present invention include C1-to-C6 alkyl esters and C5-to-C7 cycloalkyl esters, although C1-to-C4 alkyl esters are preferred. Esters of the compounds of formula I may be prepared according to conventional methods.

Examples of pharmaceutically acceptable, non-toxic amides of the present invention include amides derived from ammonia, primary C1-to-C6 alkyl amines and secondary C1-to-C6 dialkyl amines. In the case of secondary amines, the amine may also be in the form of a 5- or 6-membered heterocycle containing one nitrogen atom. Amides derived from ammonia, C1-to-C3 alkyl primary amides and C1-to-C2 dialkyl secondary amides are preferred. Amides of the compounds of formula I may be prepared according to conventional methods. It is intended that amides of the present invention include amino acid and peptide derivatives of the compounds of formula I as well.

As used herein, the term "pharmaceutically acceptable carrier" means a non-toxic, inert solid, semi-solid or liquid filler, diluent, encapsulating material or formulation auxiliary of any type. Some examples of the materials that can serve as pharmaceutically acceptable carriers are sugars, such as lactose, glucose and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository waxes; oils such as peanut oil, cottonseed oil, safflower oil, sesame oil, olive oil, corn oil and soybean oil; glycols, such as propylene glycol; polyols such as glycerin, sorbitol, mannitol and polyethylene glycol; esters such as ethyl oleate and ethyl laurate; agar; buffering agents such as magnesium hydroxide and aluminum hydroxide; alginic acid; pyrogen-free water; isotonic saline; Ringer's solution; ethyl alcohol and phosphate buffer solutions, as well as other non-toxic compatible substances used in pharmaceutical formulations. Wetting agents, emulsifiers and lubricants such as sodium lauryl sulfate and magnesium stearate, as well as coloring agents, releasing agents, coating agents, sweetening, flavoring and perfuming agents, and preservatives can also be present in the composition, according to the judgement of the formulator.

The term "prodrug". as of the compounds of formula I, refers to derivative compounds that are rapidly transformed *in vivo* to yield the parent compound of the formula I, as for example by hydrolysis in blood. T. Higuchi and V. Stella provide a thorough discussion of the prodrug concept in "Pro-drugs as Novel Delivery Systems", Vol 14 of the A.C.S. Symposium Series, American Chemical Society (1975). Examples of esters useful as prodrugs for compounds containing carboxyl groups can be found on pages 14-21 of "Bioreversible Carriers in Drug Design: Theory and Application", edited by E.B. Roche, Pergamon Press:New York (1987).

The term "prodrug ester group" refers to any of several ester-forming groups that are hydrolyzed under physiological conditions. Examples of prodrug ester groups include pivoyloxymethyl, acetoxymethyl, phthalidyl, indanyl and methoxymethyl, as well as other such groups known in the art, including a (5-R-2-oxo-1,3-dioxolen-4-yl)methyl group. Other examples of prodrug ester groups can be found in the book "Pro-drugs as Novel Delivery Systems", by Higuchi and Stella, cited above.

The term "protecting group" is well-known in the art and refers to substituents on functional groups of compounds undergoing chemical transformation which prevent undesired reactions and degradations during a synthesis; see, for example, T.H. Greene, "Protective Groups in Organic Synthesis", John Wiley & Sons, New York (1981).

The term "substituted phenyl" refers to a benzene ring having between one and five non-hydrogen substituents, each independently selected from among halogen, hydroxy, loweralkoxy, loweralkyl, hydroxy-substituted loweralkyl, amino, (loweralkyl)amino, amino(loweralkyl) and nitrogen-containing heterocycle. Examples of substituted phenyl radicals include 2-fluorophenyl, 4-fluorophenyl and 2,4-difluorophenyl.

The term "thioloweralkoxy" refers to a radical of the formula -SR³⁵ where R³⁵ is a loweralkyl group including, but not limited to, thiomethoxy and thioethoxy.

The term "thioloweralkoxy(loweralkyl)" refers to a loweralkyl radical having appended thereto a thioloweralkoxy group including, but not limited to, thiomethoxymethyl and thiomethoxyethyl.

The compounds of the invention may be administered alone or in combination or in concurrent therapy with other agents. When utilizing the compounds of the present invention for antimicrobial therapy, the specific therapeutically effective dose level for any particular patient will depend upon a variety of factors including the disorder being treated and the severity of the disorder; activity of the particular compound used; the specific composition employed; the age, body weight, general health, sex and diet of the patient; the time of administration, route of administration, and rate of excretion of the specific compound employed; the duration of the treatment; drugs used in combination or coincidently with the specific compound employed; and like factors well known in the medical arts.

The total daily dose of the compounds of this invention administered to a host in single or in divided doses can be in amounts, as for example from 0.1 to 200 mg/kg body weight or more usually from 0.25 to 100 mg/kg body weight Single dose compositions may contain such amounts or submultiples thereof as make up the daily dose.

According to the pharmaceutical compositions of the present invention, the compounds of the invention may be administered orally, parenterally, by inhalation spray, rectally, or topically in unit dosage formulations containing conventional nontoxic pharmaceutically acceptable carriers, adjuvants, diluents and/or vehicles as desired. The term "parenteral" as used herein includes subcutaneous injections, intravenous, intramuscular, intrasternal injection or infusion techniques.

Injectable preparations, as for example sterile injectable aqueous or oleaginous suspensions, may be formulated according to the known art using suitable dispersing or wetting agents and suspending agents. The sterile injectable preparation may also be a sterile injectable solution or suspension in a nontoxic parenterally acceptable diluent or solvent, as for example as a solution in 1,3-butanediol. Among the acceptable vehicles and solvents that may be employed are water, Ringer's solution, U.S.P. and isotonic sodium chloride solution. In addition, sterile, fixed oils are conventionally employed as a solvent or suspending medium. For this purpose any bland fixed oil can be employed including synthetic mono- or diglycerides. In addition, fatty acids such as oleic acid are used in the preparation of injectables.

In order to prolong the effect of a drug, it is often desirable to slow the absorption of a drug from subcutaneous or intramuscular injection. The most common way to accomplish this is to inject a suspension of crystalline or amorphous material with poor water solubility The rate of absorption of the drug becomes dependent on the rate of dissolution of the drug which is, in turn, dependent on the physical state of the drug, for example, the crystal size and the crystalline form. Another approach to delaying absorption of a drug is to administer the drug as a solution or suspension in oil. Injectable depot forms can also be made by forming microcapsule matrices of drugs and biodegradable polymers such as polylactide-polyglycolide. Depending on the ratio of drug to polymer and the composition of the polymer, the rate of drug release can be controlled. Examples of other biodegradable polymers include polyorthoesters and polyanhydrides. Depot injectables can also be made by entrapping the drug in liposomes or microemulsions which are compatible with body tissues.

Suppositories for rectal or vaginal administration of the drug can be prepared by mixing the drug with a suitable nonirritating excipient such as cocoa butter and polyethylene glycol which are solid at ordinary temperature but will melt in the rectum or in the vagina and release the drug.

Solid dosage forms for oral administration may include capsules, tablets, pills, powders, prills and granules. In such solid dosage forms the active compound may be admixed with at least one inert diluent such as sucrose, lactose or starch. Such dosage forms may also comprise, as is normal practice, additional substances other than inert diluents, e.g., tableting lubricants and other tableting aids such as magnesium stearate and microcrystalline cellulose. In the case of capsules, tablets and pills, the dosage forms may also comprise buffering agents. Tablets and pills can additionally be prepared with enteric coatings and other release-controlling coatings.

Liquid dosage forms for oral administration may include pharmaceutically acceptable emulsions, microemulsions, solutions, suspensions, syrups and elixirs containing inert diluents commonly used in the art such as water. Such compositions may also comprise adjuvants, such as wetting agents; emulsifying and suspending agents; and sweetening, flavoring and perfuming agents.

If desired, the compounds of the present invention can be incorporated into slow release or targeted delivery systems such as polymer matrices, liposomes and microspheres. They may be sterilized, for example, by filtration through a bacteria-retaining filter, or by incorporating sterilizing agents in the form of sterile solid compositions which can dissolve in sterile water, or some other sterile injectable medium immediately before use.

The active compounds can also be in micro-encapsulated form with one or more excipients as noted above.

Dosage forms for topical or transdermal administration of a compound of this invention further include ointments, pastes, creams, lotions, gels, powders, solutions, sprays, inhalants or patches. The active component is admixed under sterile conditions with a pharmaceutically acceptable carrier and any needed preservatives or buffers as may be required. Ophthalmic formulations, ear drops, eye ointments, powders and solutions are also contemplated as possible forms for administration.

The ointments, pastes, creams and gels may contain, in addition to an active compound of this invention, excipients such as animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays can contain, in addition to the compounds of this invention, excipients such as lactose, talc, silicic acid, aluminum hydroxide, calcium silicates and polyamide powder, or mixtures of these substances. Sprays can additionally contain customary propellants such as chlorofluorohydrocarbons or substitutes therefor.

Transdermal patches have the added advantage of providing controlled delivery of a compound to the body. Such dosage forms can be made by dissolving or dispersing the compound in the proper medium. Absorption enhancers can also be used to increase the flux of the compound across the skin. The rate can be controlled by either providing a rate controlling membrane or by dispersing the compound in a polymer matrix or gel.

A further possibility for delivery and/or utilization of the compounds of the present invention is by chemical conjugation of the compounds with other antibacterials such as beta-lactams. Similar dual-action conjugates (between beta-lactams and quinolones) are proposed in the published European patent application No. 597 303 of Dax *et al.* (published on May 18, 1994) and the published international patent application No. PCT/US92/08246 of White *et al.* (Publication No. WO 93/07154, published on April 15, 1993). In the manner suggested by these references, a carbon-nitrogen bond or other covalent link may be formed between, for example, either an amino substituent at the C-8 position or a carboxylic acid group at the C-3 position of a compound of the present invention, and an alkyl or other group of a beta-lactam.

In general, the compounds of the present invention are synthesized according to reaction Schemes I through III presented below, in which R¹, R², R⁶ and X correspond to the groups defined in connection with formula (I), R is a loweralkyl group, X is a halogen atom and L is a suitable leaving group, as for example a halogen atom.

Certain abbreviations are used repeatedly in the specification which follows. These include: BOC for t-butoxycarbonyl; (BOC)₂ for di-t-butyl dicarbonate; CBZ for benzyloxycarbonyl; DMF for dimethyl formamide; DMSO for dimethyl sulfoxide; HRMS for high resolution mass spectroscopy; LAH for lithium aluminum hydride; LDA for lithium diethyl amide; RaNi for Raney Nickel; and THF for tetrahydrofuran.

For the preparation of the compounds of formula (I) which are alpha-amino acid or peptide derivatives of amine groups at R², the condensation of the amino group with amino acids and peptides may be effected in accordance with conventional condensation methods such as the azide method, the mixed acid anhydride method, the DCC (dicyclohexylcarbodiimide) method, the active ester method (p-nitrophenyl ester method, N-hydroxysuccinic acid imide ester method, cyanomethyl ester method and the like), the Woodward reagent K method, the DCC-HOBT (1-hydroxy-benzotriazole) method and the like. Classical methods for amino acid condensation reactions are described in "Peptide Synthesis", Second Edition, M. Bodansky, Y.S. Klausner and M.A. Ondetti (1976). It is contemplated that the amino acid coupling reaction could be carried out before or after the amino-containing group is incorporated into the compound by displacement of the 7-fluorine atom of the appropriate intermediate.

As in conventional peptide synthesis, branched chain amino and carboxyl groups at alpha and omega positions in amino acids may be protected and deprotected if necessary. The protecting groups for amino groups which can be used involve, for example, benzyloxycarbonyl (Z), o-chloro-benzyloxycarbonyl((2-Cl)Z), p-nitrobenzyloxycarbonyl (Z(NO2)), p-methoxybenzyloxycarbonyl (Z(OMe)), t-butoxycarbonyl (Boc), t-amyloxycarbonyl (Aoc), isobornealoxycarbonyl, adamantyloxycarbonyl (Adoc), 2-(4-biphenyl)-2-propyloxy carbonyl (Bpoc), 9-fluorenyl-methoxycarbonyl (Fmoc), methylsulfonylethoxy carbonyl (Msc), trifluoroacetyl, phthalyl, formyl, 2-nitrophenylsulfenyl (Nps), diphenylphosphinothioyl (Ppt) and dimethylphosphino-thioyl (Mpt).

The examples of protecting groups for carboxyl groups involve, for example, benzyl ester (OBzl), cyclohexyl ester, 4-nitrobenzyl ester (OBzlNO2), t-butyl ester (OtBu), 4-pyridylmethyl ester (OPic) and the like.

In the course of the synthesis of certain of the compounds of the present invention, specific amino acids having functional groups other than amino and carboxyl groups in the branched chain such as arginine, cysteine, serine and the like may be protected, if necessary, with suitable protecting groups. It is preferable that, for example, the guanidino group (NG) in arginine be protected with nitro, p-toluenesulfonyl (Tos), benzyloxycarbonyl (Z), adamantyloxycarbonyl (Adoc), p-methoxybenzenesulfonyl, 4-methoxy-2,6-dimethylbenzenesulfonyl (Mts) or the like; that the thiol group in cysteine be protected with benzyl, p-methoxybenzyl, triphenylmethyl, acetamidomethyl, ethylcarbamyl, 4-methylbenzyl (4-MeBzl), 2,4,6,-trimethylbenzyl (Tmb) or the like; and that the hydroxy group in serine may be protected with benzyl (Bzl), t-butyl, acetyl, tetrahydropyranyl (THP) or the like.

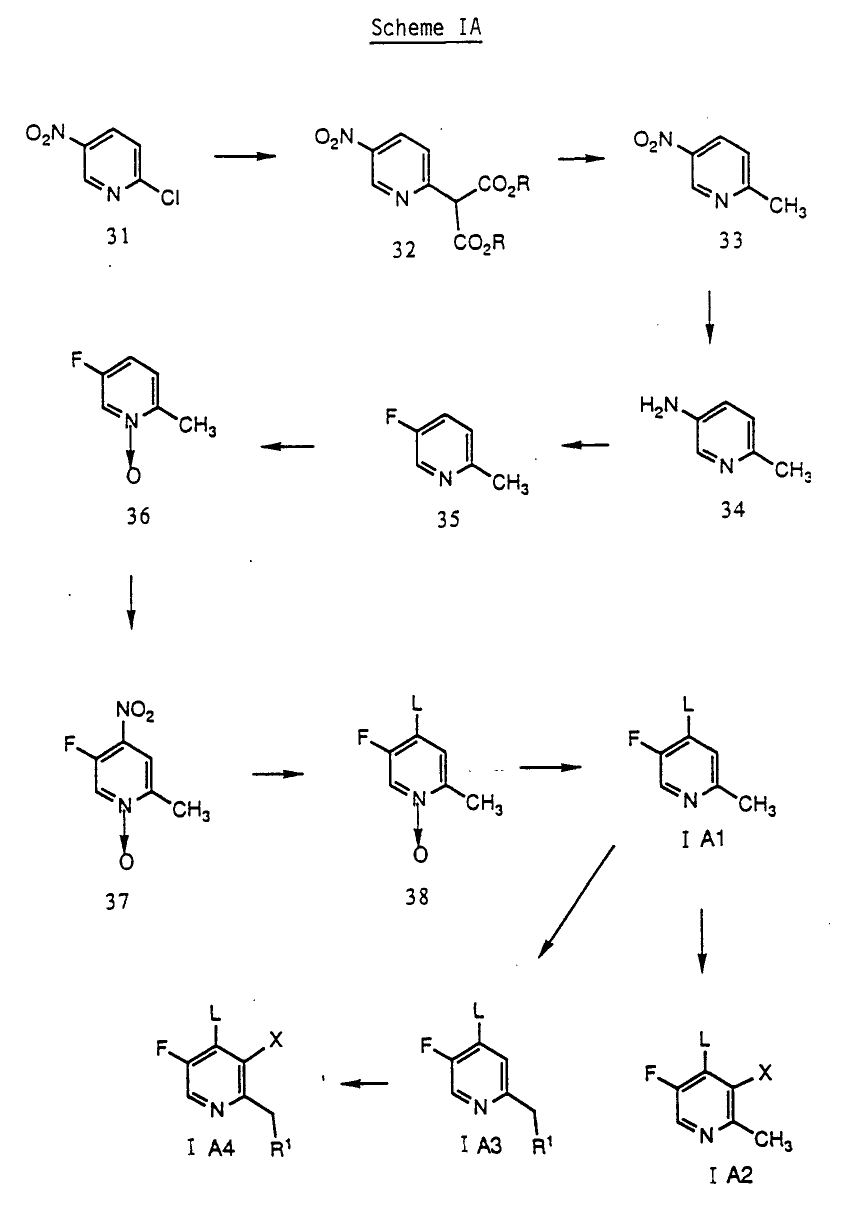

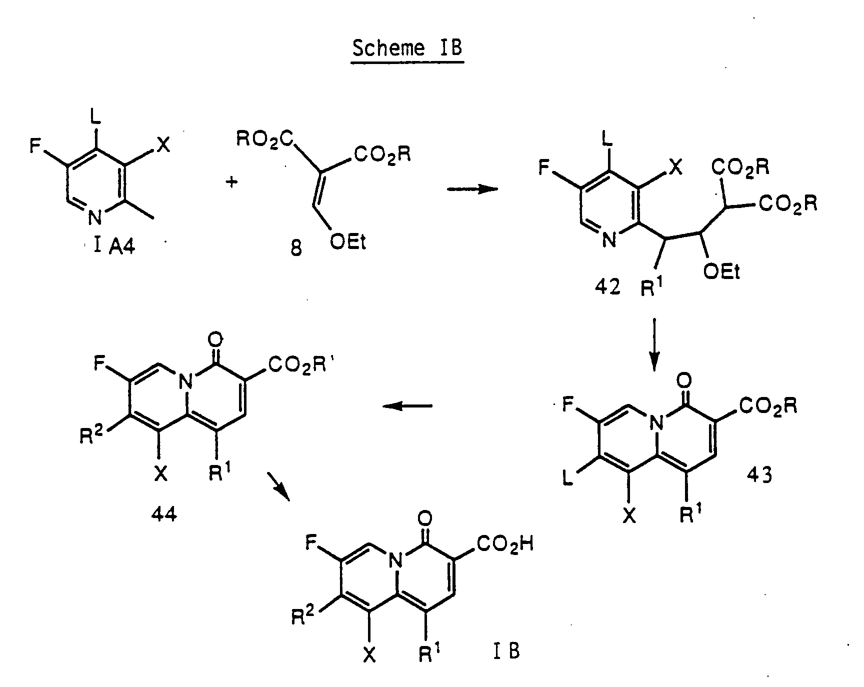

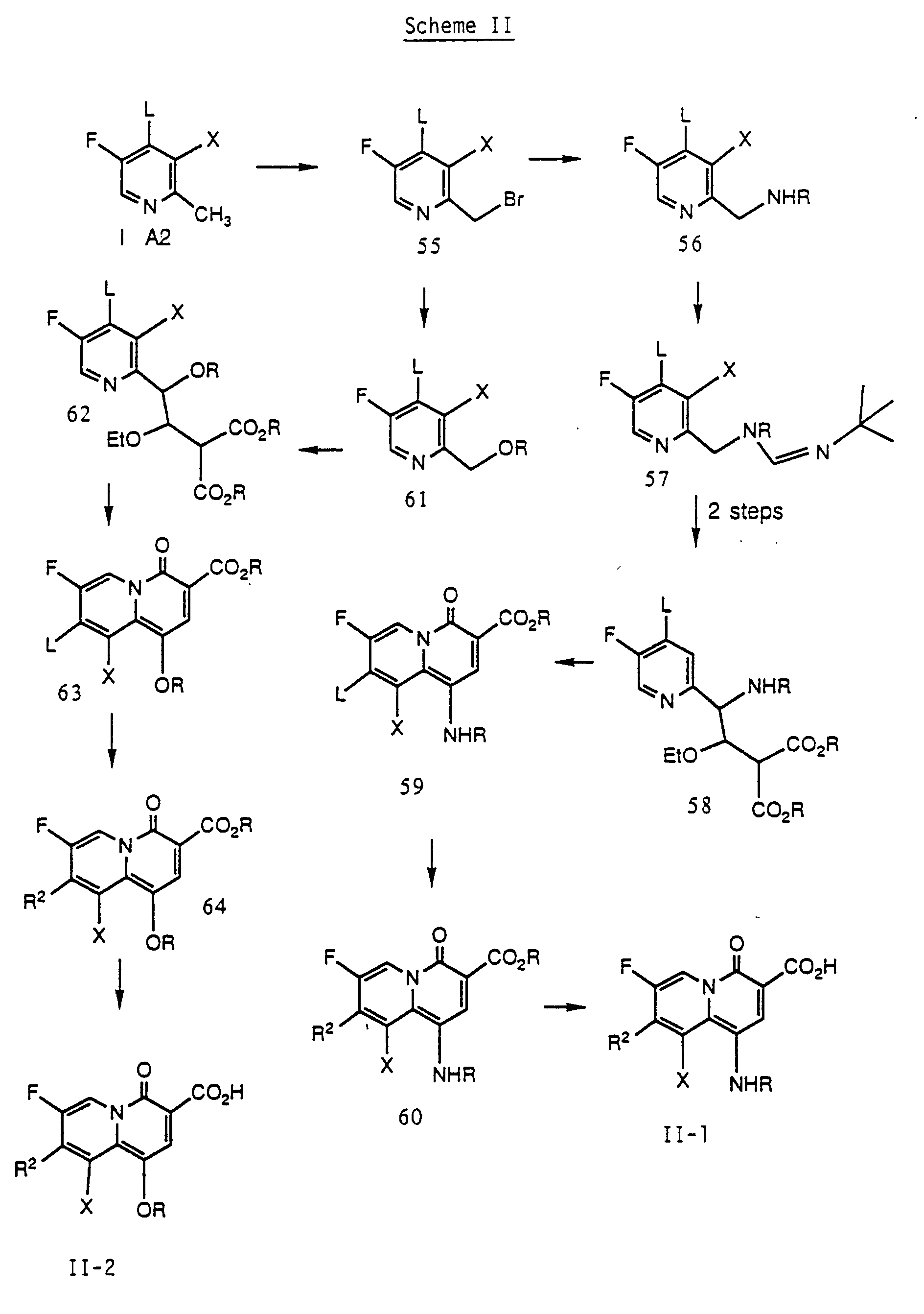

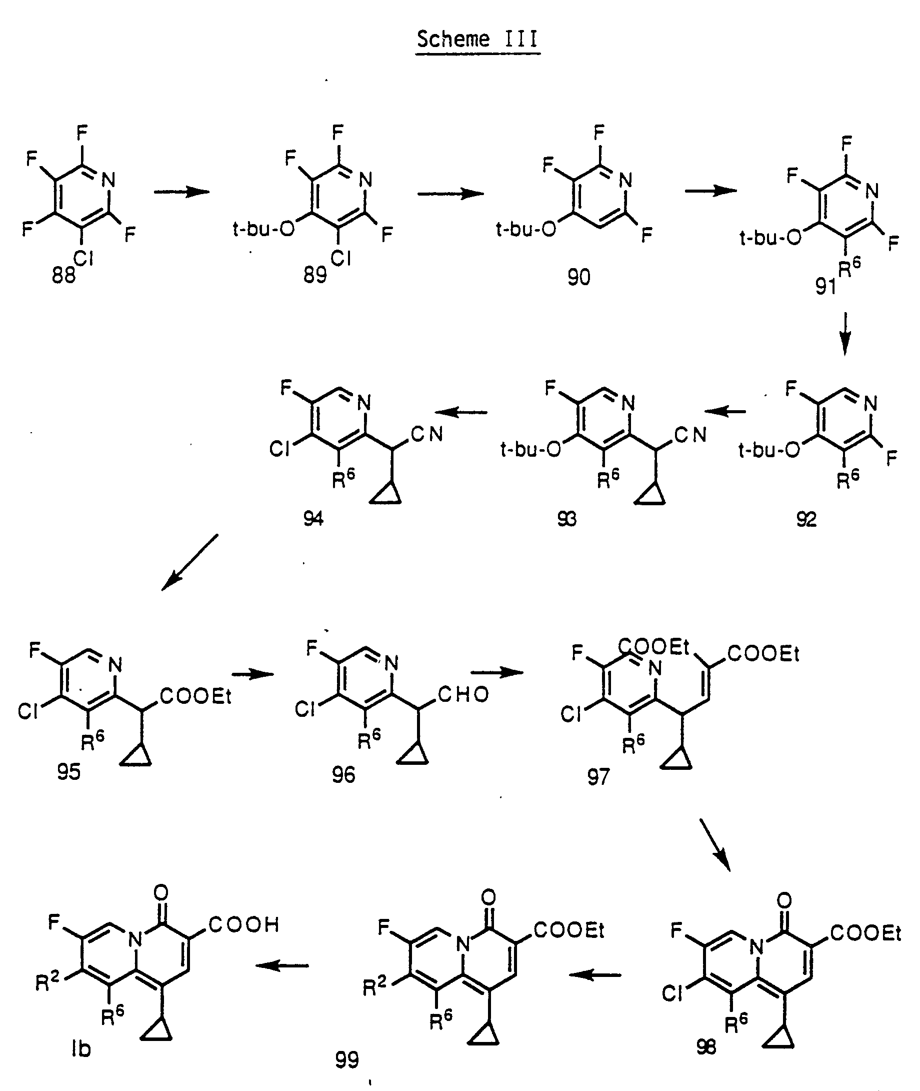

According to Scheme I A illustrated above, a compound of formula 31 is treated with a malononic acid ester, for example diethyl malonate, in the presence of a suitable base such as sodium hydride in a polar nonprotic solvent such as an ether, for example diethyl ether or THF, to afford a compound of formula 32. Compounds of formula 32 are, in turn, decarboxylated, for example by heating them in strong mineral acid such as aqueous sulfuric acid, to afford the compounds of formula 33. The nitro-compound of formula 33 is reduced to the corresponding amino-compound of formula 34. The nitro group may be reduced by catalytic hydrogenation using standard techniques or by any of a variety of known reducing agents such as using a metal, for example zinc, tin or iron, in the presence of a mineral acid, usually hydrochloric acid. The amino-compound of formula 34 is converted to the corresponding fluoro-compound of formula 35 by treatment with ethyl nitrite and tetrafluoroboric acid, followed by treatment with potassium fluoride. The compound of formula 35 is then converted into the corresponding N-oxide of formula 36 by oxidation, for example using peracetic acid. The reaction is carried out in the range from about 20°C up to the reflux temperature of the solvent employed, preferably at about 50°C. The compound of formula 36 is nitrated to afford compounds of formula 37. The nitration reaction can be carried out using a variety of known nitrating agents, for example a mixture of nitric acid and sulfuric acid or a mixture of sulfuric acid and potassium nitrate, or by using nitronium salts such as nitronium trifluoromethanesulfonate. The nitro compound of formula 37 is, in turn, convened to the corresponding halo compound of formula 38 by treatment with mineral acid at ambient or elevated temperature as desired. For example, the compound of formula 37 is treated with aqueous hydrochloric acid at a temperature of about 100-120°C to afford the compound of formula 38 wherein L is Cl. The compound of formula 38 is, in turn converted to the compound of formula I A1 by reduction, for example using a metal such as iron or zinc in the presence of an acid such as acetic acid. The compound of formula I A1 is, in turn, converted to the compound of formula I A2 by treatment with a suitable base, such as LDA, followed by treatment with a halogenating agent, for example N-chloro or N-bromo succinimide. Alternately, the compounds of formula I A1 are converted to compounds of formula I A3, wherein R¹ is alkyl, cycloalkyl or carbocyclic aryl(loweralkyl), by treatment with an alkyl, cycloalkyl or carbocyclic aryl(loweralkyl) halide in the presence of a suitable base such as LDA. The compounds of formula I A3 are further treated with a a suitable base, such as LDA, followed by treatment with a halogenating agent, for example N-chloro or N-bromo succinimide to afford the compounds of formula I A4. Compounds of formulae I A1 - I A4 are key intermediates used in the synthesis of quinolizinone compounds.

According to Schemes I B illustrated above, the compounds of formula I A4 are converted to the quinolizinone compounds of formula I B, by the following series of reactions: (1) reaction with an alkoxymethylene malonate derivative of formula 8 in the presence of a suitably strong and hindered base, for example lithium diisopropylamide (LDA), preferably at a temperature below 0°C, and conveniently at -78°C, to afford the compounds of formula 42, (2) cyclization by heating the compound of formula 42 in a solvent with a boiling point greater than 120°C, for example xylene, diglyme, triglyme, sulfolane or Dowtherm A® (a eutectic mixture of biphenyl and diphenyl ether), to afford the compounds of formula 43, (3) displacement of the leaving groups in the 8-position of the quinolizinone compound of formula 43 using 3-aminopyrrolidine with the primary amino group protected, for example with t-butoxycarbonyl, followed by removal of the protecting group to afford the compounds of formula 44, and (4) hydrolysis or hydrogenolysis of the carboxylic acid ester to the corresponding carboxylic acids of formula I B, where, for example, the ester group may be removed by hydrogenolysis when R is benzyl or with tetrabutylammonium fluoride when R is 2(trimethylsilyl)ethanol.

According to Scheme II illustrated above, compounds of formula I A2 are treated with a halogenating agent under suitable conditions for generating halogen radicals, for example using N-bromo- or N-chlorosuccinimide in the presence of a free radical initiator such as AIBN to afford the compounds of formula 55. The halogen on the alpha carbon atom is then displaced by a nucleophile, for example an alkoxide to give the compounds of formula 61 or an amine to give the compounds of formula 56. The amine function is protected during synthesis by converting it to the corresponding formamidine function affording compounds of formula 57. Compounds of formula 57 are reacted with an alkoxymethylene malonate derivative of formula 8 in the presence of a suitably strong and hindered base, for example lithium diisopropylamide (LDA), preferably at a temperature below 0°C, and conveniently at -78°C. The formamidine group is then removed by reaction with hydrazine and acetic acid to afford the compounds of formula 58. The compounds of formula 58 are cyclized as discussed in reaction Scheme III, to afford the compounds of formula 59. The leaving group, L, is then displaced as discussed in reaction Scheme IVCₜₒ afford the compounds of formula 60. The compounds of formula 50 are, in turn, convened to the compounds of formula II-1 as discussed in reaction Scheme I B.

The compounds of formula 61 are converted to the compounds of formula II-2 by the following series of reactions: (1) reaction with an alkoxymethylene malonate derivative of formula 8 in the presence of a suitably strong and hindered base, for example lithium diisopropylamide (LDA), preferably at a temperature below 0°C, and conveniently at -78°C, to afford the compounds of formula 62 (2) cyclization as discussed in reaction Scheme I B, to afford the compounds of formula 63 (3) displacement of the leaving group in the 8-position as discussed in reaction Scheme I B to afford the compounds of formula 64 and (4) conversion of the carboxylic acid ester to the corresponding carboxylic acids of formula II-2.

In accordance with Scheme III, which illustrates a process for preparing the desired compounds of formula Ib wherein R¹ is cyclopropyl, commercially available 3-chloro-2,4,5,6-tetrafluoropyridine (compound 88) is reacted with an alkali salt of t-butanol, such as for example, sodium t-butoxide or lithium t-butoxide, in a polar organic solvent such as THF, first at from 10°C to -78°C for 1-4 hours, then at room temperature for 2-72 hours, to give the compound of formula 89 (isolated from a mixture of products by chromatography). The compound of formula 89 is then reacted with hydrogen over a noble catalyst, such as Pd/C in a sodium acetate buffer, to remove the chlorine and give the compound of formula 90 (also isolated from a mixture of products by chromatography). In the instance where R⁶ is alkyl, the compound of formula 90 is then reacted with a suitable alkyl halide, for example methyl halide or the like, in the presence of a suitably strong and hindered base, for example lithium diisopropylamide (LDA), preferably at a temperature below 0°C, and conveniently at -78°C to afford the compounds of formula 91. In the instance where R⁶ is haloalkyl, for example fluoroalkyl, the compound of formula 90 is first reacted with a suitably strong and hindered base, for example lithium diisopropylamide (LDA), preferably at a temperature below 0°C, and conveniently at -78°C followed by reaction with formaldehyde to give the compound where R⁶ is hydroxymethyl which is then reacted with diaminosulfur trifluoride (DAST) in a non-polar solvent such as methylene chloride to give the compound of formula 91. Alternately, when the R⁶ group is to be a difluoromethyl, for example, the compound of formula 90 is first reacted with a suitably strong and hindered base, for example lithium diisopropylamide (LDA), preferably at a temperature below 0°C, and conveniently at -78°C followed by reaction with DMF to form the intermediate compound wherein R⁶ is CHO, and this intermediate is then reacted with DAST to prepare the compound of formula 91, wherein R6 is difluoromethyl. The compounds of formula 91 are then reacted with hydrazine under nitrogen at reflux temperature for 2-8 hours, and after removal of excess hydrazine the residue is dissolved in an organic solvent, such as methanol or benzene, for example, and air is then passed through the solution of the hydrazino product for 8-16 hours to give the compounds of formula 92. The compounds of formula 92 are then condensed with cyclopropyl acetonitrile in a polar organic solvent, such as THF, for example, in the presence of strong base, such as lithium diethylamide (LDA) or lithium diisopropylamide, at -78°C for 1-4 hours and then at 0°C for 1-4 hours or NaNH2 at -5°C to -10°C for 1 to 8 hours in order to prepare compounds of formula 93. The compounds of formula 93 are then reacted with trifluoroacetic acid under nitrogen for 1-4 hours at ambient temperature to removed the protecting t-butoxide group, and the unprotected material is then reacted with POCl3 in a suitable organic solvent, such as DMF or methylene chloride, for example, at ambient temperature for 8-24 hours in order to prepare the compounds of formula 94.

In an improved preparative method, regarded as a part of the present invention, the compounds of formula 89 may be converted directly to the compounds of formula 91 by treatment with a strong base, such as t-butyllithium or s-butyllithium, for example, in a polar solvent such as THF or the like for a period of from 0.5 to 3 hours, followed by reaction with methyl iodide at a temperature firstly below -50°C then at ambient temperature for a period of from 4 to 20 hours. The compounds of formula 91 may then be converted to the compounds of formula 92 by treatment with a hydride reducing agent, such as LAH or sodium bis-(2-methoxyethoxy)aluminum hydride (Red-Al™), for example, at from 0°C to ambient temperature for a period of from 8-24 hours. The resulting compounds of formula 93 are then reacted with POCl₃ in an organic solvent such as DMF or methylene chloride, for example, at ambient temperature for a period of from 6-20 hours in order to prepare directly the compounds of formula 94.

The cyano compounds of formula 94 are converted to esters of formula 95 by treatment with anhydrous ethanolic HCl followed by treatment with H₂O. The ester compounds of formula 95 are then reduced to the aldehyde compounds of formula 96 by reaction with lithium aluminum hydride in THF at reduced temperatures for 0.5 -2 hours, followed by reaction with oxalyl chloride and DMSO in the presence of triethyl amine at -78°C for 0.25-1.0 hours. The compounds of formula 96 are reacted with with a malonic acid diester, such as diethyl malonate, dibenzyl malonate, *t*-butyl malonate or di-*t*-butyl malonate, in the presence of a suitable base such as piperidine and a catalytic amount of an acid, such as acetic acid or sulfuric acid, in a polar solvent, such as ethanol, followed by isolation of the intemediate compounds of formula 97 with subsequent treatment thereof by heating in a polar, high-boiling solvent such as DMF or DMSO at reflux temperature or in Dowtherm A® for a period of from 0.5 to 4 hours to form the pyridopyrimidine compounds of formula 98. The chloro group of the compounds 98 is displaced by a nucleophile such as nucleophilic amine, for example N-methylpiperazine or 2-methylpiperazine, to afford the compounds of formula 99, which are in turn converted into the compounds of formula Ib as described in Scheme I B for the conversion of compounds of formula 44 into compounds of formula I B.

Representative of the chemical intermediates which are useful in the above syntheses. and which are regarded as a further aspect of the present invention, are the following compounds:
4-t-butoxy-3-chloro-2,5,6-trifluoropyridine;
4-t-butoxy-2,3,6-trifluoropyridine;
4-t-butoxy-2,3,6-trifluoro-5-methylpyridine;
4-t-butoxy-2.5-difluoro-3-methylpyridine;
2-(4-t-butoxy-5-fluoro-3-methyl-2-pyridinyl)cyclopropaneacetonitrile;
2-(4-chloro-5-fluoro-3-methyl-2-pyridinyl)cyclopropaneacetonitrile;
2-(4-chloro-5-fluoro-3-methyl-2-pyridinyl)cyclopropaneacetic acid;
ethyl 2-(4-chloro-5-fluoro-3-methyl-2-pyridinyl)cyclopropaneacetate;
2-(4-chloro-5-fluoro-3-methyl-2-pyridinyl)cyclopropaneacetaldehyde;
2-(4-chloro-5-fluoro-3-methyl-2-pyridinyl)cyclopropaneethanol;
2-(2-(4-chloro-5-fluoro-3-methyl-2-pyridinyl)-2-cyclopropylethylidinyl)-1,3-propanedicarboxylic acid, diethyl ester; and
8-chloro-1-cyclopropyl-7-fluoro-9-methyl-4-oxo-4h-quinolizine-3-carboxylic acid ethyl ester.

The foregoing may be better understood from the following examples, which are presented for the purpose of illustration.

### Examples 1-252 are missing due to their deletion.

### Example 253

### 8-(3-Amino-1-pyrrolidinyl)-1-cyclopropyl-7-fluoro-9-methyl-4-oxo-4H-quinolizine-3-carboxylic acid

### Step 253a. 4-t-Butoxy-3-chloro-2,5,6-trifluoropyridine

To 250 mL of a THF solution containing 106 g (0.571 mmol) of a mixture of 4-chlorotetrafluoropyridine and 3-chloro-tetrahydropyridine (approx 70:30 ratio, from Aldrich Chemical Co.) at -78°C was added a solution of 38.3 g (0.399 mmol) of sodium t-butoxide in 350 mL of THF, and the solution was stirred for 2 hours at -78°C and at ambient temperature for 16 hours. The mixture was poured into 500 mL of hexane, and this mixture was filtered through celite and the filtrate concentrated. The residue was purified by flash chromatography, eluting first with hexane, then ethyl acetate:hexane (1:4), to separate the desired tide product from the mixture of products. MS 238, 240 (M+H)⁺; 1H NMR (CDCl₃) ∂: 1.52 (d, J=2Hz); ¹⁹F NMR (CDCl₃, CFCl₃ as reference) ∂: 73.75 (dd, J₁=14.2, J₂=23.2 Hz), 89.71 (dd, J₁=14.2, J₂=21.98 Hz); 152.42 (apparent t, J=22 Hz).

### Step 253b. 4-t-Butoxy-2,3,6-trifluoropyridine

To the product from Step 253a above (24.92 g, 0.104 mmol) in 100 mL of methanol was added 2.5 g of Pearlman's catalyst (Aldrich Chemical Co.), and the mixture was stirred at ambient temperature for 14 hours under and atmosphere of hydrogen. An additional 2.5 g of catalyst was added, and the mixture was stirred for another 22 hours. The mixture was filtered, the filtrate was concentrated, and the residue was extracted with hexane/ether. After filtration, the solvent was removed by evaporation, and the residue was purified by flash chromatography (ethyl acetate:hexane 1:16) to yield 12.05 g of the title product. MS 206 (M+H)⁺, 233 (M+18)⁺; 1H NMR (CDCl₃) ∂: 1.52 (s, 9H), 6.51 (m, 1H); ¹⁹F NMR (CDCl₃, CFCl₃ as reference) ∂: 72.60 (dd, J₁=14.3, J₂=21.0 Hz), 89.74 (dd, J₁=14.3, J₂=21.0 Hz), 164.68 (dt, J₁=4.2, J₂=21.0 Hz).

### Step 253c 4-t-Butoxy-2,3,6-trifluoro-5-methylpyridine

A freshly prepared solution of lithium diethylamide (LDA) (58.21 mmol) in 30 mL of THF at -78°C was added to 10.0 g (48.74 mmol) of the product from Step 253b in 50 mL of THF at -78°C, and the reaction was stirred for 50 min. To the reaction mixture was added 4.3 mL (69.07 mmol) of methyl iodide, and the mixture was stirred at -78°C for 1 hour and stirred at ambient temperature for 16 hours. The reaction was quenched with saturated NH₄Cl solution, extracted with hexane, and the extracts washed with water, dried over MgSO4 and concentrated to give the title product as a pale yellow oil, which was taken directly to the next step. MS (220) (M+H)⁺; 1H NMR (CDCl₃) ∂: 1.47 (m, 9H), 2.12 (m, 3H). ¹⁹F NMR (CDCl₃, CFCl₃ as reference) ∂: 75.91 (dd apparent, J₁=15.0, J₂=22.1 Hz), 93.17 (dd apparent, J₁=15.0, J₂=22.1 Hz), 156.54 (m).

### Step 253d. 4-t-Butoxy-2,5-difluoro-3-methylpyridine

A sample of the product from Step 253c above (48.74 mmol) and 13.5 mL of hydrazine monohydrate were dissolved in 150 mL of n-propanol. The reaction was stirred at reflux temperature under nitrogen for 4 hours. The volatiles were removed, and the residue was dissolved in methylene chloride, which was washed with water and dried over MgSO₄. The solvent was removed to give the intermediate hydrazine product as a yellow liquid, which was dissolved in 110 mL of methanol. To this was added 20 mL of 20% NaOH and air was passed through the solution for 16 hours. The solvents were removed at 30°C under vacuum. The residue was dissolved in methylene chloride, which was washed with water and dried over MgSO₄. The solvent was removed and the crude product purified by flash chromatography, eluting with ethyl acetate:hexane 1:16 to give the title product as a colorless liquid after removal of the solvents. MS (202) (M+H)⁺; 1H NMR (CDCl₃) ∂: 1.43 (d, 9H, J=1.5 Hz), 2.18 (d, 3H, J=1.5 Hz), 7.85 (br s, 1H); ¹⁹F NMR (CDCl₃, CFCl₃ as reference) ∂: 73.37 (d, J=24.5 Hz), 142.17 (d, J=24.5 Hz).

### Step 253e. 2-(4-t-Butoxy-5-fluoro-3-methyl-2-pyridinyl)cyclopropaneacetonitrile

A sample of the product from Step 253d above (40.8 mmol) was dissolved in 50 mL of THF and cooled to -78°C. To this was added a freshly prepared solution of LDA (0.103 mmol) in 50 mL of THF at -78°C, and the reaction was stirred for 1 hour. The reaction was then stirred at 0°C for 1 hour, quenched with saturated NH₄Cl solution and extracted with ether. The extracts were washed with saturated NaCl solution, dried over MgSO₄, and concentrated. The residue was purified by flash chromatography, eluting with 1:4 ethyl acetate:hexane, to yield 10.33 g of the title product after removal of the solvent. MS 263 (M+H)⁺; 1H NMR (CDCl₃) ∂: 0.50 (m, 2H), 0.63 (m, 1H), 0.73 (m, 1H), 1.60 (m, 1H), 1.43 (d, 9H, J=2 Hz), 2.29 (s, 3H), 3.76 (d, 1H, J=8 Hz), 8.30 (d, 1H, J=3 Hz). IR (neat) 2240, 1580, 1470 cm⁻¹.

### Step 253f. 2-(4-Chloro-5-fluoro-3-methyl-2-pyridinyl)cyclopropaneacetonitrile

A sample of the product from Step 253e above (5.21 g, 19.86 mmol) was dissolved in 50 mL of trifluoroacetic acid, the reaction was stirred under nitrogen for 1 hour at ambient temperature, and the material concentrated to dryness. The residue was dissolved in a mixture of 15.6 mL of DMF and 90 mL of methylene chloride. This solution was cooled in a water bath as 18.8 mL (19.86 mmol) of POCl₃ was added, then the reaction was stirred at ambient temperature for 16 hours. The reaction was quenched by pouring it into ice water, and the mixture was extracted with methylene chloride. The aqueous solution was adjusted to pH7 with NaOH and reextracted with methylene chloride. The extracts were combined and washed with water, dried over MgSO₄ and concentrated. The residue was purified by flash chromatography with 1:4 ethyl acetate:hexane to give 3.26 g of the title product as a colorless liquid after removal of the solvents. MS 225, 227 (M+H)⁺; 1H NMR (CDCl₃) ∂: 0.48 (m, 1H), 0.59 (m, 1H), 0.66 (m, 1H), 0.77 (m, 1H), 1.50 (m, 1H), 2.48 (s, 3H), 3.80 (d, 1H, J=8 Hz), 8.39 (s, 1H). IR (neat) 2240, 1570, 1460 cm⁻¹.

### Step 253g. Ethyl 2-(4-chloro-5-fluoro-3-methyl-2-pyridinyl)cyclopropaneacetate

A sample of the product from Step 253f above (3.26 g, 14.51 mmol) was dissolved in 10 mL of ethanol, and gaseous HCl was introduced until 4 g had been dissolved. The solution was heated to reflux, and 0.36 mL of water was added, then the mixture was stirred for 1 hour. The reaction was cooled, then poured into water, and the mixture was adjusted to pH7 with NaHCO₃. The mixture was then extracted with methylene chloride, which was washed with water, dried over MgSO₄ and concentrated. The residue was triturated with 1:4 ethyl acetate:hexane, and filtered. The filtrate was concentrated and the residue was purified by flash chromatography with 1:4 ethyl acetate:hexane to give 2.262 g of the tide product after removal of the solvent. MS 272, 274 (M+H)⁺; 1H NMR (CDCl₃) ∂: 0.12 (m, 1H), 0.38 (m, 1H), 0.53 (m, 1H), 0.76 (m, 1H), 1.20 (t, 3H, J=7 Hz), 1.67 (m, 1H), 2.40 (s, 3H), 3.23 (d, 1H, J=9 Hz), 4.16 (q, 2H, J=7 Hz), 8.36 (s, 1H).

### Step 253h. 2-(4-Chloro-5-fluoro-3-methyl-2-pyridinyl)cyclopropane-acetaldehyde

A sample of the product from Step 253g above (1.73 g, 6.37 mmol) was dissolved in 10 mL of THF and stirred with water bath cooling and 3.2 mmol of LiAlH₄ (LAH) was added. The mixture was stirred at ambient temperature for 1 hour, then poured into water. This mixture was extracted with ether, the extracts were washed, dried and concentrated to give 1.48 g of a colorless oil. This oil was dissolved in 10 mL of methylene chloride and added to a solution of 3.8 mL (7.6 mmol) of oxalyl chloride and 1.1 mL of DMSO (15.5 mmol) in 15 mL of methylene chloride stirred at -78°C. The solution was stirred for 15 min, and 4.4 mL (31.6 mmol) of triethylamine was added. The stirring was continued at -78°C for 5 min and at -10°C for 10 min. The reaction was quenched with water, and extracted with methylene chloride. The extract was washed, dried and concentrated to give 1.49 g of the crude title product, which was taken directly to the next step without further purification. MS 228, 230 (M+H)⁺; 1H NMR (CDCl₃) ∂: 0.25 (m, 1H), 0.35 (m, 1H), 0.60 (m, 1H), 0.75 (m, 1H), 1.53 (m, 1H), 2.38 (s, 3H), 3.19 (dd, 1H, J=3, J=9 Hz), 8.37 (s, 1H), 9.86 (d, 1H, J=3 Hz).

### Step 253i. 8-Chloro-1-cyclopropyl-7-fluoro-9-methyl-4-oxo-4H-quinolizine-3-carboxylic acid ethyl ester

A sample of the product from Step 253h above (6.37 mmol) was dissolved in 50 mL of ethanol, and to this were added 1.5 mL of piperidine, 1.5 mL of acetic acid, and 5 mL of diethyl malonate (32.9 mmol). The reaction was heated at reflux under nitrogen for 4 hours. The solvents were then removed, and the residue was dissolved in ether. The ether was washed with water and brine, then dried over MgSO₄ and concentrated Purification in a kugelrohr apparatus gave 2.4 g of the crude condensation product. This intermediate product was dissolved in 20 ML of of Dowtherm A™, and this solution was added to 100 mL of Dowtherm A™ heated to 235°C. The reaction was then stirred at 220°C for 45 min. After cooling, the product was separated from the solvent by flash chromatography, eluting with hexane to remove the solvent and then with 1:4 ethyl acetate hexane to remove the product. In this manner 1.065 g of the title product was obtained after removal of the solvent. MS 324, 326 (M+H)⁺; 1H NMR (CDCl₃) ∂: 0.75 (m, 2H), 1.07 (m, 2H), 1.42 (t, 3H, J=7 Hz), 2.31 (m, 1H), 3.08 (s, 3H), 4.42 (q, 2H, J=7 Hz), 8.40 (s, 1H), 9.44 (d, 1H, J=6 Hz).

### Step 253j. 8-(3-(N-BOC-amino)pyrrolidinyl)-1-cyclopropyl-7-fluoro-9-methyl-4-oxo-4H-quinolizine-3-carboxylic acid ethyl ester

A sample of the product from Step 253i above (0.500 g, 1.544 mmol) was dissolved in 20 mL of anhydrous acetonitrile, and 0.600 g of sodium bicarbonate and 0.600 g (3.22 mmol) of 3(S)-(BOC-amino)pyrrolidine were added. The mixture was heated at reflux under nitrogen for 7 hours, then the solvent was removed and the residue was redissolved in methylene chloride. This solution was washed with water, 5% HCl, water, and concentrated.. The residue was purified by flash chromatography, eluting with 100:10 methylene chloride:methanol, followed by 100:10:0.5 methylene chloride: methanol:NH₄OH. Removal of the solvent gave 0.778 g of the title product, which was taken directly to the next step.

### Step 253k. 8-(3-(N-BOC-amino)pyrrolidinyl)-1-cyclopropyl-7-fluoro-9-methyl-4-oxo-4H-quinolizine-3-carboxylic acid

A sample of the product from Step 253j above (0.778 g, 1.645 mmol) was dissolved in 20 mL of THF, 0.570 g of LiOH•H2O and 10 mL of water were added, and the mixture was stirred under nitrogen for 3 hours. The THF was removed under vacuum, and the residue was adjusted to a pH between 2 and 4 with 1 N HCl. The solid was collected, and the filtrate was extracted with methylene chloride and washed and concentrated to give additional product. The combined solids were purified by flash chromatography eluting with 100:5:1 methylene chloride:methanol:acetic acid to yield 0.698 g of the title product after removal of the solvent. This material was taken directly to the next step.

### Step 2531. 8-(3-aminopyrrolidinyl)-1-cyclopropyl-7-fluoro-9-methyl-4-oxo-4H-quinolizine-3-carboxylic acid hydrochloride

A sample of the product from Step 253k above (0.697 g, 1.564 mmol) was dissolved in 17 mL of anhydrous methylene chloride, 5.0 mL of 4 N HCl in dioxane was added, and the reaction was stirred for 1.75 hours. Ether was added, and the precipitate was collected by filtration and washed with ether. The solid was dissolved in water, filtered through a sintered glass funnel, and freeze-dried to give the title product as a yellow solid. mp 230-232°C (dec). MS 346 (M-Cl)⁺; 1H NMR (DMSO) ∂: 0.58(m, 2H), 0.99 (m, 2H), 2.15 (m, 1H), 2.31 (m, 2H), 2.63 (s, 3H), 3.77 (m, 2H), 3.99-4.06 (m, 3H), 7.94 (s, 1H), 8.39 (br s, 3H), 9.10 (d, 1H, J=11 Hz), 13.85 (br s); IR 3440, 1695, 1610 cm⁻¹.

### Example 254

### 8-(3-(aminomethyl)pyrrolidinyl)-1-cyclopropyl-7-fluoro-9-methyl-4-oxo-4H-quinolizine-3-carboxylic acid hydrochloride

The 3-(BOC-amino)pyrrolidine of Step 253j above was replaced by 3-BOC-aminomethylpyrrolidine and the reaction product was carried forward as in Steps 253K and 253l, above, to prepare 0.085 g of the title compound. MS 360 (M-Cl)⁺; 1H NMR (DMSO) ∂: 0.60 (m, 2H), 0.99 (m, 2H), 1.81 (m, 1H), 2.18 (m, 1H), 2.30 (m, 1H), 2.60 (s, 3H), 2.98 (m, 2H), 3.66-3.81 (m, 5H), 7.90 (s, 1H), 8.09 (br s, 3H), 9.06 (d, 1H, J=11 Hz), 13.85 (br s, 1H).

### Example 255

### 8-(2S,4S-4-amino-2-methylpyrrolidinyl)-1-cyclopropyl-7-fluoro-9-methyl-4-oxo-4H-quinolizine-3-carboxylic acid hydrochloride

The 3-BOC-aminopyrrolidine of Step 253j above was replaced by (2S,4S)-4-(BOC-amino)-2-methylpyrrolidine and the reaction product was carried forward as in Steps 253K and 253l, above, to prepare 0.071 g of the title compound. MS 360 (M-Cl)⁺; 1H NMR (DMSO) ∂: 0.51 (m, 1H), 0.63 (m, 1H), 0.90 (m, 1H), 1.09 (m, 1H), 1.17 (d, 3H, J=6 Hz), 2.01 (m, 1H), 2.40 (m, 2H), 2.64 (s, 3H), 3.40 (m, 1H), 3.98 (m, 1H), 4.31 (m, 1H), 4.61 (m, 1H), 8.00 (s, 1H), 9.17 (d, 1H, J=11 Hz).

### Example 256

### 8-(3-aminoazetidinyl)-1-cyclopropyl-7-fluoro-9-methyl-4-oxo-4H-quinolizine-3-carboxylic acid hydrochloride

The 3-BOC-aminopyrrolidine of Step 253j above was replaced by 3-(BOC-amino)azetidine and the reaction product was carried forward as in Steps 253K and 253l, above, to prepare 0.094 g of the title compound. MS 332 (M-Cl)⁺; 1H NMR (DMSO) ∂: 0.61 (m, 2H), 1.00 (m, 2H), 2.30 (m, 1H), 2.61 (s, 3H), 4.15 (m, 1H), 4.56 (m, 2H), 4.86 (m, 2H), 7.89 (s, 1H), 8.51 (br s, 3H), 9.13 (d, 1H, J=10 Hz).

### Example 257

### 8-(3(S)-aminopyrrolidinyl)-1-cyclopropyl-7-fluoro-9-methyl-4-oxo-4H-quinolizine-3-carboxylic acid hydrochloride

The 3-BOC-aminopyrrolidine of Step 253j above was replaced by 3(S)-(BOC-amino)pyrrolidine and the reaction product was carried forward as in Steps 253K and 253l, above, to prepare 0.087 g of the title compound. MS 346 (M-Cl)⁺; 1H NMR (DMSO) ∂: 0.59 (m, 2H), 0.99 (m, 2H), 2.14 (m, 1H), 2.31 (m, 2H), 2.63 (s, 3H), 3.76 (m, 2H), 3.98-4.07 (m, 3H), 7.94 (s, 1H), 8.36 (br s, 3H), 9.11 (d, 1H, J=11 Hz).

### Example 258

### 1-cyclopropyl-7-fluoro-9-methyl-4-oxo-8-(3-methyl-1-piperazinyl)-4H-quinolizine-3-carboxylic acid hydrochloride

The 3-BOC-aminopyrrolidine of Step 253j above was replaced by 2-methylpiperazine (Aldrich Chemical Co.), and the reaction product was carried forward as in Steps 253K and 253l, above, to prepare 225 mg of the title compound. mp > 300°C. IR (KBr): 3420, 1720, 1650 cm⁻ ¹. MS 360 (M-Cl)⁺. ¹H NMR (CD₃OD) ∂: 0.75 (m, 2H), 1.10 (m, 2H), 1.40 (d, 3H, J=7.5 Hz), 2.90 (s, 3H), 3.45 (m, 3H), 3.71 (m, 4H), 8.23 (s, 1H), 9.40 (d, 1H, J=12 Hz). Calc. for C₁₉H₂₃ClFN₃O₃•1.25 H₂O: C, 54.55; H, 6.14; N, 10.04; Found: C, 54.78; H, 5.78; N, 10.05.

### Example 259

### 1-cyclopropyl-7-fluoro-9-methyl-4-oxo-8-piperazinyl-4H-quinolizine-3-carboxylic acid hydrochloride

The 3-BOC-aminopyrrolidine of Step 253j above was replaced by piperazine (Aldrich Chemical Co.), and the reaction product was carried forward as in Steps 253K and 253l, above, to prepare 75 mg of the title compound. mp = 279-280°C. IR (KBr): 3420, 1710, 1650, 1610 cm⁻¹. MS 346 (M-Cl)⁺. ¹H NMR (CD₃OD) ∂: 0.72 (m, 2H), 2.43 (m, 1H), 2.92 (s, 3H), 3.43 (m, 4H), 3.72 (m, 4H), 8.25 (s, 1H), 9.30 (d, 1H, J=12 Hz). Calc. for C₁₈H₂₁ClFN₃O₃•1.5 H₂O: C, 55.32; H, 5.67; N, 10.75; Found: C, 55.52; H, 5.49; N, 10.59.

### Example 260

### 1-cyclopropyl-7-fluoro-9-methyl-8-(2-((N-methyl)aminomethyl)-4-morpholinyl)-4-oxo-4H-quinolizine-3-carboxylic acid hydrochloride

### Step 260 a. 1-N-benzyl-3-(chloromethyl)morpholine

A mixture of 1.5 g (10 mmol) of N-benzyl-ethanolamine (Aldrich Chemical Co.) and 7.8 mL of epichlorohydrin was heated at 40°C for 30 min. The reaction was cooled, and the excess epichlorohydrin was removed with a rotary evaporator. The residue was dried under vacuum, dissolved in 30 mL of conc. H₂SO₄, and the mixture heated at 150°C for 30 min. The reaction was quenched by pouring onto ice, and the pH was adjusted with NaOH to pH 13. The basic solution was extracted with toulene (3x), and the extracts were dried over Na₂SO₄, filtered, and the solvent remove under vacuum. The residue was dried under vacuum to yield 193 mg of the title product.

### Step 260 b. 1-N-benzyl-3-((N-methylamino)methyl)-morpholine

A thick-walled glass tube was charged with 8.83 g of N-benzyl-3-(chloromethyl)morpholine, from step 260a above, dissolved in 15 mL of methanol. The tube and its contents were cooled and 25 mL of anhydrous methylamine was added. The tube was sealed and heated at 100°C for 24 hours. The seal was broken, and the solvent was removed under vaccum. The residue was diluted with 100 mL of 10% Na₂CO₃, then extracted 3x with methylene chloride. The extract was dried over Na₂SO₄, filtered, and the solvent was removed on a rotary evaporator to yield 8.6 g of the title product.

### Step 260 c. 1-N-benzyl-3-((N-BOC-N-methylamino)methyl)-morpholine

To a dry flask under positive N₂ atmosphere was added 8.6 g (39 mmol) of the 1-N-benzyl-3-((N-methylamino)methyl)-morpholine, from step 260b above, in 100 mL of dry methylene chloride. The solution was cooled in an ice bath and 8.6 mL (64.3 mmol) of triethylamine and 12.7 g (58.5 mmol) of di-t-butyldicarbonate was added.. The reaction mixture was stirred at 0-5°C for 30 min, then warmed to room temperature and stirred for 72 hours. The reaction contents were diluted with 100 mL of methylene chloride, which was then washed with water and dried over Na₂SO₄. The solution was filtered, the solvent was removed on a rotary evaporator, and the residue dried under vacuum to afford 12.4 g of crude title product The product was purified by column chromatography to yield 7.4 g of the title product as a colorless oil. Anal Calc. for C11H22N2O3: C, 67.47; H, 8.81;,N, 8.74; Found: C, 67.00; H, 8.53; N, 8.66.

### Step 260d. 2-(N-BOC-N-methyl-aminomethyl)morpholine

A 1.10 g (3.43 mmol sample of 1-N-benzyl-3-((N-BOC-N-methylamino)methyl)-morpholine, from step 260c above, was dissolved in 100 mL of methanol. To this was added 500 mg of 20%Pd/C, and the mixture was stirred at room temperature under 4 atm of H₂ for 16 hours. The catalyst was removed by filtration, the solvent was removed with a rotary evaporator, and the residue was dried under vacuum to yield 794 mg of the title product as a colorless oil.

### Step 260e. 1-cyclopropyl-7-fluoro-9-methyl-8-(2-((N-methyl)aminomethyl)-4-morpholinyl)-4-oxo-4H-quinolizine-3-carboxylic acid hydrochloride

The 3-BOC-aminopyrrolidine of Step 253j above was replaced by 2-(N-BOC-N-methylaminomethyl)morpholine (from step 260d above)and the reaction product was carried forward as in Steps 253K and 253l, above, to prepare 280 mg of the title compound. mp = 208-210°C. IR (KBr): 3420, 1720, 1700, 1650 cm⁻¹. MS 390 (M-Cl)⁺. ¹H NMR (CD₃OD) ∂: 0.70 (m, 2H), 1.10 (m, 2H), 2.38 (m,1H), 2.78 (s, 3H),2.90 (s, 3H), 3.10-3.30 (m, 2H), 3.50-4.15 (m, 7H), 8.12 (s, 1H), 9.20 (d, 1H, J=14 Hz). Calc. for C₂₀H₂₅ClFN₃O₄•2H₂O: C, 52.01; H, 6.33; N, 9.10; Found: C, 51.90; H, 5.92; N, 9.09.

### Example 261

### 1-cyclopropyl-7-fluoro-9-methyl-4-oxo-8-(1,2,3,4-tetrahydro-2-isoquinolinyl)-4H-quinolizine-3-carboxylic acid

The 3-BOC-aminopyrrolidine of Step 253j above was replaced by 1,2,3,4-tetrahydroisoquinoline (Aldrich Chemical Co.), and the reaction product was carried forward as in Steps 253K and 253l, above, to prepare 315 mg of the title compound. mp = 214-215°C. IR (KBr): 3420, 1730, 1680 cm⁻¹. MS 393 (M+H)⁺. ¹H NMR (CDCl₃) ∂: 0.70 (m, 2H), 1.08 (m, 2H), 2.30 (m,1H), 2.85 (s, 3H),3.10 (dd, 2H, J=6 Hz), 3.75 (m, 2H), 4.60 (s, 2H), 7.28 (m, 4H), 8.40 (s, 1H), 9.22 (d, 1H, J=12 Hz).. Calc. for C₂₃H₂₁FN₂O₃•1.25 H₂O: C, 66.58; H, 5.71; H, 6.75; Found: C, 66.56; H, 5.26; N, 6.62.

### Example 262

### 1-cyclopropyl-7-fluoro-9-methyl-4-oxo-8-(4-amino-1-piperdinyl)-4H-quinolizine-3-carboxylic acid hydrochloride

### Step 262 a. N-benzyl-4-(N-hydroxyimino)piperidine

A 3.78 g (20 mmol) sample of N-benzyl-4-oxo-piperidine (Aldrich Chemcial Co.) was dissolved in 50 mL of methanol. To this solution was added 4.16 g (60 mmol) of hydroxylamine hydrochloride and 5.2 g NaHCO3 (62 mmol) (Dissolved in 80 mL of water and added in 5 mL portions). The mixture was then stirred at room temperature for 18 hours. The mixture was filtered, and the solvent was removed from the filtrate on a rotary evaporator to give 3.05 g of the title product. mp 127-128°C.

### Step 262 b. 1-N-benzyl-4-aminopiperidine

A 2.04 g (9.98 mmol) sample of the oxime from step 262a above was dissolved in 200 mL of methanol and reduced with 10 g of Raney nickel under 4 atmosphere of H₂ at room temperature for 4 hours. The catalyst was removed by filtration, and the solvent was removed on a rotary evaporator. The residue was dried under vacuum to yield 1.79 g of the title product. MS M/Z: 191 (M+H)⁺.

### Step 262 c. 1-N-benzyl-4-BOC-aminopiperidine

In a dry system under N₂ pressure was introduced 1.78 g of the 1-N-benzyl-4-aminopiperidine, from step 262b above, dissolved in 9 mL of dry methylene chloride. To this was added 1.6 mL (12 mmol) of triethylamine and 2.45 g (11.2 mmol) of di-t-butyldicarbonate. The reaction mixture was stirred at room temperature for 96 hours. The contents were diluted with 125 mL of methylene chloride and washed with water. The organic layer was dried over Na₂SO₄, filtered, and the solvent removed on a rotary evaporator. The residue was dried under vacuum to yield 2.45 g of the title product as an off-white solid. The crude product was purified by column chromatography on silica gel, eluting with 2% methanol in methylene chloride. Removal of the solvent gave 1.74 g of product, which was the recrystallized from ethanol, and dried under vacuum. mp. 121-122°C. Anal. calc. for C17H25N2O2: C, 70.31; H, 9.02; N, 9.65; Found: C, 70.26; H, 9.02; N, 9.55.

### Step 262d. 4-BOC-aminopiperidine

The benzyl group was removed from the product of step 262c by the procedure described for Example 260d above, to afford the title product.

### Step 262 e 1-cyclopropyl-7-fluoro-9-methyl-4-oxo-8-(4-amino-1-piperdinyl)-4H-quinolizine-3-carboxylic acid hydrochloride

The 3-BOC-aminopyrrolidine of Step 253j above was replaced by 4-(BOC-amino)-methylpiperidine, from step 262d above, and the reaction product was carried forward as in Steps 253K and 253l, above, to prepare 480 mg of the title compound. mp = 231-232°C. IR (KBr): 3420, 1700, 1610 cm⁻¹. MS 360 (M-Cl)⁺. ¹H NMR (CD₃OD) ∂: 0.70 (m, 2H), 1.08 (m, 2H), 1.85 (m,1H), 2.10 (m, 1H), 2.18 (m, 2H), 2.35 (m, 2H), 2.87 (s, 3H), 3.50 (m, 2H), 3.70 (m, 1H), 8.16 (s, 1H), 9.22 (d, 2H, J=9 Hz).. Calc. for C₁₉H₂₃ClFN₃O₃•0.75 H₂O: C, 55.75; H, 6.03; H, 10.26; Found: C, 55.70; H, 6.07; N, 10.36.

### Example 263

### 1-cyclopropyl-7-fluoro-9-methyl-4-oxo-8-(3-amino-1-piperdinyl)-4H-quinolizine-3-carboxylic acid hydrochloride

The 3-BOC-aminopyrrolidine of Step 253j above was replaced by 3-amino-piperidine hydrochloride (Aldrich Chemical Co.), which was neutralized with triethylamine, and the reaction product was carried forward as in Steps 253K and 253l, above, to prepare 250 mg of the title compound. mp = 222-223°C. IR (KBr): 3400, 1700, 1680 cm⁻¹. MS 360 (M-Cl)⁺. ¹H NMR (CD₃OD) ∂: 0.70 (m, 2H, J=6 Hz), 1.10 (m, 2H, J=6 Hz), 1.70 (m,2H), 2.05 (m, 3H), 2.30 (m, 2H), 2.40 (m, 2H), 2.87 (s, 3H), 3.90 (m, 1H), 8.18 (s, 1H), 9.20 (d, 1H, J=9 Hz).. Calc. for C₁₉H₂₃ClFN₃O₃•2 H₂O: C, 52.84; H, 6.30; H, 9.73; Found: C, 52.62; H, 6.62; N, 9.36.

### Example 264

### 1-cyclopropyl-7-fluoro-9-methyl-4-oxo-8-(4-(aminomethyl)-1-piperdinyl)-4H-quinolizine-3-carboxylic acid hydrochloride

The 3-BOC-aminopyrrolidine of Step 253j above was replaced by 4-(aminomethyl)piperidine (Aldrich Chemical Co.), and the reaction product was carried forward as in Steps 253K and 253l, above, to prepare 157 mg of the title compound. mp > 300°C. IR (KBr): 3410, 1720, 1660 cm⁻¹. MS 374 (M-Cl)⁺. ¹H NMR (CD₃OD) ∂: 0.70 (m, 2H), 1.08 (m, 2H), 1.55 (m, 1H), 1.95 (m, 2H), 2.42 (m, 2H), 2.83 (s, 3H), 2.95 (m, 3H), 3.40 (m, 2H), 3.60 (m, 2H), 8.18 (s, 1H), 9.22 (d, 1H, J=9 Hz).. Calc. for C₂₀H₂₅ClFN₃O₃•1.75 H₂O: C, 54.42; H, 6.51; H, 9.52; Found: C, 53.92; H, 6.85; N, 9.73.

### Example 265

### 1-cyclopropyl-7-fluoro-9-methyl-4-oxo-8-(5-amino-1,2,3,4-tetrahydro-2-isoquinolinyl)-4H-quinolizine-3-carboxylic acid hydrochloride

### Step 265a. 5-amino-1,2,3,4-tetrahydroisoquinoline

A 1.0 g (0.69 mmol) sample of 5-aminoisoquinoline (Aldrich Chemical Co.) was dissolved in 100 mL of methanol and reduced with 250 mg PtO₂ catalyst at 25°C under 4 atmospheres of H₂ for 8 hours. The catalyst was removed by filtration, the solvent was removed on a rotary evaporator, and the residue was dired under vacuum to give 1.01 g of crude product. The material was crystallized from i-propanol and dried under vacuum, yield 602 mg. mp = 153-154°C. MS M/Z: 149 (M+H)⁺, 166 (M+NH₄)⁺.

### Step 265b, 1-cyclopropyl-7-fluoro-9-methyl-4-oxo-8-(5-amino-1,2,3,4-tetrahydro-2-isoquinolinyl)-4H-quinolizine-3-carboxylic acid hydrochloride

The 3-BOC-aminopyrrolidine of Step 253j above was replaced by 5-amino-1,2,3,4-tetrahydroisoquinoline, prepared in step 265a above, and the reaction product was carried forward as in Steps 253K and 253l, above, to prepare 507 mg of the title compound, mp = 185-187°C. IR (KBr): 3380, 1710, 1650 cm⁻¹. MS 408 (M-Cl)⁺, 390 (M+NH₄-Cl)⁺. ¹H NMR (CD₃OD) ∂: 0.72 (m, 2H, J=6, J=3 Hz), 1.10 (m, 2H, J=3 Hz), 2.40 (m, 1H), 2.90 (s, 3H), 3.07 (dd, 2H, J=7.5 Hz), 3.90 (dd, 2H, J=7.5, J=3 Hz), 4.74 (s 2H), 7.28 (m, 2H), 7.35 (m, 1H, J=9 Hz), 8.17 (s, 1H), 9.25 (d, 1H, J=12 Hz).. Calc. for C₂₃H₂₃ClFN₃O₃•0.75 H₂O: C, 60.39; H, 5.40; H, 9.19; Found: C, 60.38; H, 5.16; N, 9.10.

### Example 266

### 1-cyclopropyl-7-fluoro-9-methyl-4-oxo-8-(4-(1-pyrrolyl)-1-piperidinyl)-4H-quinolizine-3-carboxylic acid

The 3-BOC-aminopyrrolidine of Step 253j above was replaced by 4-(1-pyrrrolyl)piperidine (prepared from N-benzyl-4-hydroxypiperidine by mesylation followed by displacing the mesyl group with pyrrole and removing the benzyl group), and the reaction product was carried forward as in Steps 253K and 253l, above, to prepare 386 mg of the title compound. mp = 268-269°C. IR (KBr): 3420, 1720, 1660 cm⁻¹. MS 427 (M+NH₄)⁺, 410 (M+H)⁺. ¹H NMR (CD₃OD) ∂: 0.70 (m, 2H), 1.03 (m, 2H), 2.14 (m, 4H), 2.40 (m, 1H), 2.90 (s, 3H), 3.60 (m, 4H), 4.18 (m, 1H), 6.08 (dd, 2H, J=3 Hz), 6.84 (dd, 2H, J=3 Hz), 8.37 (s, 1H), 9.25 (d, 1H, J=12 Hz). Calc. for C₂₃H₂₄FN₃O₃•1.25 H₂O: C, 63.95; H, 6.18; H, 9.73; Found: C, 63.60; H, 6.61; N, 9.43.

### Example 267

### 1-cyclopropyl-8-(cis-3.5-dimethyl-1-piperazinyl)-7-fluoro-9-methyl-4-oxo-4H-quinolizine-3-carboxylic acid hydrochloride

The 3-BOC-aminopyrrolidine of Step 253j above was replaced by cis-3,5-dimethylpiperazine (Aldrich Chemical Co.) and the reaction product was carried forward as in Steps 253j and 253k, above, to prepare 0.46 g of the title compound. IR (KBr): 3450, 1720, 1650, 1610 cm-¹. MS 374 (M-Cl)⁺. ¹H NMR (D₆DMSO) ∂: 0.70 (m, 2H), 1.04 (m, 2H), 1.30 (d, 6H, J=7 Hz), 2.41 (m, 1H), 2.80 (s, 3H), 3.40-3.65 (m, 6H), 8.03 (s, 1H), 9.26 (d, 1H, J=9Hz), 9.60 (br s, 1H).. Calc. for C₂₀H₂₅ClFN₃O₃•0.75 H₂O: C, 56.74; H, 6.31; N, 9.92; Found: C, 56.66; H, 6.21; N, 9.74.

### Example 268

### 1-cyclopropyl-8-(2,7-diaza-7-bicyclo[3.3.0]octyl)-7-fluoro-9-methyl-4-oxo-4H-quinolizine-3-carboxylic acid hydrochloride

The 3-BOC-aminopyrrolidine of Step 253j above was replaced by 2-BOC-2,7-diaza[3.3.0]octane (prepared according to US Patent 5,071,999) and the reaction product was carried forward as in Steps 253j, k, and l, above, to prepare 0.34 g of the title compound. IR (KBr): 3400, 1700, 1650, 1605 cm⁻¹. MS 372 (M-Cl)⁺. ¹H NMR (D₆DMSO) ∂: 0.60 (m, 2H), 0.91 (m, 1H), 2.03-2.10 (m, 3H), 2.36 (m, 1H), 2.68 (s, 3H), 3.19 (m, 1H), 3.49 (m, 2H), 4.15 (m, 1H), 5.50 (m, 1H), 7.98 (s, 1H), 9.14 (d, 1H, J=10 Hz), 9.40 (br s, 1H). Calc. for C₂₀H₂₄Cl₂FN₃O₃: C, 54.06; H, 5.44; N, 9.46; Found: C, 53.86; H, 5.48; N, 9.63.

### Example 269

### 1-cyclopropyl-8-(2,8-diaza-8-bicyclo[4.3.0]nonyl)-7-fluoro-9-methyl-4-oxo-4H-quinolizine-3-carboxylic acid hydrochloride

The 3-BOC-aminopyrrolidine of Step 253j above was replaced by 8-BOC-2,8-diaza[4.3.0]nonane (prepared according to US 5,059,597), and the reaction product was carried forward as in Steps 253K and 253l, above, to prepare 0.50 g of the title compound. IR (KBr): 3400, 1690, 1650, 1600 cm⁻¹. MS 386 (M-Cl)⁺. ¹H NMR (D₆DMSO) ∂: 0.56 (m, 1H), 0.62 (m, 1H), 0.93 (m, 1H), 1.07 (m, 1H), 1.60-1.80 (m, 4H), 2.28-2.32 (m, 2H), 2.67 (s, 3H), 2.72 (m, 1H), 2.94 (m, 1H), 3.70 (m, 2H), 3.91 (m, 1H), 4.03 (m, 1H), 4.35 (m, 1H), 7.93 (s, 1H), 8.90 (br s, 1H), 9.10 (d, 1H, J=11 Hz), 9.48 (br s, 1H), 13.85 (br s, 1H). Calc. for C₂₁H₂₆Cl₂FN₃O₃: C, 55.03; H, 5.72; N, 9.17; Found: C, 54.75; H, 5.82; N, 9.38.

### Example 270

### 1-cyclopropyl-7-fluoro-9-methyl-4-oxo-8-(3(S)-(1-pyrrolyl)-1-pyrrolidinyl)-4H-quinolizine-3-carboxylic acid

A mixture of 25 mg 8-(3(S)-aminopyrrolidinyl)-1-cyclopropyl-7-fluoro-9-methyl-4-oxo-4H-quinolizine-3-carboxylic acid hydrochloride (from Example 257) and 40 mg of sodium acetate in 0.7 mL of ethyl acetate was heated to 100°C. To this solution was added 0.009 mL of dimethoxytetrahydrofuran dropwise, and the reaction was stirred at 110°C for 5 min, then quenched by addition of water. The mixture was extracted twice with methylene chloride, and the extract was washed with water, dried over MgSO₄ and concentrated. The residue was purified by preparative TLC, eluting with 100:10 chloroform:methanol, to give 13.6 mg of the title product as a yellow solid after removal of the solvent.. MS 395 (M-Cl)⁺. ¹H NMR (CDCl₃) ∂: 0.67 (m, 2H), 1.00 (m, 2H), 2.20 (m, 1H), 2.46 (m, 1H), 2.56 (m, 1H), 2.66 (s, 3H), 3.89 (m, 1H), 3.99 (m, 2H0, 4.15 (m, 1H), 4.86 (m, 1H), 6.23 (t, 2H, J=2 Hz), 6.79 (t, 2H, J=2 hz), 8.32 (s, 1H), 9.15 (d, 1H, J=10 Hz), 13.83 (br, 1H).

### Example 271

### 1-cyclopropyl-7-fluoro-8-(3-hydroxy-1-pyrrolidinyl)-9-methyl-4-oxo-4H-quinolizine-3-carboxylic acid hydrochloride

The 3-BOC-aminopyrrolidine of Step 253j above was replaced by 3-hydroxypyrrolidine (Aldrich Chemical Co.), and the reaction product was carried forward as in Steps 253j and 253k above, to prepare 0.15 g of the title compound. IR (KBr): 3425, 1690, 1650, 1600 cm⁻¹. MS 346 (M+H)⁺. ¹H NMR (DMSO-d₆) ∂: 0.59 (m, 2H), 0.93 (m, 1H), 1.03 (m, 1H), 1.96-2.01 (m, 3H), 2.29 (m, 1H), 2.49 (s, 3H), 3.43 (m, 1H), 3.69 (m, 1H), 4.01 (m, 2H), 4.42 (m, 1H), 5.15 (d, 1H, J=3 Hz), 7.89 (s, 1H), 9.05 (d, 1H, J=11 Hz), 13.86 (br s, 1H). Calc. for C₁₈H₁₉FN₂O₄: C, 62.42; H, 5.53; N, 8.09; Found: C, 62.20; H, 5.55; N, 8.09.

### Example 272

### 1-cyclopropyl-7-fluoro-8-(4-methyl-1-piperazinyl)-9-methyl-4-oxo-4H-quinolizine-3-carboxylic acid hydrochloride

The 3-BOC-aminopyrrolidine of Step 253j above was replaced by 1-methylpiperazine (Aldrich Chemical Co.), and the reaction product was carried forward as in Steps 253j and 253k, above, to prepare 0.15 g of the title compound. mp = 210-216°C (dec). MS 360 (M-Cl)⁺. ¹H NMR (CDCl₃) ∂: 0.70 (m, 2H), 1.02 (m, 2H), 2.28 (m, 1H), 2.40 (s, 3H), 2.60 (m, 4H), 2.79 (s, 3H), 3.48 (m, 4H), 8.37 (s, 1H), 9.21 (d, 1H, J=9 Hz).

### Example 273

### 1-cyclopropyl-9-chloro-7-fluoro-8-(3-amino-1-pyrrolidinyl)-4-oxo-4H-quinolizine-3-carboxylic acid trifluoroacetic acid salt

The 4-*t*-butoxy-2,3,6-trifluoro-5-methylpyridine of Step 253d above was replaced by 4-*t*-butoxy-3-chloro-2,5,6-trifluoropyridine (from step 253a above), and the methanol solvent was replace by benzene, and the reaction product was carried forward as in Steps 253d-l above, and the 4N HCl in dioxane of Step 253l was replaced with trifluoroacetic acid. to prepare 0.13 g of the title compound. MS 366 (M-CF₃CO₂)⁺. ¹H NMR (D₆-DMSO) ∂: 0.58 (m, 2H), 0.97 (m, 2H), 2.11 (m, 1H), 2.31 (m, 1H), 2.44 (m, 1H), 3.83 (m, 1H), 3.97 (m, 2H), 4.10 (m, 1H), 4.20 (m, 1H), 8.09 (s, 1H), 8.09 (br, 3H), 9.18 (d, 1H, J=11 Hz). Calc. for C₁₇H₁₇ClFN₃O₃:•CF₃COOH•0.5 H₂O: C, 46.69; H, 3.92; N, 8.60; Found: C, 46.62; H, 3.64; N, 8.45.

### Example 274

### 8-(3-amino-1-pyrrolidinyl)-1-cyclopropyl-7.9-difluoro-4-oxo-4H-quinolizine-3-carboxylic acid hydrochloride

### Step 274a. 4-t-butoxy-2,3,5,6-tetrafluoropyridine

A 158.5 g (0.938 mmol) sample of pentafluoropyridine (Aldrich Chemical Co.) was dissolved in 600 mL of THF and cooled to -78°C. To this was added 88.29 g (0.919 mmol) of sodium-t-butoxide in 800 mL of THF over a 30 min period, with stirring and while maintaining the temperature at -78°C. The mixture was stirred for another 30 min at this temperature, then the temperature of the bath was raised to -20°C, and the reaction was stirred at this temperature for 64 hours. The reaction mixture was removed from the cold bath and diluted with 1.5 L of ether, then filtered through a diatomaceous earth filter aid. The solvent was removed under vacuum to leave a yellow oil. The oil was purified by vacuum distillation to afford 141.34 g of the title product.

### Step274 b. 4-t-butoxy-2,3,5-trifluoropyridine

A 20.0 g (0.089 mmol) sample of the product from step 274a above was dissolved in 100 mL of absolute ethanol, and 26.08 mL (0.538 mol) of hydrazine monohydrate was added. The reaction was stirred for 1 hour at room temperature and 1 hour at reflux. The solvent was removed under vacuum. The residue was dissolved in ether and washed with water and brine. The organic phase was dried over MgSO₄, and the solvent was removed under vacuum to yield a yellow solid. This material was dissolved in 120 mL of toluene, 60 mL of 20% sodium hydroxide was added, and air was bubbled through the stirred solution for 18 hours. To the reaction was added 100 mL of ether, and the organic phase was separated, washed with water and brine, and dried over MgSO₄. Removal of the solvent, and purification of the residue with flash chromatography on silica gel, eluting with 1:16 ethyl acetate:hexane, gave 14.6 g of the title product as a reddish liquid.

### Step 274c. 8-(3-amino-1-pyrrolidinyl)-1-cyclopropyl-7,9-difluoro-4-oxo-4H-quinolizine-3-carboxylic acid hydrochloride

Replacing the 4-t-butoxy-2,5-difluoro-3-methylpyridine of step 253e with the 4-t-butoxy-2,3,5-trifluoropyridine from step 274b above, and carrying the product forward according to the procedures of Steps 253e-1, 76 mg of the title compound was prepared. MS M/Z: 350 (M-Cl)⁺. ¹H NMR (D₆-DMSO) ∂: 0.65 (m, 2H), 0.90 (m, 2H), 2.15-2.30 (m, 3H, 3.95-4.00 (m, 3H), 4.18 (m, 2H), 7.81 (s, 1H), 8.46 (br, 3H), 9.17 (d, 1H, J=9 Hz).

### Example 275

### 8-(3-amino-1-pyrrolidinyl)-1-cyclopropyl-7-fluoro-9-methoxy-4-oxo-4H-quinolizine-3-carboxylic acid hydrochloride

### Step 275a 4-t-butoxy-2,3,6-trifluoro-5-hydroxypyridine

A 11.16 g (54.39 mmol) sample of 4-t-butoxy-2,3,6-trifluoropyridine, from Example 253 step b above, was dissoved in 50 mL of THF, and the solution was cooled to -78°C. To this solution was added LDA (65.6 mmol) with stirring for 30 min, during which a solid preciptated. To this mixture was added 7.5 mL of trimethoxyborane, with stirring for 25 min at -78°C. To this mixture was added 10 mL of acetic acid, and the mixture was stirred and allowed to warm to room temperature. Next was added 100 mL of 30% hydrogen peroxide and 100 mL of 2N sodium hydroxide while cooling in an ice bath. The mixture was then stirred at room temperature for 16 hours, and quenched with saturated NH₄Cl solution. The mixture was extracted with ether, and the extract was washed with brine and dried over MgSO₄. The solvent was removed under vacuum, and the residue was purified by flash chromatography on silica gel, eluting with 1:8 ethyl acetate:hexane. Removal of the solvent gave 9.769 g of the title product as a colorless liquid.

### Step 275b 4-t-butoxy-2,3,6-trifluoro-5-methoxypyridine

To a solution of 237 mg (1.07 mmol) of 4-t-butoxy-2,3,6-trifluoro-5-hydroxypyridine, from step 275a above, in 3 mL of anhydrous THF was added 335 mg (1.277 mmol)of triphenyl phosphine and 0.060 mL (1.48 mmol) of methanol. To this solution was added 0.200 mL (1.270 mmol) of DEAD dropwise at room temperature. The reaction was complete in 10 min, so the solvents were removed under vacuum and the residue was purified by flash chromatography on silica gel, eluting with 1:16 ethyl acetate:hexane to give 215.6 mg of the title product as a colorless liquid after removal of the solvent.

### Step c. 8-(3-amino-1-pyrrolidinyl)-1-cyclopropyl-7-fluoro-9-methoxy-4-oxo-4H-quinolizine-3-carboxylic acid hydrochloride

Replacing the 4-t-butoxy-2,3,6-trifluoro-5-methylpyridine of Example 253 step c with the 4-t-butoxy-2,3,6-trifluoro-5-methoxypyridine of step 275b above and carrying the product forward according to the procedures of Steps 253d-l, 120 mg of the title compound was prepared. MS M/Z: 362 (M-Cl)⁺. IR (KBr): 3440, 1799, 1650, 1610 cm⁻¹. ¹H NMR (D₆-DMSO) ∂: 0.62 (m, 2H), 0.91 (m, 2H), 2.12 (m, 1H), 2.29 (m, 1H), 2.39 (m, 1H), 3.62 (s, 3H), 3.81 (m, 1H), 3.94 (m, 2H), 4.06 (m, 2H), 7.79 (s, 1H), 8.30 (br, 3H), 9.13 (d, 1H, J=10 Hz), 13.79 (br, 1H). Calc. for C₁₈H₂₀FN₃O₄•2HCl•0.5H₂O: C, 48.77; H, 5.23; N, 9.48; Found: C, 48.65; H, 5.19; N, 9.56.

### Example 276

### 1-cyclopropyl-7-fluoro-9-methyl-8-(3(S)-methylamino-1-pyrrolidinyl)-4-oxo-4H-quinolizine-3-carboxylic acid hydrochloride

### Step 276a 1-N-benzyl-3(S)-(BOC-amino)-pyrrolidine

A 4.2 g sample of (3S)-3-BOC-aminopyrrolidine (TCI America) and 4.7 mL of triethylamine were dissolved in 75 mL of methylene chloride at room temperature. To this solution was added 2.95 mL of benzyl bromide dropwise, and the reaction was heated at reflux for 6 hours. After cooling, the solution was washed with water, and the solvent was dried and evaporated to give 5.10 g of the title product as a white solid.

### Step 276b. 1-N-benzyl-3(S)-(methylamino)-pyrrolidine

The 5.10 g sample of 1-N-benzyl-3(S)-(BOC-amino)-pyrrolidine, from step 276a above, was dissolved in 25 mL of THF, and 55.6 g of LiAlH4 (1.0 M in THF) was added. The mixture was stirred and heated at reflux for 4 hours. The reaction was quenched with water, and the mixture was extracted with methylene chloride. The solvent was washed with water, dried over MgSO₄, and removed on a rotary evaporator to yield 2.43 g of the title product.

### Step 276c. 1-N-benzyl-3(S)-(N-BOC-N-methylamino)-pyrrolidine

A 2.43 g sample of 1-N-benzyl-3(S)-(methylamino)-pyrrolidine, from step 276b above, was dissolved in 100 mL of a 4:1 methanol:water mixture, and 3.34 g of di-t-butyl dicarbonate was added in portions. The reaction was stirred at room temperature for 6 hours. The methanol was removed under vacuum, and the aqueous residue was extracted with methylene chloride. The solvent was washed with water, dried over MgSO₄ and removed under vacuum. The residue was purified by chromatography over silica gel, eluting wtih 100:5:0.5 methylene chloride:methanol:NH₄OH to give 3.23 g of the title product.

### Step 276d. 3(S)-(N-BOC-N-methylamino)-pyrrolidine

The product from step 276c was treated according to the procedure of Example 171 step 5 to remove the benzy protecting group and afford 2.24 g of the title product as a white solid.

### Step 276e. 1-cyclopropyl-7-fluoro-9-methyl-8-(3(S)-methylamino-1-pyrrolidinyl)-4-oxo-4H-quinolizine-3-carboxylic acid hydrochloride

Following the procedure of Example 253 step j, replacing the 3-BOC-aminopyrrolidine of that example with the 3(S)-(N-BOC-N-methylamino)-pyrrolidine from step 276d above, and carrying the reaction product forward according to the procedures of Example 253 steps k and l, a 452 mg sample of the title product was obtained. MS: 360 (M-Cl)⁺. IR (KBr): 3450, 1710, 1650, 1610 cm⁻¹. ¹H NMR (d6-DMSO): 0.62 (m, 2H), 1.00 (m, 2H), 2.26 (m, 1H), 2.33 (m, 3H), 2.65 (s, 6H), 3.75 (m, 1H), 3.90 (m, 2H), 4.05 (m, 2H), 7.94 (s, 1H), 9.12 1H, J=10 Hz), 9.18 ( br s, 2H), 13.86 (br s, 1H). Anal. Calc. for C₁₉H₂₂FN₃O₃•HCl•H₂O: C, 55.14; H, 6.09; N, 10.15; Found: C, 55.29; H, 5.99; N, 10.18.

### Example 277

### 1-cyclopropyl-7-fluoro-9-methyl-8-(3(R)-amino-1-pyrrolidinyl)-4-oxo-4H-quinolizine-3-carboxylic acid hydrochloride

### Step 277a 1-N-benzyl-3(R)-(BOC-amino)-pyrrolidine

Following the procedure of Example 276 step a, replacing the (3S)-3-BOC-aminopyrrolidine of step 276a with (3R)-3-BOC-aminopyrrolidine (TCI America), the title compound was prepared.

### Step 277b. 3(R)-(BOC-amino)pyrrolidine

The benzyl group was removed from the product of step 277a by the procedure of step 276d above, to give the title product.

### Step 277c. 1-cyclopropyl-7-fluoro-9-methyl-8-(3(R)-amino-1-pyrrolidinyl)-4-oxo-4H-quinolizine-3-carboxylic acid hydrochloride

Following the procedure of Example 253 step j, replacing the 3-(BOC-amino)pyrrolidine of that example with the 3(R)-(BOC-amino)-pyrrolidine from step 277b above, and carrying the reaction product froward according to the procedures of Example 253 steps k and l, a 452 mg sample of the title product was obtained. MS: 346 (M-Cl)⁺. IR (KBr): 3440, 1700, 1650, 1610 cm⁻¹. ¹H NMR (d6-DMSO): 0.59 (m, 2H), 1.00 (m, 2H), 2.15 (m, 1H), 2.31 (m, 2H), 2.63 (s, 3H), 3.76 (m, 2H), 4.00-4.07 (m, 3H), 8.40 (br, 3H), 9.10 (d, 1H, J=11 Hz). C₁₈H₂₀FN₃O₃•HCl•H₂O: C, 54.07; H, 5.80; N, 10.51; Found: C, 54.19; H, 5.65; N, 10.37.

### Examples 278-285 are missing due to their deletion

### Examples 286-296

Following the procedures of Steps 253j, 253k and 253l (if required), above, replacing the 3-BOC-aminopyrrolidine of Step 253j with the reagent shown, the compounds of Examples 286-296 are prepared as shown in Table 1, below.

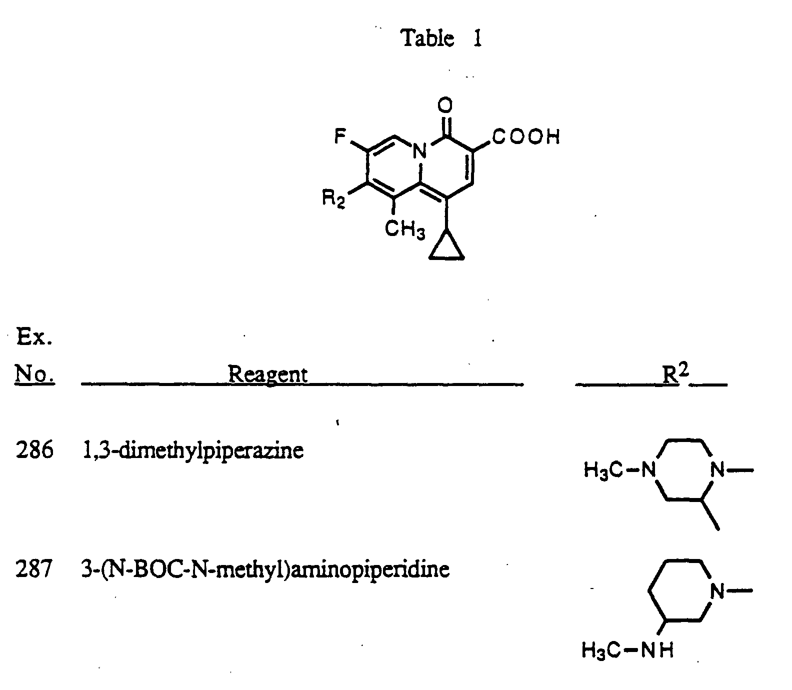

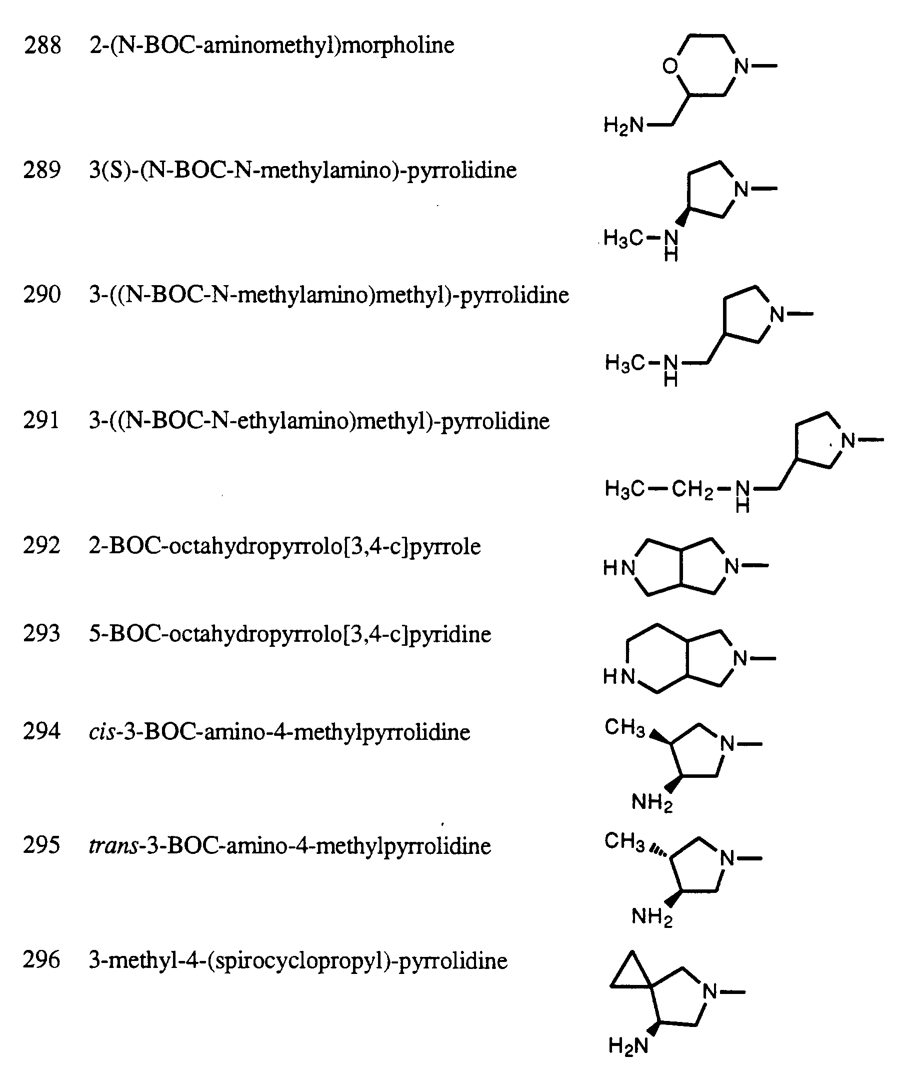

### Example 297

### 8-(2S,4S-4-amino-2-methylpyrrolidinyl)-1-cyclopropyl-7-fluoro-9-(fluoro)methyl-4-oxo-4H-quinolizine-3-carboxylic acid hydrochloride

Following the procedure of Example 253c, reacting the product of Step 253b with LDA at -78°C, then adding formaldehyde and stirring until the reaction is complete, followed by reaction of the newly formed intermediate with diethylaminosulfur trifluoride (DAST) in methylene chloride to form the intermediate product 4-t-butoxy-2,3,6-trifluoro-5-(fluoro)methylpyridine, and carrying this product through the remaining steps as in Example 253d-l, the title compound is prepared.

### Example 298

### 8-(3-Dimethylaminopyrrolidinyl)-1-cyclopropyl-7-fluoro-9-methyl-4-oxo-4H-quinolizine-3-carboxylic acid, acetic acid salt

A 81 mg sample of 8-chloro-1-cyclopropyl-7-fluoro-9-methyl-4-oxo-4H-quinolizine-3-carboxylic acid ethyl ester, from Example 253i above, was dissolved in 2.5 mL of dry pyridine under a nitrogen atmosphere. To this solution was added a solution of 114 g of 3-(dimethylamino)pyrrolidine in 2.5 mL of pyridine, and the reaction mixture was heated at 60°C for 39 hours. The pyridine was removed under vacuum, and the residue was stirred with 1N NaOH in THF/water for at 60°C for 6 hours. The solution was made acidic with acetic acid, and the product was extracted with chloroform. After drying over MgSO₄, the solvent was removed, and the residue was purified by chromatography on silica gel, eluting with 100:40:20:8 chloroform: methanol: acetic acid:water to give the title product mp 165-170°C (dec.). MS 374 (M+H)⁺; ¹H NMR (D₆-DMSO) ∂: 0.53 (m, 2H), 0.82-1.08 (m, 2H), 1.75 (s, 3H), 2.22 (s, 6H), 2.08-2.33 (m, 2H), 2.74 (m, 2H), 3.44-3.94 (m, 5H), 8.01 (br s, 1H), 8.90 (br s, 1H).

### Example 299

### (3R)-8-(3-Dimethylaminopyrrolidinyl)-1-cyclopropyl-7-fluoro-9-methyl-4-oxo-4H-quinolizine-3-carboxylic acid hydrochloride

Following the procedure of Example 298, replacing the 3-(dimethylamino)pyrrolidine with (3R)-3-(dimethylamino)pyrrolidine, the title compound was prepared. mp 146-148°C. MS 374 (M+H)⁺; ¹H NMR (D₆-DMSO) ∂: 0.64 (m, 2H), 1.02 (m, 2H), 2.23-2.43 (m, 3H), 2.66 (s, 3H), 2.83 (s, 6H), 3.78-4.17 (m, 5H), 7.95 (s, 1H), 9.12 (d, 1H, J=11 Hz), 11.14 (br s, 1H), 13.83 (br s, 1H).

### Example 300

### (3R,1S)-8-(3-(1-Aminoethyl)pyrrolidinyl)-1-cyclopropyl-7-fluoro-9-methyl-4-oxo-4H-quinolizine-3-carboxylic acid hydrochloride

A sample of 8-chloro-1-cyclopropyl-7-fluoro-9-methyl-4-oxo-4H-quinolizine-3-carboxylic acid ethyl ester, from Example 253i above, was dissolved in anhydrous acetonitrile, reacted with (3R,1S)-3-(1-(t-butoxycarbonylamino)ethyl)pyrrolidine (prepared as described by Schroeder *et al*., *J*. *Heterocyclic Chem.,* 29: 1481-1498 (**1992**)), and carried forward as described in Example 253k-l to give the title product. mp 250-255°C (dec.). MS 374 (M+H)⁺; ¹H NMR (D₆-DMSO) ∂: 0.59 (m, 2H), 1.00 (m, 2H), 1.29 (d, 3H, J=6 Hz), 1.77 (m, 1H), 2.13 (m, 1H), 2.29 (m, 1H), 2.41 (m, 1H), 2.64 (s, 3H), 3.57 (s, 1H), 3.76 (m, 3H), 3.94 (m, 1H), 7.91 (s, 1H), 8.17 (brs, 3H), 9.07 (d, 1H, J=11 Hz), 13.83 (brs, 1H).

### Example 301

### (3S,1R)-8-(3-(1-Aminoethyl)pyrrolidinyl)-1-cyclopropyl-7-fluoro-9-methyl-4-oxo-4H-quinolizine-3-carboxylic acid hydrochloride

A 0.44 g sample of 8-chloro-1-cyclopropyl-7-fluoro-9-methyl-4-oxo-4H-quinolizine-3-carboxylic acid ethyl ester, from Example 253i above, and 1.51 g of NaHCO₃ were dissolved in 40 mL of anhydrous acetonitrile, reacted with (3S,1R)-3-(1-(t-butoxycarbonylamino)ethyl)pyrrolidine (1.06 g, prepared as described by Schroeder *et al., J. Heterocyclic Chem.,* 29: 1481-1498 (**1992**)), and carried forward as described in Example 253k-l to give the title product. mp 235-240°C (dec.). MS 374 (M+H)⁺; ¹H NMR (D₆-DMSO) ∂: 0.59 (m, 2H), 1.00 (m, 2H), 1.29 (d, 3H, J=6 Hz), 1.76 (m, 1H), 2.13 (m, 1H), 2.28 (m, 1H), 2.41 (m, 1H), 2.63 (s, 3H), 3.30 (m, 1H), 3.74 (m, 3H), 3.94 (m, 1H), 7.90 (s, 1H), 8.16 (br s, 3H), 9.07 (d, 1H, J=11 Hz).

### Example 302

### (3R,1R)-8-(3-(1-Aminoethyl)pyrrolidinyl)-1-cyclopropyl-7-fluoro-9-methyl-4-oxo-4H-quinolizine-3-carboxylic acid hydrochloride

A 0.35 g sample of 8-chloro-1-cyclopropyl-7-fluoro-9-methyl-4-oxo-4H-quinolizine-3-carboxylic acid ethyl ester, from Example 253i above, and 0.73 g of sodium bicarbonate were dissolved in 24 mL of anhydrous acetonitrile, reacted with (3R,1R)-3-(1-(t-butoxycarbonylamino)ethyl)-pyrrolidine (0.51 g, prepared as described by Schroeder *et al., J. Heterocyclic Chem.,* 29: 1481-1498 (**1992**)), and carried forward as described in Example 253k-l to give the title product. mp 220-222°C. MS 374 (M+H)⁺; ¹H NMR (D₆-DMSO) ∂: 0.61 (m, 2H), 0.94 (m, 1H), 1.07 (m, 1H), 1.28 (d, 3H, J=6 Hz), 1.82 (m, 1H), 2.27 (m, 2H), 2.46 (m, 1H), 2.62 (s, 3H), 3.57 (s, 1H), 3.92 (m, 1H), 7.90 (s, 1H), 8.17 (br s, 3H), 9.07 (d, 1H, J=11 Hz), 13.84 (brs, 1H).

### Example 303

### 1-cyclopropyl-8-(R,S)-3-fluoropyrrolidine)-7-fluoro-9-methyl-4-oxo-4H-quinolizine-3-carboxylic acid

### 303a. N-CBZ-(R,S)-3-hydroxypyrrolidine

(R,S)-3-hydroxypyrrolidine (1.0 g, 0.011 mmol) was dissolved in ethyl acetate (50 mL) and to this solution at room temperature was added N-(benzyloxycarbonyl)succinimide (2.86 g, 0.011 mmol). The mixture was stirred overnight then partitioned between dilute aqueous HCl and ethyl acetate. The aqueous phase was extracted with ethyl acetate (2x). The organics were combined, dried (MgSO₄) and concentrated *in vacuo*. The crude product was purified by flash chromatography on silica gel (ethyl acetate-hexane) to give the desired compound as a clear oil, 2.1 g, 83%. MS (DCI/NH₃) m/z: 222 (M+H)⁺, 239 (M+NH₄)^{+ 1}H NMR (CDCl₃) δ: 1.85-2.10 (m, 2H), 3.37-3.65 (m, 4H), 4.44-4.55 (m, 1H), 5.15 (s,2H), 7.28-7.45 (m, 5H).

### 303b. N-CBZ-(R,S)-3-fluoropyrrolidine

The compound from step 303a above (32.01gm, 9.10mmole) was dissolved in anhydrous CH₂Cl₂ (40 mL) and cooled under nitrogen to -78°C. To the cold solution was added in one portion via syringe diethylaminosulfur trifluoride (DAST) (1.32 mL, 10.0 mmol), and the resulting solution was stirred overnight at room temperature. The product was isolated by concentrating the reaction mixture *in vacuo* with flash chromatography of the residue on silica gel(ethyl acetate-hexane) to give a clear oil, 1.53gm, 75%. MS (DCI/NH₃) m/z: 224 (M+H)⁺, 241 (M+NH₄)^{+ 1}H NMR (CDCl₃) ∂: 1.83-2.15 (m,1H), 2.16-2.35 (m, 1H), 3.43-3.90 ( m, 4H), 5.21-5.24 (m, 2.5H) 5.28-5.36, (m, 0.5H), 7.28-7.5 (m,5H).

### 303c. (R,S)-3-fluoropyrrolidine hydrochloride

The compound from step 303b above (1.53 g, 6.85 mmol) was dissolved in methanol (50 mL) to which was added 5% Pd/BaSO₄ (0.5g). The mixture was vacuum degassed (3x) then exposed to a low pressure atmosphere of hydrogen (balloon) at room temperature for 4 hours. The reaction was terminated by vacuum filtration to remove catalyst. The filtrate was cooled in an ice bath, then HCl gas was bubbled into the cold solution for one minute. The resulting solution was concentrated *in vacuo*, and the residue was triturated with ethyl acetate-ether. The solid was collected by vacuum filtration to give 0.659 g, 76% , of the hydrochloride as an off white solid. ¹HNMR (CD₃OD) d: 2.1-2.46 (m, 2H), 3.33-3.65 (m, 4H), 5.43 (db.t., 1H, J_{F,H}=51Hz).

### 303d. 1-cyclopropyl-8-((R,S)-3-fluoropyrrolidine)-7-fluoro-9-methyl-4-oxo-4H-quinolizine-3-carboxylic acid

The N-boc-3-aminopyrrolidine of Example 253j above was replaced by the (R,S)-3-fluoro pyrrolidine hydrochloride of step 303c above (0.66 g, 5.24 mmol), and the reaction product was carried forward as previously described to give 0.326 g (65%) of the title compound as a bright yellow solid. mp 227.5-230°C (dec.). MS (DCI/NH₃) m/z: 349 (M+H)⁺. ¹H NMR( CDCl₃) d: 0.58-0.78 (cm, 2H), 0.85-0.98, (cm,1H) 1.04-1.16 (cm, 1H), 2.03-2.53 (cm, 3H), 2.67 (s,3H), 3.60-3.86 (cm, 2H), 4.05-4.26 (cm, 2H), 5.43 (db.t, 1H, J_{F,H}=52Hz), 7.26 (s,1H), 8.26 (s, 1H), 8.26 (s,1H), 9.08 (d, 1H, J=10.5Hz), 13.8 (br.s., 1H). Calc. for C₁₈H₁₈N₂O₃F₂ : %C, 62.05; H, 5.22; N, 8.04. Found: %C, 62.06; H, 5.22; N, 7.86.

### Example 304

### 8-(4-(1-piperidyl)-1-piperidyl)-1-cyclopropyl-7-fluoro-9-methyl-4-oxo-4H-quinolizine-3-carboxylic acid

A 70 mg sample of 8-chloro-1-cyclopropyl-7-fluoro-9-methyl-4-oxo-4H-quinolizine-3-carboxylic acid ethyl ester, from Example 253i above, was dissolved in 2 mL of anhydrous acetonitrile, reacted with 4-(1-piperidyl)piperidine (70 mg, 0.4 mmol, Aldrich Chem. Co.), and carried forward as described in Example 253j-k to give the title product. MS 428 (M+H)⁺; ¹H NMR (CDCl₃) ∂: 0.69 (m, 2H), 1.02 (m, 2H), 1.18 (m, 4H), 2.27 (n, 1H), 2.78 (s, 3H), 2.72 (m, 1H), 3.35 (m, 3H), 3.55 (m, 1H), 3.75 (m, 1H), 8.36 (s, 1H), 9.20 (d, 1H). Anal. Calcd for C₂₄H₃₀N₃O₃F•1.5 H₂O: C, 63.42; H, 7.32; N, 9.24; Found: C, 62.99; H, 7.04; N, 8.78.

### Example 305

### 8-(4-(1-piperidyl)-1-piperidyl)-1-cyclopropyl-7-fluoro-9-methyl-4-oxo-4H-quinolizine-3-carboxylic acid trifluoroacetic acid salt

A 100 mg sample of 8-chloro-1-cyclopropyl-7-fluoro-9-methyl-4-oxo-4H-quinolizine-3-carboxylic acid ethyl ester, from Example 253i above, was dissolved in 3 mL of anhydrous acetonitrile, reacted with 4-(4-piperidyl)-piperidine (0.24 g, 0.93 mmol, obtained from Aldrich Chem. Co.), carried forward as described in Example 253j-k and converted to the TFA salt by the procedure of Example 162 to give the title product. MS 428 (M+H)⁺; ¹H NMR (CDCl₃) ∂: 0.69 (m, 2H), 1.03 (m, 2H), 1.70 (m, 2H), 1.87 (m, 2H), 1.98 (m, 2H), 2.14 (m, 2H), 2.27 (m, 1H), 2.77 (s, 3H), 2.91 (m, 2H), 3.33 (m, 2H), 3.54 (m, 4H), 8.37 (s, 1H), 9.21 (d, 1H). Anal. Calcd for C₂₄H₃₀N₃O₅F₄•1.5 H₂O: C, 54.93; H, 6.03; N, 7.39; Found: C, 54.97; H, 5.39; N, 7.24.

### Example 306

### 8-(4-(2-pyridyl)-1-piperazinyl)-1-cyclopropyl-7-fluoro-9-methyl-4-oxo-4H-quinolizine-3-carboxylic acid

A 60 mg sample of 8-chloro-1-cyclopropyl-7-fluoro-9-methyl-4-oxo-4H-quinolizine-3-carboxylic acid ethyl ester, from Example 253i above, was dissolved in 2 mL of anhydrous acetonitrile, reacted with 4-(2-pyridyl)piperazine (63.5 mg, 0.39 mmol, Aldrich Chem. Co.), and carried forward as described in Example 253j-k to give the title product. MS 423 (M+H)⁺; ¹H NMR (CDCl₃) ∂: 0.71 (m, 2H), 1.05 (m, 2H), 2.30 (m, 1H), 2.86 (s, 3H), 3.59 (m, 4H), 3.78 (m, 4H), 6.76 (m, 2H), 7.57 (m, 1H), 8.25 (m, 1H), 8.40 (s, 1H), 8.25 (d, 1H), 13,83 (bs, 1H). Anal. Calcd for C₂₃H₂₃N₄O₃F•1.5 H₂O: C, 61.46; H, 5.83; N, 12.46; Found: C, 61.76; H, 5.54; N, 11.64.

### Example 307

### 8-((2-amino)thioethoxy)-1-cyclopropyl-7-fluoro-9-methyl-4-oxo-4H-quinolizine-3-carboxylic acid trifluoroacetic acid salt

A 50 mg sample of 8-chloro-1-cyclopropyl-7-fluoro-9-methyl-4-oxo-4H-quinolizine-3-carboxylic acid ethyl ester, from Example 253i above, was dissolved in 2 mL of anhydrous acetonitrile, reacted with N-BOC-2-aminothiol (57.4 mg, 0.32 mmol, prepared by standard procedures from the unprotected compound obtained from Aldrich Chem. Co.), carried forward as described in Example 253j-k, deprotected as in step 253l, and converted to the TFA salt by the procedure of Example 162 to give the title product. MS 337 (M+H)⁺; ¹H NMR (d₆-DMSO) ∂: 0.74 (m, 2H), 1.08 (m, 2H), 3.04 (t, 2H), 3.16 (s, 3H), 3.33 (t, 2H), 8.27 (s, 1H), 9.32 (d, 1H), 13.8 (br, 1H).

### Example 308

### (3R,1S)-8-(3-(1-amino)propyl)pyrrolidinyl)-1-cyclopropyl-7-fluoro-9-methyl-4-oxo-4H-quinolizine-3-carboxylic acid hydrochloride

A 147 mg sample of 8-chloro-1-cyclopropyl-7-fluoro-9-methyl-4-oxo-4H-quinolizine-3-carboxylic acid ethyl ester, from Example 253i above, was dissolved in 3 mL of anhydrous acetonitrile, reacted with (3R,1S)-3-(1-BOC-amino)propyl)pyrrolidine (326 mg, 1.13 mmol, prepared as described by Hayakawa *et al*., U.S. Patent 5,098,912, issued Mar. 24, 1992, using modifications for chiral products described by Plummer *et al.. Tetr. Lett.* 34:7529-32 (**1993**)), and carried forward as described in Example 253j-l to give the title product. MS (high resolution) found: 388.2039; calc: 388.2036 (M+H)⁺; ¹H NMR (D₆-DMSO) ∂: 0.60 (m, 2H), 1.00 (t, 3H), 1.01 (m, 2H), 1.63 (m, 2H), 2.13 (m, 1H), 2.29 (m, 2H), 3.73 (m, 3H), 3.95 (m, 1H), 7.96 (s, 1H), 8.00 (b m, 2H), 9.08 (d, 1H), 13.83 (b s, 1H). Anal. Calcd for C₂₁H₂₇N₃O₃FCl•0.5 H₂O: C, 58.13; H, 6.74; N, 9.68; Found: C, 58.24; H, 6.51; N, 9.71.

### Example 309

### (3R,1S)-8-(3-(1-(N-methyl)amino)propyl)pyrrolidinyl)-1-cyclopropyl-7-fluoro-9-methyl-4-oxo-4H-quinolizine-3-carboxylic acid hydrochloride

A 492.9 mg sample of 8-chloro-1-cyclopropyl-7-fluoro-9-methyl-4-oxo-4H-quinolizine-3-carboxylic acid ethyl ester, from Example 253i above, was dissolved in 8 mL of anhydrous acetonitrile, reacted with (3R,1S)-3-(1-(N-methyl)amino)propyl)pyrrolidine (501 mg, 3.53 mmol, prepared as described by Hayakawa *et al.*, U.S. Patent 5,098,912, issued Mar. 24, 1992, using modifications for chiral products described by Plummer *et al. Tetr. Lett.* 34:7529-32 (**1993**)), and carried forward as described in Example 253 j-l, omitting the deprotecting step, to give the title product. MS 402 (M+H)⁺; ¹H NMR (D₆-DMSO) ∂: 0.61 (m, 2H), 0.98 (t, 3H), 1.00 (m, 2H), 1.75 (m, 5H), 2.15 (m, 1H), 2.30 (m, 1H), 2.59 (s, 3H), 2.63 (s, 3H), 3.66 (m, 1H), 3.77 (m, 2H), 3.95 (m, 1H), 7.90 (s, 1H), 8.60 (bs, 2H), 9.08 (d, 1H), 13.83 (bs, 1H) Anal. Calcd for C₂₂H₂₉N₃O₃FCl• H₂O: C, 57.95; H, 6.85; N, 9.22; Found: C, 58.24; H, 6.58; N, 9.30.

### Example 310

### (3R,1S)-8-(3-(1-amino-3-methylpropyl)pyrrolidinyl)-1-cyclopropyl-7-fluoro-9-methyl-4-oxo-4H-quinolizine-3-carboxylic acid hydrochloride

A 171 mg sample of 8-chloro-1-cyclopropyl-7-fluoro-9-methyl-4-oxo-4H-quinolizine-3-carboxylic acid ethyl ester, from Example 253i above, was dissolved in 4 mL of anhydrous acetonitrile, reacted with (3R,1S)-3-(1-amino-3-methylpropyl)pyrrolidine (400 mg, 1.32 mmol, prepared as described by Plummer *et al., Tetr. Lett.* 34:7529-32 (**1993**), and carried forward as described in Example 253j-l, omitting the deprotection reaction, to give the title product. MS (high resolution) found: 402.2174; calc: 402.2193 (M+H)⁺; ¹H NMR (D₆-DMSO) ∂: 0.60 (m, 2H), 0.95 (d, 3H), 1.06 (d, 3H), 1.75 (m, 1H), 2.13 (m, 1H), 2.29 (m, 2H), 2.50 (s, 3H), 3.66 (m, 3H), 3.78 (m, 1H), 3.97 (m, 1H), 7.88 (s, 1H), 9.08 (d, 1H), 13.82 (bs, 1H). Anal. Calcd for C₂₂H₂₉N₃O₃FCl•0.75 H₂O: C, 58.53; H, 6.81; N, 9.31; Found: C, 58.88; H, 6.70; N, 9.26.

### Example 311

### 8-(3-(1-aminocyclopropyl)pyrrolidinyl)-1-cyclopropyl-7-fluoro-9-methyl-4-oxo-4H-quinolizine-3-carboxylic acid hydrochloride

A 98 mg sample of 8-chloro-1-cyclopropyl-7-fluoro-9-methyl-4-oxo-4H-quinolizine-3-carboxylic acid ethyl ester, from Example 253i above, was dissolved in 2 mL of anhydrous acetonitrile, reacted with 1-(N-BOC-amino)cyclopropyl)pyrrolidine (172 mg, 0.76 mmol, prepared as described by Hayakawa *et al*., U.S. Patent 5,098,912, issued Mar. 24, 1992), and carried forward as described in Example 253j-l to give the title product. MS (high resolution) found: 386.1893; calc: 386.1880 (M+H)⁺; ¹H NMR (D₆-DMSO) ∂: 0.60 (m, 2H), 0.91 (m, 5H), 1.04 (m, 1H), 1.67 (m, 1H), 2.04 (m, 1H), 2.29 (m, 2H), 2.61 (s, 3H), 3.70 (m, 3H), 3.93 (m, 1H), 7.90 (s, 1H), 8.43 (bs, 2H), 9.08 (d, 1H), 13.82 (s, 1H). Anal. Calcd for C₂₂H₂₉N₃O₃FCl: C, 59.55; H, 6.12; N, 9.80; Found: C, 59.78; H, 5.97; N, 9.69.

### Example 312

### (3R,1S)-8-(3-(1-amino-2-hydroxyethyl)pyrrolidinyl)-1-cyclopropyl-7-fluoro-9-methyl-4-oxo-4H-quinolizine-3-carboxylic acid hydrochloride

### Step 312a. (S)-N-BOC-O-(methoxymethyl)serine methyl ester

A 7 g (31.96 mmol) sample of ((S)-N-BOC-serine methyl ester (obtained from Aldrich) was dissolved in CH₂Cl₂ and cooled in an ice bath. To this stirred solution was added dropwise 2.83 g (35.16 mmol) of methoxymethyl chloride, followed by dropwise addition of 4.544 g (6.12 mL, 35.16 mmol) of diisopropylethylamine. After all reagents were added the reaction was stirred for 16 hours at room temperature. The solution was washed with 0.5 % HCl, satd. NaHCO₃, H₂O, and brine, dried over MgSO₄ and filtered. The solvent was removed to leave a yellow oil. The residue was purified by chromatography on silica gel, eluting with 15-20% ethyl acetate:hexane to afford 6 g of title product after removal of the solvent. MS 264 (M+H)⁺; ¹H NMR (CDCl₃) ∂: 1.47 (s, 9H), 3.31 (s, 3H), 3.74 (dd, 1H), 3.79 (s, 3H), 4.00 (dd, 1H), 4/45 (b M, 1H), 4.60 (s, 2H), 5.43 (b m, 1H).

### Step 312b. 2-(BOC-amino)-3-(methoxymethoxy)-1-propanol

A solution of the compound from step 312a above (5.202 g, 19.78 mmol) in 15 mL of THF was added dropwise to a cooled (ice bath) suspension of 570 mg (14.84 mmol) of LAH in 15 mL of THF under N₂ atmosphere. The mixture was stirred for 1.5 hours, the reaction was quenched with water and 50% NaOH, filtered, and the filtrate evaporated to obtain the crude product. A yellow oil was obtained, which was purified by chromatography on silica gel, eluting with 35-40% ethyl acetate:hexane to give 3.475 g of the title product as a colorless oil. MS 236 (M+H)⁺

### Step 312c. 2-(BOC-amino)-3-(methoxymethoxy)-1-propanal

To a solution of the compound from step 312b above (3.47 g, 14.77 mmol) in 7 mL of DMSO cooled to 0°C was added dropwise 6.8 mL (48.74 mmol) of triethylamine. Pyridine•SO₃ complex (7.05 g, 44.31 mmol) was dissolved in 27 mL of DMSO and added to the first solution, and the reaction was stirred for one hour after the addition was complete. The solution was poured into 120 mL of cold brine, and the mixture was washed 3x with ethyl acetate. The extract was washed with water, dried over MgSO4, filtered and the solvent was removed under vacuum to give 6 g of a yellow oil, which was taken directly to the next step.

### Step 312d. 4-(BOC-amino)-5-(methoxymethoxy)-2-pentenoic acid ethyl ester

To a solution of the compound from step 312c above (14.77 mmol) in 42 mL of CH₂Cl₂ and cooled in an ice bath was added dropwise 5.454 g (15.66 mmol) of (carboethoxymethylene)triphenylphosphorane in 56 mL of CH₂Cl₂. After addition was complete, the reaction was stirred for 16 hours at room temperature. The solvent was removed, and the residue purified by column chromatography on silica gel, eluting with 10% ethyl acetate:hexane, to give 2.763 g of a colorless oil. MS 304 (M+H)⁺; ¹H NMR (CDCl₃) ∂: 1.25 (t, 3H), 1.47 (s, 9H), 3.36 (s, 3H), 3.67 (dd, 1H), 3.73 (dd, 1H), 3.72 (m, 1H), 4.20 (q, 2H), 4.62 (s, 2H), 5.99 (dd, 1H), 6.93 (dd, 1H).

### Step 312e. 4-(BOC-amino)-5-(methoxymethoxy)-3-(nitromethyl)-pentanoic acid ethyl ester

To a solution of the compound from step 312d above (2.76 g, 9.71 mmol) in 8 mL of nitromethane cooled in an ice bath was added 7 mL (6.934 g, 45.55 mmol) of 1,8-diazabicyclo[5.4.0]undec-7-ene dropwise under N₂. The mixture was warmed to room temperature and stirred for 16 hours. The solution was diluted with CH₂Cl₂ and extracted with water, 10% HCl, 10% NaHCO₃, water and brine. The solution was dried over MgSO₄, and the solvent was removed. The residue was chromatographed on silica gel, eluting with 10-15% ethyl acetate:hexane, and the solvent was removed to give 2.01 g of the title product as a white solid. MS 365 (M+H)⁺; ¹H NMR (CDCl₃) ∂: 1.27 (t, 3H), 1.47 (s, 9H), 2.46 (dd, 1H), 2.98 (br, 1H), 3.38 (s, 3H), 3.58 (ddd, 1H), 3.76 (dd, 1H), 3.97 (b m, 1H), 4.16 (q, 1H), 4.53 (dd, 1H), 4.62 (s, 2H), 4.67 (dd, 1H), 4.99 (b d, 1H).

### Step 312f. 4-(BOC-amino)-5-(methoxymethoxy)-3-(aminomethyl)-pentanoic acid ethyl ester

Two g of the compound from step 312e above was dissolved in 200 mL of ethanol and hydrogenated at 4 Atm over 4 g of Raney nickel catalyst for 24 hours. The catalyst was removed by filtration and the solvent was evaporated. The residue was taken directly to the next step.

### Step 312g. N-BOC-2-(methoxymethoxy)-1-(5-oxo-3-pyrrolidinyl)-ethylamine

The residue from step 312f above was dissolved in 150 mL of ethanol and heated at reflux for 8 hours. The solvent was removed, the residue was chromatographed on silica gel, eluting with 4% methanol/methylene chloride. Removal of the solvent gave 1.36 g of title product. MS 289 (M+H)⁺; ¹H NMR (CDCl₃) ∂: 1.47 (t, 3H), 2.17 (dd, 1H), 2.38 (dd, 1H), 2.78 (m, 1H), 3.31 (t, 1H), 3.46 (s, 3H), 3.46 (t, 1H), 3.59 (m, 2H), 3.81 (b t, 1H), 4.62 (s, 2H), 4.94 (br d, 1H), 5.43 (br, 1H).

### Step 312h. N-BOC-2-(methoxymethoxy)-1-(5-thioxo-3-pyrrolidinyl)-ethylamine

A 500 mg (1.74 mmol) sample of the compound from step 312g above and 387 mg (0.957 mmol) of Lawesson's reagent were dissolved in 4 mL of THF and stirred under N₂ for 3 hours. The solvent was removed, and the residue was dissolved in CH₂Cl₂ and chromatographed on silica gel, eluting with 35% ethyl acetate:hexane. Removal of the solvent left 500 mg of product. MS 305 (M+H)⁺; ¹H NMR (CDCl₃) ∂: 1.47 (s, 9H), 2.71 (dd, 1H), 2.89 (m, 1H), 3.00 (dd, 1H), 3.37 (s, 3H), 3.53 (dd, 2H), 3.66 (m, 2H), 3.83 (b m, 1H), 4.61 (s, 2H), 4.98 (b d, 1H).

### Step 312i. N-BOC-2-(methoxymethoxy)-3-pyrrolidinyl)-ethylamine acetic acid salt

A 250 mg (0.825 mmol) sample of the compound from step 312h above and 1.57g (6.6 mmol) of NiCl2•6H₂O were dissolved in 10 mL of a 1:1 mixture of methanol and THF, and the solution was cooled to -78°C and stirred under N₂. A 749 mg (19.8 mmol) sample of NaBH₄ was added in portions, and the mixture was stirred for 2 hours. The solvents were removed under vacuum, and dissolved in 20% methanol in chloroform. The solution was filters and the solvent removed. The residue was chromatographed on silica gel, eluting with 1:1:1:1 n-butanol:ethyl acetate:H₂O:acetic acid to provide 349 mg of title product. MS 275 (M+H)⁺; ¹H NMR (D₂O) ∂: 1.44 (s, 9H), 3.03 (m, 1H), 3.30 (m, 1H), 3.40 (s, 3H), 3.48 (m, 1H), 3.60 (t, 2H), 3.75 (m, 1H).

### Step 312j. (3R,1S)-8-(3-(1-amino-2-hydroxyethyl)pyrrolidinyl)-1-cyclopropyl-7-fluoro-9-methvl-4-oxo-4H-quinolizine-3-carboxylic acid hydrochloride

A 107 mg (0.33 mmol) sample of 8-chloro-1-cyclopropyl-7-fluoro-9-methyl-4-oxo-4H-quinolizine-3-carboxylic acid ethyl ester, from Example 253i above, was dissolved in 2.5 mL of anhydrous acetonitrile, reacted with the compound from step 312i above (0.825 mmol), and carried forward as described in Example 253j-l to give 74 mg of the title product. ¹H NMR (D₆-DMSO) ∂: 0.60 (m, 2H), 0.94 (m, 1H), 1.05 (m, 1H), 1.78 (m, 1H), 2.05 (m, 1H), 2.19 (m, 2H), 2.60 (s, 3H), 3.57 (m, 1H), 3.73 (m, 3H), 3.92 (m, 1H), 5.41 (m, 1H), 7.91 (s, 1H), 9.09 (d, 1H), 13.83 (br s, 1H).

### Example 313

### (8-(3-(1-amino-1-methylethyl)pyrrolidinyl)-1-cyclopropyl-7-fluoro-9-methyl-4-oxo-4H-quinolizine-3-carboxylic acid hydrochloride

A 150 mg sample of 8-chloro-1-cyclopropyl-7-fluoro-9-methyl-4-oxo-4H-quinolizine-3-carboxylic acid ethyl ester, from Example 253i above, was dissolved in 2 mL of anhydrous acetonitrile, reacted with 1-amino-1-methylethyl)pyrrolidine (155 mg, 0.77 mmol, prepared by standard method from the free base described by Hayakawa *et al*., U.S. Patent 5,098,912, issued Mar. 24, 1992), and carried forward as described in Example 253k-l to give the title product. MS (high resolution) found: 388.2047; calc: 388.2036 (M+H)⁺; ¹H NMR (D₆-DMSO) ∂: 0.60 (m, 2H), 0.94 (m, 1H), 1.07 (m, 1H), 1.33 (s, 3H), 1.34 (s, 1H), 2.83 (m, 1H), 2.07 (m, 1H), 2.19 (m, 2H), 2.63 (s, 3H); 3.60 (b t, 1H), 3.68 (b t, 1H), 3.81 (m, 1H), 3.93 (m, 1H), 7.90 (s, 1H), 8.11 (b s, 1H), 9.08 (d, 1H), 13.83 ( b s, 1H). Anal. Calcd for C₂₁H₂₇N₃O₃FCl•1.5 H₂O: C, 55.93; H, 6.71; N, 9.32; Found: C, 56.07; H, 6.71; N, 8.95.

### Example 314

### 8-(3-(1-aminobutyl)pyrrolidinyl)-1-cyclopropyl-7-fluoro-9-methyl-4-oxo-4H-quinolizine-3-carboxylic acid hydrochloride

### Step 314a. 4-(BOC-amino)-3-(nitromethyl)-heptanoic acid ethyl ester

Following the procedure of Example 312 step b, substituting DL-N-BOC-norvaline methyl ester (prepared from norvaline by standard methods) for the compound of step 312a thereof, and carrying the product forward via the procedures of Example 312 steps c-e, the title compound was prepared.

### Step 314b. 4-(BOC-amino)-3-(nitromethyl)-heptanol

Repeating the procedure of example 312 step b, substituting 4-(BOC-amino)-3-(nitromethyl)-heptanoic acid ethyl ester (1.3 g, 3.91 mmol), from step 314a above, for the compound of step 312a thereof, the title compound was prepared. MS 291 (M+H)⁺; ¹H NMR (CDClD₃) ∂: 0.93 (t, 3H), 1.45 (s, 9H), 1.48 (m, 5H), 1.77 (m, 1H), 2.53 (m, 1H), 3.79 (m, 3H), 4.33 (m, 1H), 4.38 (dd, 1H), 4.49 (dd, 1H).

### Step 314c. 4-(BOC-amino)-3-(nitromethyl)-heptanol, O-mesityl ether

A 610 mg (2.03 mmol) sample of the compound from step 314c above was dissolved in 2 mL of CH₂Cl₂, and the solution was cooled to -10°C. To this was added dropwise 289 mg (0.195 mL, 2.52 mmol) of methanesulfonyl chloride and 319 mg (3.15 mmol) of triethylamine. The solution was stirred for 2 hours at 0-10°C. The solution was diluted with CH₂Cl₂ and washed, once with water, once with 5% NaHCO₃, and once with brine. The solvent was dried over MgSO₄ and filtered, and the solvent was removed to give 720 mg of the title product as an oil.

### Step 314d. 3-(1-(N-BOC-amino)butyl)pyrrolidine

The 720 mg sample of the product from step 314c was dissolved in 50 mL of methanol and hydrogenated over 360 mg of 10% Pd/C catalyst at 4 Atm and room temperature for 24 hours. MS 2243 (M+H)⁺; ¹H NMR (CD₃OD) ∂: (0.94 (t, 3H), 1.34 (m, 3H), 1.44 (s, 9H), 1.48 (m, 1H), 1.70 (m, 1H), 2.13 (m, 1H), 2.37 (q, 1H), 3.04 (m, 1H), 3.22 (m, 1H), 6.71 (b d, 1H).

### Step 314f. 8-(3-(1-aminobutyl)pyrrolidinyl)-1-cyclopropyl-7-fluoro-9-methyl-4-oxo-4H-quinolizine-3-carboxylic acid hydrochloride

A 238 mg sample of 8-chloro-1-cyclopropyl-7-fluoro-9-methyl-4-oxo-4H-quinolizine-3-carboxylic acid ethyl ester, from Example 253i above, was dissolved in 5 mL of anhydrous acetonitrile, reacted with 3-(1-(N-BOC-amino)butyl)pyrrolidine (620 mg, 1.83 mmol, prepared in step 314d above), and carried forward as described in Example 253j-l to give the title product. MS (high resolution) found: 402.2199; calc: 402.2193 (M+H)⁺; ¹H NMR (D₆-DMSO) ∂: 0.60 (m, 2H), 0.89 (m, 4H), 1.05 (m, 1H), 1.49 (m, 5H), 1.17 (m, 1H), 2.14 (m, 1H), 2.27 (m, 1H), 2.62 (s, 3H), 3.77 (m, 4H), 3.94 (m, 1H), 7.89 (s, 1H), 8.54 (b m, 1H), 9.07 (d, 1H), 11.47 (br, 1H).

### Examples 315-323

Following the procedures of Steps 253j, 253k and 253l above, replacing the 3-BOC-aminopyrrolidine of Step 253j with the reagent shown, the compounds of Examples 315-323 are prepared as shown in Table 2, below.

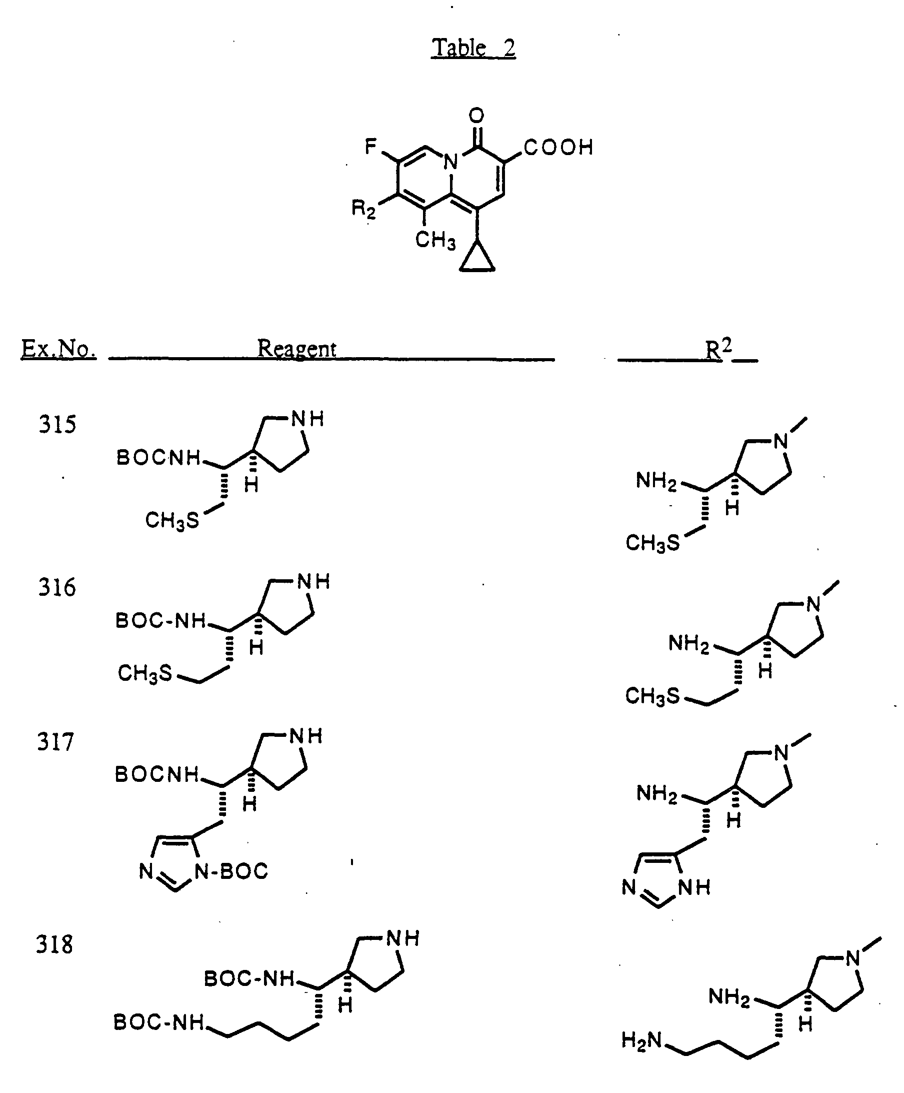

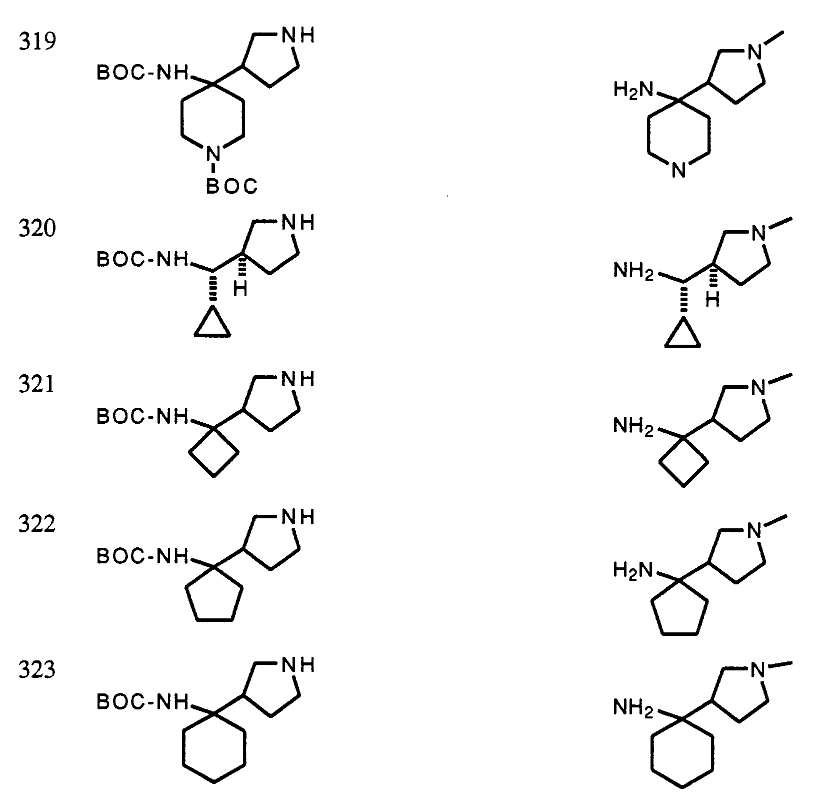

### Example 324

### 1-cyclopropyl-7-fluoro-9-methyl-4-oxo-8-(trans-4-trifluoromethyl-3-aminopyrrolidinyl)-4H-quinolizine-3-carboxylic acid hydrochloride

### Step 324a. trans-N-benzyl-4-trifluoromethyl-3-pyrrolidinecarboxvlic acid ethyl ester

Trifluoroacetic acid (3 mL, 1 N in CH₂Cl₂) was added to a stirred solution of *trans*-ethyl trifluorocrotonate (4.969 g) and N-benzyl-N-methoxymethyl)trimethylsilylamine (7.00 g) in 30 mL of CH₂Cl₂ at 0°C, and the mixture was stirred for 2 hours. After dilution with CH₂Cl₂, the solution was washed with satd. NaHCO₃ solution and water, dried over MgSO₄ and concentrated under vacuum to give a pale yellow liquid (8.75 g).

### Step 324b. trans-N-benzyl-4-trifluoromethyl-3-pyrrolidinecarboxylic acid

A sample (4.739 g ) of this liquid was hydrolyzed with 1.98 g of LiOH•H₂O in THF:H₂O (25 mL, 1.5:1) at 60°C to give after workup 3.64 g of the intermediate as a solid.

### Step 324c. trans-1-benzyl-3-(BOC-amino)-4-trifluoromethylpyrrolidine

A sample of the intermediate from 324b (3.64 g), diphenylphosphoranyl azide (3.50 mL), t-butanol (40 mL), triethylamine (2.3 mL) and 40 mL of dioxane were mixed and heated at reflux under N₂ for 17 hours. The solvents were removed under vacuum. The residue was dissolved in CH₂Cl₂, washed with satd. NaHCO₃ solution and water, dried over MgSO₄ and concentrated under vacuum. The product was purified by chromatography on silica gel, eluting with 100:5:5 CH₂Cl₂ :methanol: NH₄OH to afford 1.77 g of the title compound.

### Step 324d. trans-3-(BOC-amino)-4-trifluoromethylpyrrolidine

The compound from step 324c above (1.55 g) was hydrogenated in 50 mL of methanol over 0.45 g of 10% Pd/C catalyst under 4 Atm of H₂ for 3.5 days. The catalyst was removed by filtration, and the solvent was removed to afford the title compound as a white solid (1.09 g).

### Step 324e, 1-cyclopropyl-7-fluoro-9-methyl-4-oxo-8-(trans-4-trifluoromethvl-3-aminopyrrolidinyl)-4H-quinolizine-3-carboxylic acid hydrochloride

Following the procedure of Example 253 steps k and l, replacing the 3-BOC-aminopyrrolidine of Example 253j with the compound from step 325d above, the title compound was prepared (97 mg). MS: 414 (M+1)⁺; ¹H NMR (D₆-DMSO) ∂: 0.63 (m, 2H), 1.01 (m, 2H), 2.39 (m, 1H), 2.70 (s, 3H), 3.59 (m, 1H), 3.81 (m, 2H), 4.11-4.25 (m, 3H), 8.01 (s, 1H). Anal. Calcd for C₁₉H₁₉N₃O₃F₄•HCl·1.25 H₂O: C, 48.31; H, 4.80; N, 8.90; Found: C, 48.45; H, 4.63; N, 8.53.

### Example 325

### 1-cyclopropyl-7-fluoro-9-methyl-4-oxo-8-(trans-4-trifluoromethyl-3-aminomethylpyrrolidinyl)-4H-quinolizine-3-carboxylic acid hydrochloride

### Step 325a. trans-1-benzyl-3-(hydroxymethyl)-4-trifluoromethylpyrrolidine

A sample of the compound from Example 324 step a above (4.02 g) was dissolved in 10 mL of THF, then LAH (8.0 mL, 1.0 N in THF) was added, and the solution was stirred for 30 min at room temperature. The reaction was quenched, and the product was extracted to give 3.36 g of the title product after removal of the solvent.

### Step 325b. trans-1-benzyl-3-(aminomethyl)-4-trifluoromethylpyrrolidine

The compound from step 325a above (3.36 g), triphenylphosphine, and phthalimide were dissolved in 50 mL of THF, and DEAD (2.05 mL) was added dropwise to the above solution at room temperature. The reaction was complete almost immediately, and the solvents were removed. The residue was dissolved in 50 mL of ethanol, 0.65 mL of NH₂NH₂•H₂O was added, and the reaction was heated at reflux under N₂ for 3 hours. The solution was cooled to room temperature, 5 mL of conc. HCl was added, and the mixture was filtered. The filtrate was concentrated, and the residue was dissolved in 10% HCl and extracted (6x) with CH₂Cl₂. The aqueous layer was then adjusted to pH 11 with NaOH and extracted with CH₂Cl₂, which was washed with H₂O, dried over MgSO₄ and concentrated. The residue was dissolved in 7:25 H₂O:methanol, (BOC)₂O was added, and the reaction stirred at room temperature for 30 min. The methanol was removed under vacuum, and the aqueous residue was extracted with CH₂Cl₂. The extract was washed with H₂O, dried over MgSO₄ and concentrated. The residue was purified by chromatography on silica gel, eluting with 1:4 ethyl acetate:hexane, to give the title compound as a white solid.

### Step 325c. trans-3-(BOC-aminomethyl)-4-trifluoromethylpyrrolidine

The compound from step 325b above was hydrogenated according to the procedure of Example 324 step d to afford the title compound as a white solid.

### Step 325d. 1-cyclopropyl-7-fluoro-9-methyl-4-oxo-8-(trans-4-trifluoromethyl-3-aminomethylpyrrolidinyl)-4H-quinolizine-3-carboxylic acid hydrochloride

Following the procedure of Example 253 step j above, substituting the compound from step 325c above for the 3-BOC-aminopyrrolidine thereof, and carrying the reaction product forward as in Example 253 steps k and l above, a 77 mg sample of the title product was prepared. MS: 428 (M+1)⁺; ¹H NMR (D₆-DMSO) ∂: 0.63 (m, 2H), 1.02 (m, 2H), 2.36 (m, 1H), 2.69 (s, 3H), 2.80 (m, 1H), 3.08 (m, 2H), 3.69 (m, 1H), 3.83 (m, 1H), 3.94-4.06 (m, 3H), 7.99 (s, 1H), 9.17 (d, 1H, J=10 Hz). Anal. Calcd for C₂₀H₂₁N₃O₃F₄•HCl•H₂O: C, 49.85; H, 5.02; N, 8.72; Found: C, 49.86; H, 5.10; N, 8.93.

### Example 326

### 3(S)-1-cyclopropyl-7-fluoro-9-methyl-4-oxo-8-(3-(N-(S)-norvalylamino)pyrrolidinyl)-4H-quinolizine-3-carboxylic acid hydrochloride

Following the procedure of Example 166, replacing the starting pyrido-pyrimidine material thereof with the product of Example 253 step j, the title compound was prepared. MS: 445 (M+1)⁺; Anal. Calcd for C₂₃H₂₉N₄O₄F•1.5 HCl•0.75 H₂O: C, 53.88; H, 6.29; N, 10.93; Found: C, 53.87; H, 6.10; N, 11.10.

### Example 327

### 3(S)-1-cyclopropyl-7-fluoro-9-methyl-4-oxo-8-(3-(N-(S)-alanylamino)pyrrolidinyl)-4H-quinolizine-3-carboxylic acid hydrochloride

Following the procedure of Example 167, replacing the starting pyrido-pyrimidine material thereof with the product of Example 253 step j, 97 mg of the title compound was prepared. MS: 417 (M+1)⁺; ¹H NMR (D₆-DMSO) ∂: 0.60 (m,2H), 1.00 (m, 2H), 1.35 (d, 3H, J=7 Hz), 2.00 (m, 1H), 2.20-2.31 (m, 2H), 2.62 (s, 3H), 3.56 (m, 1H), 3.80 (m, 2H), 3.93-4.06 (m, 2H), 4.43 (m, 1H), 7.91 (s, 1H), 8.19 (br, 3H), 8.91 (d, 1H, J=6 Hz), 9.09 (d, 1H, J=10.5 Hz), 13.85 (br, 1H). Anal. Calcd for C₂₁H₂₅N₄O₄F •2 HCl: C, 51.54; H, 5.56; Found: C, 51.50; H, 5.48.

### Example 328

### 3(S)-1-cyclopropyl-7-fluoro-9-methyl-4-oxo-8-(3-(N-(S)-alanyl-(S)-alanylamino)pyrrolidinyl)-4H-quinolizine-3-carboxylic acid hydrochloride

Following the procedure of Example 168, replacing the starting pyrido-pyrimidine material thereof with the product of Example 253 step j, 680 mg of the title compound was prepared. MS: 488 (M-Cl)⁺; ¹H NMR (D₆-DMSO) ∂: 0.60 (m, 2H), 1.00 (m, 2H), 1.23 (d, 3H, J=7.5 Hz), 1.33 (d, 3H, J=7.0 Hz), 1.98 (m, 1H), 3.85-4.01 (m, 4H), 4.314.37 (m, 2H), 7.91 (s, 1H), 8.13 (br, 3H), 8.47 (d, 1H, J=6.0 Hz), 8.65 (d, 1H, J=7.5 Hz), 9.10 (d, 1H, J=10.5 Hz). Anal. Calcd for C₂₄H₃₀N₅O₅F •3 HCl•0.5 H₂O: C, 46.18; H, 5.57; N, 11.22; Found: C, 46.34; H, 5.77; N, 11.52.

### Example 329 is missing due to its deletion

### Example 330

### 1-cyclopropyl-7-fluoro-4H-8-(1-imidazolyl)-9-methyl-4-oxo-quinolizine-3-carboxylic acid hydrochloride

Following the procedure of Example 253 step j, replacing the 3-t-BOC-aminopyrrolidine thereof with imidazole, and carrying the product forward as in Example 253 step k, the title compound was prepared. HRMS: (M+H)⁺ calcd: 328.1097; found: 328.1110 ¹H NMR (CDCl₃) ∂: 0.90 (m, 2H), 1.18 (m, 2H), 2.40 (m, 1H), 2.83 (s, 3H), 7.15 (s, 1H), 7.39 (s, 1H), 7.71 (s, 1H), 8.66 (s, 1H), 9.43 (d, 1H, J=6 Hz).

### Example 331

### 8-(3-amino-1-pyrrolidinyl)-1-ethyl-7-fluoro-4H-4-oxo-9-methyl-quinolizine-3-carboxylic acid hydrochloride

Following the procedure of Example 253 step e, replacing the cyclopropylacetonitrile compound thereof with propionitrile, and carrying the product forward as in Example 253 steps e-l, the title compound was prepared. MS: 334 (M-Cl)⁺; ¹H NMR (D₆-DMSO) ∂: 2.28 (m, 3H), 2.22 (m, 1H), 2.52 (m, 4H), 2.96 (m, 2H), 3.88-4.18 (m, 5H), 8.01 (s, 1H), 9.05 (d, 1H, J=10 Hz). Anal. Calcd for C₁₇H₂₀N₃O₃FCl•HCl•1.5 H₂O: C, 51.45; H, 6.10; N, 10.59; Cl, 8.93; Found: C, 51.51; H, 5.90; N, 10.78; Cl, 8.91.

### Example 332

### 8-(3-amino-1-pyrrolidinyl)-1-cyclopropyl-9-ethyl-7-fluoro-4H-4-oxo-quinolizine-3-carboxylic acid hydrochloride

Following the procedure of Example 253 step c, replacing the methyl iodide thereof with ethyl iodide, and carrying the product forward as in Example 253 steps 253d-l, the title compound was prepared. MS: 360 (M-Cl)⁺; ¹H NMR (D₆-DMSO) ∂: 0.52 (m, 2H), 0.87 (t, 3H, J=6 Hz), 0.98 (m, 2H), 2.20 (m, 2H), 2.33 (m, 1H), 3.20 (m, 2H), 3.65-3.96 (m, 5H), 7.95 (s, 1H), 8.43 (br, 3H), 9.07 (d, 1H, J=10.5 Hz), 13.83 (br, 1H). Anal. Calcd for C₁₉H₂₂N₃O₃F•1.25 HCl•1.5 H₂O: C, 53.95; H, 6.01; N, 9.93; Found: C, 53.82; H, 5.87; N, 10.18.

### Example 333

### 1-cyclopropyl-7-fluoro-4H-9-methyl-4-oxo-8-(3-(1,2,3-triazol-1-yl)-1-pyrrolidinyl)-quinolizine-3-carboxylic acid

### Step 333a. 1-benzyl-3-(1.2.3-triazol-1-yl)pyrrolidine

A solution of 3-azido-1-benzylpyrrolidine (2.30 g) and trimethylsilylacetylene (8.0 mL) in 15 mL of toluene was heated at reflux for 18 hours. The solvents were removed to give an oily residue. The residue was dissolved in 20% HCl and heated at reflux for 16 hours. The solution was cooled, made basic with NaHCO₃, and extracted with methylene chloride. The organic layer was washed with water, dried over MgSO₄ and concentrated. The crude product was purified by chromatography on silica gel, eluting with CH₂Cl₂ :methanol:NH₄OH 100:10:1.

### Step 333b. 3-(1,2,3-triazol-1-yl)pyrrolidine

The compound from step 333a was dissolved in methanol and hydrolyzed by hydrogenation for 16 hours with a catalyst of 10% Pd/C. The mixture was filtered, and the solvent was removed to give 1.00 g of the product.

### Step 333c. 1-cyclopropyl-7-fluoro-4H-9-methyl-4-oxo-8-(3-(1,2,3-triazol-1-yl)-1-pyrrolidinyl)-quinolizine-3-carboxylic acid

Following the procedure of Example 253 step j, replacing the 3-BOC-aminopyrrolidine thereof with the compound from step 333b, and carrying the product forward as in Example 253 steps j & k, the title compound was prepared. mp 183-186°C. MS: 398 (M-Cl)⁺; ¹H NMR (D6-DMSO) ∂: 0.61 (m, 2H), 0.99 (m, 2H), 2.31 (m, 1H), 2.56 (m, 2H), 2.62 (s, 3H), 3.84 (m, 1H), 3.99 (m, 1H), 4.10 (m, 1H), 4.36 (m, 1H), 5.46 (m, 1H), 7.80 (s, 1H), 7.92 (s, 1H), 8.32 (s, 1H), 9.11 (d, 1H, J=11 Hz). Anal. Calcd for C₂₀H₂₀N₅O₃F: C, 60.45; H, 5.07; N, 17.62; Found: C, 60.46; H, 5.20; N, 17.63.

### Example 334

### 1-cyclopropyl-7-fluoro-4H-9-methyl-4-oxo-8-(cis-3-amino-4-methyl-1-pyrrolidinyl)-quinolizine-3-carboxylic acid hydrochloride

Following the procedure of Example 253 step j, replacing the 3-BOC-aminopyrrolidine thereof with *cis*-3-BOC-amino-4-methylpyrrolidine, and carrying the product forward as in Example 253 steps j-l, the title compound was prepared. MS: 360 (M-Cl)⁺; ¹H NMR (D₆-DMSO) ∂: 0.60 (m, 2H), 0.99 (m, 2H), 1.18 (d, 3H, J=7 Hz), 2.30 (m, 1H), 2.62 (s, 3H), 3.48-4.00 (m, 6H), 7.94 (s, 1H), 8.40 (m, 3H), 9.10 (d, 1H, J= 10.5 Hz). Anal. Calcd for C₁₉H₂₂N₃O₃F•HCl•1.25 H₂O: C, 54.55; H, 6.14; N, 10.04; Found: C, 54.62; H, 6.10; N, 10.08.

### Example 335

### 8-(2-aminoethyl)-1-cyclopropyl-7-fluoro-4H-9-methyl-4-oxo-quinolizine-3-carboxylic acid hydrochloride

Following the procedure of Example 253 step j, replacing the 3-BOC-aminopyrrolidine thereof with 2-aminoethylamine, and carrying the product forward as in Example 253 steps j-l, the title compound was prepared. MS: 320 (M-Cl)⁺; ¹H NMR (D₂O) ∂: 0.60 (m, 2H), 1.02 (m, 2H), 2.02 (m, 1H), 2.64 (s, 3H), 3.40 (m, 2H), 3.99 (m, 2H), 7.40 (s, 1H), 8.80 (d, 1H, J=10.5 Hz). Anal. Calcd for C₁₆H₁₈N₃O₃F•HCl•0.85 H₂O: C, 51.78; H, 5.62; N, 11.32; Found: C, 51.79; H, 5.31; N, 11.15.

### Example 336

### 8-(3-(ethylaminomethyl)pyrrolidinyl)-1-cyclopropyl-7-fluoro-4H-9-methyl-4-oxoquinolizine-3-carboxylic acid hydrochloride

Following the procedure of Example 253 step j, replacing the 3-BOC-aminopyrrolidine thereof with 3-((N-BOC-N-ethyl)amino)methylpyrrolidine, and carrying the product forward as in Example 253 steps j-l, the title compound was prepared. MS: 388 (M-Cl)⁺; ¹H NMR (CD₃OD) ∂: 0.60-0.68 (m, 2H), 1.05 (m, 2H), 1.37 (m, 3H), 1.91 (m, 1H), 2.31 (m, 2H), 2.68 (s, 3H), 2.69 (m, 1H), 3.15 (m, 2H), 3.33 (m, 2H), 3.75-3.96 (m, 4H), 8.01 (s, 1H), 9.03 (d, 1H, J=10.5 Hz). Anal. Calcd for C₂₁H₂₆N₃O₃F•1.25 HCl•H₂O: C, 55.92; H, 6.54; N, 9.32; Found: C, 56.18; H, 6.32; N, 9.27.

### Example 337

### 8-(3-(1-aminoethyl)pyrrolidinyl)-1-cyclopropyl-7-fluoro-4H-9-methyl-4-oxo-quinolizine-3-carboxylic acid hydrochloride

Following the procedure of Example 253 step j, replacing the 3-BOC-aminopyrrolidine thereof with 3-(N-BOC-aminoethyl)pyrrolidine, and carrying the product forward as in Example 253 steps j-l, the title compound was prepared. MS: 374 (M-Cl)⁺; Anal. Calcd for C₂₀H₂₄N₃O₃F•HCl•H₂O: C, 56.14; H, 6.36; Found: C, 56.27; H, 6.14.

### Example 338

### 1-cyclopropyl-7-fluoro-4H-9-methyl-8-(2-methyl-2,8-diaza-8-bicyclo[4,3,0]nonyl)-4-oxoquinolizine-3-carboxylic acid hydrochloride

Following the procedure of Example 253 step j, replacing the 3-BOC-aminopyrrolidine thereof with 2-methyl-2,8-diaza-bicyclo[4.3.0]nonane, and carrying the product forward as in Example 253 steps j-l, the title compound was prepared. MS: 400 (M-Cl)⁺; ¹H NMR (DMSO-d₆) ∂: 0.65 (m, 2H), 0.92 (m, 1H), 1.09 (m, 1H), 1.80-1.95 (m, 5H), 2.31 (m, 1H), 2.69 (s, 3H), 2.83 (m, 5H), 3.61-4.34 (m, 5H), 7.90 (s, 1H), 9.10 (d, 1H, J=10.5 Hz). Anal. Calcd for C₂₂H₂₆N₃O₃F•1.25 HCl•0.5 H₂O: C, 58.20; H, 6.27; N, 9.25; Found: C, 58.09; H, 6.27; N, 9.25.

### Example 339

### 1-cyclopropyl-7-fluoro-4H-8-((1S,4S)-2,5-diaza-bicyclo[2.2.1]heptan-2-yl)-9-methyl-4-oxoquinolizine-3-carboxylic acid hydrochloride

Following the procedure of Example 253 step j, replacing the 3-BOC-aminopyrrolidine thereof with (1S,4S)-2,5-diaza-5-BOC-bicyclo[2.2.1]heptane (prepared according to *J*. *Med Chem.,* 32:1598 (**1988**)), and carrying the product forward as in Example 253 steps j-l, the title compound was prepared. MS: 358 (M-Cl)⁺; ¹H NMR (DMSO-d₆) ∂: 0.59 (m, 1H), 0.93 (m, 1H), 1.06 (m, 1H), 2.05 (m, 1H), 2.31 (m, 2H), 2.59 (s, 3H), 3.45 (m, 2H), 3.61 (m, 1H), 4.09 (m, 1H), 4.51 (m, 1H), 4.96 (m, 1H), 7.97 (s, 1H), 9.07 (br, 1H), 9.20 (d, 1H, J=10.5 Hz), 9.54 (br, 1H). Anal. Calcd for C₁₉H₂₀N₃O₃F•1.5 HCl•1.0 H₂O: C, 53.06; H, 5.51; N, 9.77; Found: C, 53.19; H, 5.37; N, 9.58.

### Example 340

### 1-cyclopropyl-7-fluoro-4H-9-methyl-4-oxo-8-(3-(2-pyridinyl)-1-pyrrolidinyl)-quinolizine-3-carboxylic acid hydrochloride

Following the procedure of Example 253 step j, replacing the 3-BOC-aminopyrrolidine thereof with 3-(2-pyridinyl)pyrrolidine, and carrying the product forward as in Example 253 steps j-l, the title compound was prepared. MS: 408 (M-Cl)⁺; ¹H NMR (DMSO-d₆) ∂: 0.60 (m, 2H), 0.99 (m, 2H), 2.30-2.40 (m, 2H), 2.60 (m, 1H), 2.64 (s, 3H), 3.86-4.16 (m, 4H), 7.80 (m, 1H), 7.90 (s, 1H), 9.07 (d, 1H, J=11 Hz). Anal. Calcd for C₂₃H₂₃N₃O₃F •HCl•H₂O: C, 55.43; H, 5.26; N, 8.43; Found: C, 55.69; H, 4.97; N, 8.52.

### Example 341

### 8-((1R*,2S*,6R*)-2-amino-8-azabicyclo[4,3,0]nonan-8-yl))-1-cyclopropyl-7-fluoro-4H-9-methyl-4-oxo-quinolizine-3-carboxylic acid hydrochloride

### Step 341a. 1R*,2S*,6R*-2-BOC-amino-8-azabicyclo[4,3,0]nonane

Two mL of 1.0 N trifluoracetic acid was added to a stirred solution of 2.o mL of cyclohexane and 4.92 g of N-benzyl-N-(methoxymethyl)-trimethylsilylmethylamine (prepared according to *Chem. Pharm. Bull.,* 33:2762 (**1985**)) in 20 mL of methylene chloride at 0°C. The mixture was stirred at room temperature for 16 hours, then diluted with methylene chloride. The solution was washed with NaHCO₃ and water, then dried over MgSO₄. Removal of the solvent left an oily residue. The residue was dissolved in 65 mL of methanol, after which were added 2.2 g of NH₂OH•HCl, 10 mL of 10% NaOH, and 6.5 mL of methylene chloride, and the reaction was heated at 60°C for 3 hours. The solvents were removed, and the residue was dissolved in methylene chloride, which was washed with water, dried over MgSO₄ and concentrated to give an oil. The oil was dissolved in 50 mL of THF, 1.57 g of LAH were added, and the mixture was heated at reflux for 2 hours. The reaction was quenched with water, the solid was removed, and the filtrate was concentrated. The concentrate was dissolved in 40 mL of methanol and 10 mL of water. To this solution was added 5.0 g of (BOC)₂O and the reaction was stirred for 16 hours. The methanol was removed under vacuum, and the residue was extracted with methylene chloride. The extract was washed with water, dried over MgSO₄ and concentrated to give an oil. The oil was purified by chromatography on silica gel, eluting with 100:5:0.5 methylene chloride:methanol:NH₄OH to give 0.36 g of the 1R*,2S*,6R* isomer and 2.22 g of the 1R*,2R*,6R* isomer of the title compound. The 1R*,2S*,6R* isomer was stirred with 0.12g Of 10%Pd/C in 25 mL of methanol under 4 Atm of H₂ for 48 hours. The catalyst was filtered off, and the solvent was removed to give the title compound.

### 341b. 8-((1R*,2S*,6R*)-2-amino-8-azabicyclo[4.3.0]nonan-8-yl))-1-cyclopropyl-7-fluoro-4H-9-methyl-4-oxo-quinolizine-3-carboxylic acid hydrochloride

Following the procedure of Example 253 step j, replacing the 3-BOC-aminopyrrolidine thereof with 1R*,2S*,6R*-2-BOC-amino-8-azabicyclo[4.3.0]nonane, prepared in step 341a above, and carrying the product forward as in Example 253 steps j-l, the title compound was prepared. MS: 400 (M-Cl)⁺; ¹H NMR (DMSO-d₆) ∂: 0.63 (m, 2H), 0.94 (m, 1H), 1.05 (m, 1H), 1.42-1.62 (m, 4H), 1.97 (m, 1H), 2.31 (m, 2H), 2.62 (s, 3H), 2.67 (m, 1H), 3.19 (m, 1H), 3.54 (m, 1H), 3.82 (m, 1H), 4.00 (m, 2H), 7.89 (s, 1H), 8.18 (br, 3H), 9.06 (d, 1H, J=11 Hz). Anal. Calcd for C₂₂H₂₆N₃O₃F •1.25 HCl•1.5 H20: C, 55.55; H, 6.43; N, 8.83; Found: C, 55.40; H, 6.38; N, 8.72.

### Example 342

### 8-((1R*,2R*,6R*)-2-amino-8-azabicyclo[4,3,0]nonan-8-yl))-1-cyclopropyl-7-fluoro-4H-9-methyl-4-oxo-quinolizine-3-carboxylic acid hydrochloride

### Step 342a, 1R*,2R*,6R*-2-BOC-amino-8-azabicyclo[4,3,0]nonane

Removing the N-benzyl group from the 1R*,2R*,6R*-isomer of Example 341 step a, the title compound was prepared.

### Step 341b, 8-((1R*,2R*,6R*)-2-amino-8-azabicyclo[4,3,0]nonan-8-yl))-1-cyclopropyl-7-fluoro-4H-9-methyl-4-oxo-quinolizine-3-carboxylic acid hydrochloride

Following the procedure of Example 253 step j, replacing the 3-BOC-aminopyrrolidine thereof with 1R*,2R*,6R*-2-BOC-amino-8-azabicyclo[4.3.0]nonane, prepared in step 342a above, and carrying the product forward as in Example 253 steps j-l, the title compound was prepared. MS: 400 (M-Cl)⁺; ¹H NMR (DMSO-d6) ∂: 0.53-0.61 (m, 2H), 0.95-1.06 (m, 2H), 1.30 (m,2H), 1.60 (m, 2H), 1.81 (m, 2H), 2.29 (m, 1H), 2.49 (m, 1H), 2.64 (s, 3H), 2.77 (m, 1H), 3.32-3.49 (m, 3H), 4.16 (m, 2H), 7.91 (s, 1H), 8.33 (br, 3H), 9.06 (d, 1H, J=10 Hz). Anal. Calcd for C₂₂H₂₆N₃O₃F•1.0 HCl•1.25 H₂O: C, 57.64; H, 6.49; N, 9.17; Found: C, 57.70; H, 6.80; N, 9.18.

### Example 343

### 8-((1α,5α,6α)-6-amino-3-azabicyclo[3,1,0]hexan-3-yl))-1-cyclopropyl-9-methyl-7-fluoro-4H-4-oxo-quinolizine-3-carboxylic acid hydrochloride

Following the procedure of Example 253 step j, replacing the 3-BOC-aminopyrrolidine thereof with 1α,2α,6α-2-BOC-amino-8-azabicyclo[4.3.0]hexane, prepared according to U.S. Patent 5,298,629, and carrying the product forward as in Example 253 steps j-l, the title compound was prepared. MS: 358 (M-Cl)⁺; ¹H NMR (DMSO-d6) ∂: 0.61 (m, 2H), 1.01 (m, 2H), 2.12 (br s, 2H), 2.33 (m, 1H), 2.62 (s, 3H), 3.81 (m, 5H), 7.97 (s, 1H), 8.46 (br s, 3H), 9.11 (d, 1H, J=10.5 Hz), 13.83 (br, 1H). Anal. Calcd for C₁₉H₂₀N₃O₃F•1.5 HCl•0.5 H₂O: C, 54.19; H, 5.39; N, 9.98; Found: C, 54.43; H, 5.28; N, 9.87.

### Example 344

### 8-(cis-3-amino-4-fluoro-1-pyrrolidinyl))-1-cyclopropyl-9-methyl-7-fluoro-4H-4-oxoquinolizine-3-carboxylic acid hydrochloride

### Step 344a. cis-3-BOC-aminopyrrolidine

1-BOC-3,4-epoxy-pyrrolidine (20g) was dissolved in 200 mL of CH₂Cl₂. MCPBA (50-60% pure, 61.5 g) in 500 mL of CH₂Cl₂ was added to the above solution at 0°C, and the reaction was stirred at 45°C for 18 hours. The reaction mixture was filtered, and the filtrate was treated with NaHSO₃ (ca. 5 g). The solution was then poured into 1 L of 1 N NaOH, the mixture was shaken, and the organic phase was separated, washed with water, dried over MgSO₄ and concentrated. The residue was taken directly to the next step.

### Step 344b. trans-3-azido-1-benzyloxycarboxy-4-hydroxypyrrolidine

The compound from step 344a above was dissolved in 250 mL of acetone. NaN3 (20.16 g) in 200 mL of water was added, and the reaction was stirred at 60°C for 18 hours. The reaction mixture was poured into satd. NaCl solution, and the mixture was extracted (3x) with CH₂Cl₂. The extract was washed with water, dried over MgSO₄ and concentrated. The residue was purified by column chromatography on silica gel, eluting with 3% methanol in CH₂Cl₂ to yield 5.92 g of the product.

### Step 344c. cis-azido-1-benzyloxycarboxy-4-fluoropyrrolidine

The compound from step 344b above was dissolved in 15 mL of CH₂Cl₂ and cooled to -78°C. DAST (0.82 mL) was added, and the reaction was stirred at room temperature for 16 hours. The solvent was removed, the residue dissolved in ethyl acetate, and the solution was washed with satd NaHCO₃, brine, and dried over mgso4. The solvent was removed, and the residue was purified by column chromatography on silica gel, eluting with 1% methanol in CH₂Cl₂ to yield 0.88 g of the tide compound. ¹H NMR (CDCl₃) ∂: 3.62 (m, 4H), 4.22 (br d, 1H, J=11 Hz), 4.99 (br d, 1H, J=51 Hz), 5.16 (s, 2H), 7.36 (m, 5H).

### Step 344d. cis-3-(BOC-amino)-4-fluoropyrrolidine

The compound from step 344c was stirred with Raney Ni in methanol under 4 Atm H₂ for 9 hours. The catalyst was removed by filtration. The filtrate was concentrated, and the residue was treated with (BOC)₂O and the reaction was stirred for 16 hours. The methanol was removed under vacuum, and the residue was extracted with methylene chloride. The extract was washed with water, dried over MgSO₄ and concentrated. The residue was purified by chromatography on silica gel, eluting with 100:5:0.5 methylene chloride:methanol:NH₄OH to give the 1-benzyloxycarboxy compound. This protecting group was removed by hydrogenolysis over Pd/C under H₂ for 30 min. The catalyst was removed, and the filtrate was concentrated to give the title compound (331 mg). MS: 205 (M-Cl)⁺; ¹H NMR (CDCl₃) ∂: 1.46 (s, (H), 2.67 (dd, J=4.5, 12 Hz, 1H), 3.04 (ddd, J=4.5, 14, 36 Hz, 1H), 3.18 (dd, J=14, 25 Hz, 1H), 3.44 (dd, J=7.5, 12 Hz, 1H), 4.08-4.12 (m, 1H), 4.49 (br s, 1H), 4.98 (br d, J=53 Hz, 1H).

### Step 344e. 8-(cis-3-amino-4-fluoro-1-pyrrolidinyl))-1-cyclopropyl-9-methyl-7-fluoro-4H-4-oxo-quinolizine-3-carboxylic acid hydrochloride

Following the procedure of Example 253 step j, replacing the 3-BOC-aminopyrrolidine thereof with the compound from step 344d above, and carrying the product forward as in Example 253 steps j-l, the title compound (44 mg) was prepared. MS: 364 (M-Cl)⁺; HRMS: calc for C₁₈H₁₉N₃O₃F₂ (M_Cl)⁺: 364.1473; found: 364.1480. ¹H NMR (DMSO-d₆) ∂: 0.62 (m, 2H), 1.00 (m, 2H), 2.36 (m, 1H), 2.68 (s, 3H), 3.77 (m, 1H), 3.93 (m, 1H), 4.11 (m, 1H), 4.31-4.41 (m, 1H), 5.50 (br d, J=51 Hz, 1H), 7.99 (s, 1H), 8.69 (br s, 3H), 9.16 (d, J=9 Hz, 1H), Anal. Calcd for C₁₈H₁₉N₃O₃F₂•1.3 HCl•2.0 H₂O: C, 48.39; H, 5.48; N, 9.40; Found: C, 48.12; H, 5.58; N, 9.63.

### Example 345

### 1-cyclopropyl-7-fluoro-4H-8-(1-homopiperazinyl))-9-methyl-4-oxo-quinolizine-3-carboxylic acid, acetic acid salt

Following the procedure of Example 298, replacing the 3-(dimethylamino)pyrrolidine thereof with the homopiperazine, the title compound was prepared. mp 195-198°C (dec.). MS: 360 (M+H)⁺; ¹H NMR (DMSO-d₆) ∂: 0.55 (m, 2H), 0.98 (m, 2H), 1.83 (s, 6H), 2.26-2.38 (m, 2H), 2.69 (br s, 3H), 2.89 (m, 4H), 8.08 (br s, 1H), 9.04 (br s, 1H).

### Example 346 is missing due to its deletion

### Example 347

### Scaled-Up Preparation of 8-(3(S)-aminopyrrolidinyl)-1-cyclopropyl-7-fluoro-4H-9-methyl-4-oxo-quinolizine-3-carboxylic acid hydrochloride

### Step 347a. 4-t-butoxy-3-chloro-2,5,6-trifluoropyridine

A 927.55 g (5.0 mol) sample of 3-chloro-2,4,5,6-tetrafluoropyridine (from Fluorochem Ltd.) was dissolved in 4 L of anhydrous THF, and the solution was cooled to -10°C. To this solution was added 429 (5.36 mol) of lithium t-butoxide in portions over a 1-hr period, while maintaining the temperature between -5°C to -10°C. The reaction was stirred for 2 hours at -10°C, the cooling bath was removed, and the solution was warmed to room temperature over a 3 hours period. The THF was removed under reduced pressure. The residue was dissolved in 6 L of ether, and the solution was washed with 4x1 L of water. The ether solution was dried over MgSO4, and the ether was removed under reduced pressure to give 1123.44 g of the crude product. The crude product was purified by chromatography, eluting with hexane. bp 43-47°C/0.6 mm Hg.

### Step 347b. 4-t-butoxy-3-methyl-2,5,6-trifluoropyridine

A 499 g (2.08 mol) sample of the compound from step 347a above was dissolved in 4 L of THE and cooled to -70°C. While maintaining a N₂ atmosphere, 1.6 L of sec-butyllithium (2.08 mol, 1.3 M) was added, and the reaction mixture was stirred for 1 hour. Iodomethane (129.6 mL, 2.08 mol) was added rapidly dropwise, while maintaining the temperature below -50°C. The mixture was stirred while allowing the temperature to rise, and the stirring was continued for 16 hours. The reaction was quenched with 1 L of water while cooling with an ice bath, then 2 L of hexane were added, the phases mixed well, and the layers separated. The organic layer was concentrated on a rotary evaporator. The residue was dissolved in hexane, dried over MgSO4, filtered and concentrated to give 496 g of title compound, which was taken directly to the next step.

### Step 347c. 4-t-butoxy-2,5-difluoro-3-methylpyridine

Lithium aluminum hydride (56.7 g, 1.42 mol) was added to 6 L of THF, and the suspension was stirred under N₂. The temperature was adjusted to 0 to -5°C, and 476.5 g (2.27 mol) of the compound from step 347b above (dissolved in 750 mL of THF) was added in a stream over a 15 min period. The mixture was stirred at room temperature for 16 hours, then 500 mL of hexane was added. The reaction was then quenched while maintaining an internal temperature of 10-20°C by adding 57 mL of H₂O, 57 mL of 15% NaOH solution, and 171 mL of H₂O, in that order. The mixture was filtered, and the filter cake was washed with THF and hexane. The filtrate was concentrated on a rotary evaporator with a bath temperature of 35°C. The residue was purified by column chromatography on silica gel, eluting with hexane and 5% ethyl acetate in hexane to afford 141 g of the title compound. Distillation at 80-90°C and 1 mm Hg gave 103.4 g of the pure product.

### Step 347d. Alternate preparation of 4-t-butoxy-2,5-difluoro-3-methylpyridine

A 476.5 g (2.27 mmol) sample of the compound from step 347b above was dissolve in 6 L of THF and stirred under N₂. The temperature of the solution was adjusted to 0 to 5°C, and a solution of sodium bis-(2-methoxyethoxy)aluminum hydride in toluene (750 mL, 3.4 M, 2.5 mol) was added rapidly dropwise over 1 hour. The reaction mixture was stirred at room temperature for 16 hours, and 500 mL of hexane was added. The reaction was then quenched while maintaining an internal temperature of <25°C by careful addition of 500 mL of H₂O. The organic layer was decanted, and the solids were washed thoroughly with hexane. The solvents were combined and concentrated on a rotary evaporator, with a bath temperature of 55°C. The 440 g of crude product was twice purified by chromatography over silica gel, eluting with hexane and 3% ethyl acetate in hexane to give 137.5 of the pure title compound.

### Step 347e. 2-(4-t-butoxy-5-fluoro-3-methyl-2-pyridinyl)-2-cyclopropylacetonitrile

Diisopropylamine (445 mL, 3.18 mol) was dissolved in 1.5 L of anhydrous THF and stirred under N₂. The solution was cooled to -40°C, and n-butyllithium (1.274 L, 3.18 mole, 2.5 M in hexane) was added at a rate such that the internal temperature was maintained at -40 to -20°C. The solution was warmed to -10°C, then cooled to -70°C. Cyclopropylacetonitrile (257 g, 3.17 mmol) was added dropwise to maintain the temperature below -68°C, then the solution was stirred for 35 min. A sample of 4-t-butoxy-2,5-difluoro-3-methylpyridine, from step 347c or 347d above, was dissolved in 1.2 L of anhydrous THF. To this solution was added in a dropwise manner the first solution containing the lithium salt of cyclopropylacetonitrile, at a rate that the internal temperature remained below -70°C. The solution was stirred at -78°C for 1 hour, then allowed to warm to 0°C. The reaction was quenched by adding 1 L of satd aq. NH₄Cl solution and 1L of H₂O. The organic layer was separated. The aqueous layer was extracted with ethyl acetate. The organic layers were combined, washed with brine, dried with MgSO4, and concentrated on a rotary evaporator to give an oil residue. The oil was distilled at 0.2 mm Hg at 25-35°C to remove low boiling impurities and residual cyclopropylacetonitrile. The residue was twice chromatographed on silica gel, eluting with 7% ethyl acetate in hexane to afford 646 g of pure title compound. MS: 263 (M+H)⁺; ¹H NMR (CDCl₃) ∂: 0.50 (m, 2H), 0.64 (m, 1H), 0.75 (m, 1H), 1.43 (d, J= 1.5 Hz, 9H), 1.50 (m, 1H), 2.29 (s, 3H), 3.76 (d, J=7.5 Hz, 1H), 8.31 (s, 1H).

### Step 347f. 2-(4-chloro-5-fluoro-3-methyl-2-pyridinyl)-2-cyclopropylacetonitrile

To a cooled (0°C) solution of the compound from step 347e above (189.78 g, 0.72 mol) in 1.6 L of CH₂Cl₂ and 270 mL of DMF was added 300 mL (3.2 mol) of POCl₃, and the reaction was stirred for 12 hours. Another 25 mL (0.27 mol) of POCl₃ was added, and the reaction stirred for an additional 12 hours. The reaction mixture was then poured into H₂O, and this mixture was stirred for 1 hour. The organic material was extracted with CH₂Cl₂, which was washed with H₂O, sat aq NaHCO₃ solution, H₂O, dried over MgSO4, filtered and evaporated under vacuum to afford 129.3 g of the title compound as an oil. MS: 225, 227 (M+H)⁺, 191. ¹H NMR (CDCl₃) ∂: 0.48 (m, 1H), 0.58 (m, 1H), 0.66 (m, 1H), 0.77 (m, 1H), 1.50 (m, 1H), 2.49 (s, 3H), 3.80 (d, J=8 Hz, 1H), 8.39 (s, 1H).

### Step 347g. 2-(4-chloro-5-fluoro-3-methyl-2-pyridinyl)-2-cyclopropylacetic acid, ethyl ester

To 1L of ethanol saturated with ca. 400 g HCl gas and stirred under N₂ and cooled to 0°C was added a solution of 135.8 (0.6 mol) of the compound from step 347f in 90 mL of ethanol, and the reaction was stirred for 3 hours at 0°C. To this solution was added 90 mL of H₂O, and the reaction mixture was heated at 80°C for 2 hours. The mixture was poured over ice to make a total volume of 4 L. The solution was neutralized with 50% NaOH to pH 8 while maintaining the temperature below 0°C. The solid was filtered off, redissolved in CH₂Cl₂, and the residual water layer removed. The organic layer was dried over MgSO₄ and evaporated to leave a tan solid (134.4 g). MS: 272 (M+H)⁺; ¹H NMR (CDCl₃) ∂: 0.12 (m, 1H), 0.38 (m, 1H), 0.54 (m, 1H), 0.75 (m, 1H), 1.20 (t, J=7.5 Hz, 3H), 1.68 (m, 1H), 2.40 (s, 3H), 3.24 (d, J=9.3 Hz, 1H), 4.16 (q, J=7.5 Hz, 2H), 8.36 (s, 1H).

### Step 347h. 2-(4-chloro-5-fluoro-3-methyl-2-pyridinyl)-2-cyclopropylethanol

A solution of the compound from step 347g above (130.72 g, 0.48 mol) in 530 mL of anhydrous THF was stirred under N₂ at -78°C. To this was added a solution of LiAlH₄ (480 mL, 1 M in THF, 0.48 mol) dropwise while maintaining the temperature below -60°C. The reaction was stirred at -78°C for 2 hours. The reaction was quenched by addition of H₂O (16 mL). 15% NaOH (16 mL and H₂O (46 mL), and the mixture was stirred for 1 hour at room temperature. The solid was removed by filtration and washed with ether. The combined organic were washed with brine, dried over MgSO4, filtered and evaporated under vacuum to afford the title compound (108.6 g) as a white solid. MS: 230 (M+H)⁺, 196; ¹H NMR (CDCl₃) ∂: 0.21 (m, 2H), 0.44 (m, 1H), 0.60 (m, 1H), 1.21 (m, 1H), 2.39 (s, 3H), 2.56 (m, 1H), 3.52 (br s, 1H), 4.02 (m, 2H), 8.31 (s, 1H).

### Step 347i. 2-(4-chloro-5-fluoro-3-methyl-2-pyridinyl)-2-cyclopropylacetaldehyde

Anhydrous DMSO (80 mL, 1.14 mol) was dissolved in 900 mL of anhydrous CH₂Cl₂, and stirred under N₂. The solution was cooled to -78°C, and a solution of oxalyl chloride (2.0 M, 284 mL, 0.569 mol) in CH₂Cl₂ was added over a 20 min period while holding the internal temperature below -60°C and stirred for 35 min longer. The compound from step 346h (109 g, 0.475 mol) was dissolved in 400 mL of anhydrous CH₂Cl₂ and added dropwise to the first solution, while holding the internal temperature below -60°C. The reaction mixture was stirred for 30 min, and triethylamine (327 mL, 2.34 mol) was added dropwise over 10 min. The reaction was stirred as the internal temperature was raised to -10°C. The reaction was quenched with 500 mL of H₂O, and the organic layer was isolated, washed with H₂O, dried over MgSO₄ and evaporated to give 109.64 g of the title compound. MS: 228 (M+H)⁺; ¹H NMR (CDCl₃) ∂: 0.24 (m, 1H), 0.35 (m, 1H), 0.59 (s, 1H), 0.76 (m, 1H), 1.55 (m, 1H), 2.38 (s, 3H), 3.19 (dd, J=2.7, 9 Hz, 1H), 8.37 (s, 1H), 9.87 (d, J=2,7 Hz, 1H).

### Step 347j. 4-(4-chloro-5-fluoro-3-methyl-2-pyridinyl)-4-cyclopropyl-2-ethoxycarbonyl-2-butenoic acid ethyl ester

The compound from step 347i above (109.68 g, 0.48 mol) was dissolved in 1.3 L of absolute ethanol and stirred under N₂. To this solution was added diethylmalonate (351 mL, 2.31 mol), piperidine (45.5 mL, 0.46 mol) and acetic acid (45.5 mL, 0.79 mol). The solution was heated at reflux for 8 hours and cooled to room temperature. The solvent was removed with a rotary evaporator, and the residue was dissolved in ethyl acetate. This solution was washed with water, brine, dried over MgSO₄ and concentrated to give an oily residue. The residue was distilled in a short-path distillation apparatus at 0.2 mm Hg and 25-56°C to remove excess diethyl malonate and volatile impurities. The residual oil was taken directly to the next step.

### Step 347k. 8-chloro-1-cyclopropyl-7-fluoro-4H-9-methyl-4-oxo-quinolizine-3-carboxylic acid, ethyl ester

The compound from step 347j above was dissolved in 400 mL of anhydrous DMSO and heated at reflux for 1 hour. The hot reaction mixture was slowly poured into rapidly stirred ice water (3 L). The product was filtered off and washed with water (3L) and hexane (1.5 L). The product was dried in a vacuum oven for 16 hours to afford 105 g of the title compound as a yellow crystalline solid. MS: 324 (M+H)⁺; ¹H NMR (CDCl₃) ∂: 0.75 (m, 2H), 1.06 (m, 2H), 1.43 (t, 3H), 2.32 (m, 1H), 3.09 (s, 3H), 4.43 (q, 2H), 8.39 (s, 1H), 9.43 (dd, J=1, 6 Hz, 1H).

### Step 347l. 8-(3(S)-(BOC-amino)pyrrolidinyl)-1-cyclopropyl-7-fluoro-4H-9-methyl-4-oxoquinolizine-3-carboxylic acid, ethyl ester

A 93.1 g (0.29 mmol) sample of the compound from step 347k above was dissolved in 1.24 L of acetonitrile, and 137 g (0.72 mol) of 3(S)-(BOC-amino)pyrrolidine and 113 g (1.45 mol) of NaHCO₃ were added. The mixture was heated at reflux under N₂ for 1 hour. The reaction mixture was cooled to 25°C, and 700 mL of H₂O were added. The mixture was extracted with ethyl acetate, and the solvent was washed with water, 1N HCl, water and brine. The solvent was dried over MgSO₄ and concentrated to a thick tar. MS: 474 (M+H)⁺; ¹H NMR (CDCl₃) ∂: 0.60 (m, 2H)0.95 (m, 2H), 1.41 (t, 3H), 1.42 (m, 2H), 1.46 (s, 9H), 2.60 (s, 3H), 3.50 (m, 1H), 3.82 (m, 1H), 3.95 (m, 1H), 4.49 (q, 2H), 4.79 (br s, 1H), 8.2 (s, 1H), 9.25 (d, 1H).

### Step 347m. 8-(3(S)-(BOC-amino)pyrrolidinyl)-1-cyclopropyl-7-fluoro-4H-9-methyl-4-oxoquinolizine-3-carboxylic acid

The material from step 3471 above was dissolved in 900 mL of THF, and 550 mL of water and 107.5 g (2.56 mol) of LiOH•H₂O were added. The mixture was heated at reflux under N₂ for 1 hour. The mixture was diluted by pouring into a mixture of 1 L of THF and 0.5 L of water, with addition of ice to assist cooling. Conc. HCl was added with vigorous mixing to bring the acidity to pH 4, while holding the internal temperature below 15°C. The yellow precipitate was filtered off, then dissolved in CH₂Cl₂. The solution was washed with water until the washings tested neutral, then dried over MgSO₄ and concentrated. MS: 446 (M+H)⁺; ¹H NMR (CDCl₃) ∂: 0.69 (m, 2H), 1.02 (m, 2H), 1.48 (s, 9H), 2.12 (m, 2H), 2.30 (m, 1H), 2.62 (s, 3H), 3.60 (m, 1H), 3.79 (m, 1H), 3.96 (m, 2H), 4.38 (br s, 1H), 5.11 (br s, 1H), 8.13 (s, 1H), 8.99 (d, 1H), 13.82 (s, 1H).

### Step 347n. 8-(3(S)-amino)pyrrolidinyl)-1-cyclopropyl-7-fluoro-4H-9-methyl-4-oxoquinolizine-3-carboxylic acid hydrochloride

A 140 g sample of the compound from step 347m above was dissolved in 1.2 L of CH₂Cl₂, and 1.0 L of 1.0 M HCl in acetic acid was added over 5 min. The mixture was stirred under N₂ for 1 hour at room temperature. The product was collected by filtration and washed with CH₂Cl₂ until colorless. The solid was dried in a vacuum oven (50°C, 10 mm Hg) for 48 hours. This material (307.45 g) was added to 3.8 L of absolute ethanol prewarmed to 70°C. To the mixture was added 1.23 L of H₂O, and the mixture was heated to boiling and stirred until all solid dissolved. Stirring was discontinued, seed crystals were added, and the solution allowed to cool to room temperature. The mixture was then cooled at 0°C for 12 hours and at -25°C with stirring for 2 hours. The product was filtered off and washed with chilled absolute ethanol. The solid was dried in vacuum for 48 hours to give the title product (261 g) as a yellow solid. MS: 346 (M-Cl)⁺; ¹H NMR (CD₃OD) ∂: 0.69 (m, 2H), 1.06 (m, 2H), 2.26 (m, 2H), 2.52 (m, 1H), 2.73 (s, 3H), 3.88 (m, 2H), 4.05 (m, 2H), 4.18 (m, 1H), 4.88 (br s, 1H), 8.03 (s, 1H), 9.02 (d, J=10.8 Hz, 1H).

### Example 348

### 8-(spiro-1,3-dioxacyclopentane[2.3]-1-piperidinyl)-1-cyclopropyl-7-fluoro-4H-9-methyl-4-oxo-quinolizine-3-carboxylic acid

### Step 348a. N-CBZ-3-hydroxypiperidine

A sample of 3-hydroxypiperidine HCl (50.0 g) was dissolved in a small amount of water and the solution was cooled to 0°C in an ice bath. The HCL was neutralized by slow addition of 363 mL of 1 M NaOH. An additional 1.2 eq of 1 M NaOH was added quickly, and 52 mL of benzyl chloroformate in 20 mL of ether was added dropwise, then the solution was stirred for 4 hours at 0°C. The solution was diluted with 600 mL of water and extracted with methylene chloride. The organic extract was dried over Na₂SO₄, filtered, and taken to dryness to afford 89.2 g of the title compound.

### Step 348b. N-CBZ-3-oxo-piperidine

A 30.0 g sample of N-CBZ-3-hydroxypiperidine, from step 348a above, was dissolved in 250 mL of DMSO, and the solution was cooled to 0°C. To this solution, stirred at 0°, was added 142 mL of triethylamine, and next was added dropwise a solution of 60.88 g of pyridine•SO₃ complex dissolved in 250 mL of DMSO. The cooling bath was removed, and the reaction mixture was stirred at room temperature for 20 hours. The reaction mixture was diluted with water, and the mixture was extracted with methylene chloride. The extract was dried over Na₂SO₄, filtered, and taken to dryness. The DMSO was distilled off under reduced pressure, and the residue purified by distillation in a kugelrohr apparatus to yield 26.53 g of the title compound.

### Step 348c. spiro-1,3-dioxacyclopentane[2.3]piperidine

A 10.0 g sample of N-CBZ-3-oxo-piperidine, from step 348b above, was dissolved in 10 mL of toluene and 5.98 mL of ethylene glycol and 0.408 g of p-toluenesulfonic acid were added. The solution was stirred at 130°C for 96 hours, then poured into 5% NaHCO₃ solution. The mixture was extracted with methylene chloride, the extract was dried over Na₂SO₄, then the solvent was removed under vacuum and the residue was distilled in a kugelrohr apparatus to give 7.30 g of the title compound.

### Step 348d. 8-(spiro-1,3-dioxacyclopentane[2.3]-1-piperidinyl)-1-cyclopropyl-7-fluoro-4H-9-methyl-4-oxo-quinolizine-3-carboxylic acid

Following the procedure of Example 253 step j, replacing the 3-BOC-aminopyrrolidine thereof with spiro-1,3-dioxacyclopentane[2.3]piperidine, from step 348c above, and carrying the product forward as in Example 253 steps j-k, the title compound was prepared (245 mg). mp 184-187°C. MS: 403 (M+1)⁺; ¹H NMR (CDC13) ∂: 0.69 (m, 2H), 1.03 (m, 2H), 1.88 (m, 2H), 1.99 (m, 2H), 2.28 (m, 1H), 2.82 (s, 3H), 3.35 (m, 4H), 3.97 (m, 4H), 8.36 (s, 1H), 9.20 (d, 1H, J=3 Hz), 13.91 (s, 1H). Anal. Calcd for C₂₁H₂₃N₂O₅F•0.5 H₂O: C, 61.31; H, 5.88; N, 6.81; Found: C, 61.41; H, 5.91; N, 6.62.

### Example 349

### 8-(3-amino-4-methoxypyrrolidinyl)-1-cyclopropyl-7-fluoro-4H-9-methyl-4-oxo-quinolizine-3-carboxylic acid hydrochloride

### Step 349a. N-CBZ-pyrroline

A 50.0 g sample of pyrroline (Aldrich) was dissolved in 868 mL of 1M NaOH, and the solution was cooled to 0°C. Benzyl chloroformate (103.29 mL) was dissolved in 100 mL of ether and added to the solution of pyrroline dropwise over a 1 hour period. The solution was stirred for 4 hours at 0°C, then diluted with 500 mL of water and extracted with methylene chloride. The extracts were combined, dried of Na₂SO₄, filtered, and evaporated to dryness to yield 144.6 g of the title compound.

### Step 349b. N-CBZ-3,4-epoxv-pyrrolidine

In a dry system under N₂ a 15.0 g sample of N-CBZ-pyrroline, from step 349a above, was dissolved in 200 mL of methylene chloride, and the solution was cooled to 0°C. To this solution was added 46.3 g of m-chloroperbenzoic acid dissolved in 500 mL of methylene chloride dropwise over a 1 hour period. The reaction mixture was then heated at 45°C for 18 hours, then recooled to 0°C. To the cool solution was added 3 g of sodium bisulfite, and the mixture was stirred for 1 hour and poured into 1 L of 1 N NaOH. The organic layer was washed with water, dried over Na₂SO₄, filtered and evaporated to afford 14.5 g of the title compound.

### Step 349c. N-CBZ-3-azido-4-hydroxy-pyrrolidine

A 16.18 g sample of N-CBZ-3,4-epoxy-pyrrolidine was dissolved in 145 mL of acetone. A 14.39 g sample of sodium azide was dissolved in 130 mL of water and added to the acetone solution. The reaction mixture was stirred at 60°C for 16 hours, then poured into 400 mL of satd. NaCl solution. The quenched reaction mixture was extracted with methylene chloride, which was dried over Na₂SO₄, filtered and evaporated. The residue was purified by flash chromatography over silica gel to afford 21.40 g of the title compound.

### Step 349d. N-CBZ-3-azido-4-methoxy-pyrrolidine

A 3.36 g sample of NaH was suspended in 60 mL of THF in a dry flask under N₂ and cooled to 0°C. A 20.0 g sample of N-CBZ-3-azido-4-hydroxy-pyrrolidine, from step 349c above, was dissolved in 200 mL of THF, and this solution was added dropwise to the suspension of NaH. The reaction mixture was stirred for 30 min at 0°C, 30 min at room temperature, and recooled to 0°C. To this solution was added dropwise a solution of 5.70 mL of methyl iodide in 60 mL of THE. The reaction mixture was stirred at 0°C for 30 min and at room temperature for 23.5 hours. The reaction mixture was poured into 500 mL of 5% NH₄Cl solution, and the mixture was extracted with methylene chloride. The extract was dried over Na₂SO₄, filtered and evaporated. The residue was purified by flash chromatography over silica gel to afford 8.99 g of the title compound.

### Step 349e. N-CBZ-3-amino-4-methoxy-pyrrolidine

A 8.98 g sample of N-CBZ-3-azido-4-methoxy-pyrrolidine, from step 349d above, was dissolved in 100 mL of methanol and hydrogenated at room temperature under 4 Atm of H₂ in the presence of 6.8 g of RaNi for 4 days in a sealed bomb. The catalyst was removed by filtration, and the methanol was evaporated. The residue was dissolved in methylene chloride, dried over Na₂SO₄, and filtered. The solvent was removed to yield 5.60 g of the title compound.

### Step 349f. N-CBZ-3-(BOC-amino)-4-methoxy-pyrrolidine

A 5.60 g sample of N-CBZ-3-(BOC-amino)-4-methoxy-pyrrolidine was dissolved in 120 mL of methylene chloride in a dry flask under N₂ and cooled to 0°C. To this were added 6.61 mL of triethylamine and 7.76 g of di-t-butyl dicarbonate dissolved in 50 mL of methylene chloride (dropwise). The reaction mixture was stirred at )°C fro 1 hour and at room temperature for 24 hours. The reaction was quenched by addition to water. The mixture was extracted with methylene chloride. The extract was dried over Na₂SO₄, filtered and evaporated to yield 6.88 g of crude product. The residue was purified by flash chromatography over silica gel to afford 1.97 g of pure title compound.

### Step 349g. 3-(BOC-amino)-4-methoxy-pyrrolidine

A 1.97 g sample of N-CBZ-3-(BOC-amino)-4-methoxy-pyrrolidine, from step 349f above, was hydrogenated over 0.2 g of 10% Pd/C in 100 mL of methanol under 4 Atm of H₂ at room temperature for 24 hours. The catalyst was removed by filtration, the solvent was removed to yield 1.28 g of title compound.

### Step 349h. 8-(3-amino-4-methoxypyrrolidinyl)-1-cyclopropyl-7-fluoro-4H-9-methyl-4-oxoquinolizine-3-carboxylic acid hydrochloride

Following the procedure of Example 253 step j, replacing the 3-BOC-aminopyrrolidine thereof with 3-(BOC-amino)-4-methoxypyrrolidine, from step 349g above, and carrying the product forward as in Example 253 steps j-1, the title compound was prepared (369 mg). MS: 376 (M+1)⁺; ¹H NMR (CD₃OD) ∂: 0.71 (m, 2H), 1.88 (m, 2H), 2.30 (m, 1H), 2.74 (s, 3H), 3.51 (s, 3H), 3.84 (m, 2H), 3.98 (m, 1H), 4.24 (m, 3H), 8.02 (s, 1H), 9.02 (d, 1H, J=3.5 Hz). Anal. Calcd for C₁₉H₂₃N₃O₄ClF•4 H₂O: C, 46.16; H, 6.46; N, 8.68; Found: C, 47.53; H, 6.06; N, 9.36.

### Example 350

### 8-(4-amino-4-methylpyrrolidinyl)-1-cyclopropyl-7-fluoro-4H-9-methyl-4-oxo-quinolizine-3-carboxylic acid hydrochloride

### Step 350a. N-CBZ-4-hydroxypiperidine

A 35.43 g of 4-hydroxypiperidine was suspended in 420 mL of 1 M NaOH, and cooled to 0°C. To this stirred solution was added 50.0 mL of benzyl chloroformate dissolved in 100 mL of ether dropwise over a 1 hour period. The reaction mixture was stirred for 3 hours, diluted with 200 mL of water, and extracted with methylene chloride. The extract was dried over Na₂SO₄, filtered and evaporated to afford the title compound.

### Step 350b. N-CBZ-4-oxopiperidine

A 43.1 g sample of N-CBZ-4-hydroxypiperidine, from step 350a above, was dissolved in 370 mL of DMSO in a dry flask under N₂ and cooled to 0°C. To this solution was added 204 mL of triethyl amine, then a solution of 87.5 g of pyridine•SO₃ in 370 mL of DMSO was added dropwise over a period of 1 hour. The reaction was stirred for 24 hours at room temperature, then quenched by addition to 1 L of NaCl solution. The mixture was extracted with methylene chloride. The extract was dried over Na₂SO₄, filtered and evaporated. The residue was chromatographed of a silica gel column to afford 11.49 g of the title compound.

### Step 350c. N-CBZ-4-hydroxy-4-methylpiperidine

A 58 mL sample of methyl magnesium bromide was placed into a dry flask under N₂ containing 450 mL of dry ether cooled to -20°C. A 25.00 g sample of N-CBZ-4-oxopiperidine, from step 350b above, was dissolved in 100 mL of dry ether and added to the reaction vessel dropwise over a 1 hour period. The reaction mixture was stirred for 1 hour, then warmed to room temperature over a 2.5-hour period. The reaction was quenched by dropwise addition of an excess of satd NH₄Cl solution. The layers were separated, and the aqueous layer was extracted with ether. The organic layers were combined, dried over Na₂SO₄, filtered and evaporated. The residue was distilled in a kugelrohr apparatus to yield 44.3 g of the title compound.

### Step 350d. N-CBZ-4-(acetylamino)-4-methylpiperidine

A solution of 270 mL of 90% sulfuric acid and 34 mL of acetonitrile was prepared and cooled to 0°C. A 44.3 g sample of N-CBZ-4-hydroxy-4-methylpiperidine, from step 350c above, dissolved in acetonitrile was added dropwise to the stirred solution in the reaction vessel over a 2 hours period. The reaction mixture was stirred an additional 45 min at 0°C and 2.5 hours without cooling. The reaction mixture was poured over 1 kg of ice, and the mixture was adjusted to pH 12-13 with 50% NaOH. This mixture was extracted with ethyl acetate. The organic layers were combined, dried over Na₂SO₄, filtered and evaporated to give the title compound (101.5 g) as a white solid.

### Step 350e. N-CBZ-4-amino-4-methylpiperidine

A 53 g sample of N-CBZ-4-(acetylamino)-4-methylpiperidine, from step 350e above, was dissolved in 202 mL of 12 M HCl and heated at 115°C for 90 hours. The reaction mixture was poured over 800 g of ice. This mixture was extracted with methylene chloride. The organic layers were combined, dried over Na₂SO₄, filtered and evaporated to give 37.6 g of the title compound.

### Step 350f. N-CBZ-4-(BOC-amino)-4-methylpiperidine

In a dry flask under N₂ a 37.6 g sample of N-CBZ-4-amino-4-methylpiperidine, from step 350e above, was dissolved in 220 mL of CCl4, 51.3 mL of triethylamine was added, and 52.2 g of di-t-butyl dicarbonate was added in small portions. The solution was stirred at 38°C for 20 hours, then washed with water. The organic solvent was dried over Na₂SO₄, filtered and evaporated to give 23.71 g of title compound.

### Step 350g. 4-(BOC-amino)-4-methylpiperidine

A 23.71 g sample of N-CBZ-4-(BOC-amino)-4-methylpiperidine, from step 350f above, was hydrogenated as described in Example 349g above to give 15.7 g of title compound.

### 350h. 8-(4-amino-4-methylpyrrolidinyl)-1-cyclopropyl-7-fluoro-4H-9-methyl-4-oxoquinolizine-3-carboxylic acid hydrochloride

Following the procedure of Example 253 step j, replacing the 3-BOC-aminopyrrolidine thereof with 4-(BOC-amino)-4-methylpyrrolidine (Aldrich) and carrying the product forward as in Example 253 steps j-l, the title compound was prepared (513 mg). mp 205-207°C. MS: 374 (M+1)⁺; ¹H NMR (CD₃OD) ∂: 0.71 (m, 2H), 1.08 (m, 2H), 1.54 (s, 3H), 2.00 (m, 4H), 2.38 (m, 1H), 2.87 (s, 3H), 3.60 (m, 4H), 8.20 (s, 1H), 9.27 (d, 1H, J=3 Hz). Anal. Calcd for C₂₀H₂₅N₃O₃ClF•3 H₂O: C, 51.78; H, 6.73; N, 9.06; Found: C, 51.64; H, 6.39; N, 9.01.

### Example 351

### 8-(4-(2-hydroxyethyl)piperidinyl)-1-cyclopropyl-7-fluoro-4H-9-methyl-4-oxo-quinolizine-3-carboxylic acid

Following the procedure of Example 253 step j, replacing the 3-BOC-aminopyrrolidine thereof with 1-piperidineethanol, obtained from Aldrich, and carrying the product forward as in Example 253 steps j-k, the title compound was prepared (270 mg). MS: 389 (M+1)⁺; ¹H NMR (CD₃OD) ∂: 0.73 (m, 2H), 1.09 (m, 2H), 2.40 (m, 1H), 2.93 (s, 3H), 3.42 (m, 4H), 3.54 (m, 1H), 3.75 (m, 2H), 3.78 (m, 4H), 3.96 (m, 2H), 8.29 (s, 1H), 9.32 (d, 1H, J=3.3). Anal. Calcd for C₂₀H₂₄N₃O₄F•2.5 H₂O: C, 55.29; H, 6.73; N, 9.67; Found: C, 55.08; H, 6.02; N, 9.56.

### Example 352

### 8-(4-(methoxymethyl)piperidinyl)-1-cyclopropyl-7-fluoro-4H-9-methyl-4-oxo-quinolizine-3-carboxylic acid

### Step 352a. N-CBZ-4-methoxymethoxypiperidine

A 4.00 g sample of N-CBZ-4-hydroxypiperidine, prepared as in Example 350a above, was dissolved in 45 mL of methylene chloride, and 11.85 mL of diisopropylethylamine was added. To this solution was then added 3.87 mL of chloromethyl methyl ether dropwise over 10 min. The reaction mixture was stirred at room temperature for 17 hours, diluted with 50 mL of methylene chloride, and washed with 0.5 M phosphoric acid, 5% NaHCO₃ and water. The solvent was dried over Na₂SO₄, filtered and evaporated to give 4.43 g of the title compound.

### Step 352b. 4-methoxymethoxypiperidine

A 4.43 g sample of N-CBZ-4-methoxymethoxypiperidine, from step 352a above, was hydrogenated as described in Example 349g above to give 2.15 g of title compound.

### Step 352c. 8-(4-(methoxymethyl)piperidinyl)-1-cyclopropyl-7-fluoro-4H-9-methyl-4-oxoquinolizine-3-carboxylic acid

Following the procedure of Example 253 step j, replacing the 3-BOC-aminopyrrolidine thereof with 4-methoxymethylpiperidine, from step 352b above, and carrying the product forward as in Example 253 steps j-k, the title compound was prepared (270 mg). mp 128-130°C. MS: 405 (M+1)⁺; ¹H NMR (CD₃OD) ∂: 0.69 (m, 2H), 1.03 (m, 2H), 1.68 (m, 3H), 1.98 (m, 1H), 2.12 (m, 1H), 2.27 (m, 1H), 2.79 (s, 3H), 3.28 (m, 1H), 3.37 (m, 3H), 3.65 (m, 1H), 3.79 (m, 1H), 4.71 (m, 2H), 8.38 (s, 1H), 9.20 (d, 1H, J=12 Hz), 13.88 (s, 1H). Anal. Calcd for C₂₁H₂₅N₂O₅F•0.5 H₂O: C, 61.02; H, 6.11; N, 6.87; Found: C, 61.01; H, 6.34; N, 6.78.

### Example 353

### 8-(3-amino-3-methylpiperidinyl)-1-cyclopropyl-7-fluoro-4H-9-methyl-4-oxo-quinolizine-3-carboxylic acid hydrochloride

### Step 353a. N-benzyl-3-hydroxy-3-methylpiperidine

To a dried system under N₂ was added 400 mL of dry ether and 32.2 mL of methyl magnesium bromide, and the solution was cooled to -30°C. To this solution was added dropwise a solution of 16.626 g of N-benzyl-3-piperidone (Aldrich) in 50 mL of dry ether. The reaction mixture was then stirred at room temperature for 4 hours. The reaction was quenched by dropwise addition of satd NH₄Cl solution with cooling until the suspended solid separated. An additional 300 mL of 10% NH₄Cl solution was then added, and the layers were separated. The aqueous layer was washed with ether, the organic solution and extracts were combined, dried over Na₂SO₄, filtered and evaporated. The residue was distilled in a kugelrohr apparatus to give 17.942 g of the title compound.

### Step 353b. N-benzyl-3-(acetylamino)-3-methylpiperidine

A 21.961 g sample of N-benzyl-3-hydroxy-3-methylpiperidine, prepared as in step 353a above, was dissolved in 16.8 mL of acetonitrile and added dropwise over 1.5 hours to 134 mL of vigorously stirred 90% sulfuric acid cooled to 0°C. The reaction mixture was stirred for an additional 15 min at 0°C, and at room temperature for 6 hours. The reaction was quenched by pouring the reaction mixture over ice. This solution was adjusted to pH 12 with 50% NaOH solution and was then extracted with methylene chloride. The extract was dried over Na₂SO₄, filtered and evaporated to yield 19.2 of the title compound.

### Step 353c. N-benzyl-3-amino-3-methylpiperidine

The sample of N-benzyl-3-(acetylamino)-3-methylpiperidine from the previous step was stirred with 100 mL of conc. HCl at 110°C for 36 hours. The reaction mixture was poured over 800 g of ice. This mixture was extracted with methylene chloride. The organic layers were combined, dried over Na₂SO₄, filtered and evaporated to give the title compound.

### Step 353d. N-benzyl-3-(BOC-amino)-3-methylpiperidine

The N-benzyl-3-amino-3-methylpiperidine of the previous step was reacted with di-t-butyl dicarbonate according to the procedure of Example 350f above, and the title compound was isolated.

### Step 353e. 3-(BOC-amino)-3-methylpiperidine

A 3.32 g sample of N-benzyl-3-(BOC-amino)-3-methylpiperidine was hydrogenated according to the procedure of Example 350f above, and 2.50 g of the title compound was isolated.

### Step 353f. 8-(3-amino-3-methylpiperidinyl)-1-cyclopropyl-7-fluoro-4H-9-methyl-4-oxoquinolizine-3-carboxylic acid hydrochloride

Following the procedure of Example 253 step j, replacing the 3-BOC-aminopyrrolidine thereof with 3-(BOC-amino)-3-methylpiperidine, from step 353e above, and carrying the product forward as in Example 253 steps j-l, the title compound was prepared (225 mg). MS: 373 (M+1)⁺; ¹H NMR (CD₃OD) ∂: 0.69 (m, 2H), 1.05 (m, 2H), 1.53(m, 3H), 1.80 (m, 1H), 2.23 (m, 2H), 2.86 (m, 3H), 3.23 (m, 2H), 3.41 (m, 2H), 3.72 (m, 2H), 8.68 (m, 2H), 8.15 (m, 1H), 9.01 (m, 1H), 13.64 (s, 1H). Anal. Calcd for C₂₀H₂₅N₃O₃ClF•H₂O: C, 56.14; H, 6.36; N, 9.82; Found: C, 55.73; H, 6.43; N, 9.48.

### Example 354

### 8-(3-pyrrolylpiperidinyl)-1-cyclopropyl-7-fluoro-4H-9-methyl-4-oxo-quinolizine-3-carboxylic acid

### Step 354a. N-CBZ-3-(methanesulfonyloxy)piperidine

A 4.0 g sample of N-CBZ-3-hydroxypiperidine (prepared from 3-hydroxypiperidine by standard methods) was dissolved in 25 mL of methylene chloride and cooled to 0°C. To this was added 3.55 mL of triethylamine, then a solution of 1.77 mL of methanesulfonylchloride in 4 mL of methylene chloride was added dropwise. The reaction mixture was stirred at 0°C for 15 min and at room temperature for 1.5 hours. The reaction was quenched by dilution with methylene chloride and extraction with 15% NaHCO₃ solution. The layers were separated, and the organic layer dried over Na₂SO₄, filtered and evaporated to give 5.02 g of the title compound.

### Step 354b. N-CBZ-3-pyrrolylpiperidine

A 5.02 g sample of the N-CBZ-3-(methanesulfonyloxy)piperidine from step 354a above was dissolved in 8.89 g of pyrrole and heated at 100°C for 20 hours. Excess pyrrole was removed under vacuum, and the residue was washed with 5% NaHCO₃ solution, water, dried over Na₂SO₄, filtered and taken to dryness. The residue was purified by flash chromatography on silica gel, eluting with 0-1% methanol in methylene chloride to afford 0.500 g of the title compound.

### Step 354c. 3-pyrrolylpiperidine

A 612 mg sample of N-CBZ-3-pyrrolylpiperidine, from step 354b above, was hydrogenated according to the procedure of Example 350f above, and 500 mg of the title compound was isolated.

### Step 354d. 8-(3-pyrrolylpiperidinyl)-1-cyclopropyl-7-fluoro-4H-9-methyl-4-oxo-quinolizine-3-carboxylic acid

Following the procedure of Example 253 step j, replacing the 3-BOC-aminopyrrolidine thereof with 3-pyrrolylpiperidine, from step 354c above, and carrying the product forward as in Example 253 steps j-k, the title compound was prepared (157 mg). mp 182-185°C. MS: 410 (M+1)⁺; ¹H NMR (CD₃OD) ∂: 0.71 (m, 2H), 1.03 (m, 2H), 1.93 (m, 2H), 2.26 (m, 3H), 2.78 (s, 3H), 2.91-3.78 (m, 6H), 6.19 (m, 2H), 6.77 (m, 2H), 8.23 (s, 1H), 9.15 (d, 1H, J=12 Hz), 13.09 (s, 1H). Anal. Calcd for C₂₃H₂₄N₃O₃F•2.25 H₂O: C, 61.39; H, 5.83; N, 9.34; Found: C, 61.40; H, 5.63; N, 8.94.

### Example 355

### 8-(3-aminopiperidinyl)-1-cyclopropyl-7-fluoro-4H-9-methyl-4-oxo-quinolizine-3-carboxylic acid hydrochloride

### Step 355a. (R)-3-amino-2-piperidone

A sample of D-ornithine methyl ester hydrochloride was dissolved in 240 mL of methanol, and stirred with 75 g of an anion exchange resin in the OH⁻ form for 4 hours at room temperature. The suspension was filtered, and the filtrate was taken to dryness. The residue was distilled in a kugelrohr apparatus to yield 7.59 g of the title compound.

### Step 355b.(R)-3-aminopiperidine

A 7.49 g sample of (R)-3-amino-2-piperidone, from step 355a above, was dissolved in 140 mL of THF, and the solution was cooled to 0°C. To this solution was carefully added in small portions 3.00 g of lithium aluminum hydride. The reaction mixture was stirred at room temperature for 2 hours. The reaction was quenched with water and NaOH, filtered, and the filter cake was extracted with THF. The solution was dried over Na₂SO₄, filtered, and evaporated to dryness. The residue was purified by distillation.

### Step 355c. 8-(3-aminopiperidinyl)-1-cyclopropyl-7-fluoro-4H-9-methyl-4-oxo-quinolizine-3-carboxylic acid hydrochloride

Following the procedure of Example 253 step j, replacing the 3-BOC-aminopyrrolidine thereof with 3-aminopiperidine, from step 355b above, and carrying the product forward as in Example 253 steps j-l, the title compound was prepared (376 mg). MS: 360 (M+1)⁺; ¹H NMR (CD₃OD) ∂: 0.71 (m, 2H),1.09 (m, 2H), 1.67-2.44 (m, 10H), 3.82 (d, 2H, J=12 Hz), 8.20 (s, 1H), 9.25 (d, 1H, J=9 Hz). Anal. Calcd for C₁₉H₂₃N₃O₃ClF•H₂O: C, 55.14; H, 6.09; N, 10.15; Found: C, 55.50; H, 6.37; N, 9.26.

### Example 356

### 8-(3-amino-3-methylpyrrolidinyl)-1-cyclopropyl-7-fluoro-4H-9-methyl-4-oxo-quinolizine-3-carboxylic acid hydrochloride

Following the procedure of Example 253 step j, replacing the 3-BOC-aminopyrrolidine thereof with 3-(BOC-amino)-3-methylpyrrolidine, and carrying the product forward as in Example 253 steps j-l, the title compound was prepared (255 mg). MS: 360 (M+1)⁺; ¹H NMR (CD₃OD) ∂: 0.69 (m, 2H), 1.07 (m, 2H), 1.63 (s, 3H), 2.31 (m, 3H), 2.74 (s, 3H), 3.95 (m, 4H), 8.12 (s, 1H), 9.14 (d, 1H, J=9 Hz). Anal. Calcd for C₁₉H₂₃N₃O₃ClF•H₂O: C, 55.14; H, 6.09; N, 10.15; Found: C, 55.08; H, 6.01; N, 9.77.

### Example 357

### 8-(3-amino-4-(1',3'-dioxolanyl)pyrrolidinyl)-1-cyclopropyl-7-fluoro-4H-9-methyl-4-oxoquinolizine-3-carboxylic acid hydrochloride

### Step 357a. N-CBZ-3-amino-4-hydroxy-pyrrolidine

A 27.1 g sample of N-CBZ-3-azido-4-hydroxy-pyrrolidine, prepared as in step 349c above, was hydrogenated for 24 hours under the conditions of example 349e above, and 25.4 g of the title compound was obtained.

### Step 357b. N-CBZ-3-(CBZ-amino)-4-hydroxy-pyrrolidine

A 25.4 g sample of N-CBZ-3-azido-4-hydroxy-pyrrolidine, from step 357a above, was dissolved in 129 mL of 1 M NaOH, and the solution was cooled to 0°C. A 15.35 mL sample of benzyl chloroformate was dissolved in 20 mL of ethanol, and this solution was added dropwise to the vigorously stirred solution of the pyrrolidine over a 40 min period. The reaction mixture was stirred at 0°C for 4 hours, then the reaction was quenched by pouring into 200 mL of water. This mixture was extracted with methylene chloride, which was dried over Na₂SO₄, filtered, and evaporated to dryness. The residue was purified by column chromatography on silica gel, eluting with 0.5-3.5% methanol in methylene chloride to yield 18.77 g of the title compound.

### Step 357c. N-CBZ-3-(CBZ-amino)-4-pyrrolidinone

In a dry vessel under N₂ was place 385 mL of methylene chloride, and the solvent was cooled to 0°C. To this was added 17.32 mL of DMSO, then 21.89 mL of phenyl dichlorophosphate was added dropwise over a 30 min period. Next was added 34.03 mL of triethylamine over a 30 min period. To this solution was added a solution of N-CBZ-3-(CBZ-amino)-4-hydroxy-pyrrolidine, from step 357b above, in 100 mL of methylene chloride in a dropwise manner over a 45 min period. The reaction mixture was stirred at 0°C for 1 hour and at room temperature for 20 hours. The reaction was quenched by pouring it into 20% NaCl solution. The mixture was extracted with methylene chloride, which was dried over Na₂SO₄, filtered, and evaporated to dryness. The excess DMSO was removed under vacuum with heating, and the residue was purified by column chromatography on silica gel, eluting with 0 to 1 % methanol in methylene chloride to give 9.2 of the title compound.

### Step 357d. N-CBZ-3-(CBZ-amino)-4-(1'-3-dioxolanylyl)pyrrolidine

A0.932 g sample of N-CBZ-3-(CBZ-amino)-4-pyrrolidinone, from step 357c above, was dissolved in 17 mL of toluene and 0.353 mL of ethylene glycol and 24 mg of p-toluenesulfonic acid were added. The reaction mixture was stirred at 110°C for 20 hours, then the reaction was quenched by addition of 5% NaHCO₃ solution. The mixture was extracted with methylene chloride, which was dried over Na₂SO₄, filtered, and evaporated to dryness. The residue was purified by column chromatography on silica gel, eluting with 2% methanol in methylene chloride to afford 578 mg of the title compound.

### Step 357e. 3-amino-4-(1'-3-dioxolanylyl)pyrrolidine

A 2.68 g sample of N-CBZ-3-(CBZ-amino)-3-methylpiperidine was hydrogenated for 7 days according to the procedure of Example 350f above, and 937 mg of the title compound was isolated.

### Step 357f. 8-(3-amino-4-(1',3'-dioxolanyl)pyrrolidinyl)-1-cyclopropyl-7-fluoro-4H-9-methyl-4-oxo-quinolizine-3-carboxylic acid hydrochloride

Following the procedure of Example 253 step j, replacing the 3-BOC-aminopyrrolidine thereof with 3-amino-4-(1',3'-dioxolanyl)pyrrolidine, from step 357d above, and carrying the product forward as in Example 253 steps j-l, the title compound was prepared (324 mg). MS: 404 (M+1)⁺; ¹H NMR (CD₃OD) ∂: 0.69 (m, 2H), 1.06 (m, 2H), 2.33 (m, 1H), 2.75 (s, 3H), 3.88-4.02 (m, 4H), 4.16 (m, 4H), 4.21 (m, 1H), 8.16 (s, 1H), 9.21 (d, 1H, J=9 Hz). Anal. Calcd for C₂₀H₂₃N₃O₅ClF•H₂O•HCl: C, 48.59; H, 5.30; N, 8.50; Found: C, 48.80; H, 4.87; N, 8.52.

### Example 358

### 8-(3-amino-4-hydroxy-pyrrolidinyl)-1-cyclopropyl-7-fluoro-4H-9-methyl-4-oxo-quinolizine-3-carboxylic acid hydrochloride

### Step 358a. N-CBZ-3-azido-4-(methoxymethoxy)pyrrolidine

A sample of N-CBZ-3-azido-4-hydroxypyrrolidine, prepared as in Example 349c above, was dissolved in 20 mL of methylene chloride, 5.02 mL of diisopropylethylamine was added, and 1.64 mL of methoxymethyl chloride was added dropwise over a 15 min period, with cooling as necessary to maintain the temperature at ambient. The reaction mixture was stirred at room temperature for 18 hours, then washed with 0.5 M phosphoric acid, 5% NaHCO₃, dried over Na₂SO₄, filtered, and evaporated to dryness. The residue was purified by flash chromatography on silica gel, eluting with 0.5% methanol in methylene chloride to yield 1.58 g of the title compound.

### Step 358b. N-CBZ-3-amino-4-(methoxymethoxy)pyrrolidine

A 2.23 g sample of N-CBZ-3-azido-4-(methoxymethoxy)pyrrolidine, prepared as in step 358a above, was dissolved in 200 mL of ethyl acetate and hydrogenated at room temperature under 4 Atm of H₂ in the presence of RaNi for 24 hours in a sealed bomb. The catalyst was removed by filtration, and the solvent was removed under vacuum to give the title product.

### Step 358c. N-CBZ-3-(BOC-amino)-4-(methoxymethoxy)pyrrolidine

A 2.04 g sample of N-CBZ-3-amino-4-(methoxymethoxy)pyrrolidine, from step 358b above, was dissolved in 20 mL of methylene chloride, and the solution was cooled to 0°C. To this solution was added 2 mL of triethylamine, then 2.38 mL of di-t-butyl dicarbonate
dissolved in 5 mL of methylene chloride. The reaction mixture was stirred for 30 min at 0°C, at room temperature for 24 hours, and at 40°C for 8 hours, then quenched by pouring into 10% NaCl solution. The mixture was extracted with methylene chloride, which was dried over Na₂SO₄, filtered, and evaporated to dryness. The residue was purified by column chromatography on silica gel, eluting with 1% methanol in methylene chloride to give 1.35 g of the title compound.

### Step 358d. 3-(BOC-amino)-4-(methoxymethoxy)pyrrolidine

A 1.35 g sample of N-CBZ-3-(BOC-amino)-4-(methoxymethoxy)pyrrolidine, from step 358c above, was hydrogenated for 12 days according to the procedure of Example 350f above, and 874 mg of the title compound was isolated.

### Step 358e. 8-(3-amino-4-hydroxy-pyrrolidinyl)-1-cyclopropyl-7-fluoro-4H-9-methyl-4-oxo-quinolizine-3-carboxylic acid hydrochloride

Following the procedure of Example 253 step j, replacing the 3-BOC-aminopyrrolidine thereof with 3-amino-4-hydroxypyrrolidine from step 358d above, and carrying the product forward as in Example 253 steps j-l, the title compound was prepared (125 mg). MS: 362 (M+1)⁺; ¹H NMR (CD₃OD) ∂: 0.69 (m, 2H), 1.08 (m, 2H), 2.31 (m, 1H), 2.73 (s,3H), 3.69-4.53 (m, 7H), 8.08 (s, 1H), 9.10 (m, 2H). Anal. Calcd for C₁₈H₂₁N₃O₄ClF•1.5H₂O: C, 50.89; H, 5.69; N, 9.89; Found: C, 51.38; H, 5.65; N, 9.73.

### Example 359

### 8-(4-(1-(N-ethylamino)methyl)piperidinyl)-1-cyclopropyl-7-fluoro-4H-9-methyl-4-oxoquinolizine-3-carboxylic acid hydrochloride

### Step 359a. 4-(N-BOC-N-ethylaminomethyl)pyridine

A 4.00 g sample of 4-(N-ethylaminomethyl)pyridine (Aldrich) was dissolved in 50 mL of methylene chloride, and the solution was cooled to 0°C. To his solution was added 8.19 mL of triethylamine and then 8.01 g of di-t-butyl dicarbonate dissolved in 10 mL of methylene chloride was added dropwise. The reaction mixture was stirred for 1 hour at 0°C and at room temperature for 30 min, then quenched by pouring into 10% NaCl solution. The mixture was extracted with methylene chloride, which was dried over Na₂SO₄, filtered, and evaporated to dryness. The residue was purified by flash chromatography on silica gel, eluting with 1 % methanol in methylene chloride to yield 5.52 g of the title compound.

### Step 359b. 4-(N-BOC-N-ethylaminomethyl)piperidine

A 5.50 g sample of 4-(N-BOC-N-ethylaminomethyl)pyridine, prepared as in step 359a above, was dissolved in 200 mL of ethyl acetate and hydrogenated at room temperature under 4 Atm of H₂ in the presence of RaNi for 24 hours in a sealed bomb. The catalyst was removed by filtration, and the solvent was removed under vacuum to give 1.80 g of the title product.

### Step 359c. 8-(4-(1-(N-ethylamino)methyl)piperidinyl)-1-cyclopropyl-7-fluoro-4H-9-methyl-4-oxo-quinolizine-3-carboxylic acid hydrochloride

Following the procedure of Example 253 step j, replacing the 3-BOC-aminopyrrolidine thereof with 4-(N-BOC-N-ethylaminomethyl)piperidine, prepared in step 359b above, and carrying the product forward as in Example 253 steps j-l, the title compound was prepared (488 mg). MS: 402 (M+1)⁺; ¹H NMR (CD₃OD) ∂: 0.69 (m, 2H), 1.07 (m, 2H), 1.36 (t, J=7.5 Hz, 3H), 1.91 (m, 4H), 2.36 (m, 1H), 2.84 (s, 3H), 2.97 (3.37 (m, 8H), 3.41 (m, 1H), 8.20 (s, 1H), 9.26 (d, J=9 Hz, 1H). Anal. Calcd for C₂₂H₂₉N₃O₃ClF•0.5H₂O: C, 59.12; H, 6.77; N, 9.40; Found: C, 58.74; H, 6.63; N, 9.28.

### Example 360

### 1-cyclopropyl-7-fluoro-8-(3-hydroxy-4-methylaminopyrrolidinyl)-4H-9-methyl-4-oxoquinolizine-3-carboxylic acid hydrochloride

### Step 360a. N-CBZ-3-cyano-4-hydroxypyrrolidine

A sample of N-CBZ-3,4-epoxypyrrolidine, prepared as in Example 349b above, was dissolved in 100 mL of ethanol and added to a solution of 9.88 g of MgSO₄ and 13.41 g of NaCN in 195 mL of water. The reaction mixture was stirred at 65°C for 20 hours., cooled, filtered, and extracted with methylene chloride, which was dried over Na₂SO₄, filtered, and evaporated to dryness to afford 9.0 g of the title compound.

### Step 360b. N-CBZ-3-aminomethyl-4-hydroxypyrrolidine

A 13.97 g sample of N-CBZ-3-cyano-4-hydroxypyrrolidine, prepared as in step 360a above, was dissolved in 210 mL of methanol containing 40 mL of triethylamine and hydrogenated at room temperature under 4 Atm of H₂ in the presence of RaNi for 24 hours in a sealed bomb. The catalyst was removed by filtration, and the solvent was removed under vacuum to give 14.38 g of the title product.

### Step 360c. N-CBZ-3-(BOC-aminomethyl)-4-hydroxypyrrolidine

A 2.73 g sample of N-CBZ-3-aminomethyl-4-hydroxypyrrolidine, from step 360b above, was dissolved in 20 mL of methylene chloride, and the solution was cooled to 0°C. To this solution was added 2.86 g of di-t-butyl dicarbonate dissolved in 3 mL of methylene chloride, and the reaction mixture was stirred at 0°C for 1 hour and at room temperature for 18 hours. The reaction was quenched by pouring into 250 mL of water, and the mixture was extracted with methylene chloride, which was dried over Na₂SO₄, filtered, and evaporated to dryness. The residue was purified by flash chromatography on silica gel to afford the title compound.

### Step 360d. 3-hydroxy-4-methylaminopyrrolidine

A sample of N-CBZ-3-(BOC-aminomethyl)-4-hydroxypyrrolidine, from step 360c above, was hydrogenated over 10% Pd/C in 100 mL of methanol under 4 Atm of H₂ at room temperature for 24 hours. The catalyst was removed by filtration, the solvent was removed to yield 610 mg of title compound.

### Step 360e. 1-cyclopropyl-7-fluoro-8-(3-hydroxy-4-methylaminopyrrolidinyl)-4H-9-methyl-4-oxo-quinolizine-3-carboxylic acid hydrochloride

Following the procedure of Example 253 step j, replacing the 3-BOC-aminopyrrolidine thereof with 3-hydroxy-4-methylaminopyrrolidine, from step 360d above, and carrying the product forward as in Example 253 steps j-l, the title compound was prepared (540 mg). MS: 376 (M+1)⁺; ¹H NMR (CD₃OD) ∂: 0.68 (m, 3H), 0.99 (m, 2H), 2.29 (m, 1H), 2.70 (s, 3H), 3.55-4.58 (m, 9H), 8.09 (s, 1H), 9.02 (d, J=9 Hz, 1H). Anal. Calcd for C₁₉H₂₃N₃O₄ClF•2H₂O: C, 50.95; H, 6.08; N, 9.38; Found: C, 50.88; H, 5.77; N, 9.01.

### Example 361

### 8-(3-aminomethylpiperidinyl)-1-cyclopropyl-7-fluoro-4H-9-methyl-4-oxo-quinolizine-3-carboxylic acid hydrochloride

### Step 361a. 3-(N-BOC-aminomethyl)pyridine

Under dry N₂, 15.69 g of di-t-butyldicarbonate was dissolved in 100 mL of CH₂Cl₂. The flask and contents were cooled in an ice bath, and to this was added a solution of 6.12 g of 3-(aminomethyl)pyridine in CH₂Cl₂ dropwise with stirring. The solution was stirred at 0-5°C for 30 min, then stirred at room temperature for 72 hours. The reaction was diluted with additional CH₂Cl₂ (100 mL), then washed with 250 mL of water. The water was back-extracted with CH₂Cl₂, and the organic layers were combined and dried over Na₂SO₄. The solution was filtered, and the solvent was removed on a rotary evaporator to give 13 g of title compound.

### Step 361b. 3-(N-BOC-aminomethyl)piperidine

A 10.13 g sample of the compound from step 361a above was dissolved in 250 mL of methanol and reduced over 5 g of 5% Rh/C catalyst at room temperature under 4 Atm or H₂ for 18 hours. The catalyst was removed by filtration, and the solvent was removed under vacuum. The product was recrystallized from ethyl acetate, and dried under high vacuum to give 3.8 g of product. mp. 64-65°C.

### Step 361c. 8-(3-aminomethylpiperidinyl)-1-cyclopropyl-7-fluoro-4H-9-methyl-4-oxoquinolizine-3-carboxylic acid hydrochloride

Following the procedure of Example 253 step j, replacing the 3-BOC-aminopyrrolidine thereof with 3-(N-BOC-aminomethyl)piperidine, prepared in step 361b above, and carrying the product forward as in Example 253 steps j-l, the title compound was prepared (301 mg). mp 207-208°C. MS: 374 (M+1)⁺; ¹H NMR (CD₃OD) ∂: 0.70 (m, 2H), 1.05 (m, 2H), 1.45 (m, 2H), 1.90 (m, 2H), 2.10 (m, 2H), 2.35 (m, 1H), 2.84 (s, 3H), 3.00 (m, 2H), 3.20 (m, 1H), 3.30 (m, 2H), 8.09 (s, 1H), 8.32 (s, 1H), 9.17 (d, 1H, J=12 Hz). Anal. Calcd for C₂₀H₂₅N₃O₃ClF•1.5H₂O: C, 50.75; H, 6.18; N, 8.88; Found: C, 50.53; H, 6.20; N, 9.03.

### Example 362

### 8-(2-aminomethyl-4-morpholinyl)-1-cyclopropyl-7-fluoro-4H-9-methyl-4-oxo-quinolizine-3-carboxylic acid hydrochloride

### Step 362a. N-benzyl-2-chloromethylmorpholine

A flask was charged with 1.5 g (10 mmol) of N-benzyl-ethanolamine, 7.8 mL of epichlorohydrin (71 mmol). The reaction mixture was heated at 40°C for 30 min, then cooled to room temperature. The excess epichlorohydrin was removed under vacuum, and the residue was dissolved in 30 mL of conc. H₂SO₄. The solution was heated at 150°C for 30 min and poured onto 50 g of ice. The solution was adjusted to pH 13 with NaOH, and the mixture was extracted with toluene. The solution was dried over Na₂SO₄, filtered, the solvent removed, and the residue dried under vacuum to give 193 mg of the title compound. MS m/z: 226, 228 (M+H)⁺.

### Step 362b. 2-(N-benzyl-morpholinyl)-N-methylphthalimide

An oven-dried system under positive N₂ pressure was charged with 900 mg (4 mmol) of N-benzyl-3-chloromethylmorpholine dissolved in 20 mL of DMSO. To this was added 1.48 g (8 mmol) of potassium phthalimide. The reaction mixture was stirred at 100°C for 96 hours, then cooled to room temperature and poured into 50 mL of water. The mixture was extracted with methylene chloride, the extract washed with water, and the organic layer was dried over Na₂SO₄. The solution was filtered, the solvent was removed under vacuum, and the product was dried under vacuum to give 1.18 g of the title compound. The material was recrystallized from ethanol, separated by filtration, and dried under vacuum to give 884 mg of pure title compound.

### Step 362c. 4-benzyl-2-aminomethylmorpholine

A system under positive N₂ pressure was charged with 160 mg of 3-(N-benzyl-morpholinyl)-N-methylphthalimide, from step 362b above, suspended in 4 mL of ethanol. To this was added 50 µL of hydrazine hydrate, and the reaction mixture was stirred at room temperature for 3 hours and at 70°C for 24 hours. The reaction mixture was cooled to room temperature and diluted with 10 mL of water. The mixture was filtered, and the aqueous layer was adjusted to pH 12 with NaOH and extracted with methylene chloride. The organic extract was dried over Na₂SO₄, filtered, and the solvent was removed and the product was dried under vacuum to give 72 mg of the title compound.

### Step 362d. 4-benzyl-2-(BOC-aminomethyl)morpholine

An oven-dried system protected from moisture was charged with 198 mg of 1-benzyl-3-aminomethylmorpholine, prepared as in step 362c above, dissolved in 2 mL of methylene chloride. To this solution was added 250 mg of di-t-butyl-dicarbonate. The reaction mixture was stirred at room temperature for 24 hours, diluted with 30 mL of methylene chloride, and dried over Na₂SO₄. The mixture was filtered, and the solvent was removed under vacuum. The residue was purified with preparative TLC on silica gel, developing with 9% methanol in methylene chloride and collecting the band at Rf=0.48. The product was removed from the silica gel with 300 mL of 10% methanol in methylene chloride, and the solvent was removed under vacuum to give 173 mg of the title compound. MS: 307 (M+1)⁺.

### Step 362e. 2-(BOC-aminomethyl)morpholine

A 50 mg sample of 4-benzyl-2-(BOC-aminomethyl)morpholine, from step 362d above, was dissolved in 5 mL of methanol and the benzyl group was removed by hydrogenation over under 4 Atm of H₂ over 25 mg of Pd/C at room temperature for 48 hours. The catalyst was filtered off, and the solvent was removed to give 33 mg of the title compound.

### Step 362f. 8-(2-aminomethyl-4-morpholinyl)-1-cyclopropyl-7-fluoro-4H-9-methyl-4-oxoquinolizine-3-carboxylic acid hydrochloride

Following the procedure of Example 253 step j, replacing the 3-BOC-aminopyrrolidine thereof with 2-(BOC-aminomethyl)morpholine, prepared as in step 362e above, and carrying the product forward as in Example 253 steps j-l, the title compound was prepared (287 mg). mp 209-210°C. MS: 376 (M+1)⁺, 393 (M+NH₄)⁺; ¹H NMR (CD₃OD) ∂: 0.70 (dd, 2H, J=4.5, 1.5 Hz), 1.09 (dd, 2H, J=1.5, 4.5 Hz), 2.38 (m, 1H), 2.88 (s, 3H), 3.05 (m, 2H), 3.20 (m, 2H), 3.40 (m, 2H), 3.50 (m, 2H), 3.90 (m, 2H), 4.10 (dd, 1H, J=1.5, 12 Hz), 8.03 (s, 1H), 8.15 (s, 1H), 9.23 (d, 1H, J=9 Hz), Anal. Calcd for C₁₉H₂₃N₃O₄ClF•2.25 H₂O: C, 50.45; H, 6.13; N, 9.29; Found: C, 50.63; H, 6.17; N, 9.11.

### Example 363

### 8-(3-(1-(methylamino)methypiperidinyl)-1-cyclopropyl-7-fluoro-4H-9-methyl-4-oxoquinolizine-3-carboxylic acid hydrochloride

### Step 363a. 3-(N-BOC-N-methylamino)methyl)pyridine

To a dry flask under N₂ was added 84.7 mg (2.2 mmol) of NaH (minoil washed with dry hexane) and 2 mL of dry THF. The mixture was cooled in an ice bath and 416 mg of 3-(N-BOC-aminomethyl)piperidine, from Example 361b above, in 4 mL of dry THF was added dropwise. The mixture was stirred at 0-5°C for 1 hour after addition was complete, and 0.125 mL of methyl iodide was added. The mixture was stirred at 0-5°C for 30 min, then warmed to room temperature and stirred for 24 hours. The reaction was quenched by pouring it into 30 mL of satd NaCl solution, and the mixture was extracted with 3x30 mL of methylene chloride. The organic extracts were combined, dried over Na₂SO₄, filtered and concentrated on a rotary evaporator to give 430 mg of title compound.

### Step 363b. 3-(N-BOC-N-methylamino)methyl)piperidine

A 1.16 g sample of the compound from step 361a above was dissolved in 50 mL of methanol and reduced over 1.16 g of 5% Rh/C catalyst at room temperature under 4 Atm or H₂ for 18 hours. The catalyst was removed by filtration, and the solvent was removed under vacuum. The product was recrystallized from ethyl acetate, and dried under high vacuum to give 1.18 g of product. MS m/z: 229 (M+H)⁺.

### Step 363c. 8-(3-(1-(methylamino)methypiperidinyl)-1-cyclopropyl-7-fluoro-4H-9-methyl-4-oxo-quinolizine-3-carboxylic acid hydrochloride

Following the procedure of Example 253 step j, replacing the 3-BOC-aminopyrrolidine thereof with 3-(N-BOC-N-methylamino)methyl)piperidine prepared according to step 363a above, and carrying the product forward as in Example 253 steps j-l, the title compound was prepared (535 mg). mp 246-247°C. MS: 388 (M+1)⁺; ¹H NMR (CD₃OD) ∂: 0.70 (dd, 2H, J=4.5 Hz), 1.07 (dd, 2H, J=7.8 Hz), 1.50 (m, 2H), 1.90 (m, 4H), 2.10 (m, 2H), 2.21 (m, 1H), 2.72 (s, 3H), 2.85 (s, 3H), 3.00 (m, 2H), 8.10 (s, 1H), 8.32 (s, 1H), 9.18 (d, 1H, J=9 Hz), Anal. Calcd for C₂₁H₂₇N₃O₃ClF•H₂O: C, 57.08; H, 6.61; N, 9.51; Found: C, 56.93; H, 6.68; N, 10.23.

### Example 364

### 8-(3-(methyl(methylenedioxy)methyl)piperidinyl)-1-cyclopropyl-7-fluoro-4H-9-methyl-4-oxoquinolizine-3-carboxylic acid hydrochloride

Following the procedure of Example 253 step j, replacing the 3-BOC-aminopyrrolidine thereof with 3-(methyl(methylenedioxy)methyl)piperidine prepared according to European Patent Application 342, 675, and carrying the product forward as in Example 253 steps j-k, the title compound was prepared (443 mg). mp 117-118°C. MS: 419 (M+1)⁺; ¹H NMR (CDCl₃) ∂: 0.70 (m, 2H), 1.03 (m, 2H), 1.40 (m, 2H), 1.71 (m, 6H), 2.80 (s, 3H), 3.10 (dt, 1H, J=3, 12 Hz), 8.04 (dd, 2H, J=7.5 Hz), 8.32 (s, 1H), 9.18 (d, 1H, J=12 Hz); Anal. Calcd for C₂₂H₂₇N₂O₅F: C, 63.15; H, 6.50; N, 6.69; Found: C, 63.02; H, 6.42; N, 6.64.

### Example 365

### 8-(3-(S)-aminopiperidinyl)-1-cyclopropyl-7-fluoro-4H-9-methyl-4-oxo-quinolizine-3-carboxylic acid hydrochloride

Following the procedure of Example 253 step j, replacing the 3-BOC-aminopyrrolidine thereof with 3-(S)-(N-BOC-amino)piperidine and carrying the product forward as in Example 253 steps j-l, the title compound was prepared (500 mg). mp 220-221°C. MS: 360 (M+1)⁺, 377 (M+NH₄)⁺; ¹H NMR (CD₃OD) ∂: 0.70 (m, 2H, J=6 Hz), 1.10 (m, 2H, J=6 Hz), 1.72 (m, 2H), 2.05 (m, 3H), 2.28 (m, 2H), 2.40 (m, 2H), 2.86 (s, 3H), 3.90 (m, 1H), 8.18 (s, 1H), 9.22 (d, 1H, J=9 Hz); Anal. Calcd for C₁₉H₂₃N₂O₅ClF•1.5 H₂O: C,53.97; H, 6.20; N, 9.94; Found: C, 54.28; H, 6.61; N, 8.85.

### Example 366

### 8-(3-(S)-(N-ethyl-N-methylamino)piperidinyl)-1-cyclopropyl-7-fluoro-4H-9-methyl-4-oxoquinolizine-3-carboxylic acid

### Step 366a. (S)-3-acetylamino-1-benzylpyrrolidine

To 1.30 g (7.38 mmol) of 3-amino-1-benzylpyrrolidine and 1.7 mL (12 mmol) of triethylamine in 25 mL of ethyl acetate stirred at room temperature was added 1.1 mL (12 mmol) of acetic anhydride, and the reaction was stirred for 1 hour. The solvent was removed, and the residue was treated with 1:1 20% K₂CO₃:brine, then extracted with methylene chloride. The organic extract was dried over Na₂SO₄, filtered, the solvent was removed under vacuum, and the residue was dried under high vacuum for 16 hours to give 1.71 g of the title compound. MS: 219 (M+1)⁺; Anal. Calcd for C₁₃H₁₈N₂O: C,68.69; H, 8.42; N, 12.32; Found: C, 68.75; H, 8.00; N, 12.27.

### Step 366b. (S)-3-ethylamino-1-benzylpyrrolidine

To 1.70 g (7.4 mmol) of the compound from step 366a above in 20 mL of THF was added 850 mg of lithium aluminum hydride, and the mixture was stirred at room temperature for 72 hours. The reaction was quenched with water and NaOH, stirred for 1 hour, filtered, and the filter cake was extracted with methylene chloride. The aqueous layers were extracted with methylene chloride, and the organic extracts were combined. The solution was dried over Na₂SO₄, filtered, and the solvent was removed under vacuum to give 1.71 g of the title compound. ¹H NMR (CDCl₃) ∂: 1.09 (t, 3H), 1.30-1.51 (m, 1H), 1.48-1.53 (m, 1H), 2.06-2.21 (m, 1H), 2.34 (dd, 1H), 2.58 (q, 2H), 2.47-2.68 (m, 2H), 2.77 (dd, 1H), 3.26-3.37 (m, 1H), 3.50 (s, 2H), 7.19-7.40 (m, 5H).

### Step 366c. (S)-3-(N-BOC-N-ethylamino)-1-benzylpyrrolidine

To a 1.7 g sample of the compound from step 366b above dissolved in 3 mL of methylene chloride was added 1.94 g (8.9 mmol) of butoxycarbonyl anhydride, and the reaction was stirred for 16 hours. The solvent was removed under vacuum, and the residue was chromatographed on silica gel, eluting with 1;1 hexane:ethyl acetate to give 1.8 g of the title compound. MS: 305 (M+1)⁺; ¹H NMR (CDCl₃) ∂: 1.11 (t, 3H), 1.44 (s, 9H), 3.25 (q, 2H), 7.24-7.47 (m, 5H). Anal. Calcd for C₁₈H₂₈N₂O₂: C,68.00; H, 9.35; N, 8.81; Found: C, 68.05; H, 8.73; N, 8.85.

### Step 366d. (S)-3-(N-ethyl-N-methylamino)-1-benzylpyrrolidine

To a 1.8 g (5.9 mmol) sample of the compound from step 366c above in 20 mL of THF was added 800 mg of LAH, and the reaction was stirred for 48 at reflux conditions. The reaction was cooled to room temperature, and 0.8 mL of water was added dropwise with stirring, followed by 0.8 mL of 15% NaOH similarly, and finally 2.4 mL of water, and the mixture was stirred for 2 hours at room temperature. The mixture was filtered, the filter cake washed with methylene chloride, the filtrate concentrated under vacuum to give the crude title product. This material was dissolved in acetic acid and filtered, methanol was added and the solvent removed, and the residue repeatedly dissolved in methanol and stripped. The residue was taken up in water, adjusted to pH 10-11 with K₂CO₃, saturated with NaCl, then this solution was extracted with 10% methanol in CHCl₃. The extract was dried over Na₂SO₄, filtered and the solvent was removed to give 603 mg of the title product. MS: 219 (M+1)⁺; ¹H NMR (CDCl₃) ∂: 1.06 (t, 3H), 1.93-2.09 (m, 1H), 2.20 (s, 3H), 2.28-2.60 (br, 4H), 2.66-2.77 (m, 1H), 2.82 (dd, 1H), 2.96-3.14 (m, 1H), 3.60 (q, 2H), 7.18-7.41 (m, 5H).

### Step 366e. (S)-3-(N-ethyl-N-methylamino)pyrrolidine

A 1.3 g sample of the compound from step 366d above was dissolved in 50 mL of acetic acid and 0.5 mL of HCl, 0.13 g of 10% Pd/C was added and the sample hydrogenated under 4 Atm of H₂. Additional amounts of catalyst and HCl were added before the reaction was complete. The solution was filtered, then the solvent was removed with repeated addition and removal of methanol. The residue was dissolved in water, which was adjusted to pH 10-11 with K₂CO₃, saturated with NaCl, and extracted repeatedly with 10% methanol in CHCl₃. The extract was dried over Na₂SO₄, filtered, and taken to dryness to give 603 mg of the title compound. HRMS (M+1)⁺: calc: 129.1936; found, 129.1392.

### Step 366f. 8-(3-(S)-(N-ethyl-N-methylamino)piperidinyl)-1-cyclopropyl-7-fluoro-4H-9-methyl-4-oxo-quinolizine-3-carboxylic acid

Following the procedure of Example 253 step j, replacing the 3-BOC-aminopyrrolidine thereof with (S)-3-(N-ethyl-N-methylamino)-pyrrolidine from step 366e above and carrying the product forward as in Example 253 steps j-k, the title compound was prepared. MS: 416 (M+1)⁺; ¹H NMR (CDCl₃) ∂: 0.5-0.6 M, 1H), 0.6-0.7 (m, 1H), 0.8-0.95 (m, 2H), 1.1 (t, 3H), 1.4 (t, 3H), 1.9-2.0 (m, 1H), 2.1-2.2 (m, 1H), 2.25 (s, 3H), 2.33 (s, 3H), 3.6-3.7 (m, 4H), 3.7-3.9 (m, 1H), 3.9-4.0 (m, 1H), 4.12 (dd, 1H), 4.4 (q, 2H), 8.13 (s, 1H), 9.25 (d, 2H).

### Example 367

### 1-cyclopropyl-8-(4-(2'-(N-methylamino)methyl-1',3'-dioxolanyl)piperidinyl)-7-fluoro-9-methyl-4-oxo-4H-quinolizine-3-carboxylic acid hydrochloride

### Step 367a. N-CBZ-4-(4'-bromomethyl-1',3'-dioxolanyl)piperidine

A 17.48 g sample of N-CBZ-4-oxopiperidine, prepared as in Example 350b above, was dissolved in 325 mL of toluene, and 16.40 mL of 3-bromo-1,2-propanediol and 713 mg of p-toluenesulfonic acid were added. The reaction mixture was heated at reflux (120-125°C) for 24 hours while collecting the water of reaction in a Dean-Stark trap. The reaction mixture was cooled to room temperature, then washed with 5% NaHCO₃ and water, dried over Na₂SO₄, filtered, and taken to dryness. The residue was purified by flash chromatography on silica gel, eluting with 0-to-1.5% methanol in methylene chloride to yield 26.5 g of the title compound.

### Step 367b. N-CBZ-4-(4'-(methylaminomethyl)-1',3'-dioxolanyl)piperidine

A 7.29 g sample of N-CBZ-4-(4'-bromomethyl-1',3'-dioxolanyl)piperidine, from step 367a above, was heated with excess methylamine, and 3.427 g of the title compound was isolated and purified.

### Step 367c. N-CBZ-4-(4'-(N-BOC-N-methylaminomethyl)-1',3'-dioxolanyl)piperidine

A 3.43 g sample of N-CBZ-4-(4'-(methylaminomethyl)-1',3'-dioxolanyl)piperidine, from step 367b above, was dissolved in 30 mL of methylene chloride, to which was added 2.98 mL of triethylamine followed by dropwise addition of 3.50 g of di-t-butyl dicarbonate in 20 mL of methylene chloride. The reaction mixture was stirred at 35°C for 5 hours and at room temperature for 15 hours. The mixture was diluted with methylene chloride and washed with water. The extract was dried over Na₂SO₄, filtered, and taken to dryness to obtain 4.29 g of title compound.

### Step 367d. 4-(4'-(N-BOC-N-methylaminomethyl)-1',3'-dioxolanyl)piperidine

A sample of N-CBZ-4-(4'-(N-BOC-N-methylaminomethyl)-1',3'-dioxolanyl)piperidine, from step 367c above, was hydrogenated over 10% Pd/C in 200 mL of methanol under 4 Atm of H₂ at room temperature for 24 hours. The catalyst was removed by filtration, and the solvent was removed to yield the title compound.

### Step 367e. 1-cyclopropyl-8-(4-(2'-(N-methylamino)methyl-1',3'-dioxolanyl)piperidinyl)-7-fluoro-9-methyl-4-oxo-4H-quinolizine-3-carboxylic acid hydrochloride

Following the procedure of Example 253 step j, replacing the 3-BOC-aminopyrrolidine thereof with 4-(4'-(N-BOC-N-methylaminomethyl)-1',3'-dioxolanyl)piperidine, prepared in step 367d above, and carrying the product forward as in Example 253 steps j-k, 199 mg of the title compound was prepared. IR (KBr) cm⁻¹: 3300 (br), 2850 (br), 1700 (s), 1610 (m), 1530 (s), 790 (m). MS (CDI/NH3) m/z (M+H)⁺: 446 base. NMR (d₆-DMSO): 9.18 (d, 1H), 8.00 (s, 1H), 3.69-4.57 (m, 4H), 2.95-3.25 (m, 5H), 2.76 (s, 3H), 2.48 (m, 3H), 2.40 (m, 1H), 1.88 (m, 4H), 1.02 (m, 2H), 0.65 (m, 2H). Anal. Calcd for C₂₃H₂₉ClFN₃O₅:•2 H₂O: C,53.33; H, 6.42; N, 8.11; Found: C, 53.62; H, 6.38; N, 8.32.

### Example 368

### 1-cyclopropyl-8-(3-aza-6-amino-6-methylbicyclo[3.3.0]octan-1-yl)-7-fluoro-9-methyl-4-oxo-4H-quinolizine-3-carboxylic acid hydrochloride

### Step 368a. N-benzyl-3-aza-6-oxobicyclo[3.3.0]octane

A 32.69 g sample of N-methoxymethyl-N-(trimethylsilylmethyl)-benzylamine was dissolved in 30 mL of methylene chloride, and the solution was cooled to 0°C. To this solution was added 9.5 mL of 2-cyclopentene-1-one and 1.75 mL of trifluoroacetic acid, and the reaction mixture was stirred at 0°C for 0.5 hours and at room temperature for 24 hours. The reaction was quenched with water, and the mixture was extracted with methylene chloride, which was dried over Na₂SO₄, filtered, and taken to dryness to obtain 28.27 g of the title compound.

### Step 368b. N-benzyl-3-aza-6-hydroxy-6-methylbicyclo[3.3.0]octane

In dry ether and under N₂, the compound from step 368a was reacted with methyl magnesium bromide at -30°C. After standard workup, the title compound was isolated.

### Step 368c. N-benzyl-3-aza-6-(acetylamino)-6-methylbicyclo[3.3.0]octane

The compound of step 368b was reacted with acetonitrile in the presence of concentrated sulfuric acid. The reaction was quenched with water, and the product was extracted into methylene chloride, which was dried over Na₂SO₄, filtered, and taken to dryness to obtain the title compound.

### Step 368d. N-benzyl-3-aza-6-amino-6-methylbicyclo[3.3.0]octane

The acetyl group was removed from the compound of step 368c by reaction with conc. HCl. The reaction mixture was made basic with NaOH, and the product was extracted into methylene chloride, which was dried over Na₂SO₄, filtered, and taken to dryness to obtain the title compound.

### Step 368e. N-benzyl-3-aza-6-(BOC-amino)-6-methylbicyclo[3,3,0]octane

The compound from step 368d was reacted with di-t-butyl dicarbonate in the presence of triethylamine. The reaction was quenched with water, and the product was extracted into methylene chloride, which was dried over Na₂SO₄, filtered, and taken to dryness to obtain the title compound.

### Step 368f. 3-aza-6-(BOC-amino)-6-methylbicyclo[3.3.0]octane

The benzyl group was removed from the compound of step 368f by hydrogenation in the presence of Pd/C. The catalyst was removed by filtration, and the product was obtained by evaporation of the solvent.

### Step 368g. 1-cyclopropy-8-(3-aza-6-amino-6-methylbicyclo[3.3.0]octan-1-yl)-7-fluoro-9-methyl-4-oxo-4H-quinolizine-3-carboxylic acid hydrochloride

Following the procedure of Example 253 step j, replacing the 3-BOC-aminopyrrolidine thereof with 3-aza-6-(BOC-amino)-6-methylbicyclo[3.3.0]-octane, from step 369g above, and carrying the product forward as in Example 253 steps j-k, 418 mg of the title compound was prepared. IR (KBr) cm⁻¹: 3340 (br), 2860 (br), 1700 (m), 1610 (m), 1430 (s), 1370 (m). MS (CDI/NH3) m/z (M+H)⁺: 400 base. NMR (CD₃OD): 9.12 (m, 1H), 8.03 (s, 1H), 3.94 (m, 2H), 3.78 (m, 1H), 3.57 (m, 2H), 2.83 (m, 1H), 2.78 (m, 3H), 2.31 (m, 1H), 1.88 (m, 2H), 2.19 (m, 2H), 1.50 (s, 3H), 1.07 (m, 2H), 0.68 (m, 2H). Anal. Calcd for C₂₂H₂₇ClFN₃O₃:•1.5 H₂O C.57.62; H, 6.37; N, 9.06; Found: C, 58.02; H, 6.64; N, 9.23.

### Example 369

### 1-cyclopropyl-8-(3-fluoromethylpiperidinyl)-7-fluoro-9-methyl-4-oxo-4H-quinolizine

### Step 369a. N-BOC-3-hydroxymethylpiperidine

A sample of 3-hydroxymethylpiperidine (2.0g,17.4mmol) was suspended in 60 mL of water and cooled to 0C. Sodium bicarbonate (2.63g, 31mmol) was added in one portion, then benzyl chloroformate (2.60ml, 18.3mmol) was added dropwise in 10ml of diethyl ether. After stirring for 4 hours at 0C, the reaction was poured into 150ml water and extracted with methylene chloride (3X 100ml). The combined organic layers were dried over sodium sulfate, then filtered and the filtrate evaporated to dryness to yield 3.74g (86%).

### Step 369b. N-BOC-3-fluoromethylpiperidine

This compound from step 369a (3.74g, 15mmol) was then dissolved in 10ml of methylene chloride and added dropwise to a solution of diethylaminosulfur trifluoride (2.59ml, 19.5mmol) in 10ml of methylene chloride at -78C. After the addition, the reaction was stirred at room temperature for 16 hours. 10ml of water, then 30ml of 1M sodium hydroxide was added dropwise to the reaction, then the product was extracted into methylene chloride (3X75ml). The combined organic layers were dried over sodium sulfate, filtered, and the filtrate was evaporated to dryness. The product was purified by flash chromatography (100% methylene chloride) to yield 2.42g (64%).

### Step 369c. 3-fluoromethylpiperidine

The amine was deprotected under hydrogenation conditions in methanol using palladium on carbon (2g). After 16h at room temperature and 4atm, the catalyst was filtered off and the filtrate concentrated to yield: 808 mg (68%) of the desired amine.

### Step 369d. 1-cyclopropyl-8-(3-fluoromethylpiperidinyl)-7-fluoro-9-methyl-4-oxo-4H-quinolizine

Following the procedure of Example 253 step j, replacing the 3-BOC-aminopyrrolidine thereof with 3-fluoromethylpiperidine, from step 369d above, and carrying the product forward as in Example 253 steps j-k, 198 mg of the title compound was prepared. IR (KBr) cm⁻¹: 2950 (br), 1650 (s), 1470 (s), 1440 (s), 1350 (m). MS (CDI/NH3) m/z (M+H)⁺: 377 base. NMR (CDCl₃): 9.22 (d, 1H, J=9 Hz), 8.37 (s, 1H), 4.21-4.53 (m, 4H), 3.14-3.67 (m, 7H), 2.79 (s, 3H), 2.25 (m, 1H), 1.04 (m, 2H), 0.72 (m, 2H). Anal. Calcd for C₂₀H₂₂F₂N₂O₃: C, 63.82; H, 5.89; N, 7.44; Found: C, 63.35; H, 5.83; N, 6.85.

### Example 370

### 1-cyclopropyl-8-(4-(N,N-dimethyl)aminopiperidinyl)-7-fluoro-9-methyl-4-oxo-4H-quinolizine-3-carboxylic acid hydrochloride

### Step 370a. 4-(N,N-dimethyl)aminopiperidine

4-(N,N-dimethyl)aminopyridine (1.0g, 8.2mmol) was subjected to hydrogenation conditions in 100ml methanol using Rhodium (50 mg) at room temperature and 4atm for 72 hours. The catalyst was filtered off and the filtrate was evaporated to yield 100% of the desired amine.

### Step 370b. 1-cyclopropyl-8-(4-(N.N-dimethyl)aminopiperidinyl)-7-fluoro-9-methyl-4-oxo-4H-quinolizine-3-carboxylic acid hydrochloride

Following the procedure of Example 253 step j, replacing the 3-BOC-aminopyrrolidine thereof with 4-(N,N-dimethyl)aminopiperidine, from step 370a above, and carrying the product forward as in Example 253 steps j-k, 345 mg of the title compound was prepared. IR (KBr) cm⁻¹: 2950 (br), 1710 (m), 1610 (m), 1470 (s), 1440 (s). MS (CDI/NH3) m/z (M+H)⁺: 388 base. Anal. Calcd for C₂₁H₂₇ClFN₃O₃: C, 59.50; H, 6.42; N, 9.91; Found: C, 59.72; H, 6.69; N, 9.33.

### Example 371

### 1-cyclopropyl-8-(6-amino-3-azabicyclo[3.3.0]octyl)-7-fluoro-9-methyl-4-oxo-4H-quinolizine-3-carboxylic acid hydrochloride

### Step 371a. 3-aza-3-benzyl-6-(hydroxylimino)bicyclo[3.3.0]octane

A 3.24 sample of 3-aza-6-oxobicyclo[3.3.0]octane, prepared as in Example 368a above, was dissolved in 40 mL of THF. Hydroxylamine hydrochloride (3.14 g) was dissolved in 60 mL of water and 4.05 g of NaHCO₃ was added to neutralize the salt. The neutral hydroxylamine solution was added to the THF solution, and the reaction mixture was stirred vigorously at room temperature for 18 hours. The THF was removed from the mixture under vacuum, and the aqueous solution was extracted with methylene chloride, which was dried over sodium sulfate, filtered and evaporated to dryness to yield 2.80 g of the title compound.

### Step 371b. 3-aza-3-benzyl-6-aminobicyclo[3.3.0]octane

A 29.37 g sample of 3-aza-6-(hydroxylimino)bicyclo[3.3.0]octane, prepared as in step 371a above, was dissolved in 1 L of methanol and hydrogenated at 4 Atm of H₂ over 58.74 g of RaNi catalyst for 24 hours. The catalyst was filtered off, and the solvent was evaporated to afford the title compound.

### Step 371c. 3-aza-3-benzyl-6-(BOC-amino)bicyclo[3.3.0]octane

A 2.63 g sample of 3-aza-3-benzyl-6-aminobicyclo[3.3.0]octane, from step 371b above, was dissolved in 25 mL of methylene chloride, 3.39 mL of triethylamine was added, and the solution was cooled to 0°C. A 3.98 g sample of di-t-butyl dicarbonate was dissolved in 6 mL of methylene chloride and added dropwise to the first solution. The reaction mixture was stirred 30 min at 0°C and at room temperature for 18 hours, the quenched by rapid addition to water. The mixture was extracted with methylene chloride, which was dried over sodium sulfate, filtered and evaporated to dryness. The residue was purified by column chromatography, eluting with 2% methanol in methylene chloride to afford the title compound.

### Step 371d. 3-aza-6-(BOC-amino)bicyclo[3.3.0]octane

The compound from step 371c above was dissolved in 150 mL of methanol and hydrogenated for 23 hours at room temperature and 4 Atm of H₂ over 1.5 g of 10% Pd/C catalyst. The catalyst was filtered off, and the solvent was evaporated to afford the title compound.

### Step 371e. 1-cyclopropyl-8-(6-amino-3-azabicyclo[3.3.0]octyl)-7-fluoro-9-methyl-4-oxo-4H-quinolizine-3-carboxylic acid hydrochloride

Following the procedure of Example 253 step j, replacing the 3-BOC-aminopyrrolidine thereof with 6-(BOC-amino)-3-azabicyclo[3.3.0]octane, from step 371d above, and carrying the product forward as in Example 253 steps j-k, 298 mg of the final compound was prepared. IR (KBr) cm⁻¹: 2900 (br), 1700 (m), 1610 (m), 1430 (s), 1380 (m). MS (CDI/NH3) m/z (M+H)⁺: 386 base. NMR (CD₃OD): 9.04 (d, 1H, J=9 Hz), 7.97 (s, 1H), 3.92 (m, 2H), 3.78 (m, 2H), 3.57 (m, 1H), 3.15 (m, 1H), 3.04 (m, 1H), 2.76 (s, 3H), 2.29 (m, 1H), 1.69-2.21 (m, 3H), 1.08 (m, 2H), 0.67 (m, 2H). Anal. Calcd for C₂₁H₂₅ClFN₃O₃•1.5 H₂O• HCl: C, 51.97; H, 6.02; N, 8.66; Found: C, 52.07; H, 5.81; N, 8.48.

### Example 372

### 1-cyclopropyl-8-((2-aza-4-(dimethylaminomethyl)bicyclo[4.3.0]non-2-yl)-7-fluoro-9-methyl-4-oxo-4H-quinolizine carboxylic acid hydrochloride

### Step 372a. 2-aza-4-dimethylaminomethylbicyclo[3.3.0]nonane

A 1 g sample of 3-dimethylaminomethylindole was hydrogenated over Pd/C in acetic acid/HCl, the catalyst removed by filtration, and the solvent diluted with water, adjusted to pH 11, and extracted with ethyl acetate. The solvent was dried and evaporated to afford the title compound.

### Step 372b. 1-cyclopropyl-8-((2-aza-4-(dimethylaminomethyl)bicyclo[4,3,0]non-2-yl)-7-fluoro-9-methyl-4-oxo-4H-quinolizine carboxylic acid hydrochloride

Following the procedure of Example 253 step j, replacing the 3-BOC-aminopyrrolidine thereof with 2-aza-4-(dimethylaminomethyl)bicyclo[4.3.0]-nonane, from step 372a above, and carrying the product forward as in Example 253 steps j-k, 354 mg of the final compound was prepared. IR (KBr) cm⁻¹: 3400 (br), 2950 (m), 2600 (br), 1720 (m), 1610 (m), 1430 (s), 1380 (m). MS (CDI/NH3) m/z (M+H)⁺: 442 base. NMR (CDCl₃): 9.07 (d, 1H, J=9 Hz), 8.28 (s, 1H), 4.47 (m, 1H), 4.04 (m, 1H), 3.60 (m, 1H), 3.18 (m, 2H), 2.75 (s, 3H), 2.49 (m, 1H), 2.27 (m, 1H), 1.26 (m, 2H), 1.00-1.90 (m, 9H), 2.91 (s, 6H), 0.70 (m, 2H). Anal. Calcd for C₂₅H₃₃ClFN₃O₃•1.25 H₂O: C, 60.59; H, 7.73; N, 8.48; Found: C, 60.07; H, 7.71; N, 8.15.

### Example 373

### 1-cyclopropyl-8-(3-aza-6-(L-alanylamino)-6-methylbicyclo[3.3.0]octane)-7-fluoro-9-methyl-4-oxo-4H-quinolizine carboxylic acid hydrochloride

A 50 mg sample of 1-cyclopropyl-8-(3-aza-6-(L-alanylamino)-6-methylbicyclo[3.3.0]octane)-7-fluoro-9-methyl-4-oxo-4H-quinolizine carboxylic acid hydrochloride, from Example 368, was dissolved in 3 mL of DMF, and the solution was cooled to 0°C. A 0.044 mL sample of diisopropylethylamine was added, followed by 35 mg of N-BOC-L-alanyl-N-hydroxysuccinimide. and the reaction was stirred at 0°C for 20 min and at room temperature for 48 hours. The solution was poured into a large volume of water, and the product was filtered off and dried. IR (KBr) cm⁻¹: 2950 (br), 1680 (m), 1430 (s). MS (CDI/NH3) m/z (M+H)⁺: 471 base. Anal. Calcd for C₂₅H₃₂ClFN₄O₄•H₂O: C, 57.19; H. 6.14; N, 10.67; Found: C, 57.16: H, 6.48; N, 9.90.

### Example 374

### (3R, 1R)-8-(3-(1-(N-methyl)amino)propyl)pyrrolidinyl)-1-cyclopropyl-7-fluoro-9-methyl-4-oxo-4H-quinolizine-3-carboxylic acid hydrochloride

A sample of 8-chloro-1-cyclopropyl-7-fluoro-9-methyl-4-oxo-4H-quinolizine-3-carboxylic acid ethyl ester, from Example 253i above, was dissolved in 8 mL of anhydrous acetonitrile, reacted with (3R,lR)-3-(1-(N-methyl)amino)propyl)pyrrolidine (prepared as described by Hayakawa *et al*., U.S. Patent 5,098,912, issued Mar. 24, 1992, using modifications for chiral products described by Plummer *et al., Tetr. Lett.* 34:7529-32 (**1993**)), and carried forward as described in Example 253 j-1, omitting the deprotecting step, to give the title product. MS 402 (M+H)⁺; ¹H NMR (D₆-DMSO) ∂: 0.6-0.7 (m, 3H), 0.9 (t, 3H), 1-1.5 (m, 2H), 16-1.95 (m, 4H), 2.1-2.2 (m, 1H), 2.6-2.65 (m, 1H), 2.60 (s, 3H), 2.7 (s, 3H), 3.45-3.55 (m, 1H), 3.7-3.75 (m, 2H), 3.95-4 (m, 1H), 8.25 (s, 1H), 9.1 (d, 2H)

### Examples 375-408

Following the procedures of Steps 253j, 253k and 253l above, replacing the 3-BOC-aminopyrrolidine of Step 253j with the appropriate unprotected or BOC-protected reagent. the compounds of Examples 375-412 are prepared as shown in Table 3, below.

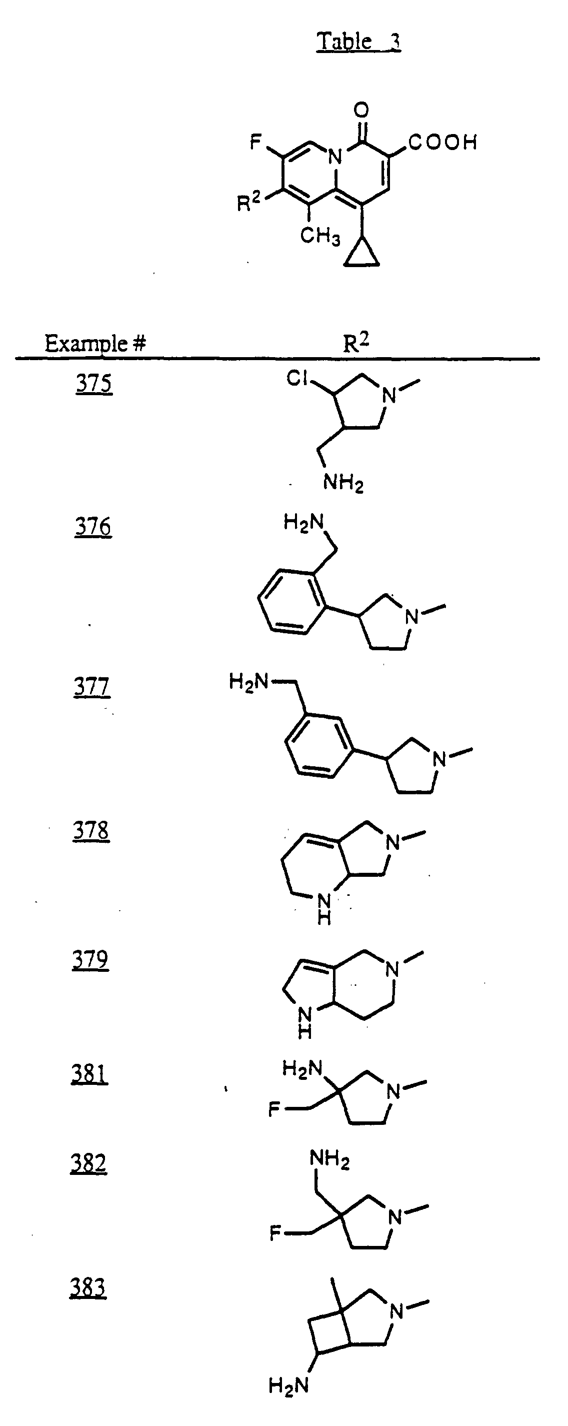

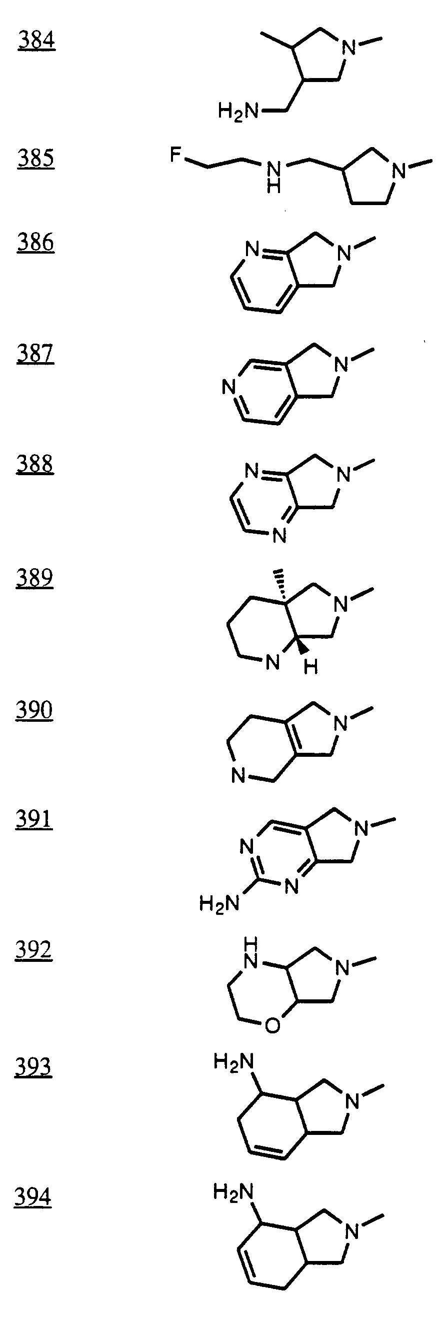

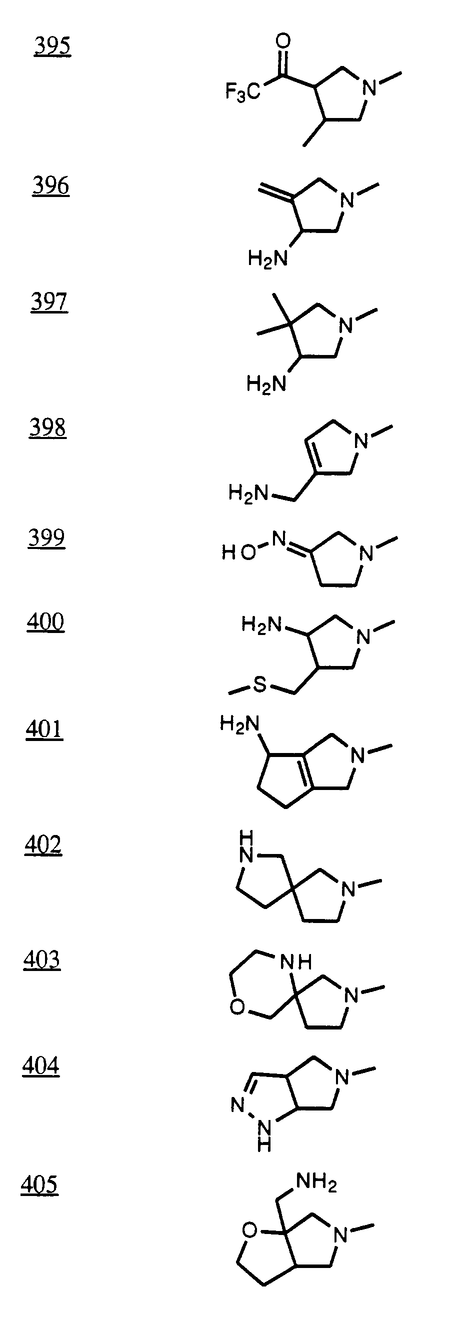

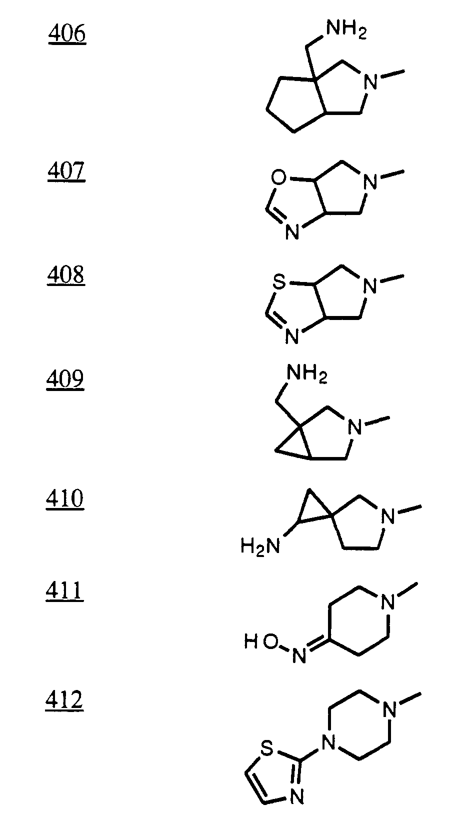

### Example 413

### (3R,1S)-8-(3-(1-amino-2-methoxyethyl)pyrrolidinyl)-1-cyclopropyl-7-fluoro-9-methyl-4-oxo-4H-quinolizine-3-carboxylic acid hydrochloride

### Step 413a. (S)-N-BOC-O-(t-butyldimethylsilyl)serine methyl ester

A 7 g (31.96 mmol) sample of ((S)-N-BOC-serine methyl ester (obtained from Aldrich) was dissolved in pyridine and cooled in an ice bath. To this stirred solution was added dropwise 5.54 g (36.76 mmol) of t-butyldimethylsilyl chloride (TBDMSC) dissolved in 40 mL of pyridine. After all reagents were added the reaction was stirred for 4 hours at room temperature. An additional 0.5 g of TBDMSC was added and the reaction was stirred for an additional 2 hours. To the mixture was then added 2.5 equivalents of imidazole in 14 mL of DMF, and the reaction was stirred for 2 hours. The solvents were removed under reduced pressure, and the residue was dissolved in ethyl acetate, which was washed with water and brine. The solvent was removed to give the title compound as a yellow oil. MS 334 (M+H)⁺; ¹H NMR (CDCl₃) ∂: 0.11 (s, 6H), 0.86 (s, 9H), 1.46 (s, 9H), 3.74 (s, 3H), 3.82 (dd, 1H), 4.04 (dd, 1H), 4.36 (m, 1H), 5.35 (br, 1H).

### Step 413b. (S)-2-(BOC-amino)-3-(t-butyldimethylsilyloxy)-1-propanol

A solution of the compound from step 413a above (9.6 g, 28.83 mmol) in 44 mL of THF was added dropwise to a cooled (ice bath) suspension of 570 mg (14.84 mmol) of LAH in 15 mL of THF under N₂ atmosphere. The mixture was stirred for 1.5 hours, the reaction was quenched with water and 50% NaOH, filtered, and the filtrate evaporated to obtain the crude product. An oil was obtained, which was purified by chromatography on silica gel, eluting with 15-20% ethyl acetate:hexane to give 3.465 g of the tide product as a colorless oil. MS 306 (M+H)⁺; ¹H NMR (CDCl₃) ∂: 0.08(s, 6H), 0.90 (s, 9H), 1.45 (s, 9H), 2.68 (br, 1H), 3.68 (m, 2H), 3.81 (d, 2H), 3.85 (m, 1H), 5.15 (br, 1H).

### Step 413c. (S)-2-(BOC-amino)-3-(t-butyldimethylsilyloxy)-1-propanal

To a solution of the compound from step 413b above (3.47 g, 11.36 mmol) in 6 mL of DMSO cooled to 0°C was added dropwise 5.2 mL (37.49 mmol) of triethylamine. Pyridine•SO₃ complex (5.424 g, 34.08 mmol) was dissolved in 21 mL of DMSO and added to the first solution, and the reaction was stirred for 1.5 hours after the addition was complete. The solution was poured into 120 mL of cold brine, and the mixture was washed 3x with ethyl acetate. The extract was washed with water, dried over MgSO4, filtered and the solvent was removed under vacuum to give 3.9 g of a yellow oil, which was taken directly to the next step.

### Step 413d. (S)-4-(BOC-amino)-5-(t-butyldimethylsilyloxy)-2-pentenoic acid ethyl ester

To a solution of the compound from step 413c above (11.36 mmol) in 24 mL of CH₂Cl₂ and cooled in an ice bath was added dropwise 3.958 g (11.36 mmol) of (carboethoxymethylene)triphenylphosphorane in 13 mL of CH₂Cl₂. After addition was complete, the reaction was stirred for 16 hours at room temperature. The solvent was removed, and the residue was purified by column chromatography on silica gel, eluting with 3-10% ethyl acetate:hexane, to give 3.93 g of a colorless oil. MS 374 (M+H)⁺; ¹H NMR (CDCl₃) ∂: 0.05 (d, 6H), 0.88 (s, 9H), 1.27 (t, 3H), 1.46 (s, 9H), 3.72 (m, 2H), 4.19 (q, 2H), 4.36 (br, 1H), 5.98 (dd, 1H), 6.91 (dd, 1H).

### Step 413e. (S)-4-(BOC-amino)-5-(t-butyldimethylsilyloxy)-3-(nitromethyl)-pentanoic acid ethyl ester

To a solution of the compound from step 413d above (3.9 g, 10.46 mmol) in 6 mL of nitromethane cooled in an ice bath was added 1.56 mL (10.46 mmol) of 1,8-diazabicyclo[5.4.0]undec-7-ene dropwise under N₂. The mixture was warmed to room temperature and stirred for 16 hours. The solution was diluted with CH₂Cl₂ and extracted with water, 10% HCl, 10% NaHCO₃, water and brine. The solution was dried over MgSO4, and the solvent was removed. The residue was chromatographed on silica gel, eluting with 5-10% ethyl acetate:hexane, and the solvent was removed to give 3.6 g of the title product as a white solid. MS 435 (M+H)⁺; ¹H NMR (CDCl₃) ∂: 0.09 (s, 6H), 0.91 (s, 9H), 1.28 (t, 3H), 1.45 (s, 9H), 2.45 (dd, 1H), 2.60 (dd, 1H), 2.93 (m, 1H), 3.68 (dd, 1H), 3.78 (dd, 1H), 3.84 (m, 1H), 4.15 (q, 2H), 4.52 (dd, 1H), 4.67 (dd, 1H), 4.84.

### Step 413f. (S)-4-(BOC-amino)-5-(t-butyldimethylsilyloxy)-3-(aminomethyl)-pentanoic acid ethyl ester

A 4.74 g sample of the compound from step 413e above was dissolved in 250 mL of ethanol and hydrogenated at 4 Atm over 14.2 g of Raney nickel catalyst for 24 hours. The catalyst was removed by filtration and the solvent was evaporated. The residue (mp 152-154°C) was taken directly to the next step.

### Step 413g. (S)-4-(1-(BOC-amino)-2-(t-butyldimethylsilyloxy)ethyl)-2-oxo-4-pyrrolidine

The residue from step 413f above was dissolved in 150 mL of ethanol and heated at reflux for 8 hours. The solvent was removed, the residue was chromatographed on silica gel, eluting with 4% methanol/methylene chloride. Removal of the solvent gave the title product.

### Step 413h. (S)-4-(1-(BOC-amino)-2-(t-butyldimethylsilyloxy)ethyl)-1-benzyl-2-oxopyrrolidine

A 200 mg (0.558 mg) sample of the compound from step 413g above was dissolved in 1 mL of THF and added dropwise to a 0°C suspension of NaH (47 mg, 1.172 mmol) in 2 mL of THF, and the reaction mixture was stirred for 1 hour. To this mixture was then added 124 mg of benzyl bromide, and the reaction was stirred at room temperature for 3 hours. The reaction was quenched with water, and the mixture was extracted with ethyl acetate. The organic phase was acidified with citric acid solution, and the mixture was extracted with ethyl acetate. The solvent was washed with brine and dried over MgSO₄, filtered and evaporated. The residue was purified by column chromatography on silica gel, eluting with 30-35% ethyl acetate:hexane, to give 168 mg of the title compound. MS 449 (M+H)⁺; ¹H NMR (CDCl₃) ∂: 0.03 (s, 6 H), 0.87 (s, 9H), 1.42 (s, 9H), 2.26 (dd, 1H), 2.52 (dd, 1H), 2.58 (m, 1H), 3.16 (br t, 1H), 3.27 (dd, 1H), 3.61 (br m, 3H), 4.28 (d, 1H), 4.59 (d, 1H), 4.70 (d, 1H), 7.23 (m, 2H), 7.32 (m, 3H).

### Step 413i. (S)-4-(1-(BOC-amino)-2-hydroxyethyl)-1-benzyl-2-oxopyrrolidine

A 143 mg sample of the compound from step 413h above was dissolved in 1 mL of THF and reacted with 1 equivalent of tetra-n-butyl ammonium fluoride at room temperature for 1.5 hours. The solvent was removed, and the residue was dissolved in methylene chloride and purified by column chromatography on silica gel, eluting with 5% methanol in methylene chloride, to give 110 mg of the title compound. MS 335 (M+H)⁺; ¹H NMR (CDCl₃) ∂: 1.42 (s, 9H), 2.28 (m, 1H), 2.59 (m, 3H), 3.15 (m, 1H), 3.31 (m, 1H), 3.61 (m, 2H), 3.70 (m, 1H), 4.30 (d, 1H), 4.58 (d, 1H), 4.78 (d, 1H), 7.23 (m, 2H), 7.32 (m, 3H).

### Step 413j. (S)-4-(1-(BOC-amino)-2-methoxyethyl)-1-benzyl-2-oxopyrrolidine

A sample of the compound from step 413i above (7.34 mmol) was dissolved in 22 mL of THF and added to a suspension of 8.72 mg (16.148 mmol) of sodium methoxide in 40 mL of THF, and the reaction mixture was stirred at room temperature under nitrogen for 1 hour. To this solution was then added 3.958 g of methyl iodide in 5 mL of THF, and the reaction mixture was stirred for 16 hours. The solvents were removed under vacuum, and the residue was dissolved in ethyl acetate, which was washed with sodium thiosulfate and brine and dried over MgSO₄, filtered and evaporated. The residue was dissolved in methylene chloride and purified by column chromatography on silica gel, eluting with 5% methanol in methylene chloride, to give the title compound. MS 349 (M+H)⁺; ¹H NMR (CDCl₃) ∂: 1.42 (s, 9h), 2.28 (dd, 1H), 2.56 (m, 3H), 3.14 (br t, 1H), 3.28 (dd, 1H), 3.30 (s, 3H), 3.37 (d, 2H), 3.71 (br, 1H), 4.24 (dd, 1H), 4.52 (dd, 1H), 4.80 (d, 1H), 7.23 (m, 2H), 7.31 (m, 3H).

### Step 413k. (S)-4-(1-(BOC-amino)-2-methoxyethyl)-1-benzyl-2-thioxopyrrolidine

A 50 mg (0.14 mmol) sample of the compound from step 413j above and 29 mg (0.07 mmol) of Lawesson's reagent were dissolved in 0.3 mL of THF and stirred under N2 for 3 hours. The solvent was removed, and the residue was dissolved in CH₂Cl₂ and chromatographed on silica gel, eluting with 30% ethyl acetate:hexane. Removal of the solvent left 51 mg of product. MS 365 (M+H)⁺; ¹H NMR (CDCl₃) ∂: 1.41 (s, 9H), 2.64 (dd, 1H), 2.87 (dd, 1H), 3.16 (dd, 1H), 3.29 (s, 3H), 3.36 (d, 2H), 3.55 (m, 2H), 3.70 (m, 1H), 4.70 (d, 1H), 4.83 (d, 1H), 5.21 (d, 1H), 7.33 (m, 5H).

### Step 413l. (S)-3-(1-(BOC-amino)-2-methoxyethyl)-1-benzylpyrrolidine

A 45.7 mg (0.125 mmol) sample of the compound from step 413k above and 239 mg (1.0 mmol) of NiCl2•6H₂O were dissolved in 2 mL of a 1:1 mixture of methanol and THF, and the solution was cooled to -78°C and stirred under N₂. A 114 mg (3.0 mmol) sample of NaBH₄ was added in portions, and the mixture was stirred for 2 hours. The solvents were removed under vacuum, and dissolved in 20% methanol in chloroform. The solution was filtered and the solvent removed. The residue was chromatographed on silica gel, eluting with 5%methanol in chloroform to provide 23 mg of title product. MS 335 (M+H)⁺; ¹H NMR (CDCl₃) ∂: 1.45 (s, 9H), 2.01 (m, 1H), 2.37 (m, 1H), 2.49 (m, 2H), 2.61 (m, 1H), 2.71 (m, 1H), 3.32 (s, 3H), 3.35 (m, 2H), 3.44-3.67 (m, 4H), 7.23-7.33 (m, 5H).

### Step 413m. (S)-3-(1-(BOC-amino)-2-methoxyethyl)-pyrrolidine

A 203 mg sample of the compound from step 413l above was dissolved in 25 mL of methanol and hydrogenated at 4 Atm over 50 mg of 10% Pd/C catalyst for 22 hours. The catalyst was removed by filtration and the solvent was evaporated to give 160 mg of the title compound as a viscous oil. MS 245 (M+H)⁺; ¹H NMR (CD₃OD) ∂: 1.43 (s, 9H), 1.92 (m, 1H), 2.24 (m, 1H), 2.43 (m, 1H), 2.75 (m, 1H), 2.90 (m, 1H).

### Step 413n. (3R,1S)-8-(3-(1-amino-2-methoxyethyl)pyrrolidinyl)-1-cyclopropyl-7-fluoro-9-methyl-4-oxo-4H-quinolizine-3-carboxylic acid hydrochloride

A 77 mg (0.238 mmol) sample of 8-chloro-1-cyclopropyl-7-fluoro-9-methyl-4-oxo-4H-quinolizine-3-carboxylic acid ethyl ester, from Example 253i above, was reacted with the (S)-3-(1-(BOC-amino)-2-methoxyethyl)-pyrrolidine from step 413m above and carried forward as described in Example 253 steps j-l, to give 62 mg of the title product. mp. 62-64°C. HRMS calc: 404.1986; found: 404.1990 (M+H)⁺; ¹H NMR (D6-DMSO) ∂: 0.60 (m, 2H), 0.94, (m, 1H), 2.13 (m, 1H), 2.28 (m, 2H), 2.61 (s, 3H), 3.26 (s, 3H), 3.52 (m, 2H), 3.62 (dd, 1H), 3.71 (m, 2H), 3.91 (m, 1H), 7.91 (s, 1H), 8.10 (br, 2H), 9.08 (d, 1H).

### Example 414

### In Vitro Assay of Antibacterial Activity

The *in vitro* antibacterial activity of the compounds of the present invention was demonstrated as follows: Minimum inhibitory concentrations (MICs) were determined by the agar dilution method, in which twelve petri dishes were prepared, each containing successive aqueous 2-fold dilutions of the test compounds mixed with 10 mL of sterilized Brain Heart Infusion (BHI) agar. Each plate was inoculated with 1:100 (or 1:10 for slow-growing strains, primarily *Micrococcus* and *Streptococcus)* dilutions of up to 32 different microorganisms, using a Steers replicator block calibrated to deliver approximately 10⁴ colony forming units (CFUs). The inoculated plates were incubated at from about 35°C to about 37°C for approximately 20-24 hours. In addition, a control plate using BHI agar containing no test compound was prepared and incubated at the beginning and at the end of each test. The quinolone antibacterial ciprofloxacin was used as a control ("Cntl").

After incubation, each petri dish was observed for the presence or absence of microorganism growth. The MIC was defined as the lowest concentration of test compound yielding no growth (a slight haze or sparsely isolated colonies at the inoculum spot) as compared to the growth control containing no test compound.

The results of the above tests, shown in Tables 4, 5, 6 and 7 below, demonstrate that the compounds of the present invention (Tables 5 and 7) are surprisingly effective in combating bacterial growth. Moreover, the 9-methyl quinolizinone compounds of the invention (in which R⁶ of formula (I) is methyl) are shown to have excellent activity even against the ciprofloxacin-resistant pathogen *Staphylococcus aureus* 1775, demonstrating the potential usefulness of these compounds in treating infections not susceptible to this widely-used agent.

Comparison may be made with the reference examples in Tables 4 and 6 and reference Examples 329 and 346 in Table 7, where the reference examples are:
Example 1: 3-Fluoro-9-(4-fluorophenyl)-2-(4-methylpiperazin-1-yl)-6H-6-oxo-pyrido[1,2-a]pyrimidine-7-carboxylic acid;
Example 2: 3-Fluoro-9-(4-fluorophenyl)-2-(4-methylpiperazin-1-yl)-6H-6-oxo-pyrido[1,2-a]pyrimidine-7-carboxylic acid;
Example 62: 8-(3-Amino-1-pyrrolidinyl)-1-ethyl-4H-quinolizin-4-one-3-carboxylic acid hydrochloride;
Example 64: 1-Ethyl-8-(3-methyl-1-piperazinyl)-4H-quinolizin-4-one-3-carboxylic acid hydrochloride;
Example 65: 1-Ethyl-8-(4-methylpiperazin-1-yl)-4H-quinolizin-4-one-3-carboxylic acid hydrochloride;
Example 157: 2-(3-Aminopyrrolidin-1-yl)-9-cyclopropyl-3-fluoro-6H-6-oxo-pyrido[1,2-a]pyrimidine-7-carboxylic acid hydrochloride salt;
Example 158: 2-(3-Aminopyrrolidin-1-yl)-9-cyclopropyl-3-fluoro-6H-6-oxo-pyrido[1,2-a]pyrimidine-7-carboxylic acid;
Example 159: 9-(2,4-Difluorophenyl)-3-fluoro-2-(4-methylpiperazin-1-yl)-6H-6-oxopyrido[1,2-a]pyrimidine-7-carboxylic acid;
Example 160: 2-(3-N-*t*-butoxycarbonyl)aminopyrrolidin-1-yl)-9-(2,4-difluorophenyl)-3-fluoro-6H-6-oxopyrido[1,2-a]pyrimidine-7-carboxylic acid;
Example 161: 2-(3-Aminopyrrolidin-1-yl)-9-(2,4-difluorophenyl)-3-fluoro-6H-6-oxopyrido[1,2-a]pyrimidine-7-carboxylic acid;
Example 162: 2-(3-Aminopyrrolidin-1-yl)-9-(2,4-difluorophenyl)-3-fluoro-6H-6-oxopyrido[1,2-a]pyrimidine-7-carboxylic acid trifluoroacetic acid salt;
Example 163: 9-Cyclopropyl-3-fluoro-2-(4-methylpiperazin-1-yl)-6H-6-oxo-pyrido[1,2-a] pyrimidine-7-carboxylic acid;
Example 164: 9-Cyclopropyl-3-fluoro-2-(piperazin-1-yl)-6H-6-oxo-pyrido[1,2-a]pyrimidine-7-carboxylic acid;
Example 165: 9-Cyclopropyl-3-fluoro-2-(morpholin-1-yl)-6H-6-oxo-pyrido[1,2-a]pyrimidine-7-carboxylic acid;
Example 166: 9-(2,4-Difluorophenyl)-3-fluoro-2-(3-(N-(S)-norvalyl)aminopyrrolidin-1-yl)-6H-6-oxopyrido[1,2-a]pyrimidine-7-carboxylic acid hydrochloride salt;
Example 167: 2-(3-(N-(S)-Alanyl)aminopyrrolidin-1-yl)-9-(2,4-difluorophenyl)-3-fluoro-6H-6-oxopyrido[1,2-a]pyrimidine-7-carboxylic acid hydrochloride;
Example 168: 2-(3-(N-(S)-Alanyl-(S)-alanyl)aminopyrrolidin-1-yl)-9-(2,4-difluorophenyl)-3-fluoro-6H-6-oxopyrido[1,2-a]pyrimidine-7-carboxylic acid hydrochloride;
Example 169: 2-((2S, 4S)-4-Acetamido-2-methylpyrrolidin-1-yl)-9-(2,4-difluorophenyl)-3-fluoro-6H-6-oxopyrido[1,2-a]pyrimidine-7-carboxylic acid;
Example 170: 9-(2,4-Difluorophenyl)-3-fluoro-2-(3-hydroxypyrrolidin-1-yl)-6H-6-oxopyrido[1,2-a]pyrimidine-7-carboxylic acid;
Example 171: 2-((2S, 4S)-4-Amino-2-methylpyrrolidin-1-yl)-9-(2,4-difluorophenyl)-3-fluoro-6H-6-oxopyrido[1,2-a]pyrimidine-7-carboxylic hydrochloride;
Example 172: 2-(3-Aminopyrrolidin-1-yl)-3-fluoro-9-(2,3,4,5,6-pentafluorophenyl)-6H-6-oxopyrido[1,2-a]pyrimidine-7-carboxylic acid hydrochloride salt;
Example 173: 2-((2S, 4S)-4-(N-(S)-Alanyl-(S)-alanyl)amino-2-methylpyrrolidin-1-yl)-9-(2,4-difluorophenyl)-3-fluoro-6H-6-oxopyrido[1,2-a]pyrimidine-7-carboxylic acid hydrochloride;
Example 278: (3R)-9-fluoro-3-methyl-10-(4-methyl)-1-piperidinyl)-2H,3H,6H-6-oxo-pyrano[2.3.4-ij]quinolizine-5-carboxylic acid hydrochloride;
Example 279: 3(S)-9-fluoro-3-methyl-10-(4-methyl-1-piperazinyl)-2H,3H,6H-6-oxo-pyrano[2.3.4-ij]quinolizine-5-carboxylic acid hydrochloride;
Example 280: 9-fluoro-10-(1-morpholinyl)-2H,3H,6H-6-oxo-pyrano[2.3.4-ij]quinolizine-5-carboxylic acid;
Example 281: 3(R)-10-(3-amino-1-pyrrolidinyl)-9-fluoro-3-methyl-2H,3H,6H-6-oxo-pyrano[2.3.4.-ij]quinolizine-5-carboxylic acid;
Example 282: 3(R)-10-(3-aminomethyl-1-pyrrolidinyl)-9-fluoro-3-methyl-2H,3H,6H-6-oxo-pyrano[2.3.4.-ij]quinolizine-5-carboxylic acid hydrochloride;
Example 284: 3(R)-9-fluoro-10-(3-hydroxy-1-pyrrolidinyl)-3-methyl-2H,3H,6H-6-oxo-pyrano[2.3.4.-ij]quinolizine-5-carboxylic acid;
Example 285: 9-fluoro-10-(4-methyl-1-piperazinyl)-2H,3H,6H-6-oxo-pyrano[2.3.4.-ij]quinolizine-5-carboxylic acid hydrochloride.
Example 329: 1-Cyclopropyl-7-fluoro-6-methyl-4-oxo-8-(3-aminopyrrolidinyl)-4H-quindizine-3-carboxylic acid hydrochloride
Example 346: 7,9-difluoro-4H-8-(4-methylpiperazinyl)-4-oxo-1-phenyl-quinolizine-3-carboxylic acid hydrochloride

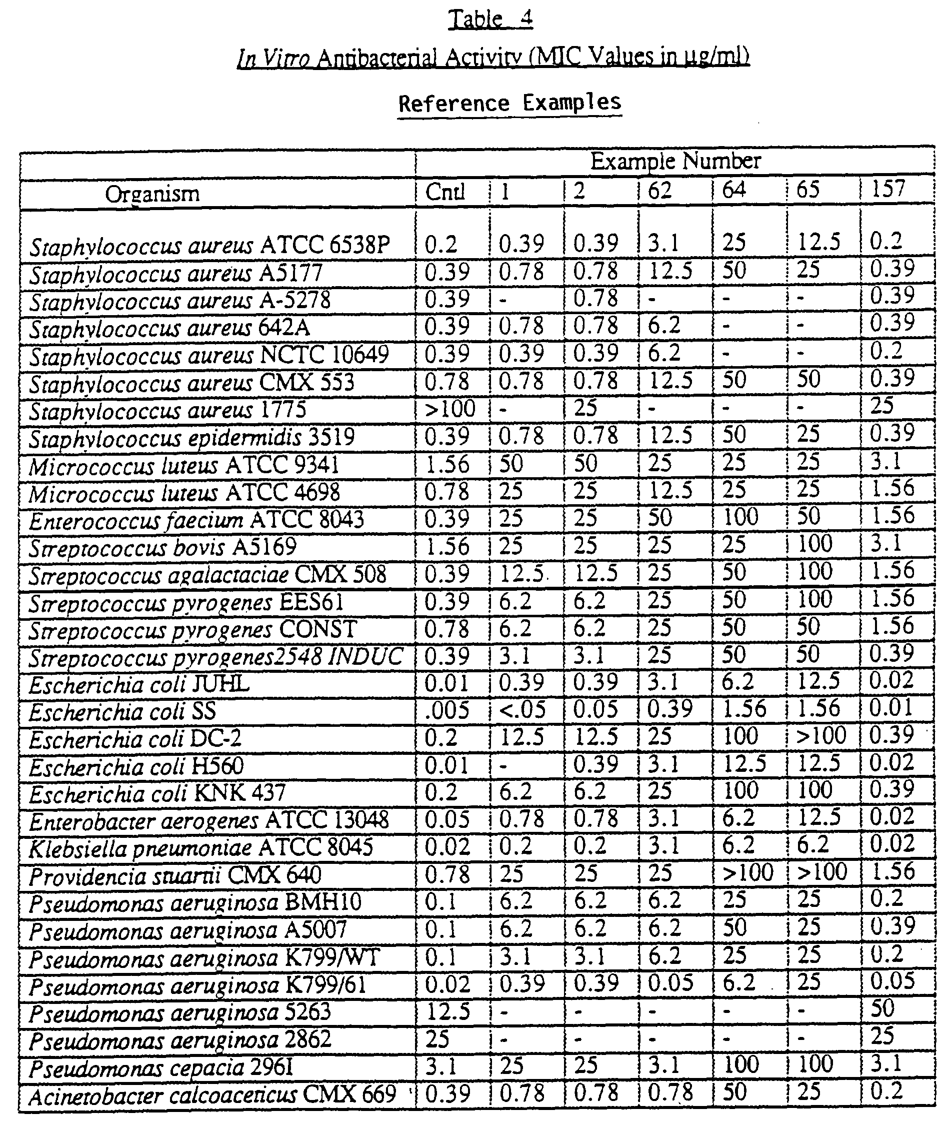

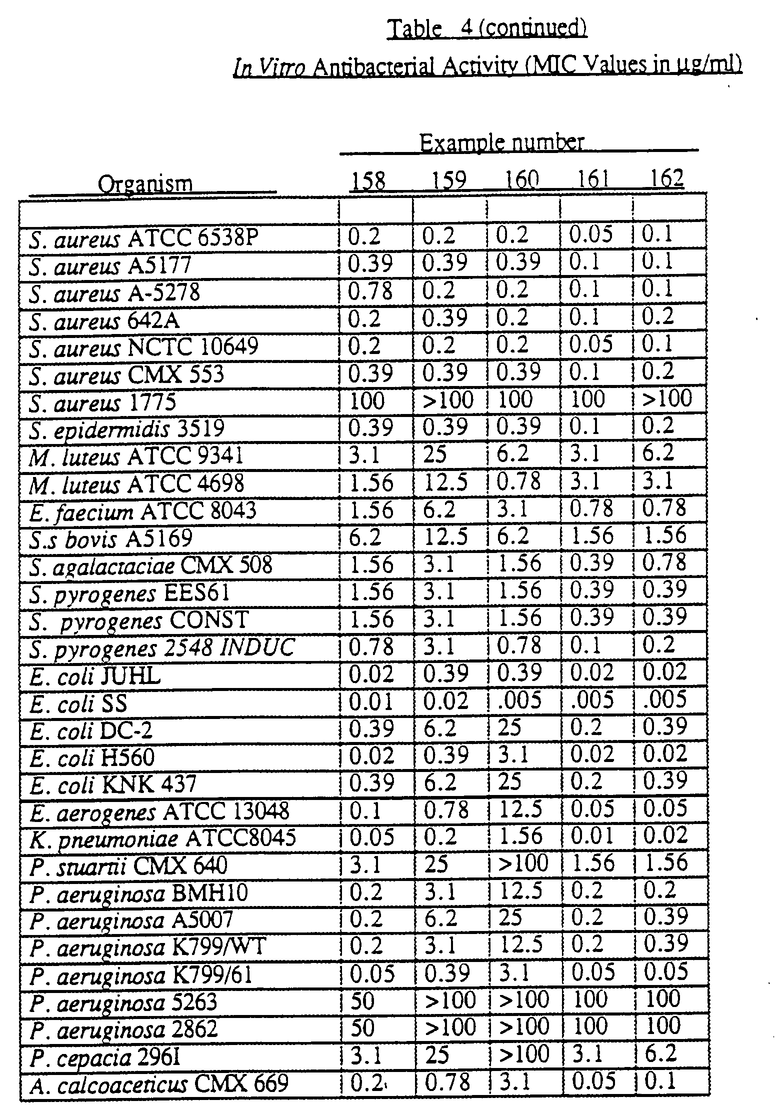

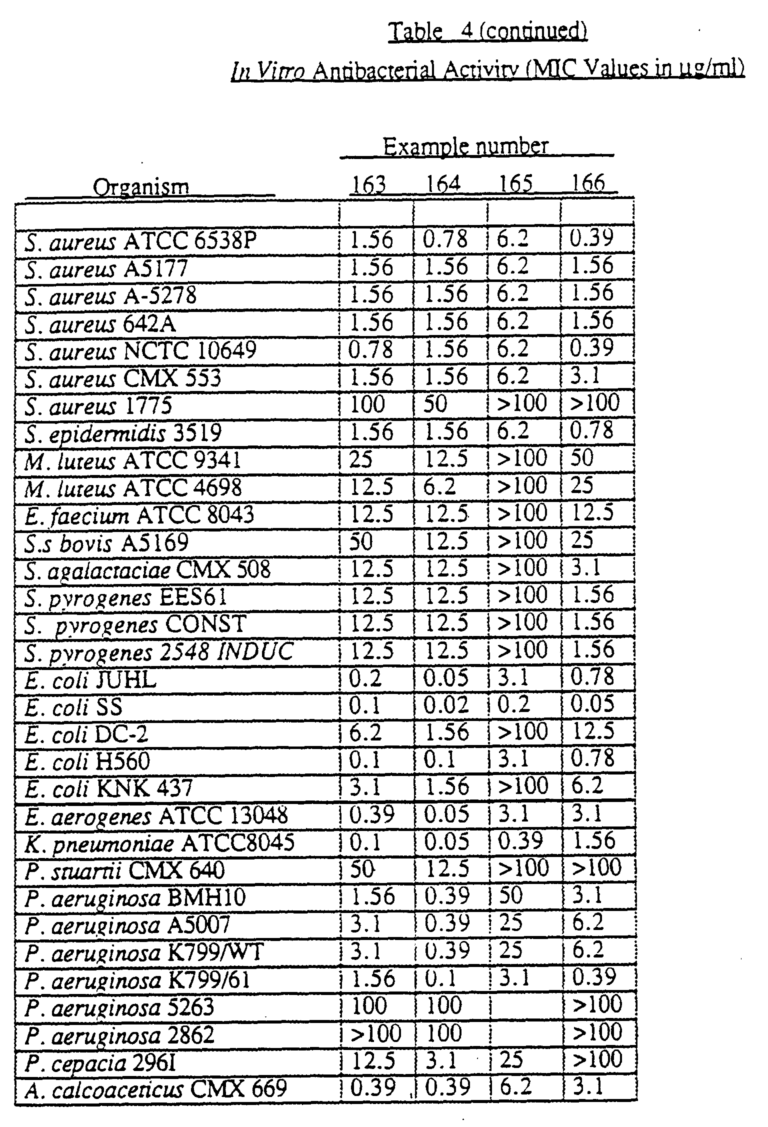

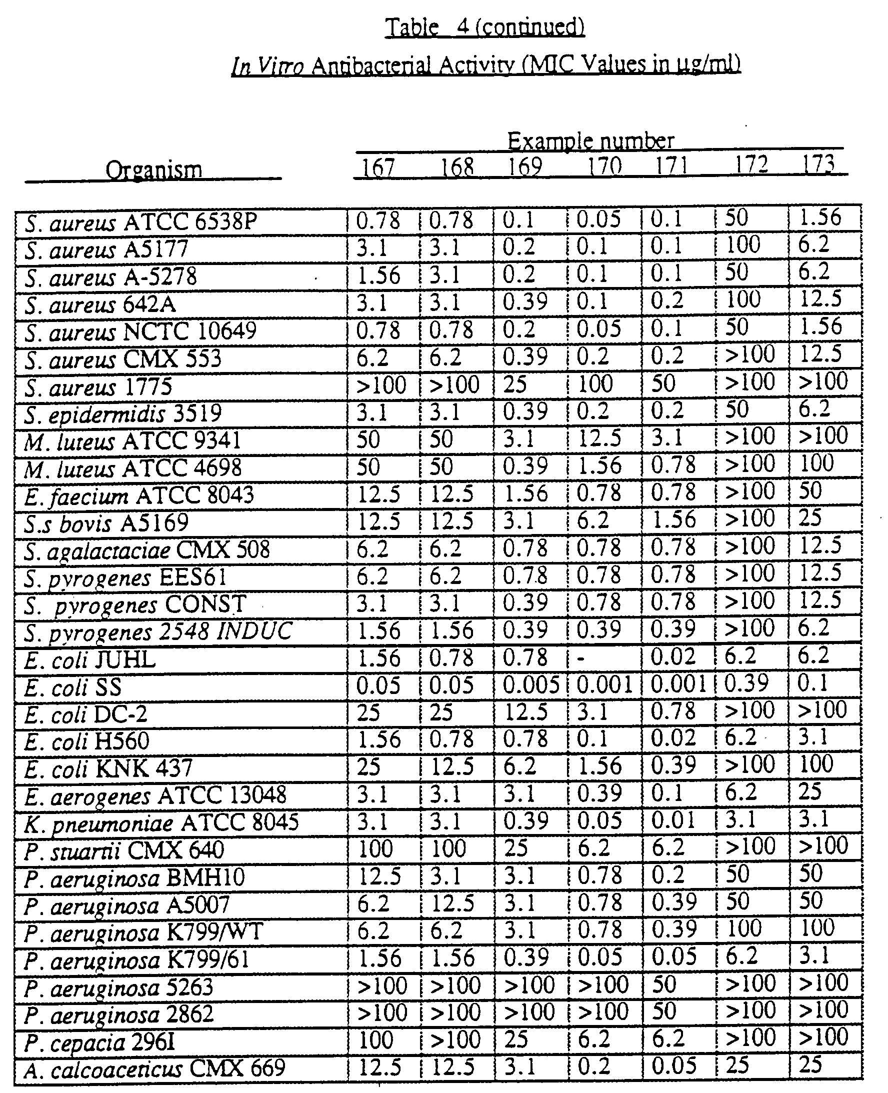

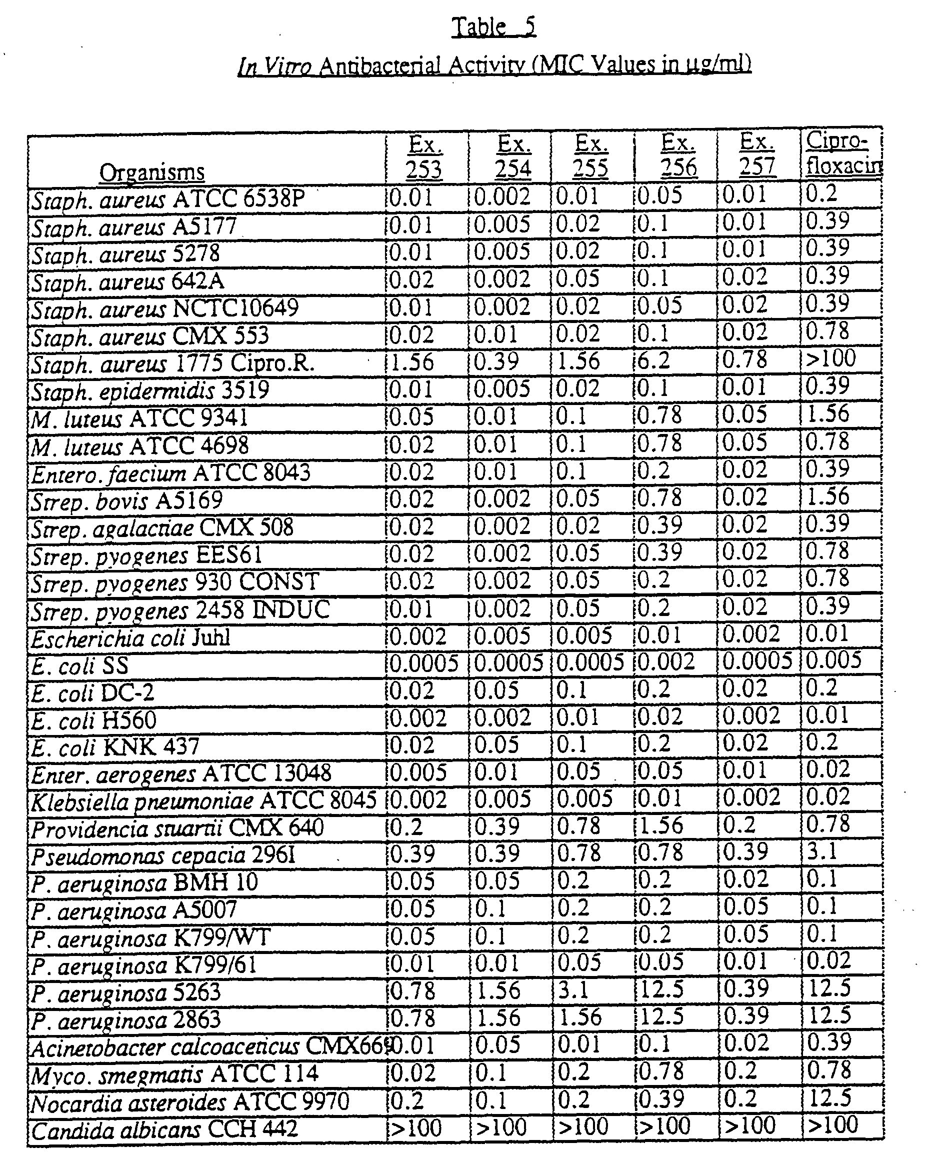

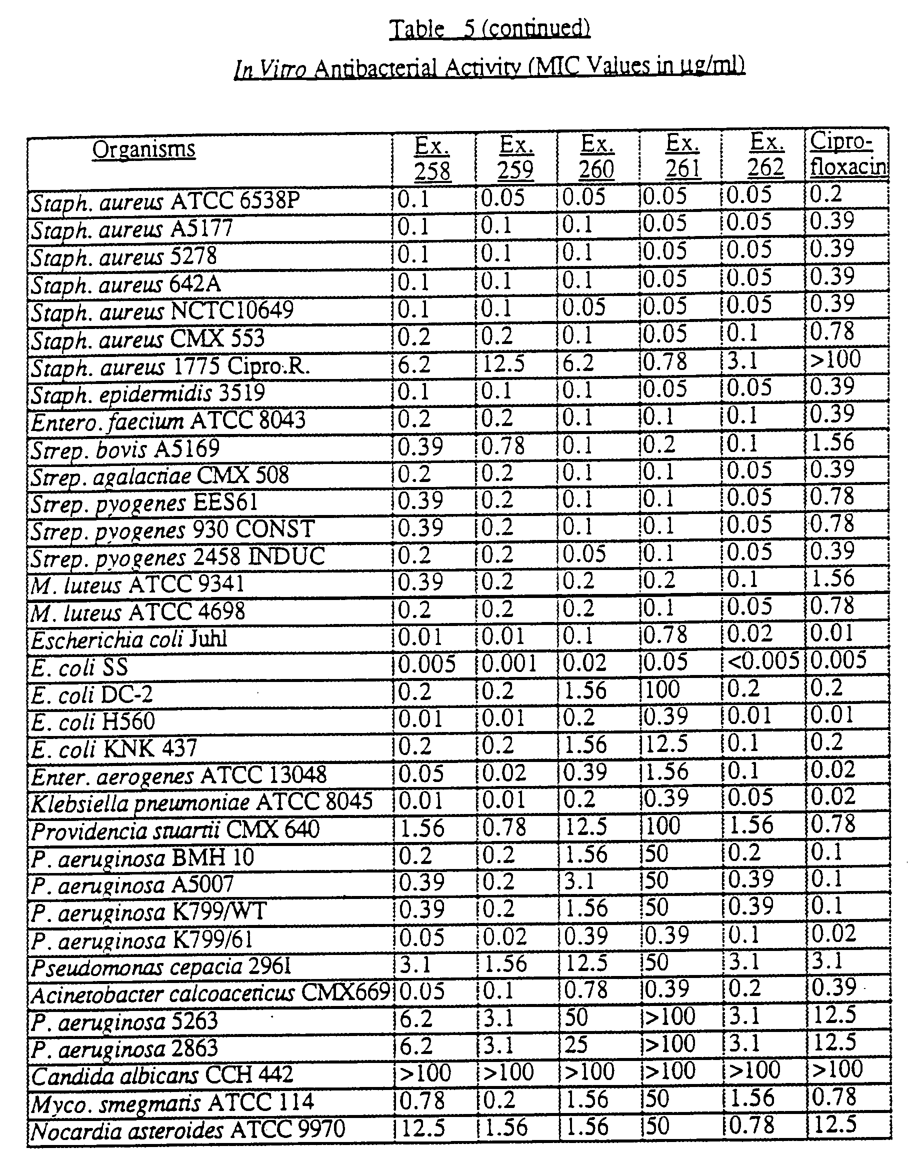

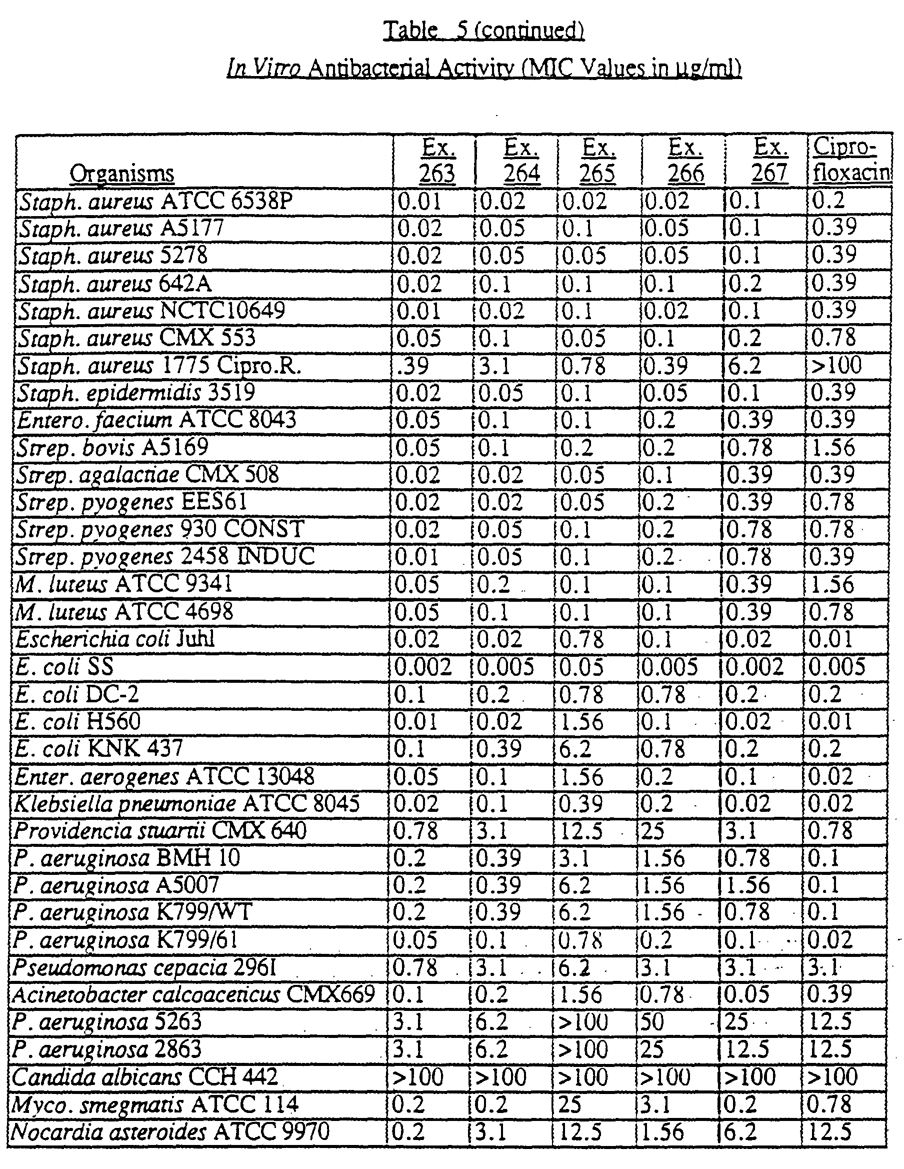

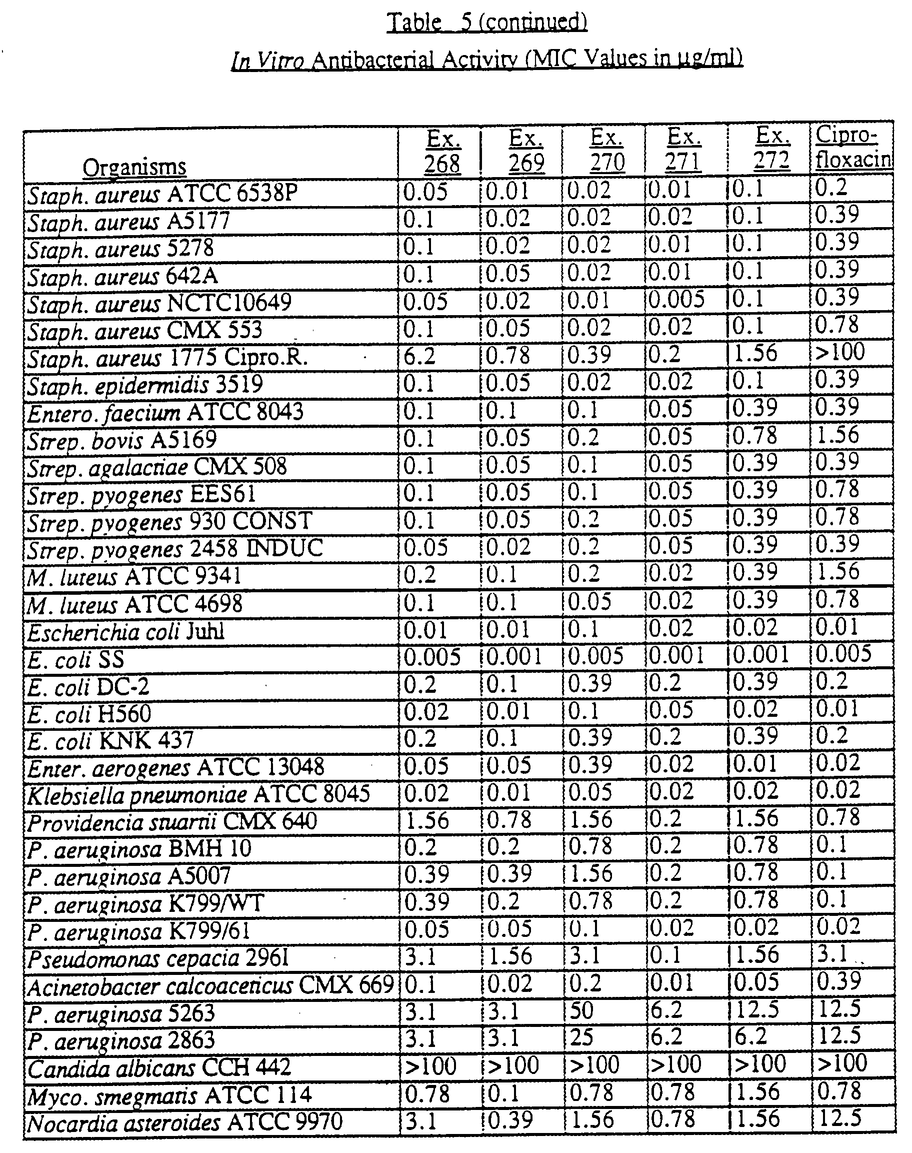

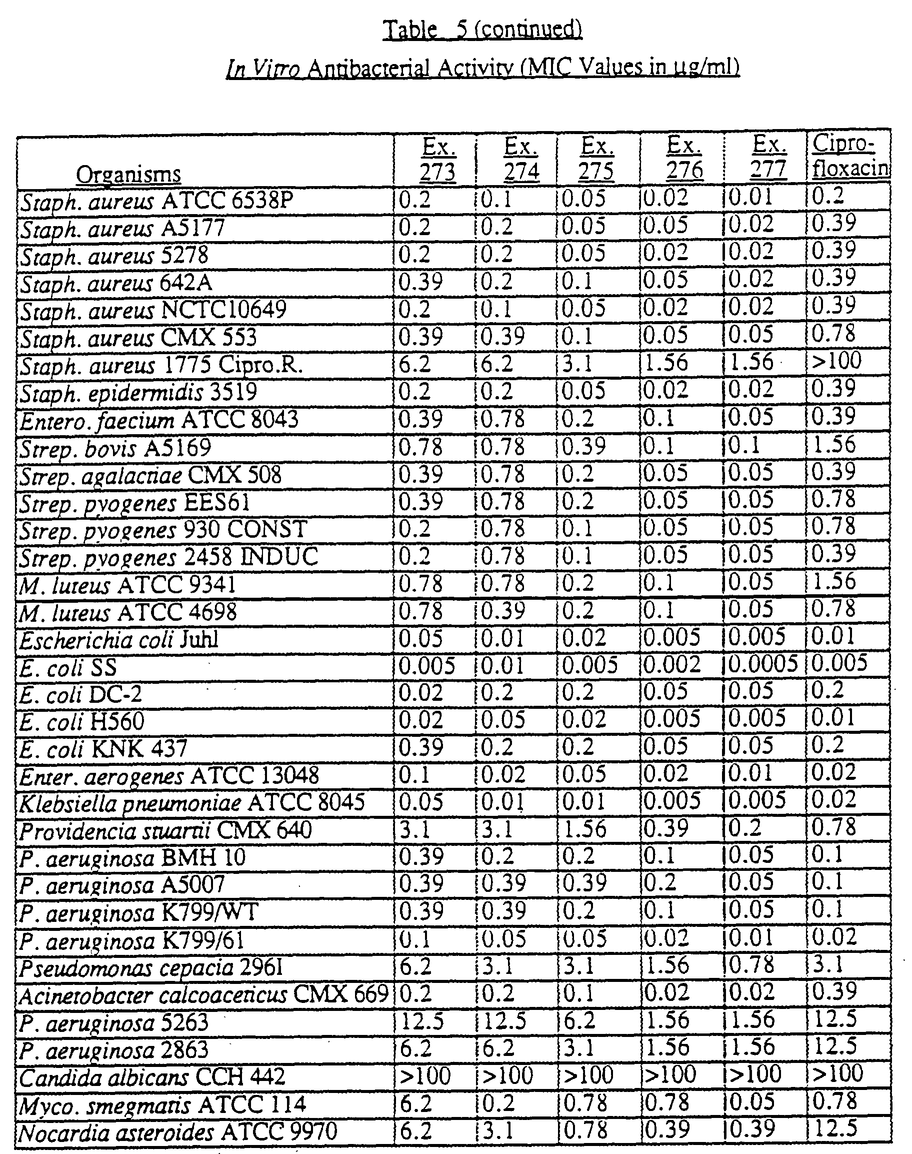

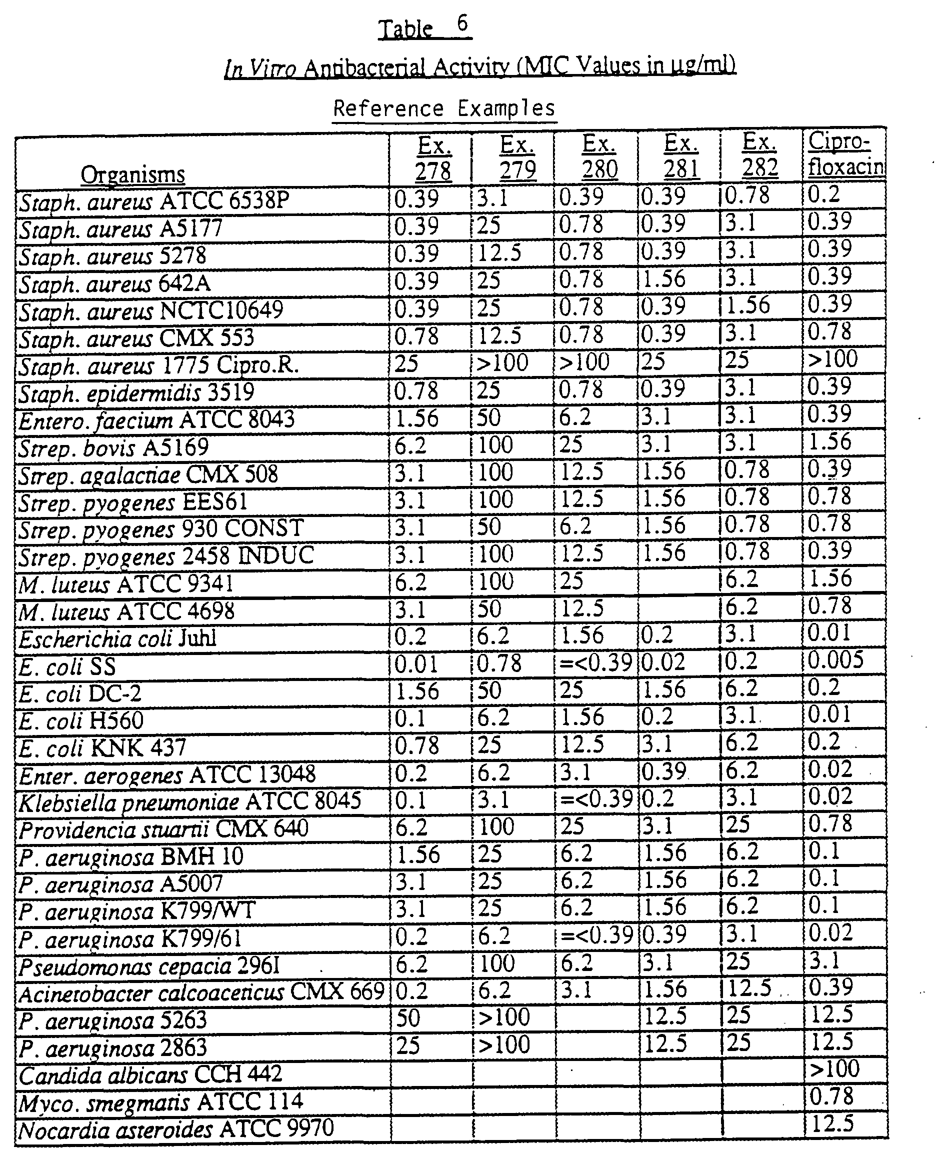

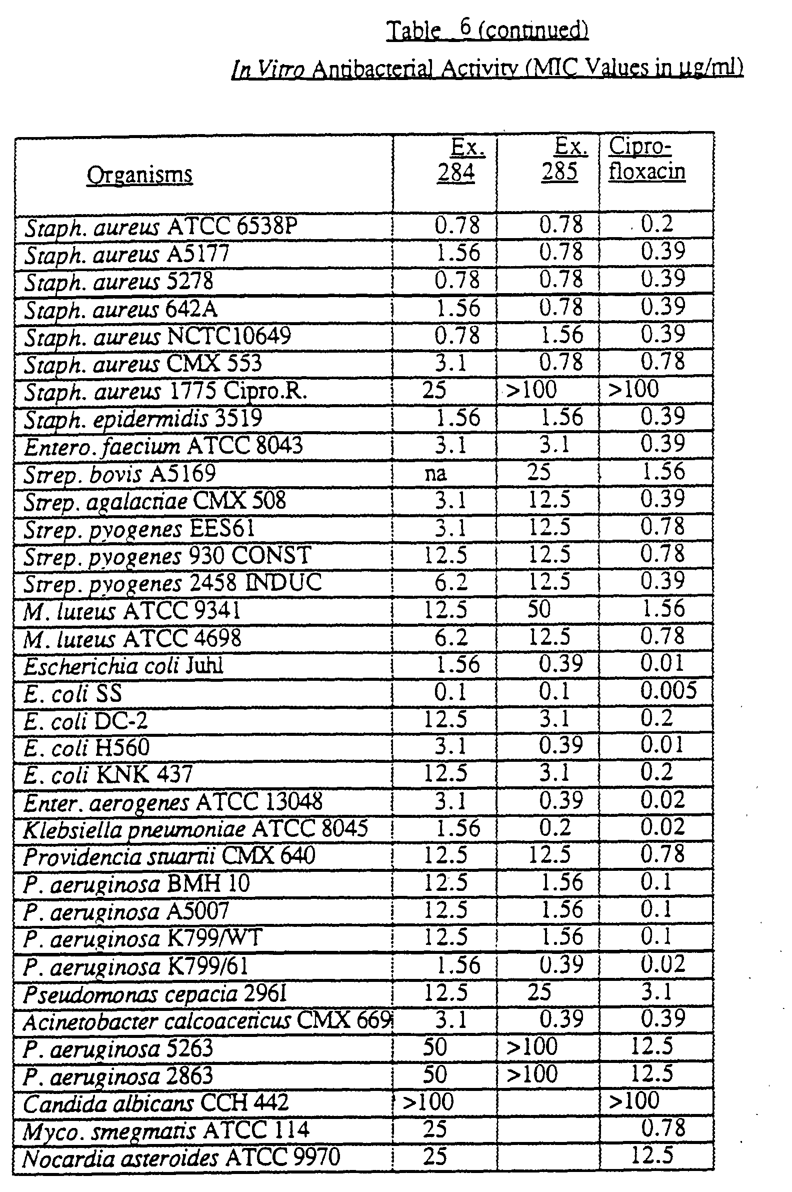

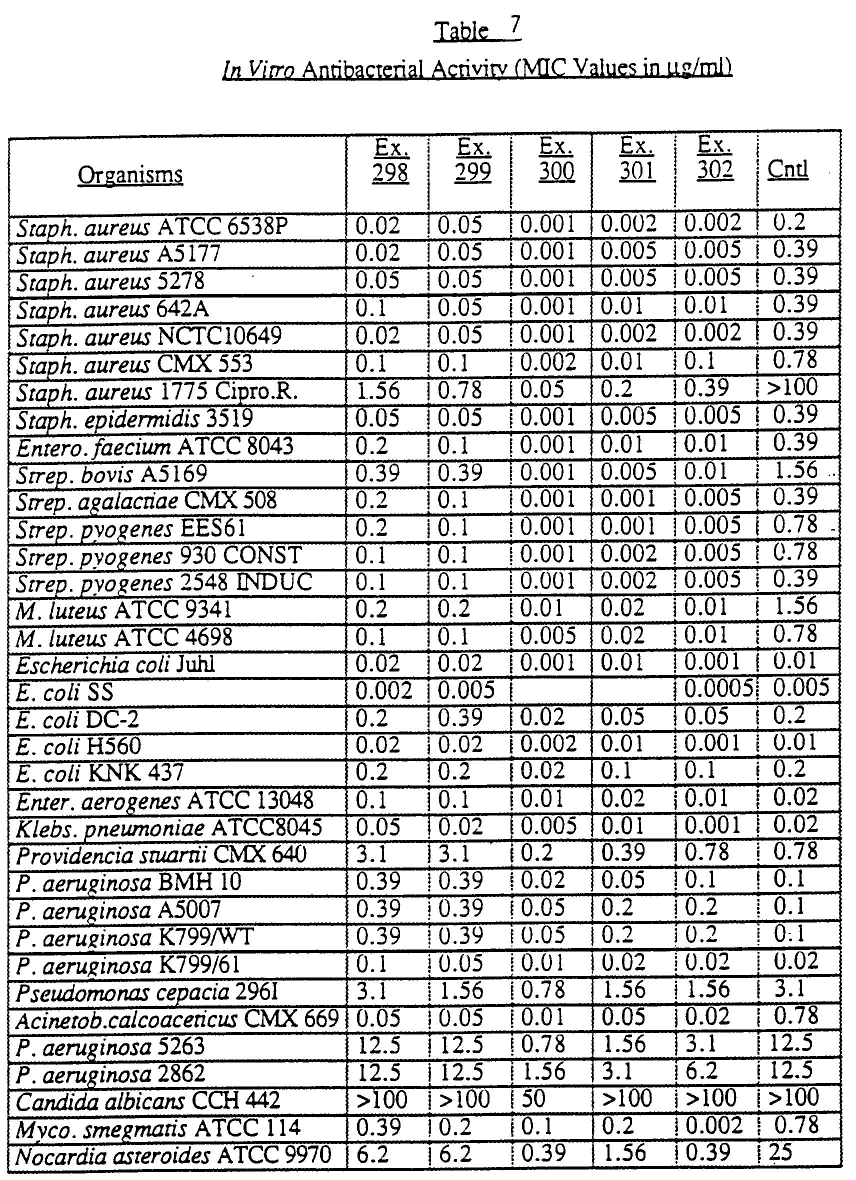

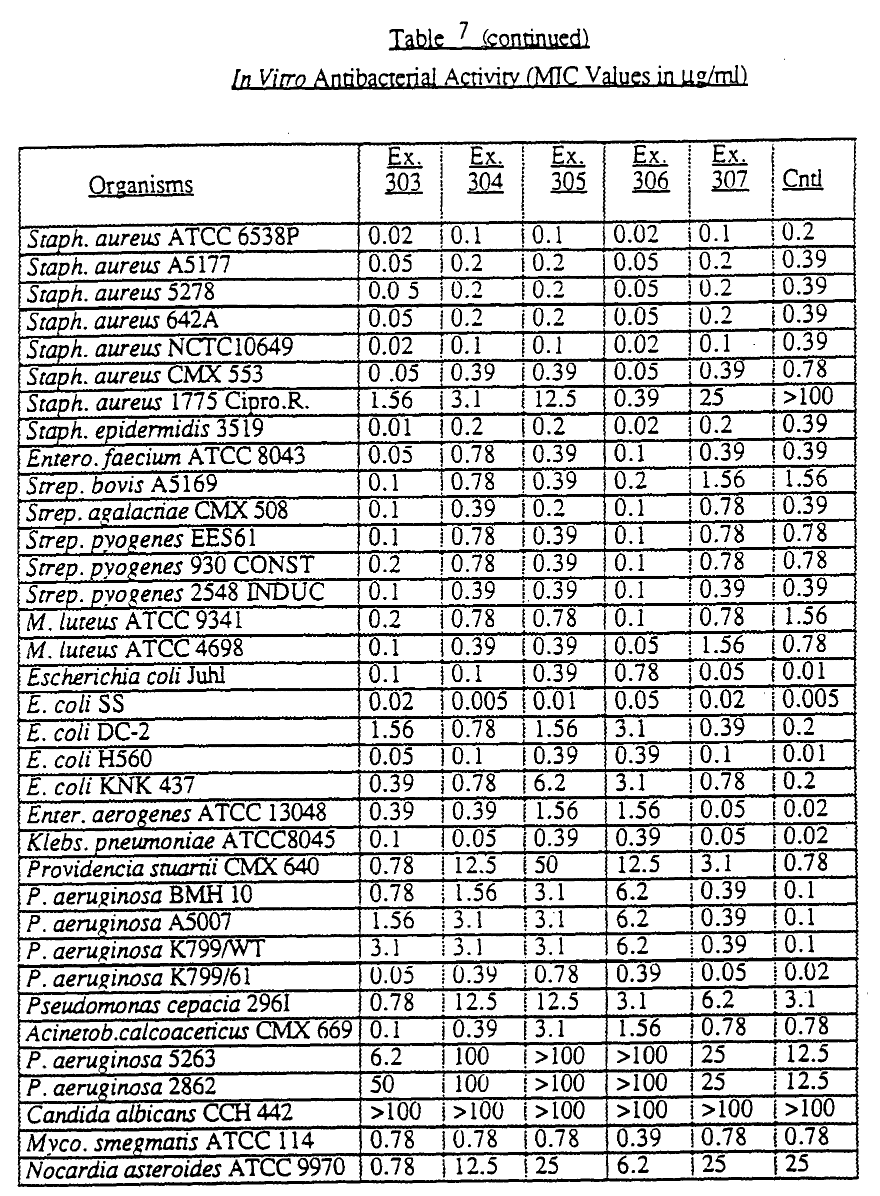

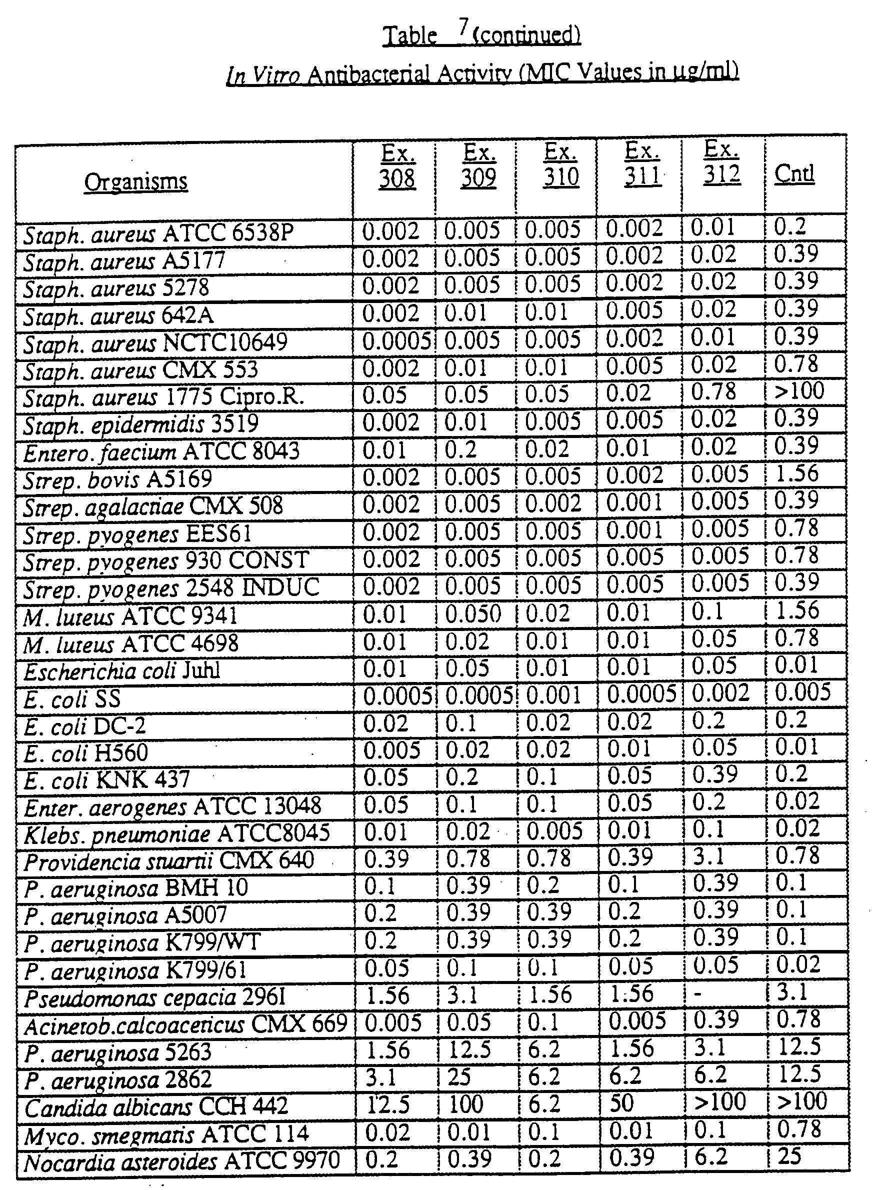

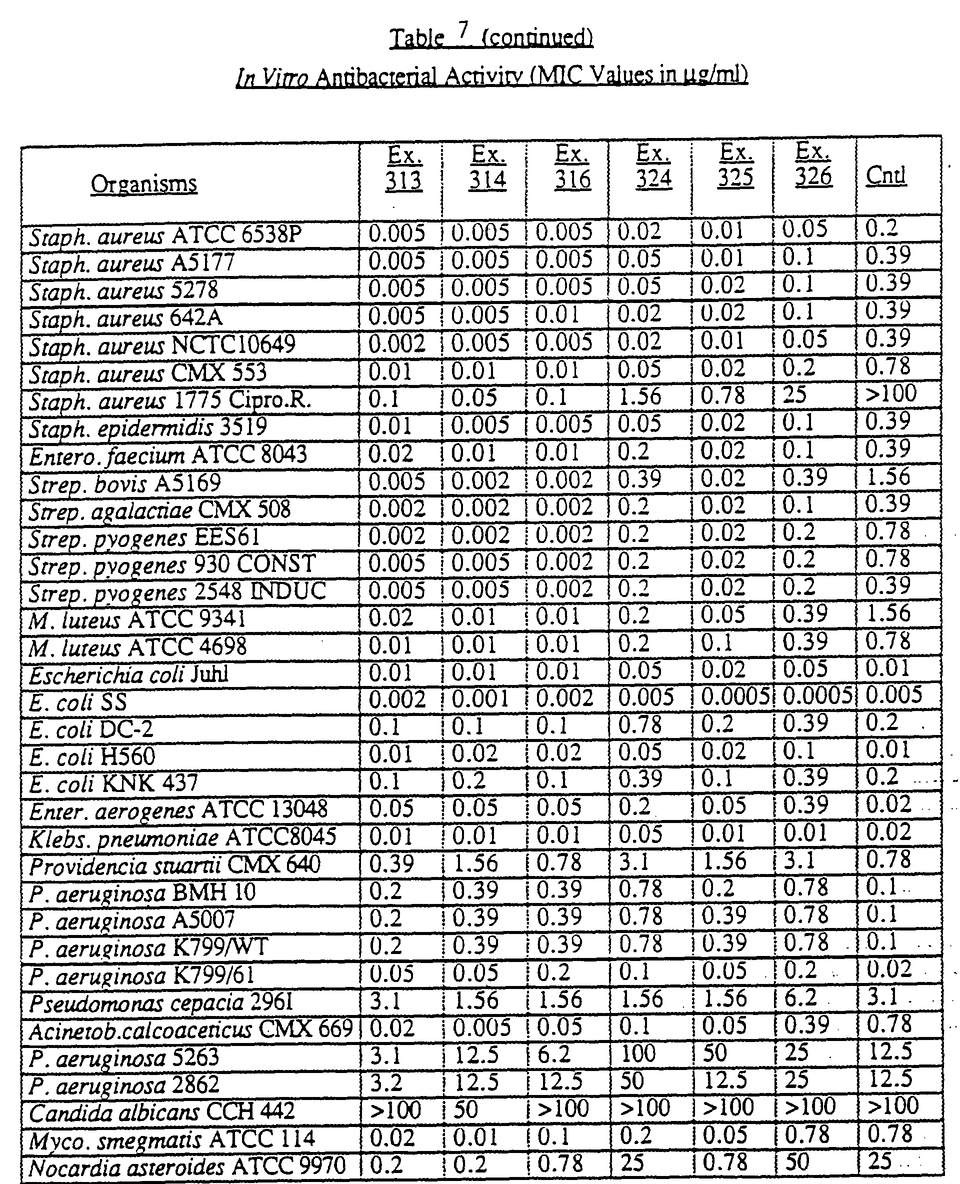

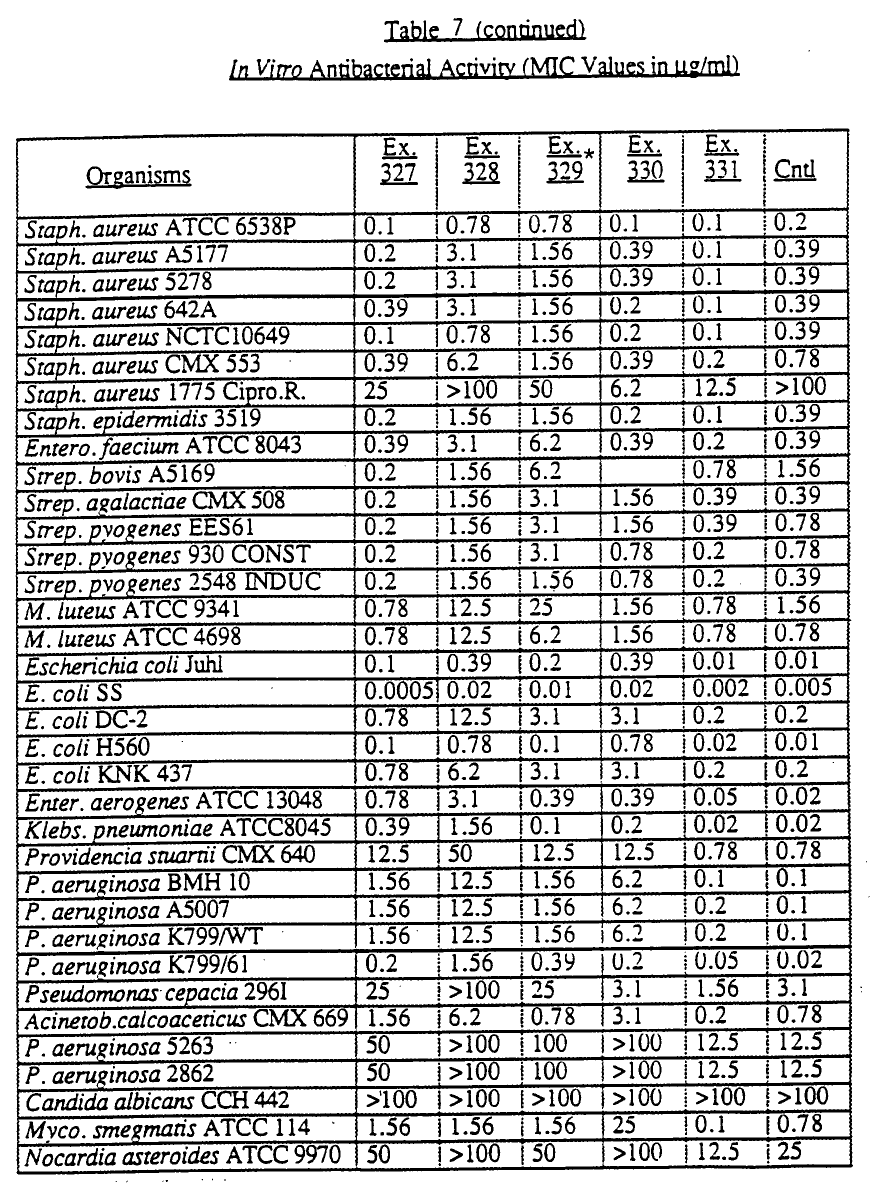

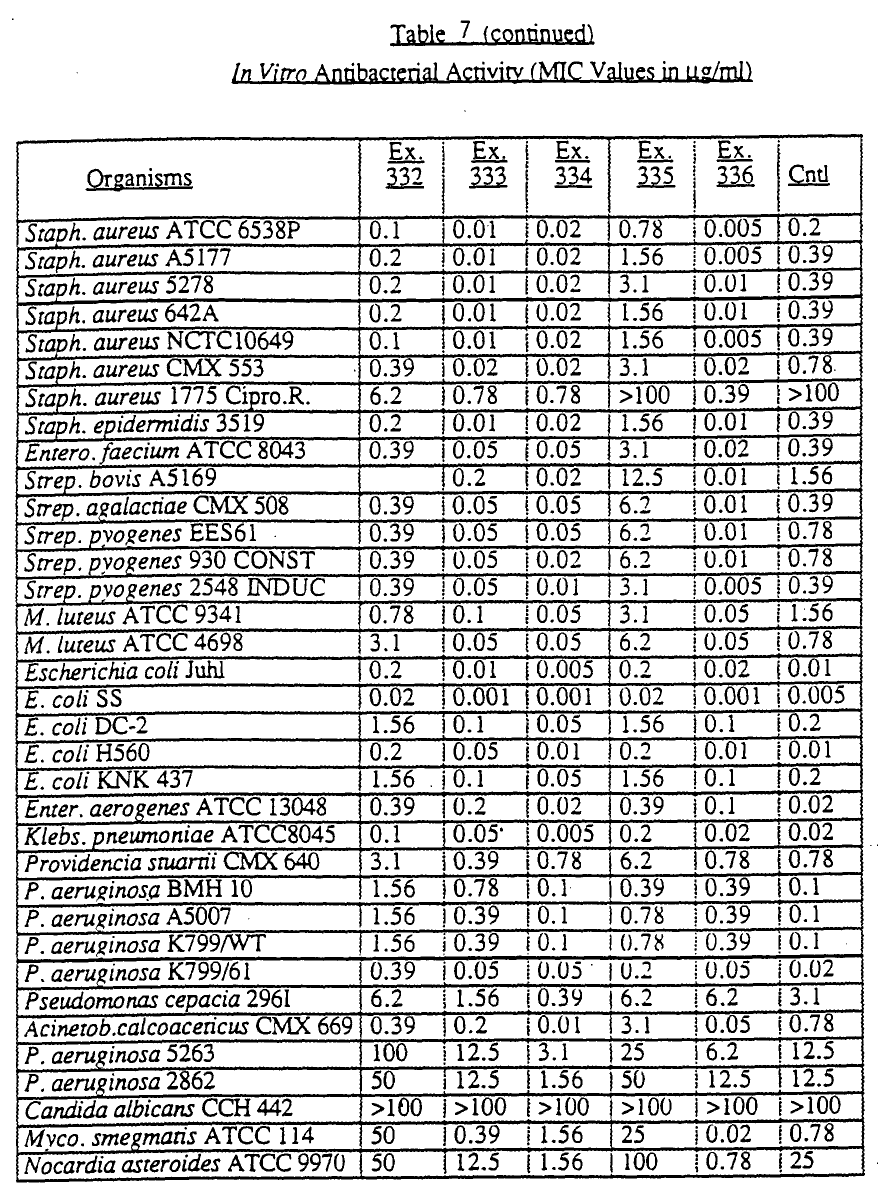

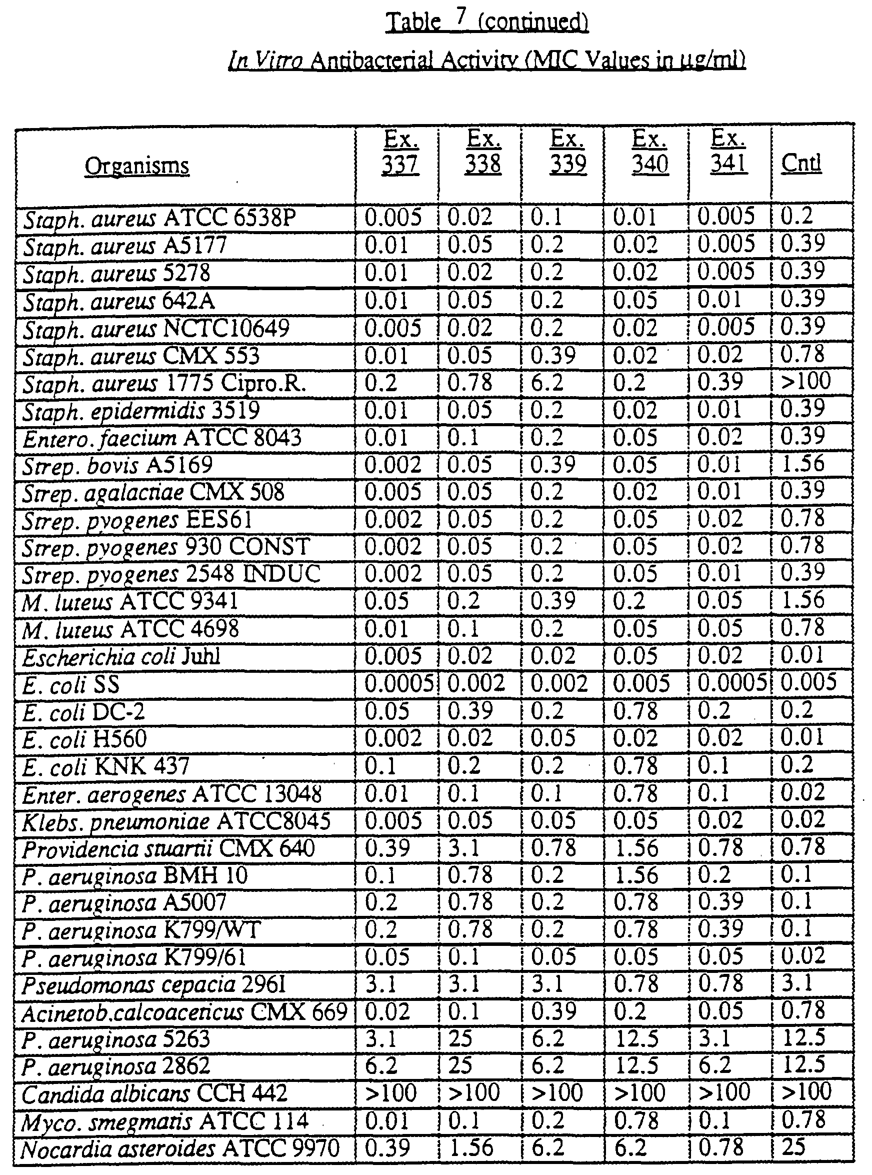

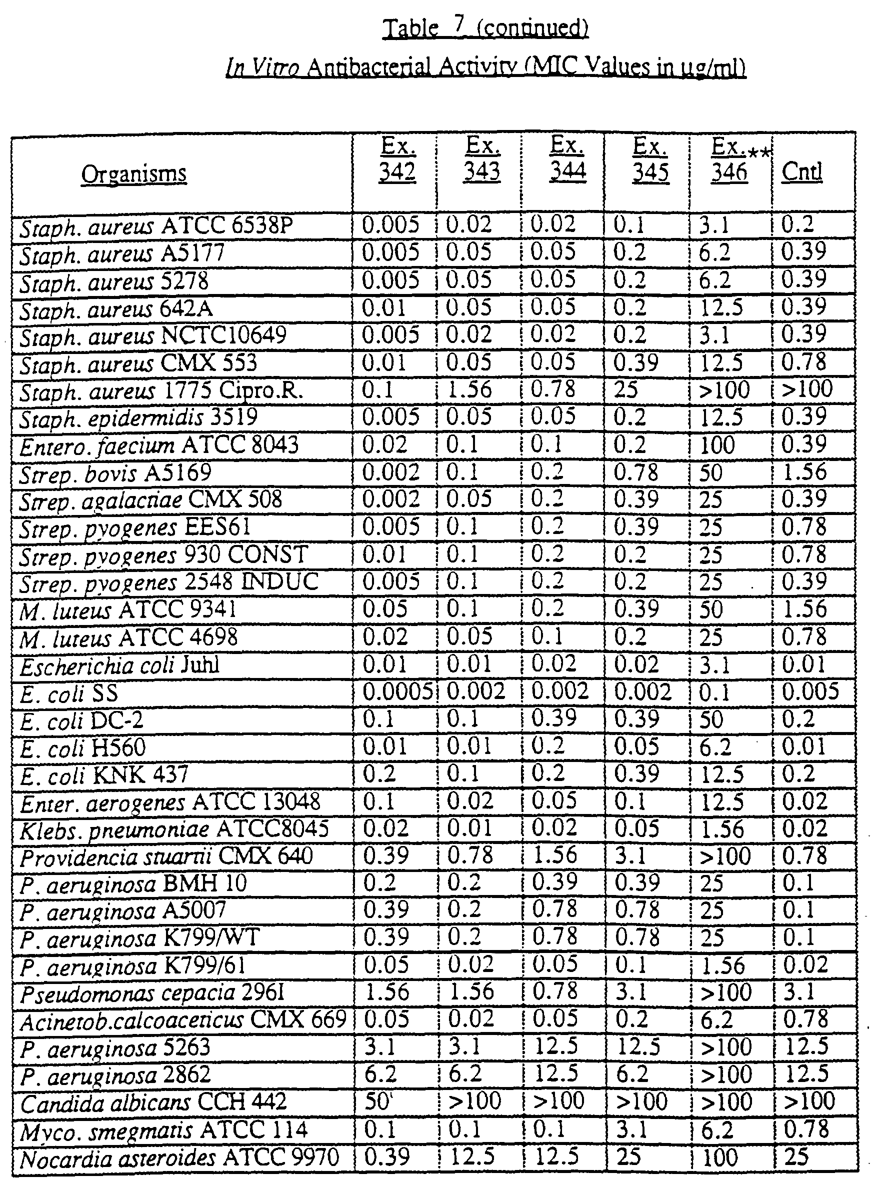

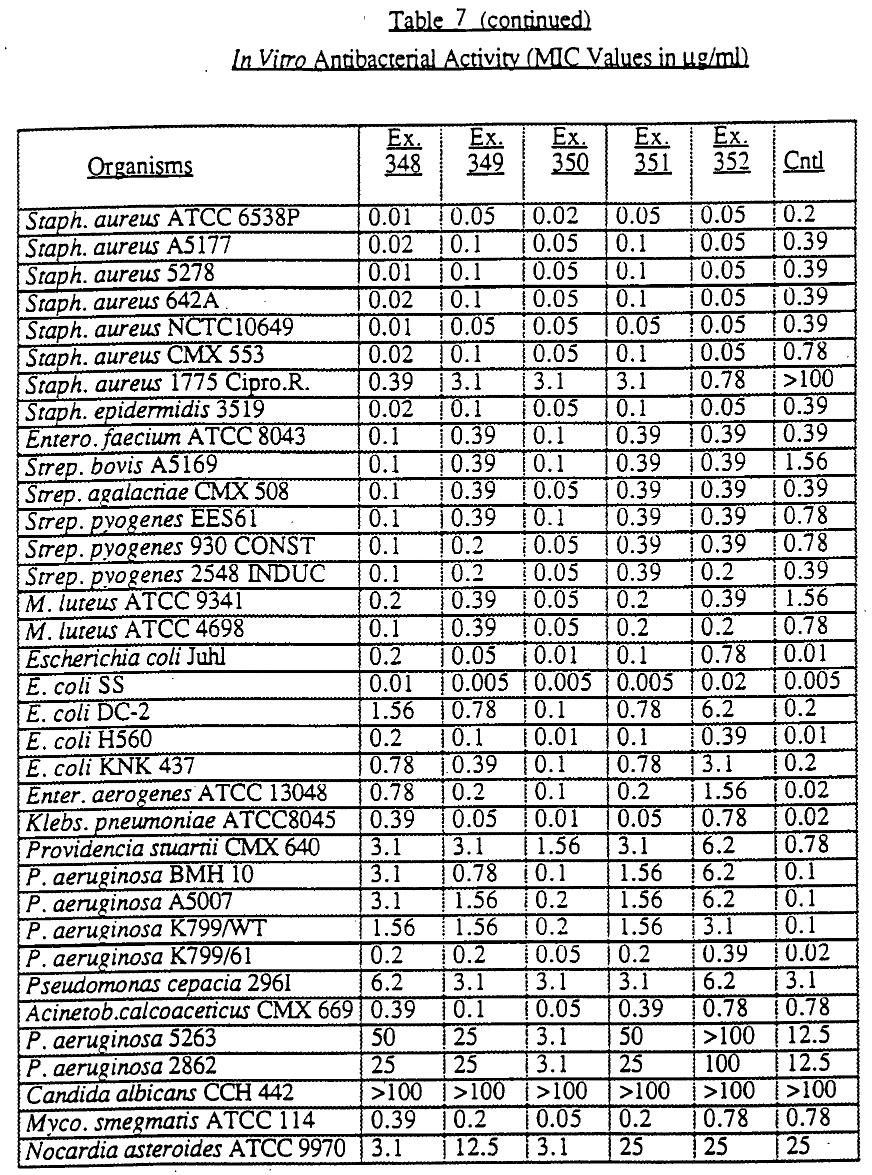

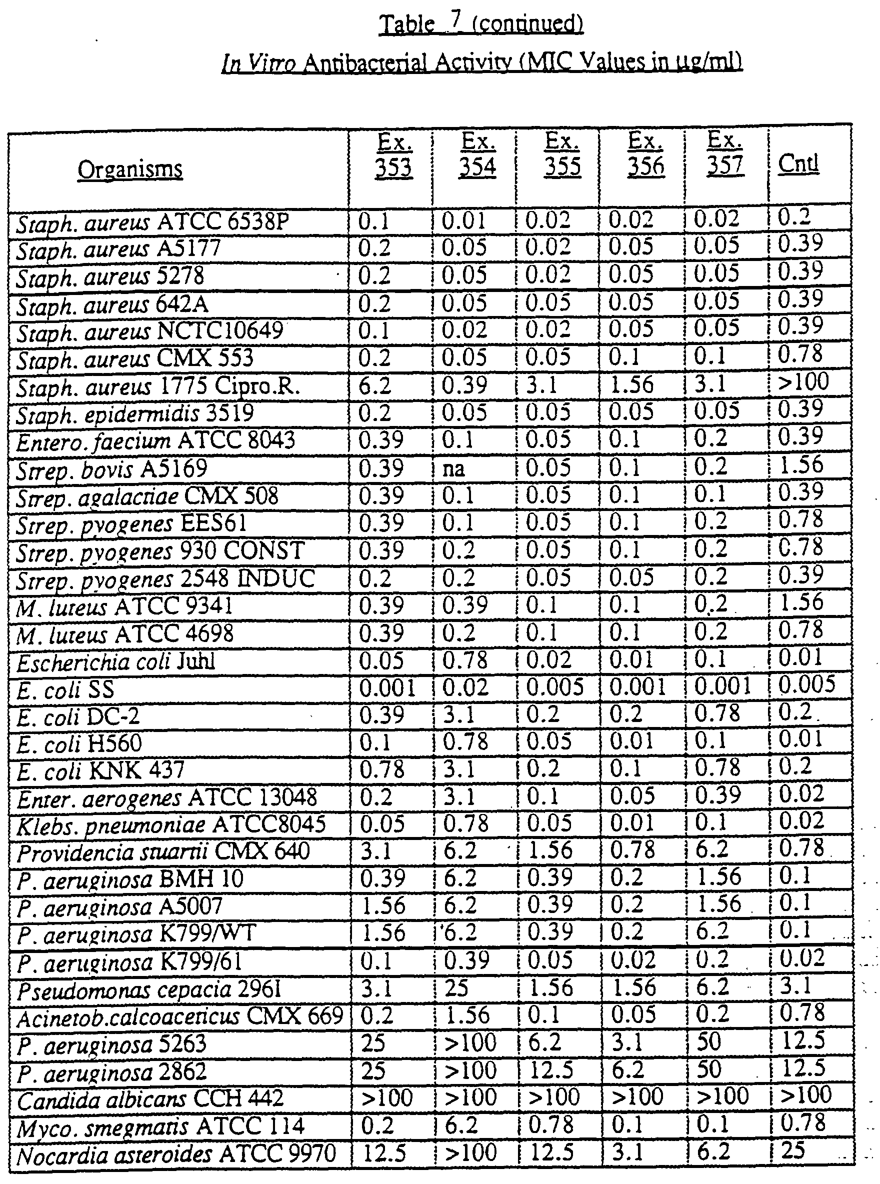

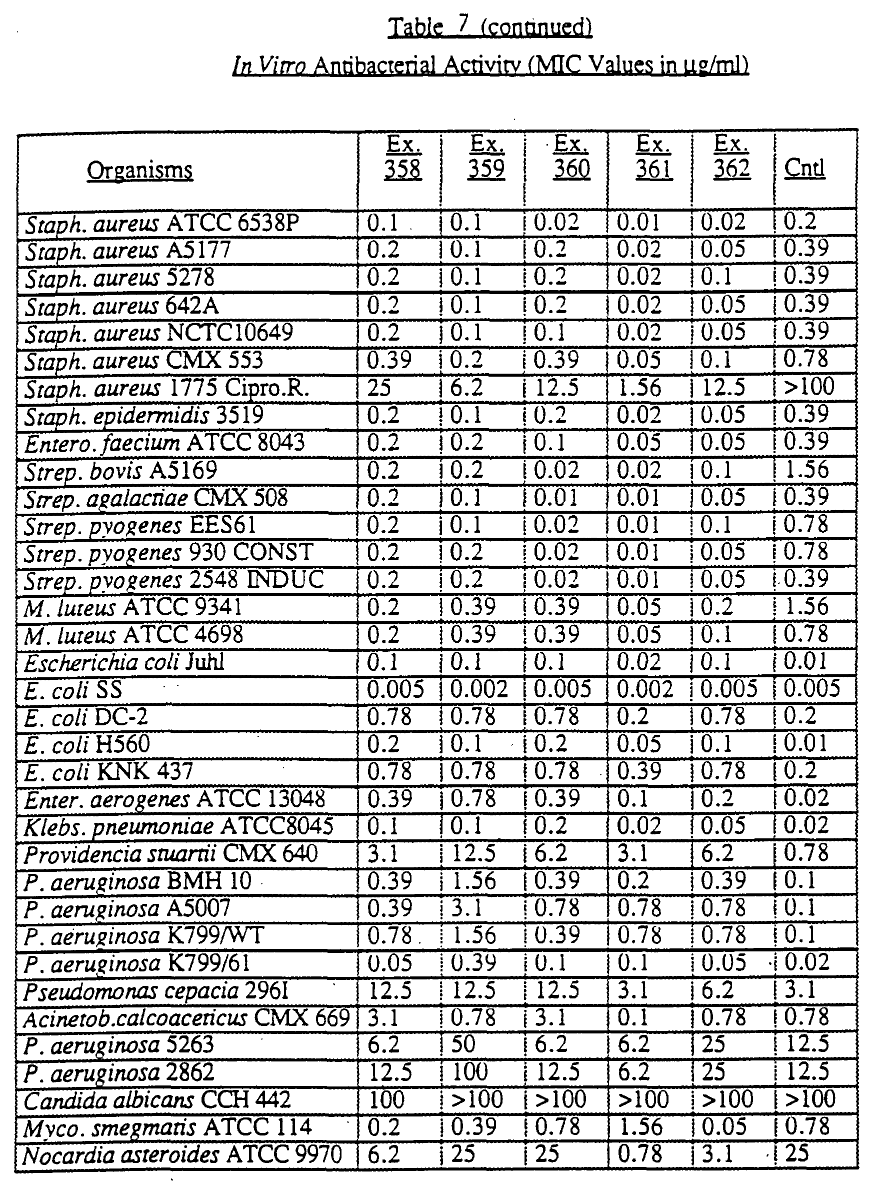

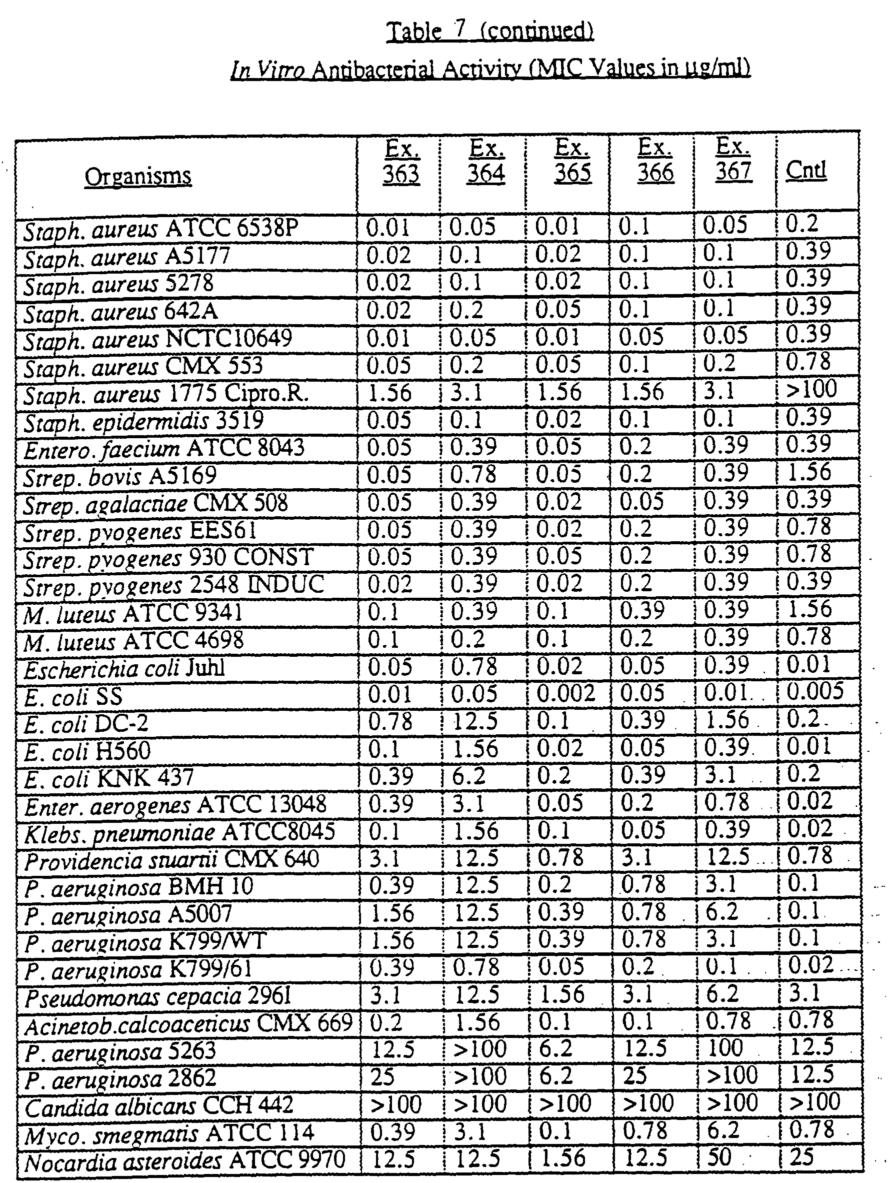

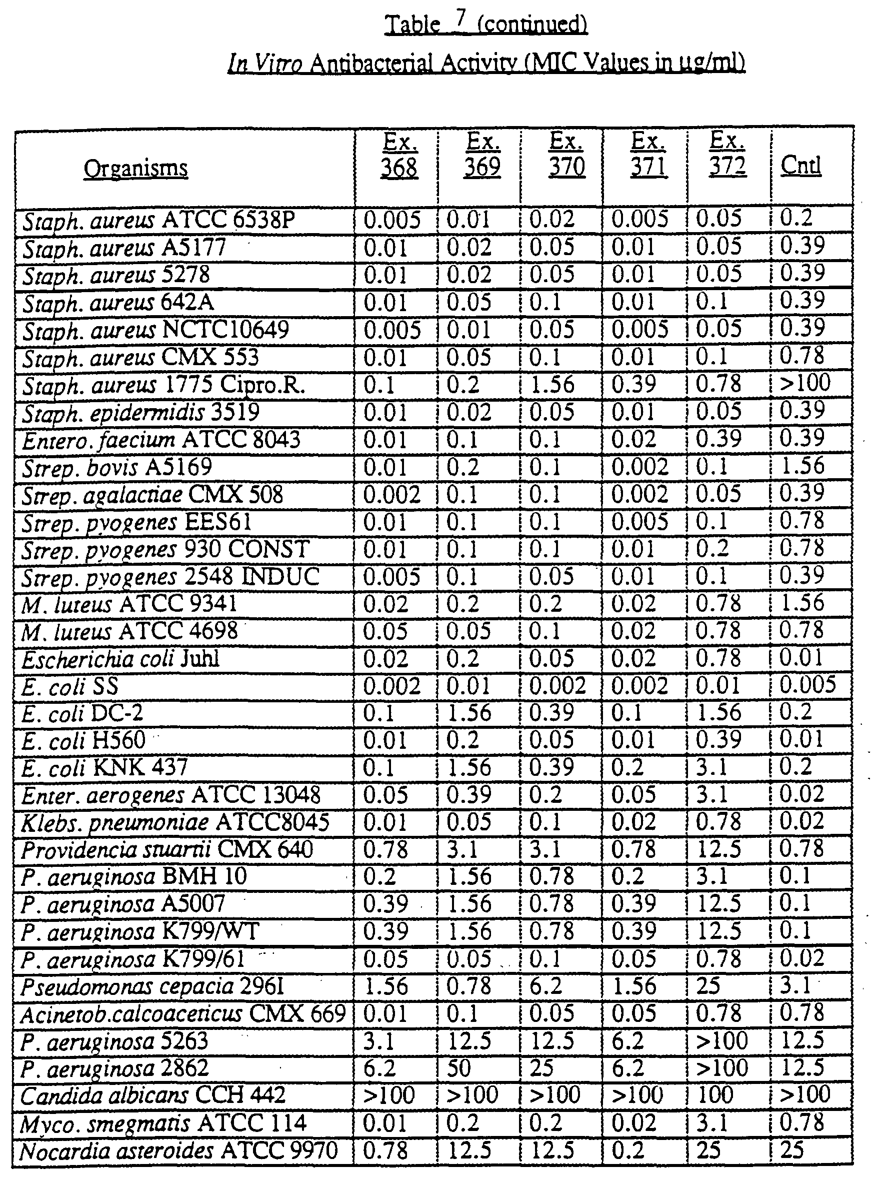

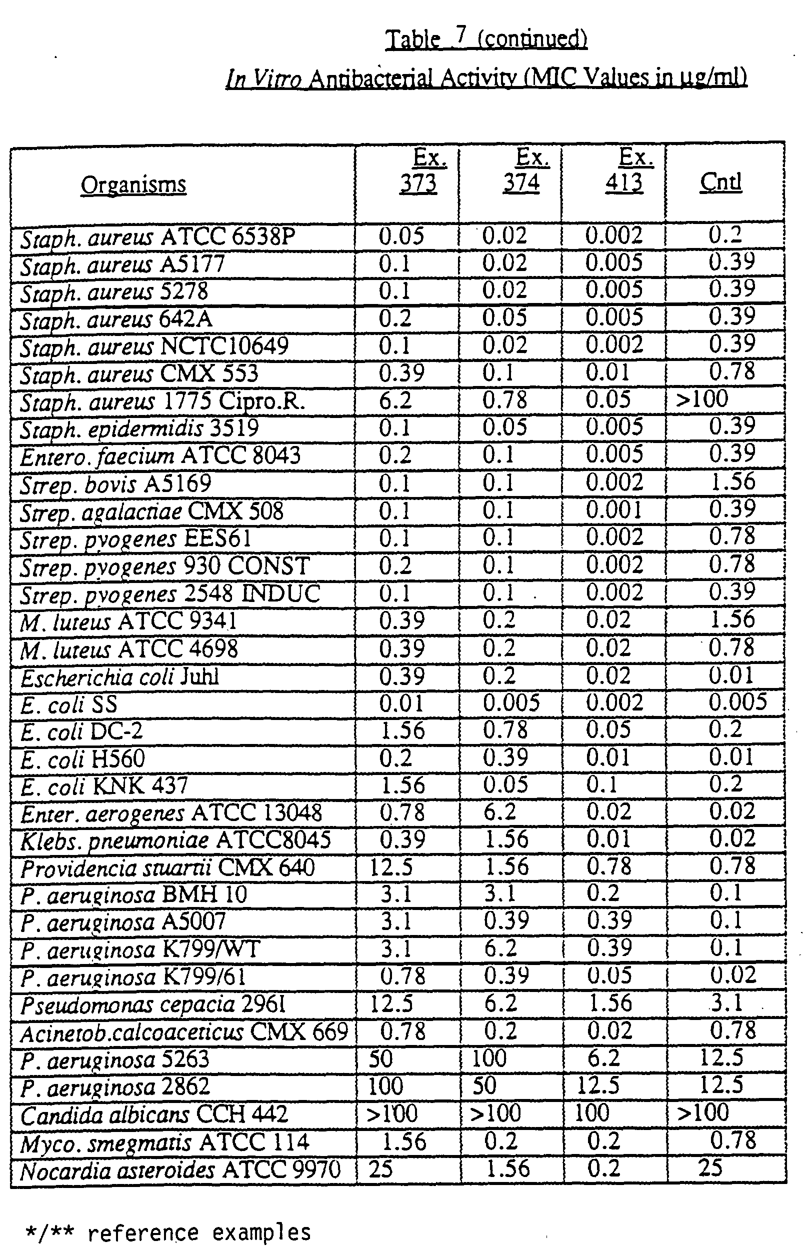

## Claims

1. A compound of the formula 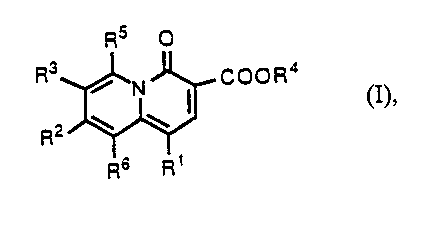 or a pharmaceutically acceptable salt, ester, or amide thereof, wherein
R¹ is selected from the group consisting of (a) C₁-C₆ alkyl, (b) C₂-C₆ alkenyl, (c) C₁-C₆ haloalkyl, (d) C₁-C₆ alkoxy, (e) C₃-C₈ cycloalkyl, (f) phenyl, (g) substituted phenyl, (h) halo, (i) cyano, (j) nitro, (k) bicycloalkyl, (l) C₂-C₆ alkynyl, (m) (C₁-C₆ alkoxy)carbonyl, (n) nitrogen-containing aromatic heterocycle, (o) halo-substituted nitrogen-containing aromatic heterocycle, (p) a 4-, 5- or 6-membered cyclic ether, and (q) -NR⁷R⁸ wherein R⁷ and R⁸ are independently selected from the group consisting of hydrogen, C₁-C₆ alkyl and C₁-C₈ alkanoyl or, taken together with the nitrogen atom to which they are attached, R⁷ and R⁸ form a 5-, 6- or 7-membered heterocycle;
R² is selected from the group consisting of (a) halo, (b) C₁-C₆ alkyl, (c) C₂-C₆ alkenyl, (d) C₃-C₈ cycloalkyl, (e) C₄-C₈ cycloalkenyl, (f) C₁-C₆ alkoxy, (g) aryloxy, (h) aryl-C₁-C₆ alkoxy, (i) aryl-C₁-C₆ alkyl, (j) cycloalkyl-C₁-C₆ alkyl, (k) amino, (l) mono- or di-(C₁-C₆ alkyl)amino, (m) aryl(C₁-C₆ alkyl)amino, (n) hydroxy-substituted (C₁-C₆ alkyl)amino, (o) phenyl, (p) substituted phenyl, (q) bicyclic nitrogen-containing heterocycle, (r) nitrogen-containing aromatic heterocycle, and (s) nitrogen-containing heterocycle having the formula 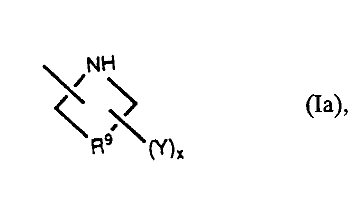 where x is between 0 and 3;
R⁹ is selected from the group consisting of (i) -(CH₂)ₘ- where m is between 1 and 3, and (ii) -(CH₂)ₙR¹⁰(CH₂)ₚ- where R¹⁰ is selected from the group consisting of -S-, -O- and-NH, n is 1 or 2, and p is 1 or 2; and
Y is independently selected at each occurrence from the group consisting of
(i) C₁-C₆ alkyl,
(ii) hydroxy,
(iii) halo,
(iv) C₁-C₆ haloalkyl,
(v) C₁-C₆ alkoxy,
(vi) C₁-C₆ alkoxy(C₁-C₆ alkyl),
(vii) C₁-C₆ alkoxy(C₁-C₆ alkoxy)(C₁-C₆ alkyl),
(viii) hydroxy-substituted C₁-C₆ alkyl,
(ix) imino,
(x) amino(C₁-C₆ alkyl),
(xi) (C₁-C₆ haloalkyl)amino(C₁-C₆ alkyl),
(xii) C₁-C₆ thioalkoxy(C₁-C₆ alkyl),
(xiii) C₁-C₆ thioalkoxy(C₁-C₆ alkoxy),
(xiv) amino(C₁-C₆ thioalkoxy),
(xv) C₃-C₆ cycloalkyl,
(xvi) C₃-C₈ cycloalkyl(C₁-C₆ alkyl),
(xvii) phenyl,
(xviii) substituted phenyl,
(xix) nitrogen-containing aromatic heterocycle,
(xx) -NR¹¹R¹² where R¹¹ and R¹² are independently selected from the group consisting of hydrogen and C₁-C₆ alkyl or, when one of R¹¹ and R¹² is hydrogen, the other is selected from the group consisting of C₁-C₈ alkanoyl, an alpha-amino acid, and a polypeptide residue of from 2 to 5 amino acids, and
(xxi) -C(R²¹)(R²²)NH₂ where R²¹ and R²² are independently selected from the group consisting of hydrogen, C₁-C₆ alkyl, hydroxy-substituted C₁-C₆ alkyl, amino(C₁-C₆ alkyl), C₁-C₆ alkoxy(C₁-C₆ alkyl), C₁-C₆ thioalkoxy(C₁-C₆ alkyl), C₃-C₆ cycloalkyl, and C₁-C₆ alkyl substituted with a nitrogen-containing aromatic heterocycle, or, taken together with the carbon atom to which they are attached, R²¹ and R²² form a ring structure selected from C₃-C₆ cycloalkyl and nitrogen-containing heterocycle;
R³ is selected from the group consisting of hydrogen, halo and C₁-C₆ alkoxy;
R⁴ is selected from the group consisting of hydrogen, C₁-C₆ alkyl, a pharmaceutically acceptable cation and a prodrug ester group which is an ester-forming group that is hydrolyzed under physiological conditions;
R⁵ is selected from the group consisting of (a) hydrogen, (b) halo, (c) hydroxy, (d) C₁-C₆ alkyl, (e) C₁-C₆ haloalkyl, (f) C₁-C₆ alkoxy, and (g) -NR¹³R¹⁴ where R¹³ and R¹⁴ are independently selected from the group consisting of hydrogen, C₁-C₆ alkyl, hydroxy-substituted C₁-C₆ alkyl, C₁-C₆ alkoxy(C₁-C₆ alkyl), and C₁-C₈ alkanoyl; and
R⁶ is C₁-C₆ alkyl.

2. A compound according to Claim 1 wherein **R**^{**3**} is halogen.

3. A compound according to Claim 2 wherein **R**^{**5**} is selected from the group consisting of hydrogen, C₁-C₆ alkyl, C₁-C₆ haloalkyl and -NR¹³R¹⁴ where R¹³ and R¹⁴ are as previously defined.

4. A compound according to Claim 3 wherein **R**^{**1**} is selected from the group consisting of cycloalkyl of from three to eight carbon atoms and substituted phenyl.

5. A compound according to Claim 4 wherein **R**^{**2**} is selected from the group consisting of bicyclic nitrogen-containing heterocycle and a nitrogen-containing heterocycle having the formula 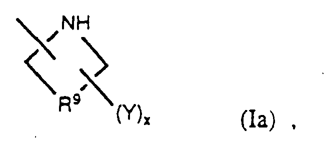 where R⁹, Y and x are as previously defined.

6. A compound according to Claim 5 wherein **R**^{**2**} is selected from the group consisting of 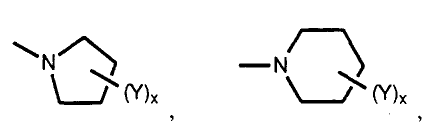 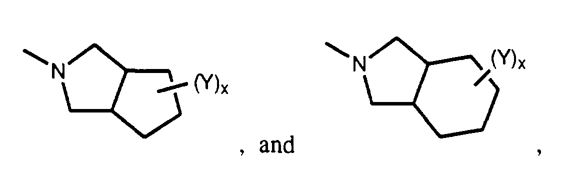 where Y and x are as previously defined.

7. A compound according to Claim 6 wherein x is one or two and **Y** is selected from the group consisting of -NR¹¹R¹² and -C(R²¹)(R²²)NH₂, where R¹¹, R¹², R²¹ and R²² are as previously defined.

8. A compound according to any of Claims 2, 3, 4, 5, 6 or 7 wherein **R**^{**6**} is methyl.

9. A compound according to Claim 1 having the formula 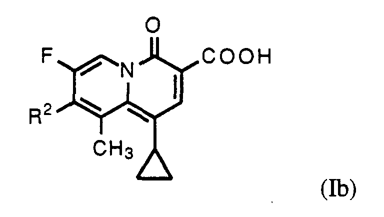 or a pharmaceutically acceptable salt, ester or amide thereof, wherein
**R**^{**2**} is selected from the group consisting of bicyclic nitrogen-containing heterocycle and a nitrogen-containing heterocycle having the formula 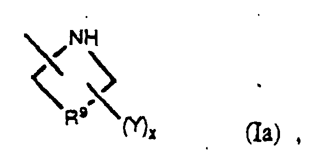
and where R⁴, R⁹, Y and x are as previously defined.

10. A compound according to Claim 9 wherein **R**^{**2**} is selected from the group consisting of 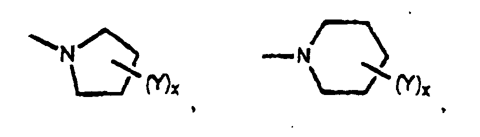 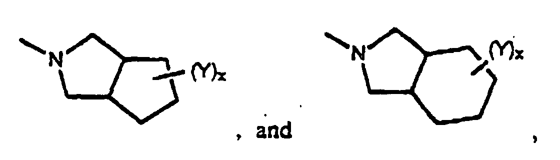 where Y and x are as previously defined.

11. A compound according to Claim 10 wherein x is one or two and Y is selected from the group consisting of -NR¹¹R¹² and -C(R²¹)(R²²)NH₂, where R¹¹, R¹², R²¹ and R²² are as previously defined.

12. A compound according to Claim 1 selected from the group consisting of:
8-(3-amino-1-pyrrolidinyl)-1-cyclopropyl-7-fluoro-9-methyl-4-oxo-4H-quinolizine-3-carboxylic acid hydrochloride;
8-(3-(aminomethyl)pyrrolidinyl)-1-cyclopropyl-7-fluoro-9-methyl-4-oxo-4H-quinolizine-3-carboxylic acid hydrochloride;
8-(2S,4S-4-amino-2-methylpyrrolidinyl)-1-cyclopropyl-7-fluoro-9-methyl-4-oxo-4H-quinolizine-3-carboxylic acid hydrochloride;
8-(3-aminoazetidinyl)-1-cyclopropyl-7-fluoro-9-methyl-4-oxo-4H-quinolizine-3-carboxylic acid hydrochloride;
8-(3(S)-aminopyrrolidinyl)-1-cyclopropyl-7-fluoro-9-methyl-4-oxo-4H-quinolizine-3-carboxylic acid hydrochloride;
1-cyclopropyl-7-fluoro-9-methyl-4-oxo-8-(3-methyl-1-piperazinyl)-4H-quinolizine-3-carboxylic acid hydrochloride;
1-cyclopropyl-7-fluoro-9-methyl-4-oxo-8-piperazinyl-4H-quinolizine-3-carboxylic acid hydrochloride;
1-cyclopropyl-7-fluoro-9-methyl-8-(2-((N-methyl)aminomethyl)-4-morpholinyl)-4-oxo-4H-quinolizine-3-carboxylic acid hydrochloride;
1-cyclopropyl-7-fluoro-9-methyl-4-oxo-8-(1,2,3,4-tetrahydro-2-isoquinolinyl)-4H-quinolizine-3-carboxylic acid;
1-cyclopropyl-7-fluoro-9-methyl-4-oxo-8-(4-amino-1-piperdinyl)-4H-quinolizine-3-carboxylic acid hydrochloride;
1-cyclopropyl-7-fluoro-9-methyl-4-oxo-8-(3-amino-1-piperdinyl)-4H-quinolizine-3-carboxylic acid hydrochloride;
1-cyclopropyl-7-fluoro-9-methyl-4-oxo-8-(4-(aminomethyl)-1-piperdinyl)-4H-quinolizine-3-carboxylic acid hydrochloride:
1-cyclopropyl-7-fluoro-9-methyl-4-oxo-8-(5-amino-1,2,3,4-tetrahydro-2-isoquinolinyl)-4H-quinolizine-3-carboxylic acid hydrochloride;
1-cyclopropyl-7-fluoro-9-methyl-4-oxo-8-(4-(1-pyrrolyl)-1-piperidinyl)-4H-quinolizine-3-carboxylic acid;
1-cyclopropyl-8-(*cis*-3.5-dimethyl-1-piperazinyl)-7-fluoro-9-methyl-4-oxo-4H-quinolizine-3-carboxylic acid hydrochloride;
1-cyclopropyl-8-(2,7-diaza-7-bicyclo[3.3.0]octyl)-7-fluoro-9-methyl-4-oxo-4H-quinolizine-3-carboxylic acid hydrochloride;
1-cyclopropyl-8-(2,8-diaza-8-bicyclo[4.3.0]nonyl)-7-fluoro-9-methyl-4-oxo-4H-quinolizine-3-carboxylic acid hydrochloride;
1-cyclopropyl-7-fluoro-9-methyl-4-oxo-8-(3 (S)-(1-pyrrolyl)-1-pyrrolidinyl)-4H-quinolizine-3-carboxylic acid;
1-cyclopropyl-7-fluoro-8-(3-hydroxy-1-pyrrolidinyl)-9-methyl-4-oxo-4H-quinolizine-3-carboxylic acid hydrochloride;
1-cyclopropyl-7-fluoro-8-(4-methyl-1-piperazinyl)-9-methyl-4-oxo-4H-quinolizine-3-carboxylic acid hydrochloride;
1-cyclopropyl-8-(2,7-diaza-7-bicyclo[3.3.0]octyl)-7-fluoro-9-methyl-4-oxo-4H-quinolizine-3-carboxylic acid hydrochloride;
1-cyclopropyl-8-(2,8-diaza-8-bicyclo[4.3.0]nonyl)-7-fluoro-9-methyl-4-oxo-4H-quinolizine-3-carboxylic acid hydrochloride;
1-cyclopropyl-7-fluoro-9-methyl-4-oxo-8-(3(S)-(1-pyrrolyl)-1-pyrrolidinyl)-4H-quinolizine-3-carboxylic acid;
1-cyclopropyl-7-fluoro-8-(3-hydroxy-1-pyrrolidinyl)-9-methyl-4-oxo-4H-quinolizine-3-carboxylic acid hydrochloride;
1-cyclopropyl-7-fluoro-8-(4-methyl-1-piperazinyl)-9-methyl-4-oxo-4H-quinolizine-3-carboxylic acid hydrochloride;
1-cyclopropyl-7-fluoro-9-methyl-8-(3(S)-methylamino-1-pyrrolidinyl)-4-oxo-4H-quinolizine-3-carboxylic acid hydrochloride;
1-cyclopropyl-7-fluoro-9-methyl-8-(3(R)-amino-1-pyrrolidinyl)-4-oxo-4H-quinolizine-3-carboxylic acid hydrochloride;
8-(2,4-dimethyl-1-piperazinyl)-1-cyclopropyl-7-fluoro-9-methyl-4-oxo-4H-quinolizine-3-carboxylic acid hydrochloride;
8-(3-(methylamino)-1-piperazinyl)-1-cyclopropyl-7-fluoro-9-methyl-4-oxo-4H-quinolizine-3-carboxylic acid hydrochloride;
8-(3-(methylamino)-1-morpholinyl)-1-cyclopropyl-7-fluoro-9-methyl-4-oxo-4H-quinolizine-3-carboxylic acid hydrochloride;
8-(3-(S)-(methylamino)-1-pyrrolidinyl)-1-cyclopropyl-7-fluoro-9-methyl-4-oxo-4H-quinolizine-3-carboxylic acid hydrochloride;
8-(3-(S)-(1-(methylamino)methyl)-1-pyrrolidinyl)-1-cyclopropyl-7-fluoro-9-methyl-4-oxo-4H-quinolizine-3-carboxylic acid hydrochloride;
8-(3-(S)-(1-(ethylamino)methyl)-1-pyrrolidinyl)-1-cyclopropyl-7-fluoro-9-methyl-4-oxo-4H-quinolizine-3-carboxylic acid hydrochloride;
8-(octahydropyrrolo[3,4-c]pyrrol-1-yl)-1-cyclopropyl-7-fluoro-9-methyl-4-oxo-4H-quinolizine-3-carboxylic acid hydrochloride;
8-(octahydropyrrolo[3,4-c]pyridin-5-yl)-1-cyclopropyl-7-fluoro-9-methyl-4-oxo-4H-quinolizine-3-carboxylic acid hydrochloride;
8-(cis-4-amino-3-methylpyrrolidinyl)-1-cyclopropyl-7-fluoro-9-methyl-4-oxo-4H-quinolizine-3-carboxylic acid hydrochloride;
8-(trans-4-amino-3-methylpyrrolidinyl)-1-cyclopropyl-7-fluoro-9-methyl-4-oxo-4H-quinolizine-3-carboxylic acid hydrochloride;
8-(3-methyl-4-spirocyclopropylpyrrolidinyl)-1-cyclopropyl-7-fluoro-9-methyl-4-oxo-4H-quinolizine-3-carboxylic acid hydrochloride;
8-(3-dimethylaminopyrrolidinyl)-1-cyclopropyl-7-fluoro-9-methyl-4-oxo-4H-quinolizine-3-carboxylic acid, acetic acid salt;
(3R)-8-(3-dimethylaminopyrrolidinyl)-1-cyclopropyl-7-fluoro-9-methyl-4-oxo-4H-quinolizine-3-carboxylic acid hydrochloride;
(3R,1S)-8-(3-(1-aminoethyl)pyrrolidinyl)-1-cyclopropyl-7-fluoro-9-methyl-4-oxo-4H-quinolizine-3-carboxylic acid hydrochloride;
(3S,1R)-8-(3-(1-aminoethyl)pyrrolidinyl)-1-cyclopropyl-7-fluoro-9-methyl-4-oxo-4H-quinolizine-3-carboxylic acid hydrochloride;
(3R, 1R)-8-(3-(1-aminoethyl)pyrrolidinyl)-1-cyclopropyl-7-fluoro-9-methyl-4-oxo-4H-quinolizine-3-carboxylic acid hydrochloride;
1-cyclopropyl-8-((R,S)-3-fluoropyrrolidine)-7-fluoro-9-methyl-4-oxo-4H-quinolizine-3-carboxylic acid;
8-(4-(1-piperidyl)-1-piperidyl)-1-cyclopropyl-7-fluoro-9-methyl-4-oxo-4H-quinolizine-3-carboxylic acid;
8-(4-(1-piperidyl)-1-piperidyl)-1-cyclopropyl-7-fluoro-9-methyl-4-oxo-4H-quinolizine-3-carboxylic acid trifluoroacetic acid salt;
8-(4-(2-pyridyl)-1-piperazinyl)-1-cyclopropyl-7-fluoro-9-methyl-4-oxo-4H-quinolizine-3-carboxylic acid;
8-((2-amino)thioethoxy)-1-cyclopropyl-7-fluoro-9-methyl-4-oxo-4H-quinolizine-3-carboxylic acid trifluoroacetic acid salt;
(3R,1S)-8-(3-(1-amino)propyl)pyrrolidinyl)-1-cyclopropyl-7-fluoro-9-methyl-4-oxo-4H-quinolizine-3-carboxylic acid hydrochloride;
(3R,1S)-8-(3-(1-(N-methyl)ammo)propyl)pyrrolidinyl)-1-cyclopropyl-7-fluoro-9-methyl-4-oxo-4H-quinolizine-3-carboxylic acid hydrochloride;
(3R,1S)-8-(3-(1-amino-3-methylpropyl)pyrrolidinyl)-1-cyclopropyl-7-fluoro-9-methyl-4-oxo-4H-quinolizine-3-carboxylic acid hydrochloride;
8-(3-(1-aminocyclopropyl)pyrrolidinyl)-1-cyclopropyl-7-fluoro-9-methyl-4-oxo-4H-quinolizine-3-carboxylic acid hydrochloride;
(3R,1S)-8-(3-(1-amino-2-hydroxyethyl)pyrrolidinyl)-1-cyclopropyl-7-fluoro-9-methyl-4-oxo-4H-quinolizine-3-carboxylic acid hydrochloride;
(8-(3-(1-amino-1-methylethyl)pyrrolidinyl)-1-cyclopropyl-7-fluoro-9-methyl-4-oxo-4H-quinolizine-3-carboxylic acid hydrochloride;
8-(3-(1-aminobutyl)pyrrolidinyl)-1-cyclopropyl-7-fluoro-9-methyl-4-oxo-4H-quinolizine-3-carboxylic acid hydrochloride;
1-cyclopropyl-7-fluoro-9-methyl-4-oxo-8-(trans-4-trifluoromethyl-3-aminopyrrolidinyl)-4H-quinolizine-3-carboxylic acid hydrochloride;
1-cyclopropyl-7-fluoro-9-methyl-4-oxo-8-(trans-4-trifluoromethyl-3-aminomethylpyrrolidinyl)-4H-quinolizine-3-carboxylic acid hydrochloride;
3(S)-1-cyclopropyl-7-fluoro-9-methyl-4-oxo-8-(3-(N-(S )-norvalylamino)pyrrolidinyl)-4H-quinolizine-3-carboxylic acid hydrochloride;
3(S)-1-cyclopropyl-7-fluoro-9-methyl-4-oxo-8-(3-(N-(S )-alanylamino)pyrrolidinyl)-4H-quinolizine-3-carboxylic acid hydrochloride;
3(S)-1-cyclopropyl-7-fluoro-9-methyl-4-oxo-8-(3-(N-(S)-alanyl-(S)-alanylamino)pyrrolidinyl)-4H-quinolizine-3-carboxylic acid hydrochloride;
1-cyclopropyl-7-fluoro-4H-8-(1-imidazolyl)-9-methyl-4-oxo-quinolizine-3-carboxylic acid hydrochloride;
8-(3-amino-1-pyrrolidinyl)-1-ethyl-7-fluoro-4H-4-oxo-9-methyl-quinolizine-3-carboxylic acid hydrochloride;
8-(3-amino-1-pyrrolidinyl)-1-cyclopropyl-9-ethyl-7-fluoro-4H-4-oxo-quinolizine-3-carboxylic acid hydrochloride;
1-cyclopropyl-7-fluoro-4H-9-methyl-4-oxo-8-(3-(1,2,3-triazol-1-yl)-1-pyrrolidinyl)-quinolizine-3-carboxylic acid;
1-cyclopropyl-7-fluoro-4H-9-methyl-4-oxo-8-(cis-3-amino-4-methyl-1-pyrrolidinyl)-quinolizine-3-carboxylic acid hydrochloride;
8-(2-aminoethyl)-1-cyclopropyl-7-fluoro-4H-9-methyl-4-oxo-quinolizine-3-carboxylic acid hydrochloride;
8-(3-(ethylaminomethyl)pyrrolidinyl)-1-cyclopropyl-7-fluoro-4H-9-methyl-4-oxo-quinolizine-3-carboxylic acid hydrochloride;
8-(3-(1-aminoethyl)pyrrolidinyl)-1-cyclopropyl-7-fluoro-4H-9-methyl-4-oxo-quinolizine-3-carboxylic acid hydrochloride;
1-cyclopropyl-7-fluoro-4H-9-methyl-8-(2-methyl-2,8-diaza-8-bicyclo[4.3.0]nonyl)-4-oxo-quinolizine-3-carboxylic acid hydrochloride;
1-cyclopropyl-7-fluoro-4H-8-((1S,4S)-2,5-diaza-bicyclo[2.2.1]heptan-2-yl)-9-methyl-4-oxo-quinolizine-3-carboxylic acid hydrochloride;
1-cyclopropyl-7-fluoro-4H-9-methyl-4-oxo-8-(3-(2-pyridinyl)-1-pyrrolidinyl)-quinolizine-3-carboxylic acid hydrochloride;
8-((1R*,2S*,6R*)-2-amino-8-azabicyclo[4.3.0]nonan-8-yl))-1-cyclopropyl-7-fluoro-4H-9-methyl-4-oxo-quinolizine-3-carboxylic acid hydrochloride;
8-((1R*,2R*,6R*)-2-amino-8-azabicyclo[4.3.0]nonan-8-yl))-1-cyclopropyl-7-fluoro-4H-9-methyl-4-oxo-quinolizine-3-carboxylic acid hydrochloride;
8-((1a,5a,6a)-6-amino-3-azabicyclo[3.1.0]hexan-3-yl))-1-cyclopropyl-9-methyl-7-fluoro-4H-4-oxo-quinolizine-3-carboxylic acid hydrochloride;
8-(*cis*-3-amino-4-fluoro-1-pyrrolidinyl)-1-cyclopropyl-9-methyl-7-fluoro-4H-4-oxo-quinolizine-3-carboxylic acid hydrochloride;
1-cyclopropyl-7-fluoro-4H-8-(1-homopiperazinyl))-9-methyl-4-oxo-quinolizine-3-carboxylic acid, acetic acid salt;
8-(spiro-1,3-dioxacyclopentane[2.3]-1-piperidinyl)-1-cyclopropyl-7-fluoro-4H-9-methyl-4-oxo-quinolizine-3-carboxylic acid;
8-(3-amino-4-methoxypyrrolidinyl)-1-cyclopropyl-7-fluoro-4H-9-methyl-4-oxo-quinolizine-3-carboxylic acid hydrochloride;
8-(4-amino-4-methylpyrrolidinyl)-1-cyclopropyl-7-fluoro-4H-9-methyl-4-oxo-quinolizine-3-carboxylic acid hydrochloride;
8-(4-(2-hydroxyethyl)piperidinyl)-1-cyclopropyl-7-fluoro-4H-9-methyl-4-oxo-quinolizine-3-carboxylic acid;
8-(4-(methoxymethyl)piperidinyl)-1-cyclopropyl-7-fluoro-4H-9-methyl-4-oxo-quinolizine-3-carboxylic acid;
8-(3-amino-3-methylpiperidinyl)-1-cyclopropyl-7-fluoro-4H-9-methyl-4-oxo-quinolizine-3-carboxylic acid hydrochloride;
8-(3-pyrrolylpiperidinyl)-1-cyclopropyl-7-fluoro-4H-9-methyl-4-oxo-quinolizine-3-carboxylic acid;
8-(3-aminopiperidinyl)-1-cyclopropyl-7-fluoro-4H-9-methyl-4-oxo-quinolizine-3-carboxylic acid hydrochloride;
8-(3-amino-3-methylpyrrolidinyl)-1-cyclopropyl-7-fluoro-4H-9-methyl-4-oxo-quinolizine-3-carboxylic acid hydrochloride;
8-(3-amino-4-(1',3'-dioxolanyl)pyrrolidinyl)-1-cyclopropyl-7-fluoro-4H-9-methyl-4-oxo-quinolizine-3-carboxylic acid hydrochloride;
8-(3-amino-4-hydroxy-pyrrolidinyl)-1-cyclopropyl-7-fluoro-4H-9-methyl-4-oxo-quinolizine-3-carboxylic acid hydrochloride;
8-(4-(1-(N-ethylamino)methyl)piperidinyl)-1-cyclopropyl-7-fluoro-4H-9-methyl-4-oxo-quinolizine-3-carboxylic acid hydrochloride;
1-cyclopropyl-7-fluoro-8-(3-hydroxy-4-methylaminopyrrolidinyl)-4H-9-methyl-4-oxo-quinolizine-3-carboxylic acid hydrochloride;
8-(3-aminomethylpiperidinyl)-1-cyclopropyl-7-fluoro-4H-9-methyl-4-oxo-quinolizine-3-carboxylic acid hydrochloride;
8-(2-aminomethyl-4-morpholinyl)-1-cyclopropyl-7-fluoro-4H-9-methyl-4-oxo-quinolizine-3-carboxylic acid hydrochloride;
8-(3-(1-(methylamino)methypiperidinyl)-1-cyclopropyl-7-fluoro-4H-9-methyl-4-oxo-quinolizine-3-carboxylic acid hydrochloride;
8-(3-(methyl(methylenedioxy)methyl)piperidinyl)-1-cyclopropyl-7-fluoro-4H-9-methyl-4-oxo-quinolizine-3-carboxylic acid hydrochloride;
8-(3-(S)-aminopiperidinyl)-1-cyclopropyl-7-fluoro-4H-9-methyl-4-oxo-quinolizine-3-carboxylic acid hydrochloride;
8-(3-(S)-(N-ethyl-N-methylamino)piperidinyl)-1-cyclopropyl-7-fluoro-4H-9-methyl-4-oxo-quinolizine-3-carboxylic acid;
1-cyclopropyl-8-(4-(2'-(N-methylamino)methyl-1',3'-dioxolanyl)piperidinyl)-7-fluoro-9-methyl-4-oxo-4H-quinolizine-3-carboxylic acid hydrochloride;
1-cyclopropyl-8-(3-aza-6-amino-6-methylbicyclo[3.3.0] octan-1-yl)-7-fluoro-9-methyl-4-oxo-4H-quinolizine-3-carboxylic acid hydrochloride;
1-cyclopropyl-8-(3-fluoromethylpiperidinyl)-7-fluoro-9-methyl-4-oxo-4H-quinolizine;
1-cyclopropyl-8-(4-(N,N-dimethyl)aminopiperidinyl)-7-fluoro-9-methyl-4-oxo-4H-quinolizine-3-carboxylic acid hydrochloride;
1-cyclopropyl-8-(6-amino-3-azabicyclo[3.3.0]octyl)-7-fluoro-9-methyl-4-oxo-4H-quinolizine-3-carboxylic acid hydrochloride;
1-cyclopropyl-8-((2-aza-4-(dimethylaminomethyl)bicyclo[4.3.0]non-2-yl)-7-fluoro-9-methyl-4-oxo-4H-quinolizine carboxylic acid hydrochloride;
1-cyclopropyl-8-(3-aza-6-(L-alanylamino)-6-methylbicyclo[3.3.0] octane)-7-fluoro-9-methyl-4-oxo-4H-quinolizine carboxylic acid hydrochloride; and
(3R,1R)-8-(3-(1-(N-methyl)amino)propyl)pyrrolidinyl)-1-cyclopropyl-7-fluoro-9-methyl-4-oxo-4H-quinolizine-3-carboxylic acid hydrochloride;
(3R,1S)-8-(3-(1-amino-2-methoxyethyl)pyrrolidinyl)-1-cyclopropyl-7-fluoro-9-methyl-4-oxo-4H-quinolizine-3-carboxylic acid hydrochloride;
and the pharmaceutically acceptable salts, esters and amides thereof.

13. A compound according to Claim 12 selected from the group consisting of:
8-(3-(aminomethyl)pyrrolidinyl)-1-cyclopropyl-7-fluoro-9-methyl-4-oxo-4H-quinolizine-3-carboxylic acid hydrochloride;
8-(3(S)-amino-1-pyrrolidinyl)-1-cyclopropyl-7-fluoro-9-methyl-4-oxo-4H-quinolizine-3-carboxylic acid hydrochloride;
8-(3-amino- 1-pyrrolidinyl)-1-cyclopropyl-7-fluoro-9-methyl-4-oxo-4H-quinolizine-3-carboxylic acid hydrochloride;
(3R,1S)-8-(3-(1-amino)propyl)pyrrolidinyl)-1-cyclopropyl-7-fluoro-9-methyl-4-oxo-4H-quinolizine-3-carboxylic acid hydrochloride;
8-(3-(1-aminobutyl)pyrrolidinyl)-1-cyclopropyl-7-fluoro-9-methyl-4-oxo-4H-quinolizine-3-carboxylic acid hydrochloride;
(3R,1S)-8-(3-(1-amino-2-methoxyethyl)pyrrolidinyl)-1-cyclopropyl-7-fluoro-9-methyl-4-oxo-4H-quinolizine-3-carboxylic acid hydrochloride;
1-cyclopropyl-7-fluoro-9-methyl-4-oxo-8-(4-amino-1-piperdinyl)-4H-quinolizine-3-carboxylic acid hydrochloride;
1 -cyclopropyl-7-fluoro-9-methyl-4-oxo-8-(3-amino-1-piperdinyl)-4H-quinolizine-3-carboxylic acid hydrochloride;
8-(3-(S)-aminopiperidinyl)-1-cyclopropyl-7-fluoro-4H-9-methyl-4-oxo-quinolizine-3-carboxylic acid hydrochloride;
1-cyclopropyl-8-(3-aza-6-amino-6-methylbicyclo[3.3.0]octan-1-yl)-7-fluoro-9-methyl-4-oxo-4H-quinolizine-3-carboxylic acid hydrochloride;
1-cyclopropyl-8-(6-amino-3-azabicyclo[3.3.0]octyl)-7-fluoro-9-methyl-4-oxo-4H-quinolizine-3-carboxylic acid hydrochloride;
8-((1R*,2S*,6R*)-2-amino-8-azabicyclo[4.3.0]nonan-8-yl))-1-cyclopropyl-7-fluoro-4H-9-methyl-4-oxo-quinolizine-3-carboxylic acid hydrochloride;
8-((1R*,2R*,6R*)-2-amino-8-azabicyclo[4.3.0]nonan-8-yl))-1-cyclopropyl-7-fluoro-4H-9-methyl-4-oxo-quinolizine-3-carboxylic acid hydrochloride;
8-(3-(1-aminoethyl)pyrrolidinyl)-1-cyclopropyl-7-fluoro-4H-9-methyl-4-oxo-quinolizine-3-carboxylic acid hydrochloride;
(8-(3-(1-amino-1-methylethyl)pyrrolidinyl)-1-cyclopropyl-7-fluoro-9-methyl-4-oxo-4H-quinolizine-3-carboxylic acid hydrochloride;
(3R,1S)-8-(3-(1-(N-methyl)amino)propyl)pyrrolidinyl)-1-cyclopropyl-7-fluoro-9-methyl-4-oxo-4H-quinolizine-3-carboxylic acid hydrochloride;
8-(3-aminopiperidinyl)-1-cyclopropyl-7-fluoro-4H-9-methyl-4-oxo-quinolizine-3-carboxylic acid hydrochloride;
8-(3-(1-aminocyclopropyl)pyrrolidinyl)-1-cyclopropyl-7-fluoro-9-methyl-4-oxo-4H-quinolizine-3-carboxylic acid hydrochloride;
(3S,1R)-8-(3-(1-aminoethyl)pyrrolidinyl)-1-cyclopropyl-7-fluoro-9-methyl-4-oxo-4H-quinolizine-3-carboxylic acid hydrochloride;
(3R,1S)-8-(3-(1-aminoethyl)pyrrolidinyl)-1-cyclopropyl-7-fluoro-9-methyl-4-oxo-4H-quinolizine-3-carboxylic acid hydrochloride; and
(3R,1R)-8-(3-(1-aminoethyl)pyrrolidinyl)-1-cyclopropyl-7-fluoro-9-methyl-4-oxo-4H-quinolizine-3-carboxylic acid hydrochloride;
and the pharmaceutically acceptable salts, esters and amides thereof.

14. A compound according to Claim 12 selected from the group consisting of:
8-(3(S)-amino-1-pyrrolidinyl)-1-cyclopropyl-7-fluoro-9-methyl-4-oxo-4H-quinolizine-3-carboxylic acid hydrochloride;
8-(3-amino- 1-pyrrolidinyl)-1-cyclopropyl-7-fluoro-9-methyl-4-oxo-4H-quinolizine-3-carboxylic acid hydrochloride;
(3R,1S)-8-(3-(1-amino-2-methoxyethyl)pyrrolidinyl)-1-cyclopropyl-7-fluoro-9-methyl-4-oxo-4H-quinolizine-3-carboxylic acid hydrochloride;
(8-(3-(1-amino-1-methylethyl)pyrrolidinyl)-1-cyclopropyl-7-fluoro-9-methyl-4-oxo-4H-quinolizine-3-carboxylic acid hydrochloride:
8-(3-(1-aminocyclopropyl)pyrrolidinyl)-1-cyclopropyl-7-fluoro-9-methyl-4-oxo-4H-quinolizine-3-carboxylic acid hydrochloride: and
(3R,1S)-8-(3-(1-aminoethyl)pyrrolidinyl)-1-cyclopropyl-7-fluoro-9-methyl-4-oxo-4H-quinolizine-3-carboxylic acid hydrochloride;
and the pharmaceutically acceptable salts, esters and amides thereof.

15. The compound 8-(3(S)-aminopyrrolidinyl)-1-cyclopropyl-7-fluoro-9-methyl-4-oxo-4H-quinolizine-3-carboxylic acid hydrochloride, and the pharmaceutically acceptable salts. esters and amides thereof.

16. A pharmaceutical composition comprising a compound according to any of Claims 1, 9, 12 or 15 in combination with a pharmaceutically acceptable carrier.

17. A compound according to any of claims 1, 9, 12 or 15 for use as a therapeutic agent.

18. Use of a compound according to any of Claims 1, 9, 12 or 15 for manufacturing a medicament for treating a bacterial infection in a human or veterinary patient.

19. A synthetic intermediate selected from the group consisting of:
4-t-butoxy-3-chloro-2,5,6-trifluoropyridine;
4-t-butoxy-2,3,6-trifluoropyridine;
4-t-butoxy-2,3,6-trifluoro-5-methylpyridine;
4-t-butoxy-2,5-difluoro-3-methylpyridine;
2-(4-t-butoxy-5-fluoro-3-methyl-2-pyridinyl)cyclopropaneacetonitrile;
2-(4-chloro-5-fluoro-3-methyl-2-pyridinyl)cyclopropaneacetonitrile;
2-(4-chloro-5-fluoro-3-methyl-2-pyridinyl)cyclopropaneacetic acid;
ethyl 2-(4-chloro-5-fluoro-3-methyl-2-pyridinyl)cyclopropaneacetate;
2-(4-chloro-5-fluoro-3-methyl-2-pyridinyl )cyclopropaneacetaldehyde;
2-(4-chloro-5-fluoro-3-methyl-2-pyridinyl)cyclopropaneethanol;
2-(2-(4-chloro-5-fluoro-3-methyl-2-pyridinyl)-2-cyclopropylethylidinyl)-1,3-propanedicarboxylic acid, diethyl ester; and
8-chloro-1-cyclopropyl-7-fluoro-9-methyl-4-oxo-4h-quinolizine-3-carboxylic acid ethyl ester.

## Patentansprüche

1. Eine Verbindung der Formel 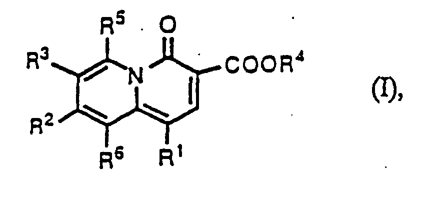 oder ein pharmazeutisch verträgliches Salz, Ester oder Amid davon, worin
R¹ gewählt ist aus der Gruppe bestehend aus (a) C₁-C₆ Alkyl, (b) C₂-C₆ Alkenyl, (c) C₁-C₆ Haloalkyl, (d) C₁-C₆ Alkoxy, (e) C₃-C₈ Cycloalkyl, (f) Phenyl, (g) substituiertes Phenyl, (h) Halo, (i) Cyano, (j) Nitro, (k) Bicycloalkyl, (l) C₂-C₆ Alkinyl, (m) (C₁-C₆ Alkoxy)carbonyl, (n) Stickstoff-haltigem aromatischen Heterozyklus, (o) Halo-substituiertem Stickstoff-haltigen aromatischen Heterozyklus, (p) einem 4-, 5- oder 6-gliedrigen zyklischen Ether, und (q) -NR⁷R⁸, worin R⁷ und R⁸ unabhängig gewählt sind aus der Gruppe bestehend aus Wasserstoff, C₁-C₆ Alkyl und C₁-C₈ Alkanoyl, oder R⁷ und R⁸ bilden zusammengenommen mit dem Stickstoffatom, an das sie gebunden sind, einen 5-, 6-oder 7-gliedrigen Heterozyklus;
R² gewählt ist aus der Gruppe bestehend aus (a) Halo, (b) C₁-C₆ Alkyl, (c) C₂-C₆ Alkenyl, (d) C₃-C₈ Cycloalkyl, (e) C₄-C₈ Cycloalkenyl, (f) C₁-C₆ Alkoxy, (g) Aryloxy, (h) Aryl-C₁-C₆ Alkoxy, (i) Aryl-C₁-C₆ Alkyl, (j) Cycloalkyl-C₁-C₆ Alkyl, (k) Amino, (l) mono- oder di-(C₁-C₆ Alkyl)amino, (m) Aryl(C₁-C₆ Alkyl)amino, (n) Hydroxy-substituiertem (C₁-C₆ Alkyl)amino, (o) Phenyl, (p) substituiertem Phenyl, (q) bizyklischem Stickstoff-enthaltenden Heterozyklus, (r) Stickstoff-enthaltendem aromatischen Heterozyklus und (s) Stickstoff-enthaltendem Heterozyklus, welcher folgende Formel hat 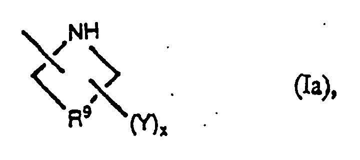 worin x zwischen 0 und 3 ist;
R⁹ ist gewählt aus der Gruppe bestehend aus (i) -(CH₂)ₘ- worin m zwischen 1 und 3 ist und (ii) -(CH₂)ₙR¹⁰(CH₂)ₚ- worin R¹⁰ gewählt ist aus der Gruppe bestehend aus -S-, -O- und -NH, n ist 1 oder 2 und p ist 1 oder 2; und
Y ist, jedesmal wenn es vorkommt, unabhängig gewählt aus der Gruppe bestehend aus
(i) C₁-C₆ Alkyl,
(ii) Hydroxy,
(iii) Halo,
(iv) C₁-C₆ Haloalkyl,
(v) C₁-C₆ Alkoxy,
(vi) C₁-C₆ Alkoxy(C₁-C₆ Alkyl),
(vii) C₁-C₆ Alkoxy(C₁-C₆ Alkoxy) (C₁-C₆ Alkyl),
(viii) Hydroxy-substituiertem C₁-C₆ Alkyl,
(ix) Imino,
(x) Amino(C₁-C₆ Alkyl),
(xi) (C₁-C₆ Haloalkyl) amino (C₁-C₆ Alkyl),
(xii) C₁-C₆ Thioalkoxy (C₁-C₆ Alkyl),
(xiii) C₁-C₆ Thioalkoxy(C₁-C₆ Alkoxy),
(xiv) Amino (C₁-C₆ Thioalkoxy),
(xv) C₃-C₆ Cycloalkyl,
(xvi) C₃-C₈ Cycloalkyl (C₁-C₆ Alkyl),
(xvii) Phenyl,
(xviii) substituiertem Phenyl,
(xix) Stickstoff-enthaltendem aromatischen Heterozyklus,
(xx) -NR¹¹R¹², worin R¹¹ und R¹² unabhängig gewählt sind aus der Gruppe bestehend aus Wasserstoff und C₁-C₆ Alkyl, oder, wenn eins von R¹¹ und R¹² Wasserstoff ist, ist das andere gewählt aus der Gruppe bestehend aus C₁-C₈ Alkanoyl, einer alpha-Aminosäure und einem Polypeptidrest aus 2 bis 5 Aminosäuren, und
(xxi) -C(R²¹) (R²²)NH₂, worin R²¹ und R²² unabhängig gewählt sind aus der Gruppe bestehend aus Wasserstoff, C₁-C₆ Alkyl, Hydroxy-substituiertem C₁-C₆ Alkyl, Amino(C₁-C₆ Alkyl), C₁-C₆ Alkoxy (C₁-C₆ Alkyl), C₁-C₆ Thioalkoxy (C₁-C₆ Alkyl), C₃-C₆ Cycloalkyl und C₁-C₆ Alkyl, substituiert mit einem Stickstoff-haltigen aromatischen Heterozyklus, oder R²¹ und R²² bilden zusammengenommen mit dem Kohlenstoffatom, an welches sie gebunden sind, eine Ringstruktur gewählt aus C₃-C₆ Cycloalkyl und Stickstoff-enthaltendem Heterozyklus;
R³ ist gewählt aus der Gruppe bestehend aus Wasserstoff, Halo und C₁-C₆ Alkoxy;
R⁴ ist gewählt aus der Gruppe bestehend aus Wasserstoff, C₁-C₆ Alkyl, einem pharmazeutisch verträglichen Kation und einer Prodrug-Estergruppe, die eine Ester-bildende Gruppe ist, die unter physiologischen Bedingungen hydrolysiert wird;
R⁵ ist gewählt aus der Gruppe bestehend aus (a) Wasserstoff, (b) Halo, (c) Hydroxy, (d) C₁-C₆ Alkyl, (e) C₁-C₆ Haloalkyl, (f) C₁-C₆ Alkoxy und (g) -NR¹³R¹⁴, worin R¹³ und R¹⁴ unabhängig gewählt sind aus der Gruppe bestehend aus Wasserstoff, C₁-C₆ Alkyl, Hydroxy-substituiertem C₁-C₆ Alkyl, C₁-C₆ Alkoxy(C₁-C₆ Alkyl) und C₁-C₆ Alkanoyl; und
R⁶ ist C₁-C₆ Alkyl.

2. Eine Verbindung gemäß Anspruch 1, worin R³ Halogen ist.

3. Eine Verbindung gemäß Anspruch 2, worin R⁵ gewählt ist aus der Gruppe bestehend aus Wasserstoff, C₁-C₆ Alkyl, C₁-C₆ Haloalkyl und -NR¹³R¹⁴, worin R¹³ und R¹⁴ wie vorher definiert sind.

4. Eine Verbindung gemäß Anspruch 3, worin R¹ gewählt ist aus der Gruppe bestehend aus Cycloalkyl von drei bis acht Kohlenstoffatomen und substituiertem Phenyl.

5. Eine Verbindung gemäß Anspruch 4, worin R² gewählt ist aus der Gruppe bestehend aus bizyklischem Stickstoff-enthaltenden Heterozyklus und einem Stickstoff-enthaltenden Heterozyklus, der folgende Formel hat 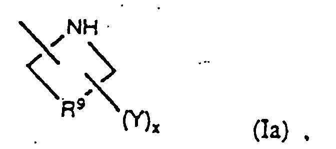 worin R⁹, Y und x wie vorher definiert sind.

6. Eine Verbindung gemäß Ansrpuch 5, worin R² gewählt ist aus der Gruppe besthend aus 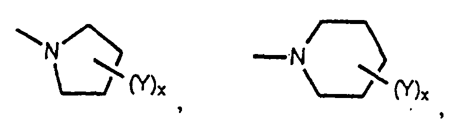 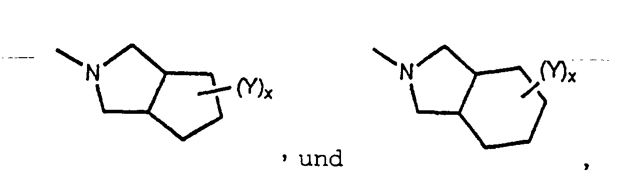 worin Y und x wie vorher definiert sind.

7. Eine Verbindung gemäß Anspruch 6, worin x eins oder zwei ist, und Y gewählt ist aus der Gruppe bestehend aus -NR¹¹R¹² und -C(R²¹)(R²²)NH₂, worin R¹¹, R¹², R²¹ und R²² wie vorher definiert sind.

8. Eine Verbindung gemäß irgendeinem der Ansprüche 2, 3, 4, 5, 6 oder 7, worin R⁶ Methyl ist.

9. Ein Verbindung gemäß Anspruch 1 mit der Formel 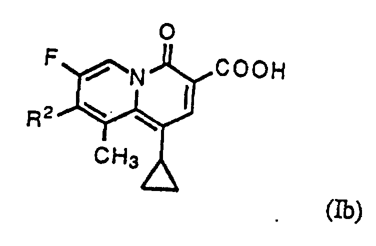 oder ein pharmazeutisch verträgliches Salz, Ester oder Amid davon, worin
R² gewählt ist aus der Gruppe bestehend aus bizyklischem Stickstoff-haltigen Heterozyklus und einem Stickstoff-haltigen Heterozyklus mit der Formel 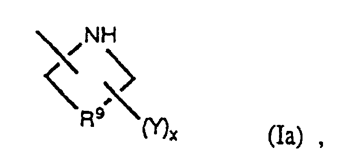 und worin R⁴, R⁹, Y und x wie zuvor definiert sind.

10. Eine Verbindung gemäß Anspruch 9, worin R² gewählt ist aus der Gruppe bestehend aus 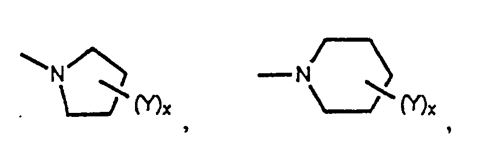 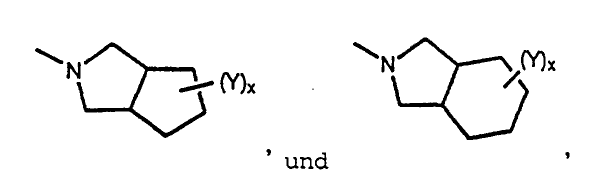 worin Y und x wie zuvor definiert sind.

11. Eine Verbindung gemäß Anspruch 10, worin x eins oder zwei ist, und Y gewählt ist aus der Gruppe bestehend aus -NR¹¹R¹² und -C(R²¹) (R²²)NH₂, worin R¹¹, R¹², R²¹ und R²² wie vorher definiert sind.

12. Eine Verbindung gemäß Anspruch 1, gewählt aus der Gruppe bestehend aus:
8-(3-Amino-1-pyrrolidinyl)-1-cylopropyl-7-fluor-9-methyl-4-oxo-4H-chinolizin-3-carbonsäure Hydrochlorid;
8-(3-Aminomethyl)pyrrolidinyl)-1-cylopropyl-7-fluor-9-methyl-4-oxo-4H-chinolizin-3-carbonsäure Hydrochlorid;
8-(2S,4S-4-Amino-2-methylpyrrolidinyl)-1-cylopropyl-7-fluor-9-methyl-4-oxo-4H-chinolizin-3-carbonsäure Hydrochlorid;
8-(3-Aminoazetidinyl)-1-cyclopropyl-7-fluor-9-methyl-4-oxo-4Hchinolizin-3-carbonsäure Hydrochlorid;
8-(3(S)-Aminopyrrolidinyl)-1-cylopropyl-7-fluor-9-methyl-4-oxo-4H-chinolizin-3-carbonsäure Hydrochlorid;
1-Cyclopropyl-7-fluor-9-methyl-4-oxo-8-(3-methyl-1-piperazinyl)-4H-chinolizin-3-carbonsäure Hydrochlorid;
1-Cyclopropyl-7-fluor-9-methyl-4-oxo-8-piperazinyl-4H-chinolizin-3-carbonsäure Hydrochlorid;
1-Cyclopropyl-7-fluor-9-methyl-8-(2-((N-methyl)aminomethyl)-4-morpholinyl)-4-oxo-4H-chinolizin-3-carbonsäure Hydrochlorid;
1-Cyclopropyl-7-fluor-9-methyl-4-oxo-8-(1,2,3,4-tetrahydro-2-isochinolinyl)-4H-chinolizin-3-carbonsäure;
1-cyclopropyl-7-fluor-9-methyl-4-oxo-8-(4-amino-1-piperidinyl)-4H-chinolizin-3-carbonsäure Hydrochlorid;
1-Cyclopropyl-7-fluor-9-methyl-4-oxo-8-(3-amino-1-piperidinyl)-4H-chinolizin-3-carbonsäure Hydrochlorid;
1-Cyclopropyl-7-fluor-9-methyl-4-oxo-8-(4-(aminomethyl)-1-piperidinyl)-4H-chinolizin-3-carbonsäure Hydrochlorid;
1-Cyclopropyl-7-fluor-9-methyl-4-oxo-8-(5-amino-1,2,3,4-tetrahydro-2-isochinolinyl)-4H-chinolizin-3-carbonsäure Hydrochlorid;
1-Cyclopropyl-7-fluor-9-methyl-4-oxo-8-(4(1-pyrrolyl)-1-piperidinyl)-4H-chinolizin-3-carbonsäure;
1-Cyclopropyl-8-(*cis*-3,5-dimethyl-1-piperazinyl)-7-fluor-9-methyl-4-oxo-4H-chinolizin-3-carbonsäure Hydrochlorid;
1-cyclopropyl-8-(2,7-diaza-7-bicyclo[3.3.0]octyl)-7-fluor-9-methyl-4-oxo-4H-chinolizin-3-carbonsäure Hydrochlorid;
1-Cyclopropyl-8-(2,8-diaza-8-bicyclo[4.3.0]nonyl)-7-fluor-9-methyl-4-oxo-4H-chinolizin-3-carbonsäure Hydrochlorid;
1-Cyclopropyl-7-fluor-9-methyl-4-oxo-8-(3(S)-(1-pyrrolyl)-1-pyrrolidinyl)-4H-chinolizin-3-carbonsäure;
1-Cyclopropyl-7-fluor-8-(3-hydroxy-1-pyrrolidinyl)-9-methyl-4-oxo-4H-chinolizin-3-carbonsäure Hydrochlorid;
1-Cyclopropyl-7-fluor-8-(4-methyl-1-piperazinyl)-9-methyl-4-oxo-4H-chinolizin-3-carbonsäure Hydrochlorid;
1-Cyclopropyl-8-(2,7-diaza-7-bicyclo[3.3.0]octyl)-7-fluor-9-methyl-4-oxo-4H-chinolizin-3-carbonsäure Hydrochlorid;
1-Cyclopropyl-8-(2,8-diaza-8-bicyclo[4.3.0]nonyl)-7-fluor-9-methyl-4-oxo-4H-chinolizin-3-carbonsäure Hydrochlorid;
1-Cyclopropyl-7-fluor-9-methyl-4-oxo-8-(3(S)-(1-pyrrolyl)-1-pyrrolidinyl)-4H-chinolizin-3-carbonsäure;
1-Cyclopropyl-7-fluor-8-(3-hydroxy-1-pyrrolidinyl)-9-methyl-4-oxo-4H-chinolizin-3-carbonsäure Hydrochlorid;
1-Cyclopropyl-7-fluor-8-(4-methyl-1-piperazinyl)-9-methyl-4-oxo-4H-chinolizin-3-carbonsäure Hydrochlorid;
1-Cyclopropyl-7-fluor-9-methyl-8-(3(S)-methylamino-1-pyrrolidinyl)-4-oxo-4H-chinolizin-3-carbonsäure Hydrochlorid;
1-cyclopropyl-7-fluor-9-methyl-8-(3(R)-amino-1-pyrrolidinyl)-4-oxo-4H-chinolizin-3-carbonsäure Hydrochlorid;
8-(2;4-Dimethyl-1-piperazinyl)-1-cyclopropyl-7-fluor-9-methyl-4-oxo-4H-chinolizin-3-carbonsäure Hydrochlorid;
8-(3-(Methylamino)-1-piperazinyl)-1-cyclopropyl-7-fluor-9-methyl-4-oxo-4H-chinolizin-3-carbonsäure Hydrochlorid;
8-(3-(Methylamino)-1-morpholinyl)-1-cyclopropyl-7-fluor-9-methyl-4-oxo-4H-chinolizin-3-carbonsäure Hydrochlorid;
8-(3-(S)-(Methylamino)-1-pyrrolidinyl)-1-cyclopropyl-7-fluor-9-methyl-4-oxo-4H-chinolizin-3-carbonsäure Hydrochlorid;
8-(3-(S)-(1-(Nethylamino)methyl)-1-pyrrolidinyl)-1-cyclopropyl-7-fluor-9-methyl-4-oxo-4H-chinolizin-3-carbonsäure Hydrochlorid; 8-(3-(S)-(1-(Ethylamino)methyl)-1-pyrrolidinyl)-1-cyclopropyl-7-fluor-9-methyl-4-oxo-4H-chinolizin-3-carbonsäure Hydrochlorid;
8-(Octahydropyrrolo[3,4-c]pyrrol-1-yl)-1-cyclopropyl-7-fluor-9-methyl-4-oxo-4H-chinolizin-3-carbonsäure Hydrochlorid;
8-(Octahydropyrrolo[3,4-c]pyidin-5-yl)-1-cyclopropyl-7-fluor-9-methyl-4-oxo-4H-chinolizin-3-carbonsäure Hydrochlorid;
8-(cis-4-Amino-3-methylpyrrolidinyl)-1-cyclopropyl-7-fluor-9-methyl-4-oxo-4H-chinolizin-3-carbonsäure Hydrochlorid;
8-(trans-4-Amino-3-methylpyrrolidinyl)-1-cyclopropyl-7-fluor-9-methyl-4-oxo-4H-chinolizin-3-carbonsäure Hydrochlorid;
8-(3-Methyl-4-spirocyclopropylpyrrolidinyl)-1-cyclopropyl-7-fluor-9-methyl-4-oxo-4H-chinolizin-3-carbonsäure Hydrochlorid;
8-(3-Dimethylaminopyrrolidinyl)-1-cyclopropyl-7-fluor-9-methyl-4-oxo-4H-chinolizin-3-carbonsäure, Essigsäuresalz;
(3R)-8-(3-Dimethylaminopyrrolidinyl)-1-cyclopropyl-7-fluor-9-methyl-4-oxo-4H-chinolizin-3-carbonsäure Hydrochlorid;
(3R,1S)-8-(3-(1-Aminoethyl)pyrrolidinyl)-1-cyclopropyl-7-fluor-9-methyl-4-oxo-4H-chinolizin-3-carbonsäure Hydrochlorid;
(3S,1R)-8-(3-(1-Aminoethyl)pyrrolidinyl)-1-cyclopropyl-7-fluor-9-methyl-4-oxo-4H-chinolizin-3-carbonsäure Hydrochlorid;
(3R,1R)-8-(3-(1-Aminoethyl)pyrrolidinyl)-1-cyclopropyl-7-fluor-9-methyl-4-oxo-4H-chinolizin-3-carbonsäure Hydrochlorid;
1-Cyclopropyl-8-((R,S)-3-fluorpyrrolidin)-7-fluor-9-methyl-4-oxo-4H-chinolizin-3-carbonsäure;
8-(4-(1-Piperidyl)-1-piperidyl)-1-cyclopropyl-7-fluor-9-methyl-4-oxo-4H-chinolizin-3-carbonsäure;
8-(4-(1-Piperidyl)-1-piperidyl)-1-cyclopropyl-7-fluor-9-methyl-4-oxo-4H-chinolizin-3-carbonsäure Trifluoressigsäuresalz;
8-(4-(2-Pyridyl)-1-piperazinyl)-1-cyclopropyl-7-fluor-9-methyl-4-oxo-4H-chinolizin-3-carbonsäure;
8-((2-Amino)thioethoxy)-1-cyclopropyl-7-fluor-9-methyl-4-oxo-4Hchinolizin-3-carbonsäure Trifluoressigsäuresalz;
(3R,1S)-8-(3-(1-Amino)propyl)pyrrolidinyl)-1-cyclopropyl-7-fluor-9-methyl-4-oxo-4H-chinolizin-3-carbonsäure Hydrochlorid;
(3R,1S)-8-(3-(1-(N-Methyl)amino)propyl)pyrrolidinyl)-1-cyclopropyl-7-fluor-9-methyl-4-oxo-4H-chinolizin-3-carbonsäure Hydrochlorid;
(3R,1S)-8-(3-(1-Amino-3-methylpropyl)pyrrolidinyl)-1-cyclopropyl-7-fluor-9-methyl-4-oxo-4H-chinolizin-3-carbonsäure Hydrochlorid;
8-(3-(1-Aminocyclopropyl)pyrrolidinyl)-1-cyclopropyl-7-fluor-9-methyl-4-oxo-4H-chinolizin-3-carbonsäure Hydrochlorid;
(3R,1S)-8(3-(1-Amino-2-hydroxyethyl)pyrrolidinyl)-1-cyclopropyl-7-fluor-9-methyl-4-oxo-4H-chinolizin-3-carbonsäure Hydrochlorid;
(8-(3-(1-Amino-1-methylethyl)pyrrolidinyl)-1-cyclopropyl-7-fluor-9-methyl-4-oxo-4H-chinolizin-3-carbonsäure Hydrochlorid;
(8-(3-(1-Aminobutyl)pyrrolidinyl)-1-cyclopropyl-7-fluor-9-methyl-4-oxo-4H-chinolizin-3-carbonsäure Hydrochlorid;
1-Cyclopropyl-7-fluor-9-methyl-4-oxo-8-(trans-4-trifluormethyl-3-aminopyrrolidinyl)-4H-chinolizin-3-carbonsäure Hydrochlorid;
1-Cyclopropyl-7-fluor-9-methyl-4-oxo-8-(trans-4-trifluormethyl-3-aminomethylpyrrolidinyl)-4H-chinolizin-3-carbonsäure Hydrochlorid;
3(S)-1-Cyclopropyl-7-fluor-9-methyl-4-oxo-8-(3-(N-(S)-norvalylamino)pyrrolidinyl)-4H-chinolizin-3-carbonsäure Hydrochlorid;
3(S)-1-Cyclopropyl-7-fluor-9-methyl-4-oxo-8-(3-(N-(S)-alanylamino)pyrrolidinyl)-4H-chinolizin-3-carbonsäure Hydrochlorid;
3(S)-1-Cyclopropyl-7-fluor-9-methyl-4-oxo-8-(3-(N-(S)-alanyl-(S)-alanylamino)pyrrolidinyl)-4H-chinolizin-3-carbonsäure Hydrochlorid;
1-Cyclopropyl-7-fluor-4H-8-(1-imidazolyl)-9-methyl-4-oxochinolizin-3-carbonsäure Hydrochlorid;
8-(3-Amino-1-pyrrolidinyl)-1-ethyl-7-fluor-4H-4-oxo-9-methylchinolizin-3-carbonsäure Hydrochlorid;
8-(3-Amino-1-pyrrolidinyl)-1-cyclopropyl-9-ethyl-7-fluor-4H-4-oxo-chinolizin-3-carbonsäure Hydrochlorid;
1-Cyclopropyl-7-fluor-4H-9-methyl-4-oxo-8-(3-(1,2,3-triazol-1-yl)-1-pyrrolidinyl)-chinolizin-3-carbonsäure;
1-Cyclopropyl-7-fluor-4H-9-methyl-4-oxo-8-(cis-3-amino-4-methyl-1-pyrrolidinyl)-chinolizin-3-carbonsäure Hydrochlorid;
8-(2-Aminoethyl)-1-cyclopropyl-7-fluor-4H-9-methyl-4-oxochinolizin-3-carbonsäure Hydrochlorid;
8-(3-(Ethylaminomethyl)pyrrolidinyl)-1-cyclopropyl-7-fluor-4H-9-methyl-4-oxo-chinolizin-3-carbonsäure Hydrochlorid;
8-(3-(1-Aminoethyl)pyrrolidinyl)-1-cyclopropyl-7-fluor-4H-9-methyl-4-oxo-chinolizin-3-carbonsäure Hydrochlorid;
1-Cyclopropyl-7-fluor-4H-9-methyl-8-(2-methyl-2,8-diaza-8-bicyclo[4.3.0)nonyl)-4-oxo-chinolizin-3-carbonsäure Hydrochlorid; -
1-Cyclopropyl-7-fluor-4H-8-((1S,4S)-2,5-diazabicyclo[2.2.1]heptan-2-yl)-9-methyl-4-oxo-chinolizin-3-carbonsäure Hydrochlorid;
1-Cyclopropyl-7-fluor-4H-9-methyl-4-oxo-8-(3-(2-pyridinyl)-1-pyrrolidinyl)-chinolizin-3-carbonsäure Hydrochlorid;
8-((1R*,2S*,6R*)-2-Amino-8-azabicyclo[4.3.0]nonan-8-yl))-1-cyclopropyl-7-fluor-4H-9-methyl-4-oxo-chinolizin-3-carbonsäure Hydrochlorid;
8-((1R*,2R*,6R*)-2-Amino-8-azabicyclo[4.3.0]nonan-8-yl))-1-cyclopropyl-7-fluor-4H-9-methyl-4-oxo-chinolizin-3-carbonsäure Hydrochlorid;
8-((1a,5a,6a)-6-Amino-3-azabicyclo[3.1.0]hexan-3-yl))-1-cyclopropyl-9-methyl-7-fluor-4H-4-oxo-chinolizin-3-carbonsäure Hydrochlorid;
8-(*cis*-3-Amino-4-fluor-1-pyrrolidinyl))-1-cyclopropyl-9-methyl-7-fluor-4H-4-oxo-chinolizin-3-carbonsäure Hydrochlorid;
1-Cyclopropyl-7-fluor-4H-8-(1-homopiperazinyl))-9-methyl-4-oxochinolizin-3-carbonsäure, Essigsäuresalz;
8-(spiro-1,3-Dioxacyclopentan[2.3]-1-piperidinyl)-1-cyclopropyl-7-fluor-4H-9-methyl-4-oxo-chinolizin-3-carbonsäure;
8-(3-Amino-4-methoxypyrrolidinyl)-1-cyclopropyl-7-fluor-4H-9-methyl-4-oxo-chinolizin-3-carbonsäure Hydrochlorid;
8-(4-Amino-4-methylpyrrolidinyl)-1-cyclopropyl-7-fluor-4H-9-methyl-4-oxo-chinolizin-3-carbonsäure Hydrochlorid;
8-(4-(2-Hydroxyethyl)piperidinyl)-1-cyclopropyl-7-fluor-4H-9-methyl-4-oxo-chinolizin-3-carbonsäure;
8-(4-(Methoxymethyl)piperidinyl)-1-cyclopropyl-7-fluor-4H-9-methyl-4-oxo-chinolizin-3-carbonsäure;
8-(3-Amino-3-methylpiperidinyl)-1-cyclopropyl-7-fluor-4H-9-methyl-4-oxo-chinolizin-3-carbonsäure Hydrochlorid;
8-(3-Pyrrolylpiperidinyl)-1-cyclopropyl-7-fluor-4H-9-methyl-4-oxo-chinolizin-3-carbonsäure;
8-(3-Aminopiperidinyl)-1-cyclopropyl-7-fluor-4H-9-methyl-4-oxochinolizin-3-carbonsäure Hydrochlorid;
8-(3-Amino-3-methylpyrrolidinyl)-1-cyclopropyl-7-fluor-4H-9-methyl-4-oxo-chinolizin-3-carbonsäure Hydrochlorid;
8-(3-Amino-4-(1',3'-dioxolanyl)pyrrolidinyl)-1-cyclopropyl-7-fluor-4H-9-methyl-4-oxo-chinolizin-3-carbonsäure Hydrochlorid;
8-(3-Amino-4-hydroxy-pyrrolidinyl)-1-cyclopropyl-7-fluor-4H-9-methyl-4-oxo-chinolizin-3-carbonsäure Hydrochlorid;
8-(4-(1-(N-Ethylamino)methyl)piperidinyl)-1-cyclopropyl-7-fluor-4H-9-methyl-4-oxo-chinolizin-3-carbonsäure Hydrochlorid;
1-Cyclopropyl-7-fluor-8-(3-hydroxy-4-methylaminopyrrolidinyl)-4H-9-methyl-4-oxo-chinolizin-3-carbonsäure Hydrochlorid;
8-(3-Aminomethylpiperidinyl)-1-cyclopropyl-7-fluor-4H-9-methyl-4-oxo-chinolizin-3-carbonsäure Hydrochlorid;
8-(2-Aminomethyl-4-morpholinyl)-1-cyclopropyl-7-fluor-4H-9-methyl-4-oxo-chinolizin-3-carbonsäure Hydrochlorid;
8-(3-(1-(Methylamino)methylpiperidinyl)-1-cyclopropyl-7-fluor-4H-9-methyl-4-oxo-chinolizin-3-carbonsäure Hydrochlorid;
8-(3-(Methyl(methylendioxy)methyl)piperidinyl)-1-cyclopropyl-7-fluor-4H-9-methyl-4-oxo-chinolizin-3-carbonsäure Hydrochlorid;
8-(3-(S)-Aminopiperidinyl)-1-cyclopropyl-7-fluor-4H-9-methyl-4-oxo-chinolizin-3-carbonsäure Hydrochlorid;
8-(3-(S)-(N-Ethyl-N-methylamino)piperidinyl)-1-cyclopropyl-7-fluor-4H-9-methyl-4-oxo-chinolizin-3-carbonsäure;
1-Cyclopropyl-8-(4-(2'-(N-methylamino)methyl-1',3'dioxolanyl)piperidinyl)-7-fluor-9-methyl-4-oxo-4H-chinolizin-3-carbonsäure-Hydrochlorid;
1-cyclopropyl-8-(3-aza-6-amino-6-methylbicyclo[3.3.0]octan-1-yl)-7-fluor-9-methyl-4-oxo-4H-chinolizin-3-carbonsäure-Hydrochlorid;
1-Cyclopropyl-8-(3-fluormethylpiperidinyl)-7-fluor-9-methyl-4-oxo-4H-chinolizin;
1-Cyclopropyl-8-(4-(N,N-dimethyl)aminopiperidinyl)-7-fluor-9-methyl-4-oxo-4H-chinolizin-3-carbonsäure Hydrochlorid;
1-Cyclopropyl-8-(6-amino-3-azabicyclo[3.3.0]octyl)-7-fluor-9-methyl-4-oxo-4H-chinolizin-3-carbonsäure Hydrochlorid;
1-Cyclopropyl-8-((2-aza-4-(dimethylaminomethyl)bicyclo[4.3.0]non-2-yl)-7-fluor-9-methyl-4-oxo-4H-chinolizincarbonsäure Hydrochlorid;
1-Cyclopropyl-8-(3-aza-6-(L-alanylamino)-6-methylbicyclo[3.3.0]octan)-7-fluor-9-methyl-4-oxo-4Hchinolizincarbonsäure Hydrochlorid; und
(3R,1R)-8-(3-(1-(N-Methyl)amino)propyl)pyrrolidinyl)-1-cyclopropyl-7-fluor-9-methyl-4-oxo-4H-chinolizin-3-carbonsäure Hydrochlorid;
(3R,1S)-8-(3-(1-Amino-2-methoxyethyl)pyrrolidinyl)-1-cyclopropyl-7-fluor-9-methyl-4-oxo-4H-chinolizin-3-carbonsäure Hydrochlorid;
und die pharmazeutisch verträglichen Salze, Ester und Amide davon.

13. Eine Verbindung gemäß Anspruch 12 gewählt aus der Gruppe bestehend aus:
8-(3-(Aminomethyl)pyrrolidinyl)-1-cyclopropyl-7-fluor-9-methyl-4-oxo-4H-chinolizin-3-carbonsäure Hydrochlorid;
8-(3(S)-Amino-1-pyrrolidinyl)-1-cyclopropyl-7-fluor-9-methyl-4-oxo-4H-chinolizin-3-carbonsäure Hydrochlorid;
8-(3-(Amino-1-pyrrolidinyl)-1-cyclopropyl-7-fluor-9-methyl-4-oxo-4H-chinolizin-3-carbonsäure Hydrochlorid;
(3R,1S)-8-(3-(1-Amino)propyl)pyrrolidinyl)-1-cyclopropyl-7-fluor-9-methyl-4-oxo-4H-chinolizin-3-carbonsäure Hydrochlorid;
8-(3-(1-Aminobutyl)pyrrolidinyl)-1-cyclopropyl-7-fluor-9-methyl-4-oxo-4H-chinolizin-3-carbonsäure Hydrochlorid;
(3R,1S)-8-(3-(1-Amino-2-methoxyethyl)pyrrolidinyl)-1-cyclopropyl-7-fluor-9-methyl-4-oxo-4H-chinolizin-3-carbonsäure Hydrochlorid;
1-cyclopropyl-7-fluor-9-methyl-4-oxo-8-(4-amino-1-piperidinyl)-4H-chinolizin-3-carbonsäure Hydrochlorid;
1-cyclopropyl-7-fluor-9-methyl-4-oxo-8-(3-amino-1-piperidinyl)-4H-chinolizin-3-carbonsäure Hydrochlorid;
8-(3-(S)-Aminopiperidinyl)-1-cyclopropyl-7-fluor-4H-9-methyl-4-oxo-chinolizin-3-carbonsäure Hydrochlorid;
1-Cyclopropyl-8-(3-aza-6-Amino-6-methylbicyclo[3.3.0]octan-1-yl)-7-fluor-9-methyl-4-oxo-4H-chinolizin-3-carbonsäure Hydrochlorid;
1-Cyclopropyl-8-(6-amino-3-azabicyclo[3.3.0]octyl)-7-fluor-9-methyl-4-oxo-4H-chinolizin-3-carbonsäure Hydrochlorid;
8-((1R*,2S*,6R*)-2-Amino-8-azabicyclo[4,3,0]nonan-8-yl))-1-cyclopropyl-7-fluor-4H-9-methyl-4-oxo-chinolizin-3-carbonsäure Hydrochlorid;
8-((1R*,2R*,6R*)-2-Amino-8-azabicyclo[4.3.0]nonan-8-yl))-1-cyclopropyl-7-fluor-4H-9-methyl-4-oxo-chinolizin-3-carbonsäure Hydrochlorid;
8-(3-(1-Aminoethyl)pyrrolidinyl)-1-cyclopropyl-7-fluor-4H-9-methyl-4-oxo-chinolizin-3-carbonsäure Hydrochlorid;
8-(3-(1-Amino-1-methylethyl)pyrrolidinyl)-1-cyclopropyl-7-fluor-9-methyl-4-oxo-4H-chinolizin-3-carbonsäure Hydrochlorid;
(3R,1S)-8-(3-(1-(N-Methyl)amino)propyl)pyrrolidinyl)-1-cyclopropyl-7-fluor-9-methyl-4-oxo-4H-chinolizin-3-carbonsäure Hydrochlorid;
8-(3-Aminopiperidinyl)-1-cyclopropyl-7-fluor-4H-9-methyl-4-oxochinolizin-3-carbonsäure Hydrochlorid;
8-(3-(1-Aminocyclopropyl)pyrrolidinyl)-1-cyclopropyl-7-fluor-9-methyl-4-oxo-4H-chinolizin-3-carbonsäure Hydrochlorid;
(3S,1R)-8-(3-(1-Aminoethyl)pyrrolidinyl)-1-cyclopropyl-7-fluor-9-methyl-4-oxo-4H-chinolizin-3-carbonsäure Hydrochlorid;
(3R,1S)-8-(3-(1-Aminoethyl)pyrrolidinyl)-1-cyclopropyl-7-fluor-9-methyl-4-oxo-4H-chinolizin-3-carbonsäure Hydrochlorid; und
(3R,1R)-8-(3-(1-Aminoethyl)pyrrolidinyl)-1-cyclopropyl-7-fluor-9-methyl-4-oxo-4H-chinolizin-3-carbonsäure Hydrochlorid;
und die pharmazeutisch verträglichen Salze, Ester und Amide davon.

14. Eine Verbindung gemäß Anspruch 12, gewählt aus der Gruppe bestehend aus:
8-(3(S)-Amino-1-pyrrolidinyl)-1-cyclopropyl-7-fluor-9-methyl-4-oxo-4H-chinolizin-3-carbonsäure Hydrochlorid;
8-(3-Amino-1-pyrrolidinyl)-1-cyclopropyl-7-fluor-9-methyl-4-oxo-4HH-chinolizin-3-carbonsäure Hydrochlorid;
(3R,1S)-8-(3-(1-Amino-2-methoxyethyl)pyrrolidinyl)-1-cyclopropyl-7-fluor-9-methyl-4-oxo-4H-chinolizin-3-carbonsäure Hydrochlorid;
(8-(3-(1-Amino-1-methylethyl)pyrrolidinyl)-1-cyclopropyl-7-fluor-9-methyl-4-oxo-4H-chinolizin-3-carbonsäure Hydrochlorid;
8-(3-(1-Aminocyclopropyl)pyrrolidinyl)-1-cyclopropyl-7-fluor-9-methyl-4-oxo-4H-chinolizin-3-carbonsäure Hydrochlorid; und
(3R,1S)-8-(3-(1-Aminoethyl)pyrrolidinyl)-1-cyclopropyl-7-fluor-9-methyl-4-oxo-4H-chinolizin-3-carbonsäure Hydrochlorid;
und die pharmazeutisch verträglichen Salze, Ester und Amide davon.

15. Die Verbindung 8-(3(S)-Aminopyrrolidinyl)-1-cyclopropyl-7-fluor-9-methyl-4-oxo-4H-chinolizin-3-carbonsäureydrochlorid,
und die pharmazeutisch verträglichen Salze, Ester und Amide davon.

16. Eine pharmazeutische Zusammensetzung einschließlich einer Verbindung gemäß irgendeinem der Ansprüche 1, 9, 12 oder 15 in Kombination mit einem pharmazeutisch verträglichen Träger.

17. Eine Verbindung gemäß irgendeinem der Ansprüche 1, 9, 12 oder 15 zur Verwendung als ein therapeutischer Wirkstoff.

18. Verwendung einer Verbindung gemäß irgendeinem der Ansprüche 1, 9, 12 oder 15 zur Herstellung eines Medikamentes zur Behandlung einer bakteriellen Infektion in einem menschlichen oder tierischen Patienten.

19. Ein synthetischer Zwischenstoff gewählt aus der Gruppe bestehend aus:
4-t-Butoxy-3-chlor-2,5,6-trifluorpyridin;
4-t-Butoxy-2,3,6-trifluorpyridin;
4-t-Butoxy-2,3,6-trifluor-5-methylpyridin;
4-t-Butoxy-2,5-difluor-3-methylpyridin;
2-(4-t-Butoxy-5-fluor-3-methyl-2-pyridinyl)cyclopropanacetonitril;
2-(4-Chlor-5-fluor-3-methyl-2-pyridinyl)cyclopropanacetonitril;
2-(4-Chlor-5-fluor-3-methyl-2-pyridinyl)cyclopropanessigsäure;
Ethyl 2-(4-Chlor-5-fluor-3-methyl-2-pyridinyl)cyclopropanacetat;
2-(4-Chlor-5-fluor-3-methyl-2-pyridinyl)cyclopropanacetaldehyd;
2-(4-Chlor-5-fluor-3-methyl-2-pyridinyl)cyclopropanethanol;
2-(2-(4-Chlor-5-fluor-3-methyl-2-pyridinyl)-2-cyclopropylethylidinyl)-1,3-propandicarbonsäure, Diethylester; und
8-Chlor-1-cyclopropyl-7-fluor-9-methyl-4-oxo-4h-chinolizin-3-carbonsäure Ethylester.

## Revendications

1. Composé de formule 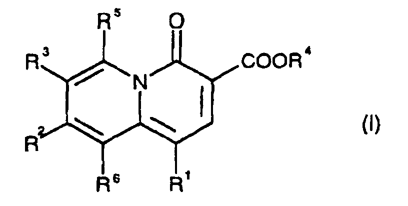 ou un sel, un ester ou un amide de celui-ci pharmaceutiquement acceptable, dans lequel
R¹ est choisi dans le groupe constitué par (a) un groupe alkyle en C₁-C₆, (b) alcényle en C₂-C₆, (c)halogénoalkyle en C₁-C₆, (d) alcoxy en C₁-C₆, (e) cycloalkyle en C₃-C₈, (f) phényle, (g) phényle substitué, (h) halogéno, (i) nitro, (k) bicycloalkyle, (l) alcynyle en C₂-C₆, (m) (alcoxy en C₁-C₆)carbonyle, (n) hétérocycle aromatique contenant un atome d'azote, (o) hétérocycle aromatique contenant un atome d'azote halogéno-substitué, (p) éther cyclique à 4, 5 ou 6 chaînons, et (q) -NR⁷R⁸ où R⁷ et R⁸ sont choisis indépendamment dans le groupe constitué par un atome d'hydrogène, un groupe alkyle en C₁-C₆ et alcanoyle en C₁-C₈ ou, pris conjointement avec l'atome d'azote sur lequel ils sont fixés, R⁷ et R⁸ forment un hétérocyle à 5, 6 ou 7 chaînons ;
R² est choisi dans le groupe constitué par (a) un groupe halogéno, (b) alkyle en C₁-C₆, (c) alcényle en C₂-C₆, (d) cycloalkyle en C₃-C₈, (e) cycloalcényle en C₄-C₈, (f) alcoxy en C₁-C₆, (g) aryloxy, (h) arylalcoxy en C₁-C₆, (i) arylalkyle en C₁-C₆, (j) cycloalkylalkyle en C₁-C₆, (k) amino, (l) mono- ou di-(alkyl en C₁-C₆)amino, (m) aryl(alkyl en C₁-C₆)amino, (n) (alkyl en C₁-C₆)amino hydroxy-substitué, (o) phényle, (p) phényle substitué, hétérocycle contenant un atome d'azote bicyclique, (r) hétérocyclique aromatique contenant un atome d'azote, et (s) hétérocycle contenant un atome d'azote répondant à la formule 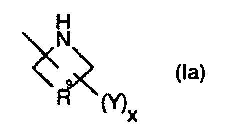
dans laquelle x est compris entre 0 et 3 ;
R⁹ est choisi dans le groupe constitué par (i) -(CH₂)ₘ- où m est compris entre 1 et 3, et (ii) -(CH₂)ₙR¹⁰ (CH₂)ₚ-, où R¹⁰ est choisi dans le groupe constitué par -S-, -O- et -NH-, n vaut 1 ou 2 et p vaut 1 ou 2 ; et
Y est choisi indépendamment à chaque fois dans le groupe constitué par
(i) alkyle en C₁-C₆,
(ii) hydroxy,
(iii) halogéno,
(iv) halogénoalkyle en C₁-C₆,
(v) alcoxy en C₁-C₆,
(vi) (alcoxy en C₁-C₆)alkyle en C₁-C₆,
(vii) (alcoxy en C₁-C₆) (alcoxy en C₁-C₆)alkyle en C₁-C₆,
(viii) alkyle en C₁-C₆ hydroxy-substitué,
(ix) imino,
(x) aminoalkyle en C₁-C₆,
(xi) (halogénoalkyl en C₁-C₆)aminoalkyle en C₁-C₆,
(xii) (thioalcoxy en C₁-C₆)alkyle en C₁-C₆,
(xiii) (thioalcoxy en C₁-C₆)alcoxy en C₁-C₆,
(xiv) aminothioalcoxy en C₁-C₆,
(xv) cycloalkyle en C₃-C₆,
(xvi) (cycloalkyl en C₃-C₈)alkyle en C₁-C₆,
(xvii) phényle,
(xviii) phényle substitué,
(xix) hétérocycle contenant un atome d'azote,
(xx) -NR¹¹R¹² où R¹¹ et R¹² sont choisis indépendamment dans le groupe constitué par un atome d'hydrogène et un groupe alkyle en C₁-C₆ ou bien, lorsque l'un des radicaux R¹¹ et R¹² est un atome d'hydrogène, l'autre est choisi dans le groupe constitué par un groupe alcanoyle en C₁-C₈, alpha-aminoacide et un résidu de polypeptide de 2 à 5 aminoacides, et
(xxi) -C(R²¹) (R²²)NH₂, où R²¹ et R²² sont choisis indépendamment dans le groupe constitué par un atome d'hydrogène, un groupe alkyle en C₁-C₆, alkyle en C₁-C₆ hydroxy-substitué, aminoalkyle en C₁-C₆, (alcoxy en C₁-C₆)alkyle en C₁-C₆, (thioalcoxy en C₁-C₆)alkyle en C₁-C₆, cycloalkyle en C₃-C₆ et alkyle en C₁-C₆ substitué par un hétérocycle aromatique contenant un atome d'azote ou bien, pris conjointement avec l'atome de carbone sur lequel ils sont fixés, R²¹ et R²² forment un structure de noyau choisie parmi cycloalkyle en C₃-C₆ et un hétérocycle contenant un atome d'azote ;
R³ est choisi dans le groupe constitué par un atome d'hydrogène, un groupe halogéno et alcoxy en C₁-C₆ ;
R⁴ est choisi dans le groupe constitué par un atome d'hydrogène, un groupe alkyle en C₁-C₆, un cation pharmaceutiquement acceptable et un ester précurseur d'un médicament qui est un groupe formant un ester qui est hydrolysé dans des conditions physiologiques ;
R⁵ est choisi dans le groupe constitué par (a) un atome d'hydrogène, (b) un groupe halogéno, (c) hydroxy, (d) alkyle en C₁-C₆, (e) halogénoalkyle en C₁-C₆, (f) alcoxy en C₁-C₆ et (g) -NR¹³R¹⁴ où R¹³ et R¹⁴ sont choisis indépendamment dans le groupe constitué par un atome d'hydrogène, un groupe alkyle en C₁-C₆, alkyle en C₁-C₆ hydroxy-substitué, (alcoxy en C₁-C₆)alkyle en C₁-C₆ et alcanoyle en C₁-C₈ ; et
R⁶ est alkyle en C₁-C₆.

2. Composé selon la revendication 1, dans lequel R³ est un atome d'halogène.

3. Composé selon la revendication 2, dans lequel R⁵ est choisi dans le groupe constitué par un atome d'hydrogène, un groupe alkyle en C₁-C₆, halogénoalkyle en C₁-C₆ et -NR¹³R¹⁴ où R¹³ et R¹⁴ sont tels que définis précédemment.

4. Composé selon la revendication 3, dans lequel R¹ est choisi dans le groupe constitué par un groupe cycloalkyle de trois à huit atomes de carbone et phényle substitué.

5. Composé selon la revendication 4, dans lequel R² est choisi dans le groupe constitué par un hétérocycle contenant un atome d'azote bicyclique et un hétérocycle contenant un atome d'azote de formule 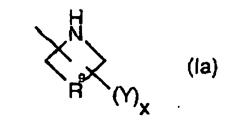 où R⁹, Y et x sont tels que définis précédemment.

6. Composé selon la revendication 5, dans lequel R² est choisi dans le groupe constitué par 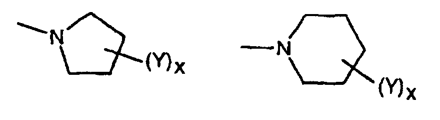 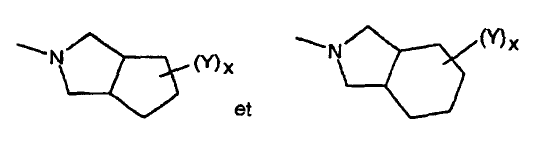 où Y et x sont tels que définis précédemment.

7. Composé selon la revendication 6, dans lequel x vaut un ou deux et Y est choisi dans le groupe constitué par -NR¹¹R¹² et C(R²¹)(R²²)NH₂, où R¹¹, R¹², R²¹ et R²² sont tels que définis précédemment.

8. Composé selon l'une quelconque des revendications 2, 3, 4, 5, 6 ou 7 dans lequel R⁶ est un groupe méthyle.

9. Composé selon la revendication 1 répondant à la formule 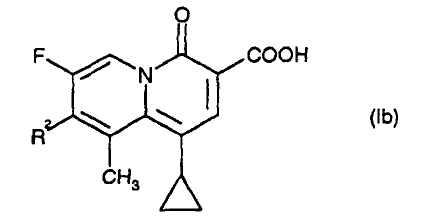 où un sel, un ester ou un amide pharmaceutiquement acceptable de celui-ci, dans lequel
R² est choisi dans le groupe constitué par un hétérocycle contenant un atome d'azote bicyclique et un hétérocycle contenant un atome d'azote répondant à la formule 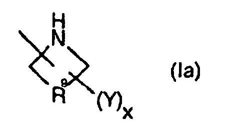 et où R⁴, R⁹, Y et x sont tels que définis précédemment.

10. Composé selon la revendication 9, dans lequel R² est choisi dans le groupe constitué par 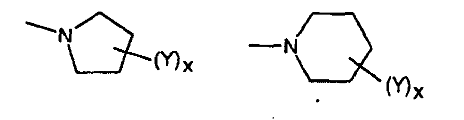 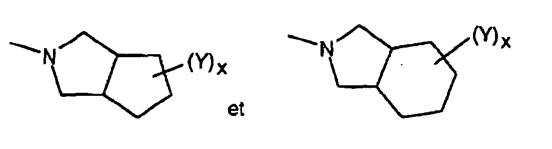 où Y et x sont tels que définis précédemment.

11. Composé selon la revendication 10, dans lequel x vaut un ou deux et Y est choisi dans le groupe constitué par -NR¹¹R¹² et C(R²¹) (R²²)NH₂, où R¹¹, R¹², R²¹ et R²² sont tels que définis précédemment.

12. Composé selon la revendication 1 choisi dans le groupe constitué par :
le chlorhydrate d'acide 8-(3-amino-1-pyrrolidinyl)-1-cyclopropyl-7-fluoro-9-méthyl-4-oxo-4H-quinolizine-3-carboxylique ;
le chlorhydrate d'acide 8-(3-(aminométhyl) pyrrolidinyl)-1-cyclopropyl-7-fluoro-9-méthyl-4-oxo-4H-quinolizine-3-carboxylique ;
le chlorhydrate d'acide 8-(2S,4S)-4-amino-2-méthylpyrrolidinyl)-1-cyclopropyl-7-fluoro-9-méthyl-4-oxo-4H-guinolizine-3-carboxylique ;
le chlorhydrate d'acide 8-(3-aminoazétidinyl)-1-cyclopropyl-7-fluoro-9-méthyl-4-oxo-4H-quinolizine-3-carboxylique ;
le chlorhydrate d'acide 8-(3(S)-aminopyrrolidinyl)-1-cyclopropyl-7-fluoro-9-méthyl-4-oxo-4H-quinolizine-3-carboxylique ;
le chlorhydrate d'acide 1-cyclopropyl-7-fluoro-9-méthyl-4-oxo-8-(3-méthyl-1-pipérazinyl)-4H-quinolizine-3-carboxylique ;
le chlorhydrate d'acide 1-cyclopropyl-7-fluoro-9-méthyl-4-oxo-8-pipérazinyl-4H-quinolizine-3-carboxylique ;
le chlorhydrate d'acide 1-cyclopropyl-7-fluoro-9-méthyl-8-(2-((N-méthyl)aminométhyl)-4-morpholinyl)-4-oxo-4H-quinolizine-3-carboxylique;
le chlorhydrate d'acide 1-cyclopropyl-7-fluoro-9-méthyl-4-oxo-8-(1,2,3,4-tétrahydro-2-isoquinolinyl)-4H-quinolizine-3-carboxylique ;
le chlorhydrate d'acide 1-cyclopropyl-7-fluoro-9-méthyl-4-oxo-8-(4-amino-1-pipéridinyl)-4H-quinolizine-3-carboxylique ;
le chlorhydrate d'acide 1-cyclopropyl-7-fluoro-9-méthyl-4-oxo-8-(3-amino-1-pipéridinyl)-4H-quinolizine-3-carboxylique ;
le chlorhydrate d'acide 1-cyclopropyl-7-fluoro-9-méthyl-4-oxo-8-(4-(aminoéthyl)-1-pipéridinyl)-4H-quinolizine-3-carboxylique ;
le chlorhydrate d'acide 1-cyclopropyl-7-fluoro-9-méthyl-4-oxo-8-(5-amino-1,2,3,4-tétrahydro-2-isoquinolinyl)-4H-quinolizine-3-carboxylique ;
l'acide 1-cyclopropyl-7-fluoro-9-méthyl-4-oxo-8-(4-(1-pyrrolyl)-1-pipéridinyl)-4H-quinolizine-3-carboxylique ;
le chlorhydrate d'acide 1-cyclopropyl-8-(cis-3,5-diméthyl-1-pipérazinyl)-7-fluoro-9-méthyl-4-oxo-4H-quinolizine-3-carboxylique ;
le chlorhydrate d'acide 1-cyclopropyl-8-(2,7-diaza-7-bicyclo[3.3.0]octyl)-7-fluoro-9-méthyl-4-oxo-4H-quinolizine-3-carboxylique ;
le chlorhydrate d'acide 1-cyclopropyl-8-(2,8-diaza-8-bicyclo[4.3.0]nonyl)-7-fluoro-9-méthyl-4-oxo-4H-quinolizine-3-carboxylique ;
l'acide 1-cyclopropyl-7-fluoro-9-méthyl-4-oxo-8-(3(S)-(1-pyrrolyl)-1-pyrrolidinyl) -4H-quinolizine-3-carboxylique ;
le chlorhydrate d'acide 1-cyclopropyl-7-fluoro-8-(3-hydroxy-1-pyrrolidinyl)-9-méthyl-4-oxo-4H-quinolizine-3-carboxylique ;
le chlorhydrate d'acide 1-cyclopropyl-7-fluoro-8-(4-méthyl-1-pipérazinyl)-9-méthyl-4-oxo-4H-quinolizine-3-carboxylique ;
le chlorhydrate d'acide 1-cyclopropyl-8-(2,7-diaza-7-bicyclo[3.3.0]octyl)-7-fluoro-9-méthyl-4-oxo-4H-quinolizine-3-carboxylique ;
le chlorhydrate d'acide 1-cyclopropyl-8-(2,8-diaza-8-bicyclo[4.3.0]nonyl)-7-fluoro-9-méthyl-4-oxo-4H-quinolizine-3-carboxylique ;
l'acide 1-cyclopropyl-7-fluoro-9-méthyl-4-oxo-8-(3(S)-(1-pyrrolyl)-1-pyrrolidinyl)-4H-quinolizine-3-carboxylique ;
le chlorhydrate d'acide 1-cyclopropyl-7-fluoro-8-(3-hydroxy-1-pyrrolidinyl)-9-méthyl-4-oxo-4H-quinolizine-3-carboxylique ;
le chlorhydrate d'acide 1-cyclopropyl-7-fluoro-8-(4-méthyl-1-pipérazinyl)-9-méthyl-4-oxo-4H-quinolizine-3-carboxylique ;
le chlorhydrate d'acide 1-cyclopropyl-7-fluoro-9-méthyl-8-(3(S)-méthylamino-1-pyrrolidinyl)-4-oxo-4H-quinolizine-3-carboxylique ;
le chlorhydrate d'acide 1-cyclopropyl-7-fluoro-9-méthyl-8-(3(R) -amino-1-pyrrolidinyl) -4-oxo-4H-quinolizine-3-carboxylique ;
le chlorhydrate d'acide 8-(2,4-diméthyl-1-pipérazinyl)-1-cyclopropyl-7-fluoro-9-méthyl-4-oxo-4H-quinolizine-3-carboxylique ;
le chlorhydrate d'acide 8-(3-(méthylamino)-1-pipérazinyl)-1-cyclopropyl-7-fluoro-9-méthyl-4-oxo-4H-quinolizine-3-carboxylique ;
le chlorhydrate d'acide 8-(3-(méthylamino)-1-morpholinyl)-1-cyclopropyl-7-fluoro-9-méthyl-4-oxo-4H-quinolizine-3-carboxylique ;
le chlorhydrate d'acide 8-(3-(S)-(méthylamino)-1-pyrrolidinyl)-1-cyclopropyl-7-fluoro-9-méthyl-4-oxo-4H-quinolizine-3-carboxylique ;
le chlorhydrate d'acide 8-(3-(S)-(1-(méthylamino) méthyl-1-pyrrolidinyl)-1-cyclopropyl-7-fluoro-9-méthyl-4-oxo-4H-quinolizine-3-carboxylique ;
le chlorhydrate d'acide 8-(3-(S)-(1-(éthylamino) méthyl-1-pyrrolidinyl)-1-cyclopropyl-7-fluoro-9-méthyl-4-oxo-4H-quinolizine-3-carboxylique;
le chlorhydrate d'acide 8-(octahydropyrrolo[3.4-c]pyrrol-1-yl)-1-cyclopropyl-7-fluoro-9-méthyl-4-oxo-4H-quinolizine-3-carboxylique ;
le chlorhydrate d'acide 8-(octahydropyrrolo[3.4-c]pyridin-5-yl)-1-cyclopropyl-7-fluoro-9-méthyl-4-oxo-4H-quinolizine-3-carboxylique;
le chlorhydrate d'acide 8-(3-cis-4-amino-3-méthylpyrrolidinyl)-1-cyclopropyl-7-fluoro-9-méthyl-4-oxo-4H-quinolizine-3-carboxylique ;
le chlorhydrate d'acide 8-(3-trans-4-amino-3-méthylpyrrolidinyl)-1-cyclopropyl-7-fluoro-9-méthyl-4-oxo-4H-quinolizine-3-carboxylique;
le chlorhydrate d'acide 8-(3-méthyl-4-spirocyclopropylpyrrolidinyl)-1-cyclopropyl-7-fluoro-9-méthyl-4-oxo-4H-quinolizine-3-carboxylique ;
le sel de l'acide acétique de l'acide 8-(3-diméthylaminopyrrolidinyl)-1-cyclopropyl-7-fluoro-9-méthyl-4-oxo-4H-quinolizine-3-carboxylique;
le chlorhydrate d'acide (3R)-8-(3-diméthylaminopyrrolidinyl)-1-cyclopropyl-7-fluoro-9-méthyl-4-oxo-4H-quinolizine-3-carboxylique ;
le chlorhydrate d'acide (3R,1S)-8-(3-(1-aminoéthyl) pyrrolidinyl)-1-cyclopropyl-7-fluoro-9-méthyl-4-oxo-4H-quinolizine-3-carboxylique ;
le chlorhydrate d'acide (3S,1R)-8-(3-(1-aminoéthyl) pyrrolidinyl)-1-cyclopropyl-7-fluoro-9-méthyl-4-oxo-4H-quinolizine-3-carboxylique ;
le chlorhydrate d'acide (3R,1R)-8-(3-(1-aminoéthyl) pyrrolidinyl)-1-cyclopropyl-7-fluoro-9-méthyl-4-oxo-4H-quinolizine-3-carboxylique;
l'acide 1-cyclopropyl-8-((R,S)-3-fluoropyrrolidine)-7-fluoro-9-méthyl-4-oxo-4H-quinolizine-3-carboxylique ;
l'acide 8-(4-(1-pipéridyl)-1-pipéridyl)-1-cyclopropyl-7-fluoro-9-méthyl-4-oxo-4H-quinolizine-3-carboxylique ;
le sel de l'acide trifluoroacétique de l'acide 8-(4-(1-pipéridyl)-1-pipéridyl)-1-cyclopropyl-7-fluoro-9-méthyl-4-oxo-4H-quinolizine-3-carboxylique ;
l'acide 8-(4-(2-pipéridyl)-1-pipéridyl)-1-cyclopropyl-7-fluoro-9-méthyl-4-oxo-4H-quinolizine-3-carboxylique ;
le sel de l'acide trifluoroacétique de l'acide 8-((2-amino)thioéthoxy)-1-cyclopropyl-7-fluoro-9-méthyl-4-oxo-4H-quinolizine-3-carboxylique;
le chlorhydrate d'acide (3R,1R)-8-(3-(1-aminopropyl) pyrrolidinyl)-1-cyclopropyl-7-fluoro-9-méthyl-4-oxo-4H-quinolizine-3-carboxylique;
le chlorhydrate d'acide (3R,1R)-8-(3-(1-(N-méthyl) amino)propyl)pyrrolidinyl)-1-cyclopropyl-7-fluoro-9-méthyl-4-oxo-4H-quinolizine-3-carboxylique ;
le chlorhydrate d'acide (3R,1R)-8-(3-(1-amino-3-méthoxypropyl)pyrrolidinyl)-1-cyclopropyl-7-fluoro-9-méthyl-4-oxo-4H-quinolizine-3-carboxylique ;
le chlorhydrate d'acide 8-(3-(1-aminocyclopropyl)-pyrrolidinyl)-1-cyclopropyl-7-fluoro-9-méthyl-4-oxo-4H-quinolizine-3-carboxylique ;
le chlorhydrate d'acide (3R,1R)-8-(3-(1-amino-2-hydroxyéthyl)pyrrolidinyl)-1-cyclopropyl-7-fluoro-9-méthyl-4-oxo-4H-quinolizine-3-carboxylique ;
le chlorhydrate d'acide 8-(3-(1-amino-1-méthyléthyl) pyrrolidinyl)-1-cyclopropyl-7-fluoro-9-méthyl-4-oxo-4H-quinolizine-3-carboxylique;
le chlorhydrate d'acide 8-(3-(1-aminobutyl) pyrrolidinyl)-1-cyclopropyl-7-fluoro-9-méthyl-4-oxo-4H-quinolizine-3-carboxylique;
le chlorhydrate d'acide 1-cyclopropyl-7-fluoro-9-méthyl-4-oxo-8-(trans-4-trifluorométhyl-3-aminopyrrolidinyl)-4H-quinolizine-3-carboxylique ;
le chlorhydrate d'acide 1-cyclopropyl-7-fluoro-9-méthyl-4-oxo-8-(trans-4-trifluorométhyl-3-aminométhylpyrrolidinyl)-4H-quinolizine-3-carboxylique ;
le chlorhydrate d'acide 3(S)-1-cyclopropyl-7-fluoro-9-méthyl-4-oxo-8-(3-(N-(S)-norvalylamino)pyrrolidinyl)-4H-quinolizine-3-carboxylique;
le chlorhydrate d'acide 3(S)-1-cyclopropyl-7-fluoro-9-méthyl-4-oxo-8-(3-(N-(S)-alanylamino)pyrrolidinyl)-4H-quinolizine-3-carboxylique ;
le chlorhydrate d'acide 3(S)-1-cyclopropyl-7-fluoro-9-méthyl-4-oxo-8-(3-(N-(S)-alanyl-(S)-alanylamino)-pyrrolidinyl)-4H-quinolizine-3-carboxylique ;
le chlorhydrate d'acide 1-cyclopropyl-7-fluoro-4H-8-(1-imidazolyl)-9-méthyl-4-oxo-quinolizine-3-carboxylique ;
le chlorhydrate d'acide 8-(3-amino-1-pyrrolidinyl)-1-éthyl-7-fluoro-4H-4-oxo-9-méthyl-quinolizine-3-carboxylique ;
le chlorhydrate d'acide 8-(3-amino-1-pyrrolidinyl)-1-cyclopropyl-9-éthyl-7-fluoro-4H-4-oxo-quinolizine-3-carboxylique ;
l'acide 1-cyclopropyl-7-fluoro-4H-9-méthyl-4-oxo-8-(3-(1,2,3-triazol-1-yl)-pyrrolidinyl)-quinolizine-3-carboxylique ;
le chlorhydrate d'acide 1-cyclopropyl-7-fluoro-4H-9-méthyl-4-oxo-8-(cis-3-amino-4-méthyl-1-pyrrolidinyl)-quinolizine-3-carboxylique;
le chlorhydrate d'acide 8-(2-aminoéthyl)-1-cyclopropyl-7-fluoro-4H-9-méthyl-4-oxo-quinolizine-3-carboxylique ;
le chlorhydrate d'acide 8-(3-(éthylaminométhyl)-pyrrolidinyl)-1-cyclopropyl-7-fluoro-4H-9-méthyl-4-oxo-quinolizine-3-carboxylique ;
le chlorhydrate d'acide 8-(3-(1-aminométhyl)-pyrrolidinyl)-1-cyclopropyl-7-fluoro-4H-9-méthyl-4-oxo-quinolizine-3-carboxylique ;
le chlorhydrate d'acide 1-cyclopropyl-7-fluoro-4H-9-méthyl-8-(2-méthyl-2,8-diaza-8-bicyclo[4.3.0]nonyl)-4-oxo-quinolizine-3-carboxylique ;
le chlorhydrate d'acide 1-cyclopropyl-7-fluoro-4H-8-((1S,4S)-2,5-diaza-bicyclo[2.2.1]heptan-2-yl)-9-méthyl-4-oxo-quinolizine-3-carboxylique ;
le chlorhydrate d'acide 1-cyclopropyl-7-fluoro-4H-9-méthyl-4-oxo-8-(3-(2-pyridinyl)-1-pyrrolidinyl)-quinolizine-3-carboxylique ;
le chlorhydrate d'acide 8-((1R*,2S*,6R*)-2-amino-8-azabicyclo[4.3.0]nonan-8-yl))-1-cyclopropyl-7-fluoro-4H-9-méthyl-4-oxo-quinolizine-3-carboxylique ;
le chlorhydrate d'acide 8-((1R*,2R*,6R*)-2-amino-8-azabicyclo[4.3.0]nonan-8-yl))-1-cyclopropyl-7-fluoro-4H-9-méthyl-4-oxo-quinolizine-3-carboxylique;
le chlorhydrate d'acide 8-((1a,5a,6a)-6-amino-3-azabicyclo[3.1.0]hexan-3-yl))-1-cyclopropyl-9-méthyl-7-fluoro-4H-4-oxo-quinolizine-3-carboxylique ;
le chlorhydrate d'acide 8-(cis-3-amino-4-fluoro-1-pyrrolidinyl)-1-cyclopropyl-9-méthyl-7-fluoro-4H-4-oxoquinolizine-3-carboxylique ;
le sel de l'acide acétique de l'acide 1-cyclopropyl-7-fluoro-4H-8-(1-homopipérazinyl)-9-méthyl-4-oxoquinolizine-3-carboxylique ;
l'acide 8-(spiro-1,3-dioxacyclopentane[2.3]-1-pipéridinyl)-1-cyclopropyl-7-fluoro-4H-9-méthyl-4-oxo-quinolizine-3-carboxylique ;
le chlorhydrate d'acide 8-(3-amino-4-méthoxypyrrolidinyl)-1-cyclopropyl-7-fluoro-4H-9-méthyl-4-oxo-quinolizine-3-carboxylique ;
le chlorhydrate d'acide 8-(4-amino-4-méthylpyrrolidinyl)-1-cyclopropyl-7-fluoro-4H-9-méthyl-4-oxo-quinolizine-3-carboxylique ;
l'acide 8-(4-(2-hydroxyéthyl)pipéridinyl)-1-cyclopropyl-7-fluoro-4H-9-méthyl-4-oxo-quinolizine-3-carboxylique ;
l'acide 8-(4-(méthoxyméthyl)pipéridinyl)-1-cyclopropyl-7-fluoro-4H-9-méthyl-4-oxo-quinolizine-3-carboxylique ;
le chlorhydrate d'acide 8-(4-amino-3-méthylpipéridinyl)-1-cyclopropyl-7-fluoro-4H-9-méthyl-4-oxo-quinolizine-3-carboxylique ;
l'acide 8-(3-pyrrolylpipéridinyl)-1-cyclopropyl-7-fluoro-4H-9-méthyl-4-oxo-quinolizine-3-carboxylique ;
le chlorhydrate d'acide 8-(3-aminopipéridinyl)-1-cyclopropyl-7-fluoro-4H-9-méthyl-4-oxo-quinolizine-3-carboxylique ;
le chlorhydrate d'acide 8-(3-amino-3-méthylpipéridinyl)-1-cyclopropyl-7-fluoro-4H-9-méthyl-4-oxo-quinolizine-3-carboxylique ;
le chlorhydrate d'acide 8-(3-amino-4-(1',3'-dioxanyl)pyrrolidinyl)-1-cyclopropyl-7-fluoro-4H-9-méthyl-4-oxo-quinolizine-3-carboxylique ;
le chlorhydrate d'acide 8-(3-amino-4-hydroxypyrrolidinyl)-1-cyclopropyl-7-fluoro-4H-9-méthyl-4-oxo-quinolizine-3-carboxylique ;
le chlorhydrate d'acide 8-(4-(1-(N-éthylamino)-méthylpipéridinyl)-1-cyclopropyl-7-fluoro-4H-9-méthyl-4-oxo-quinolizine-3-carboxylique ;
le chlorhydrate d'acide 1-cyclopropyl-7-fluoro-8-(3-hydroxy-4-méthylaminopyrrolidinyl)-4H-9-méthyl-4-oxo-quinolizine-3-carboxylique ;
le chlorhydrate d'acide 8-(3-aminométhylpipéridinyl)-1-cyclopropyl-7-fluoro-4H-9-méthyl-4-oxo-quinolizine-3-carboxylique ;
le chlorhydrate d'acide 8-(2-aminométhyl-4-morpholinyl)-1-cyclopropyl-7-fluoro-4H-9-méthyl-4-oxo-quinolizine-3-carboxylique ;
le chlorhydrate d'acide 8-(3-(1-méthylamino)-méthylpipéridinyl)-1-cyclopropyl-7-fluoro-4H-9-méthyl-4-oxo-quinolizine-3-carboxylique ;
le chlorhydrate d'acide 8-(3-(méthyl (méthylènedioxy)méthyl)pipéridinyl)-1-cyclopropyl-7-fluoro-4H-9-méthyl-4-oxo-quinolizine-3-carboxylique ;
le chlorhydrate d'acide 8-(3-(S)-aminopipéridinyl)-1-cyclopropyl-7-fluoro-4H-9-méthyl-4-oxo-quinolizine-3-carboxylique ;
le chlorhydrate d'acide 8-(3-(S)-(N-éthyl-N-méthylamino)pipéridinyl)-1-cyclopropyl-7-fluoro-4H-9-méthyl-4-oxo-quinolizine-3-carboxylique ;
le chlorhydrate d'acide 1-cyclopropyl-8-(4-(2'-(N-méthylamino)méthyl-1',3'-dioxanyl)pipéridinyl)-7-fluoro-9-méthyl-4-oxo-4H-quinolizine-3-carboxylique ;
le chlorhydrate d'acide 1-cyclopropyl-8-(3-aza-6-amino-6-méthylbicyclo [3.3.0]octan-1-yl) -7-fluoro-9-méthyl-4-oxo-4H-quinolizine-3-carboxylique;
la 1-cyclopropyl-8-(3-fluorométhylpipéridinyl)-7-fluoro-9-méthyl-4-oxo-4H-quinolizine ;
le chlorhydrate d'acide 1-cyclopropyl-8-(4-(N,N-diméthyl)aminopipéridinyl)-7-fluoro-9-méthyl-4-oxo-4H-quinolizine-3-carboxylique ;
le chlorhydrate d'acide 1-cyclopropyl-8-(6-amino-3-azabicyclo[3.3.0]octyl)-7-fluoro-9-méthyl-4-oxo-4H-quinolizine-3-carboxylique ;
le chlorhydrate d'acide 1-cyclopropyl-8-((2-aza-4-(diméthylaminoéthyl)-bicyclo[4.3.0]non-2-yl)-7-fluoro-9-méthyl-4-oxo-4H-quinolizine-3-carboxylique ;
le chlorhydrate d'acide 1-cyclopropyl-8-(3-aza-6-(L-alanylamino)-6-méthylbicyclo[3.3.0]octane)-7-fluoro-9-méthyl-4-oxo-4H-quinolizine-3-carboxylique;
le chlorhydrate d'acide (3R,1R)-8-(3-(1-(N-méthyl) amino)propyl)pyrrolidinyl)-1-cyclopropyl-7-fluoro-9-méthyl-4-oxo-4H-quinolizine-3-carboxylique ; et
le chlorhydrate d'acide (3R,1S)-8-(3-(1-amino-2-méthoxyéthyl)pyrrolidinyl)-1-cyclopropyl-7-fluoro-9-méthyl-4-oxo-4H-quinolizine-3-carboxylique ;
et les sels, les esters et les amides pharmaceutiquement acceptables de ceux-ci.

13. Composé selon la revendication 12 choisi dans le groupe constitué par :
le chlorhydrate d'acide 8-(3-(aminométhylpyrrolidinyl)-1-cyclopropyl-7-fluoro-9-méthyl-4-oxo-4H-quinolizine-3-carboxylique ;
le chlorhydrate d'acide 8-(3-(S)-amino-1-pyrrolidinyl)-1-cyclopropyl-7-fluoro-9-méthyl-4-oxo-4H-quinolizine-3-carboxylique ;
le chlorhydrate d'acide 8-(3-amino-1-pyrrolidinyl)-1-cyclopropyl-7-fluoro-9-méthyl-4-oxo-4H-quinolizine-3-carboxylique ;
le chlorhydrate d'acide (3R,lS)-8-(3-(1-amino) propyl)-pyrrolidinyl)-1-cyclopropyl-7-fluoro-9-méthyl-4-oxo-4H-quinolizine-3-carboxylique ;
le chlorhydrate d'acide 8-(3-(1-aminobutyl)-pyrrolidinyl)-1-cyclopropyl-7-fluoro-9-méthyl-4-oxo-4H-quinolizine-3-carboxylique ;
le chlorhydrate d'acide (3R,1S)-8-(3-(1-amino-2-méthoxyéthyl)pyrrolidinyl)-1-cyclopropyl-7-fluoro-9-méthyl-4-oxo-4H-quinolizine-3-carboxylique;
le chlorhydrate d'acide 1-cyclopropyl-7-fluoro-9-méthyl-4-oxo-8-(4-amino-1-pipéridinyl)-4H-quinolizine-3-carboxylique ;
le chlorhydrate d'acide 1-cyclopropyl-7-fluoro-9-méthyl-4-oxo-8-(3-amino-1-pipéridinyl)-4H-quinolizine-3-carboxylique ;
le chlorhydrate d'acide 8-(3-(S)-aminopipéridinyl)-1-cyclopropyl-7-fluoro-4H-9-méthyl-4-oxo-quinolizine-3-carboxylique ;
le chlorhydrate d'acide 1-cyclopropyl-8-(3-aza-6-amino-6-méthylbicyclo[3.3.0]octan-1-yl)-7-fluoro-9-méthyl-4-oxo-4H-quinolizine-3-carboxylique;
le chlorhydrate d'acide 1-cyclopropyl-8-(6-amino-3-azabicyclo[3.3.0]octyl)-7-fluoro-9-méthyl-4-oxo-4H-quinolizine-3-carboxylique ;
le chlorhydrate d'acide 8-((1R*,2S*,6R*)-2-amino-8-azabicyclo[4.3.0]nonan-8-yl))-1-cyclopropyl-7-fluoro-4H-9-méthyl-4-oxo-quinolizine-3-carboxylique ;
le chlorhydrate d'acide 8-((1R*,2R*,6R*)-2-amino-8-azabicyclo[4.3.0]nonan-8-yl))-1-cyclopropyl-7-fluoro-4H-9-méthyl-4-oxo-quinolizine-3-carboxylique;
le chlorhydrate d'acide 8-(3-(1-aminoéthyl) pyrrolidinyl)-1-cyclopropyl-7-fluoro-4H-9-méthyl-4-oxoquinolizine-3-carboxylique ;
le chlorhydrate d'acide 8-(3-(1-amino-1-méthyléthyl) pyrrolidinyl)-1-cyclopropyl-7-fluoro-9-méthyl-4-oxo-4H-quinolizine-3-carboxylique ;
le chlorhydrate d'acide (3R,1S)-8-(3-(1-(N-méthyl) amino)-propyl)pyrrolidinyl)-1-cyclopropyl-7-fluoro-9-méthyl-4-oxo-4H-quinolizine-3-carboxylique ;
le chlorhydrate d'acide 8-(3-aminopipéridinyl)-1-cyclopropyl-7-fluoro-4H-9-méthyl-4-oxo-quinolizine-3-carboxylique ;
le chlorhydrate d'acide 8-(3-(1-aminocyclopropyl)-pyrrolidinyl)-1-cyclopropyl-7-fluoro-9-méthyl-4-oxo-4H-quinolizine-3-carboxylique ;
le chlorhydrate d'acide (3S,1R)-8-(3-(1-aminoéthyl) pyrrolidinyl)-1-cyclopropyl-7-fluoro-9-méthyl-4-oxo-4H-quinolizine-3-carboxylique;
le chlorhydrate d'acide (3R,1S)-8-(3-(1-aminoéthyl) pyrrolidinyl)-1-cyclopropyl-7-fluoro-9-méthyl-4-oxo-4H-quinolizine-3-carboxylique ; et
le chlorhydrate d'acide (3R,1R)-8-(3-(1-aminoéthyl) pyrrolidinyl)-1-cyclopropyl-7-fluoro-9-méthyl-4-oxo-4H-quinolizine-3-carboxylique;
et les sels, les esters et les amides pharmaceutiquement acceptables de ceux-ci.

14. Composé selon la revendication 12 choisi dans le groupe constitué par :
le chlorhydrate d'acide 8-(3-(S)-amino-1-pyrrolidinyl)-1-cyclopropyl-7-fluoro-9-méthyl-4-oxo-4H-quinolizine-3-carboxylique ;
le chlorhydrate d'acide 8-(3-amino-1-pyrrolidinyl)-1-cyclopropyl-7-fluoro-9-méthyl-4-oxo-4H-quinolizine-3-carboxylique ;
le chlorhydrate d'acide (3R,lS)-8-(3-(1-amino-2-méthoxyéthyl)pyrrolidinyl)-1-cyclopropyl-7-fluoro-9-méthyl-4-oxo-4H-quinolizine-3-carboxylique ;
le chlorhydrate d'acide 8-(3-(1-amino-1-méthyléthyl) pyrrolidinyl)-1-cyclopropyl-7-fluoro-9-méthyl-4-oxo-4H-quinolizine-3-carboxylique ;
le chlorhydrate d'acide 8-(3-(1-aminocyclopropyl)-pyrrolidinyl)-1-cyclopropyl-7-fluoro-9-méthyl-4-oxo-4H-quinolizine-3-carboxylique; et
le chlorhydrate d'acide (3R,1S)-8-(3-(1-aminoéthyl) pyrrolidinyl)-1-cyclopropyl-7-fluoro-9-méthyl-4-oxo-4H-quinolizine-3-carboxylique ; et
et les sels, les esters et les amides pharmaceutiquement acceptables de ceux-ci.

15. Composé chlorhydrate d'acide 8-(3-(S)-aminopyrrolidinyl)-1-cyclopropyl-7-fluoro-9-méthyl-4-oxo-4H-quinolizine-3-carboxylique
et les sels, les esters et les amides pharmaceutiquement acceptables de celui-ci.

16. Composition pharmaceutique comprenant un composé selon l'une quelconque des revendications 1, 9, 12 ou 15, en combinaison avec un véhicule pharmaceutiquement acceptable.

17. Composé selon l'une quelconque des revendications 1, 9, 12 ou 15, destiné à une utilisation comme agent thérapeutique.

18. Utilisation d'un composé selon l'une quelconque des revendications 1, 9, 12 ou 15, pour la fabrication d'un médicament pour traiter une infection bactérienne chez un homme ou un patient vétérinaire.

19. Intermédiaire de synthèse choisi dans le groupe constitué par :
la 4-t-butoxy-3-chloro-2,5,6-trifluoropyridine;
la 4-t-butoxy-2,3,6-trifluoropyridine ;
la 4-t-butoxy-2,3,6-crifluoro-5-méthylpyridine ;
la 4-t-butoxy-2,5-difluoro-5-méthylpyridine ;
le 2-(4-t-butoxy-5-fluoro-3-méthyl-5-pyridinyl)-cyclopropaneacétonitrile ;
le 2-(4-chloro-5-fluoro-3-méthyl-2-pyridinyl)-cyclopropaneacétonitrile ;
l'acide 2-(4-chloro-5-fluoro-3-méthyl-2-pyridinyl)-cyclopropaneacétique ;
le 2-(4-chloro-5-fluoro-3-méthyl-2-pyridinyl)-cyclopropaneacétate d'éthyle ;
le 2-(4-chloro-5-fluoro-3-méthyl-2-pyridinyl)-cyclopropaneacétaldéhyde ;
le 2-(4-chloro-5-fluoro-3-méthyl-2-pyridinyl)-cyclopropaneéthanol ;
l'ester diéthylique de l'acide 2-(2-(4-chloro-5-fluoro-3-méthyl-2-pyridinyl) -2-cyclopropyléthylidinyl)-1,3-propanedicarboxylique ; et
l'ester éthylique de l'acide 8-chloro-1-cyclopropyl-7-fluoro-9-méthyl-4-oxo-4H-quinolizine-3-carboxylique.
